# EUROPEAN PATENT APPLICATION

(11) **EP 2 813 242 A1**
(43) Date of publication of application: **17.12.2014**
(21) Application number: 13075040.9
(22) Date of filing: 13.06.2013
(51) Int. Cl.: A61K 39/00, A61K 47/34

(54) **Low molecular weight immune-modulators as adjuvants for specific immunotherapy**

(71) Applicant: PLS-Design GmbH, 20255 Hamburg (DE); Klinikum rechts der Isar der Technischen Universität München, 81675 München (DE); Helmholtz Zentrum München Forschungszentrum für Gesundheit und Umwelt GmbH, 85764 Neuherberg (DE)
(72) Inventor: Bredehorst, Reinhard, 20255 Hamburg (DE); Grunwald, Thomas, 22459 Hamburg (DE); Ollert, Markus, 82131 Gauting (DE); Schmidt-Weber, Carsten, 81249 München (DE)
(74) Representative: Seuss, Thomas

(57) **Abstract**

The invention relates to a pharmaceutical composition for local modulation of T cell and B cell responses at the site of allergen or antigen presentation, made of one or more preparations comprising one or more antigens or allergens, and a therapeutically effective dose of two or more low molecular weight immune modulators selected from four groups of tolerance-inducing therapeutics comprising inhibitors of complement-mediated functions, inhibitors of tumor necrosis factor receptor 1 (TNFR1)-mediated functions, inhibitors of interleukin 4- and interleukin 13-mediated functions', and therapeutic agents suitable for vitamin D3 supplementation wherein preferably two or more low molecular weight immune modulators and one or more antigens or allergens are coated or adsorbed on or embedded in a matrix, wherein the matrix is selected as to enable sustained release of one or more antigens or allergens and two or more immune modulators for the treatment of T cell-mediated diseases, including allergy, allergic asthma, and autoimmune disease such as type 1 diabetes, rheumatoid arthritis, multiple sclerosis.

SEQUENCE LISTING

<110> PLS-Design GmbH

<120> Low Molecular Weight Immune-Modulators

<160> 10

<170> BiSSAP 1.2

<210> 1
<211> 13
<212> PRT
<213> Artificial Sequence

<220>
<223> cyclic peptide C3 inhibitor

<400> 1
Ile Cys Val Val Gln Asp Trp Gly His His Arg Cys Thr
1               5                   10

<210> 2
<211> 2258
<212> DNA
<213> Artificial Sequence

<220>
<221> source
<222> 1..2258
<223> /organism="Artificial Sequence"
      /note="NM_001065.3"
      /mol_type="unassigned DNA"

<400> 2
   ctcctccagc tcttcctgtc ccgctgttgc aacactgcct cactcttccc ctcccacctt      60
   ctctcccctc ctctctgctt taattttctc agaattctct ggactgaggc tccagttctg     120
   gcctttgggg ttcaagatca ctgggaccag gccgtgatct ctatgcccga gtctcaaccc     180
   tcaactgtca ccccaaggca cttgggacgt cctggacaga ccgagtcccg ggaagcccca     240
   gcactgccgc tgccacactg ccctgagccc aaatggggga gtgagaggcc atagctgtct     300
   ggcatgggcc tctccaccgt gcctgacctg ctgctgccac tggtgctcct ggagctgttg     360
   gtgggaatat acccctcagg ggttattgga ctggtccctc acctagggga cagggagaag     420
   agagatagtg tgtgtcccca aggaaaatat atccaccctc aaaataattc gatttgctgt     480
   accaagtgcc acaaaggaac ctacttgtac aatgactgtc caggcccggg gcaggatacg     540
   gactgcaggg agtgtgagag cggctccttc accgcttcag aaaaccacct cagacactgc     600
   ctcagctgct ccaaatgccg aaaggaaatg ggtcaggtgg agatctcttc ttgcacagtg     660
   gaccgggaca ccgtgtgtgg ctgcaggaag aaccagtacc ggcattattg gagtgaaaac     720
   cttttccagt gcttcaattg cagcctctgc ctcaatggga ccgtgcacct ctcctgccag     780
   gagaaacaga acaccgtgtg cacctgccat gcaggtttct ttctaagaga aaacgagtgt 840
   gtctcctgta gtaactgtaa gaaaagcctg gagtgcacga agttgtgcct accccagatt 900
   gagaatgtta agggcactga ggactcaggc accacagtgc tgttgcccct ggtcattttc 960
   tttggtcttt gccttttatc cctcctcttc attggtttaa tgtatcgcta ccaacggtgg 1020
   aagtccaagc tctactccat tgtttgtggg aaatcgacac ctgaaaaaga gggggagctt 1080
   gaaggaacta ctactaagcc cctggcccca aacccaagct tcagtcccac tccaggcttc 1140
   acccccaccc tgggcttcag tcccgtgccc agttccacct tcacctccag ctccacctat 1200
   acccccggtg actgtcccaa ctttgcggct ccccgcagag aggtggcacc accctatcag 1260
   ggggctgacc ccatccttgc gacagccctc gcctccgacc ccatccccaa cccccttcag 1320
   aagtgggagg acagcgccca caagccacag agcctagaca ctgatgaccc cgcgacgctg 1380
   tacgccgtgg tggagaacgt gcccccgttg cgctggaagg aattcgtgcg gcgcctaggg 1440
   ctgagcgacc acgagatcga tcggctggag ctgcagaacg ggcgctgcct gcgcgaggcg 1500
   caatacagca tgctggcgac ctggaggcgg cgcacgccgc ggcgcgaggc cacgctggag 1560
   ctgctgggac gcgtgctccg cgacatggac ctgctgggct gcctggagga catcgaggag 1620
   gcgctttgcg gccccgccgc cctcccgccc gcgcccagtc ttctcagatg aggctgcgcc 1680
   cctgcgggca gctctaagga ccgtcctgcg agatcgcctt ccaaccccac ttttttctgg 1740
   aaaggagggg tcctgcaggg gcaagcagga gctagcagcc gcctacttgg tgctaacccc 1800
   tcgatgtaca tagcttttct cagctgcctg cgcgccgccg acagtcagcg ctgtgcgcgc 1860
   ggagagaggt gcgccgtggg ctcaagagcc tgagtgggtg gtttgcgagg atgagggacg 1920
   ctatgcctca tgcccgtttt gggtgtcctc accagcaagg ctgctcgggg gcccctggtt 1980
   cgtccctgag cctttttcac agtgcataag cagttttttt tgtttttgtt ttgttttgtt 2040
   ttgtttttaa atcaatcatg ttacactaat agaaacttgg cactcctgtg ccctctgcct 2100
   ggacaagcac atagcaagct gaactgtcct aaggcagggg cgagcacgga acaatggggc 2160
   cttcagctgg agctgtggac ttttgtacat acactaaaat tctgaagtta aagctctgct 2220
   cttggaaaaa aaaaaaaaaa aaaaaaaaaa aaaaaaaa 2258

<210> 3
<211> 390
<212> DNA
<213> Homo sapiens

<220>
<221> source
<222> 1..390

<223> /organism="Homo sapiens"
      /note="Human Interleukin-4, Pro-peptide sequence"
      /mol_type="unassigned DNA"

<400> 3
cacaagtgcg atatcacctt acaggagatc atcaaaactt tgaacagcct cacagagcag      60

aagactctgt gcaccgagtt gaccgtaaca gacatctttg ctgcctccaa gaacacaact     120

gagaaggaaa ccttctgcag ggctgcgact gtgctccggc agttctacag ccaccatgag     180

aaggacactc gctgcctggg tgcgactgca cagcagttcc acaggcacaa gcagctgatc     240

cgattcctga aacggctcga caggaacctc tggggcctgg cgggcttgaa ttcctgtcct     300

gtgaaggaag ccaaccagag tacgttggaa aacttcttgg aaaggctaaa gacgatcatg     360

agagagaaat attcaaagtg ttcgagctga                                      390


<210> 4
<211> 129
<212> PRT
<213> Homo sapiens

<220>
<223> Human Interleukin-4, Pro-peptide Translated DNA sequence

<400> 4


<210> 5
<211> 453
<212> DNA
<213> Homo sapiens

<220>
<221> source
<222> 1..453
<223> /organism="Homo sapiens"
      /mol_type="unassigned DNA"

<400> 5

atgggtagca gccatcatca tcatcatcac tccagcggtc tggttcctcg tggtagtcat 60

atgcacaaat gtgacatcac cctgcaggag attatcaaaa cactgaatag cctgacggag 120

cagaaaacac tgtgtaccga actgacggtt accgacatct ttgccgcctc caaaaacacc 180

accgagaaag aaaccttttg tcgtgctgcc actgtactgc gtcaattcta tagccaccat 240

gaaaaagaca cccgttgcct gggagcgact gcccaacaat tccatcgtca taaacagctg 300

atccgcttcc tgaaacgtct ggatcgtaat ctgtggggac tggcaggtct gaattcatgt 360

ccggtgaaag aggcaaatca gtcaaccctg gagaactttc tggaacgcct gaaaaccatt 420

atggatgaaa aagactccaa atgctcgagc tga 453

<210> 6
<211> 150
<212> PRT
<213> Homo sapiens

<220>
<223> Human Interleukin-4, N-terminal His-tagged mature peptide
      sequence

<400> 6
Met Gly Ser Ser His His His His His His Ser Ser Gly Leu Val Pro
1               5                   10                  15
Arg Gly Ser His Met His Lys Cys Asp Ile Thr Leu Gln Glu Ile Ile
            20                  25                  30
Lys Thr Leu Asn Ser Leu Thr Glu Gln Lys Thr Leu Cys Thr Glu Leu
        35                  40                  45
Thr Val Thr Asp Ile Phe Ala Ala Ser Lys Asn Thr Thr Glu Lys Glu
    50                  55                  60
Thr Phe Cys Arg Ala Ala Thr Val Leu Arg Gln Phe Tyr Ser His His
65                  70                  75                  80
Glu Lys Asp Thr Arg Cys Leu Gly Ala Thr Ala Gln Gln Phe His Arg
                85                  90                  95
His Lys Gln Leu Ile Arg Phe Leu Lys Arg Leu Asp Arg Asn Leu Trp
            100                 105                 110
Gly Leu Ala Gly Leu Asn Ser Cys Pro Val Lys Glu Ala Asn Gln Ser
        115                 120                 125
Thr Leu Glu Asn Phe Leu Glu Arg Leu Lys Thr Ile Met Asp Glu Lys
    130                 135                 140
Asp Ser Lys Cys Ser Ser
145                 150

<210> 7
<211> 363
<212> DNA
<213> Mus musculus

<220>
<221> source
<222> 1..363
<223> /organism="Mus musculus"
      /note="Murine Interleukin-4, Pro-peptide sequence"
      /mol_type="unassigned DNA"

<400> 7
catatccacg gatgcgacaa aaatcacttg agagagatca tcggcatttt gaacgaggtc      60

acaggagaag ggacgccatg cacggagatg gatgtgccaa acgtcctcac agcaacgaag     120

aacaccacag agagtgagct cgtctgtagg gcttccaagg tgcttcgcat attttattta     180

aaacatggga aaactccatg cttgaagaag aactctagtg ttctcatgga gctgcagaga     240

ctctttcggg cttttcgatg cctggattca tcgataagct gcaccatgaa tgagtccaag     300

tccacatcac tgaaagactt cctggaaagc ctaaagagca tcatgcaaat ggattactcg     360

tag                                                                   363

<210> 8
<211> 120
<212> PRT
<213> Mus musculus

<400> 8
His Ile His Gly Cys Asp Lys Asn His Leu Arg Glu Ile Ile Gly Ile
1               5                   10                  15
Leu Asn Glu Val Thr Gly Glu Gly Thr Pro Cys Thr Glu Met Asp Val
            20                  25                  30
Pro Asn Val Leu Thr Ala Thr Lys Asn Thr Thr Glu Ser Glu Leu Val
        35                  40                  45
Cys Arg Ala Ser Lys Val Leu Arg Ile Phe Tyr Leu Lys His Gly Lys
    50                  55                  60
Thr Pro Cys Leu Lys Lys Asn Ser Ser Val Leu Met Glu Leu Gln Arg
65                  70                  75                  80
Leu Phe Arg Ala Phe Arg Cys Leu Asp Ser Ser Ile Ser Cys Thr Met
                85                  90                  95
Asn Glu Ser Lys Ser Thr Ser Leu Lys Asp Phe Leu Glu Ser Leu Lys
            100                 105                 110
Ser Ile Met Gln Met Asp Tyr Ser
        115                 120

<210> 9
<211> 429
<212> DNA
<213> Mus musculus

<220>
<221> source
<222> 1..429
<223> /organism="Mus musculus"
      /mol_type="unassigned DNA"

<400> 9
atgggtagca gccatcatca tcatcatcac tccagcggtc tggttcctcg tggtagtcat      60

atgcacattc acgggtgtga caaaaatcat ctgcgcgaga ttatcggtat tctgaacgaa     120

gtgaccggag aaggcactcc ttgtacggaa atggatgtcc cgaacgtcct gacagcgacg     180

aaaaacacaa cggaatcgga actggtttgc cgtgccagca aagtcctgcg catcttctat     240

ctgaaacatg gtaaaacgcc gtgtctgaaa aaaaacagca gcgttctgat ggaactgcaa 300

cgcctgtttc gtgctttccg ctgcctggat agcagtatca gctgtacgat gaacgagtcc 360

aaatcaacct ccctgaaaga cttcctggaa tcactgaaat cgatcatgga tatggatgac 420

agctgataa 429

<210> 10
<211> 141
<212> PRT
<213> Mus musculus

<400> 10
Met Gly Ser Ser His His His His His His Ser Ser Gly Leu Val Pro
1               5                   10                  15
Arg Gly Ser His Met His Ile His Gly Cys Asp Lys Asn His Leu Arg
            20                  25                  30
Glu Ile Ile Gly Ile Leu Asn Glu Val Thr Gly Glu Gly Thr Pro Cys
        35                  40                  45
Thr Glu Met Asp Val Pro Asn Val Leu Thr Ala Thr Lys Asn Thr Thr
    50                  55                  60
Glu Ser Glu Leu Val Cys Arg Ala Ser Lys Val Leu Arg Ile Phe Tyr
65                  70                  75                  80
Leu Lys His Gly Lys Thr Pro Cys Leu Lys Lys Asn Ser Ser Val Leu
                85                  90                  95
Met Glu Leu Gln Arg Leu Phe Arg Ala Phe Arg Cys Leu Asp Ser Ser
            100                 105                 110
Ile Ser Cys Thr Met Asn Glu Ser Lys Ser Thr Ser Leu Lys Asp Phe
        115                 120                 125
Leu Glu Ser Leu Lys Ser Ile Met Asp Met Asp Asp Ser
    130                 135                 140

## Description

### INTRODUCTION

For the treatment of allergy, asthma and autoimmune diseases including type I diabetes, rheumatoid arthritis, and multiple sclerosis, allergen- or antigen-specific immunotherapy has the potential of restoring lasting immunological tolerance and is associated with the re-induction of allergen- or antigen-specific regulatory T cells (Tregs).

The activation, proliferation and effector functions of a large spectrum of immune-competent cells such as CD4+ cells, CD8+ cells, NK cells, NKT cells, dendritic cells, macrophages, and B cells are susceptible to suppression mediated by regulatory T cells (Tregs). The induction of Treg-suppressive activity is specific and requires antigenic or allergenic stimulation through the T cell receptor (TCR). The suppressive activity of Tregs, however, is not antigen/allergen-specific. Therefore, a wide range of immune responses can be inhibited by Tregs via 'bystander' suppression. The exact mechanisms of Treg-mediated suppression remain to be elucidated, but cell-to-cell contacts and several molecules such as IL-10, TGF-beta, CTLA-4 (cytotoxic T lymphocyte antigen 4; CD152) and granzyme/perforin are reported to contribute to the suppressive activity of Tregs (for a review, see Chen and Oppenheim, 2010).

There are two Treg populations, the naturally occurring Tregs (nTregs) and the induced or adaptive Tregs (iTregs). nTregs develop in the thymus and are exported to the periphery, whereas iTregs are converted from naive CD4+ T cells by TGF-beta in conjunction with TCR stimulation in the periphery. One population of Tregs are CD4+ T cells expressing CD25 and the transcription factor FoxP3 (forkhead box P3), a master regulator of Treg development and function. The characteristic phenotype of these Tregs is regulated by FoxP3 and includes high expression of CD25 (alpha-chain of the IL-2 receptor), CTLA-4, GITR (glucocorticoid-induced TNFR family related protein, TNFRSF 18) and low expression of CD127 (alpha-chain of the IL-7 receptor). However, highly suppressive CD4+FoxP3+ Tregs which do not express CD25 also exist and are increased at inflammatory sites (Chen et al., 2010). CD4+FoxP3+ Tregs comprise approximately 10% of peripheral CD4+ cells and play a crucial role in maintaining immune system homeostasis, preventing autoimmunity, and limiting chronic inflammation.

In addition to FoxP3+ Tregs, there are other types of Tregs that can be induced from naive CD4+ cells in the periphery including IL-10- and TGF-beta-producing Tr1 cells and TGF-beta-producing Th3 cells. These FoxP3-non-expressing T cells also play significant parts in the maintenance of peripheral tolerance and immune suppression. For example, Tr1 cells specific for common environmental allergens represent the dominant subset in individuals such as bee keepers which developed tolerance after exposure to relatively high doses of allergen. Increased numbers of local Tr1 cells have been associated with elevated levels of serum IgG4 capable of blocking allergen presentation by IgE (for a review, see Akdis and Akdis, 2011). Furthermore, specific allergen immunotherapy (SIT) is associated with the induction of both Th1 and Tr1 responses (for a review, see Larche, 2006). Following SIT, IL-10 production has been reported in T cells, B cells, and monocytes/macrophages. Thus, SIT appears to modify both antigen-presenting cell and T cell responses through the induction of IL-10 and, in some reports, TGF-beta. Peripheral blood T cells isolated after SIT were able to suppress allergen-specific proliferation of pretreatment T cells in an IL-10-dependent and TGF-beta-dependent manner since neutralizing antibodies to these cytokines abrogated suppression (for a review, see Larche et al., 2006).

The majority of research on regulatory T cells has concentrated on CD4+ Tregs, but human CD8+ Treg cells are also known to be implicated in asthma and autoimmune diseases (for a review, see Tsai and Lin, 2011). Numbers of CD8+ Treg cells are relatively small in peripheral blood, but CD8+CD25+Foxp3+ T cells can be generated by continuous antigen stimulation and T cell receptor stimulation (Mahic et al., 2008). CD8+ Treg cells can suppress cellular proliferation of CD4+ naive and effector T cells via cell-cell contact lysis or soluble factors such as IL-10 and TGF-beta. Based on recent studies, CD8+ Treg cell play an important role in limiting allergic disease. For example, systemic immunization with allergen in mice has been demonstrated to induce CD8+ Treg cells that can inhibit the development of allergic diarrhea (Siegmund et al., 2009). Furthermore, CD8+ Treg cells, but not CD4+ Treg cells, can enhance CD4+CD45ROhi+ cell apoptosis, a down-regulatory mechanism of allergen-specific Th2 cells during specific immunotherapy (SIT) that prevents the continuous activation of Th2 immune responses by allergen (Tsai et al., 2010).

### Treg function in allergic and autoimmune diseases.

It is well established that autoimmune responses result from the breakdown of immune tolerance maintained by Tregs. Either low Treg numbers with normal function or normal Treg numbers with compromised suppressive function have been made responsible for allergic and autoimmune diseases. Clinical data, however, provide a more complex scenario.

In rheumatoid arthritis (RA) the frequency of Tregs with an activated phenotype and enhanced suppressive potential in the synovial fluid of inflamed joints is increased in comparison to the periphery. Similarly, activated Tregs also accumulate in the inflamed joint of other arthropathies such as juvenile idiopathic arthritis and spondyloarthropathies. Apparently, Tregs are often increased at the site of autoimmune inflammation, presumably resulting from active recruitment and in situ proliferative expansion. Detailed analyses have demonstrated that peripheral blood CD4+CD25+ T cells of RA patients are fully competent in inhibiting the proliferation of responder CD4+CD25- T cells but they are unable to suppress the production of proinflammatory cytokines by activated T cells and monocytes (Ehrenstein et al., 2004). This failure of Tregs to inhibit cytokine production could be restored by anti-TNF therapy (Ehrenstein et al., 2004). However, subsequent analyses demonstrated that in RA patients anti-TNF therapy induced Tr1 and/or Th3 suppressor cells, but did not expand or restore the function of pre-existing nTregs (Nadkarni et al., 2007). As summarized in a recent review, current published evidence does not lend support to the notion that anti-TNF therapy can simply restore or promote Treg activity in RA patients (Chen and Oppenheim, 2010). Apparently, therapeutic approaches need to be developed capable of expanding pre-existing Tregs and enhancing their suppressive effect on effector T cells.

In contrast to the situation in RA patients, the main principle of specific immunotherapy in atopic patients is to increase the number of Tregs capable of controlling allergen-specific effector T cells. Based on current knowledge, allergen tolerance is mediated by peripherally induced regulatory T cells as evidenced by a deficit of allergen-specific IL-10 producing T cells in the peripheral blood of allergic patients (for reviews, see Schmidt-Weber *et al.,* 2005; Schmidt-Weber *et al*., 2006).

In patients with allergic asthma the situation is more complicated. Several studies have shown that the number and function of Tregs in patients with allergic asthma is impaired or altered compared with healthy individuals (for a review, see Thorburn and Hansbro, 2010). However, analysis of the number of Tregs in patients with allergic asthma revealed variability between children and adults and between peripheral blood and airway tissue (for a review, see Langier et al., 2012). In paediatric patients with allergic asthma, fewer CD4+CD25+ T cells were found in blood and broncho-alveloar lavage (BAL) samples than in healthy control subjects (Lee et al., 2007; Hart1 et al., 2007), whereas in the blood of paediatric patients with persistent severe allergic asthma higher numbers of CD4+CD25+ T cells were observed (Lee et al., 2007). In the blood of adult patients with moderate to severe allergic asthma low numbers of Tregs were reported, but patients with mild disease had higher numbers of Tregs than the other two groups (Abdulamir et al., 2009). In contrast, in the BAL of adult patients with moderate to severe allergic asthma an increase in the number of CD4+FoxP3+ Tregs was observed as compared to patients with mild disease and to healthy controls (Smyth et al., 2010). The interpretation of these data, however, requires caution since Tregs characterized by CD4 and CD25 positivity alone in some of the studies may also represent activated effector T cells. Furthermore, assessment of Treg numbers in blood may not represent Treg numbers that have migrated to the site of inflammation. Despite these controversial observations, however, there is convincing evidence that supports the requirement for Tregs in the control of asthma. For example, glucocorticoid treatment of asthma is effective in suppressing inflammation and symptoms. These therapeutics induce a short-term up-regulation of FoxP3 expression and Tregs in patients with asthma (Karagiannidis et al., 2004). Further support is provided by mouse models of allergic airways disease. Adoptive transfer of CD4+CD25+ Tregs into sensitized mice before antigen challenge has been demonstrated to suppress the development of allergic disease (Kearley et al., 2005; Strickland et al., 2006). Furthermore, adoptive transfer of Tregs after onset of the disease attenuates established inflammation (Kearley et al., 2008).

There is also evidence that human patients with diabetes type 1 or multiple sclerosis have a reduced frequency or functionality of CD4+ CD25+ regulatory T cells (Kukreja et al., 2002; Viglietta et al., 2004). The necessity for increasing the number of CD4+ CD25⁻ regulatory T cells in patients with type I diabetes is supported by the observation that several therapeutic approaches which protect from diabetes, are correlated with an increase in CD4+ CD25+ regulatory T cells (for a review, see Juedes and von Herrath, 2004). For example, increased numbers of circulating CD4+ CD25+ regulatory T cells have been reported in patients receiving successful islet transplants and were treated with the anti-CD3 mAb hOKT3γ1(Ala-Ala) (Hering et al., 2004).

Taken together, the role of Tregs in allergic and autoimmune diseases indicates that control of the development, survival, and function of Tregs is instrumental for effective control of immune responses. As evident from the foregoing, however, restoring lasting immunological tolerance by specific immunotherapy in combination with the re-induction of allergen- or antigen specific Treg cells requires individual treatment protocols for allergy, allergic asthma, type I diabetes, rheumatoid arthritis, and multiple sclerosis.

### Treg modulation by complement.

In recent years, a variety of mechanisms modulating the immune response have been elucidated in detail including the complement system, TNF-alpha-mediated pathways, and other cytokine-mediated pathways.

As a central component of the innate immune system, complement is a key player in the body's defense against invading microorganisms, but it is also involved in the clearance of self-antigens and apoptotic cells, and it is a vital participant in the induction of adaptive responses (for a review, see Ricklin et al., 2010). Several studies have demonstrated that C3a and C5a can up-regulate TH1 responses by modulating DC activation and function (for a review, see Zhou, 2012). C3aR-mediated signaling has also been associated with the inhibition of TH2 responses by promoting IL-12 (a TH1-driving interleukin) production in DCs. Accordingly, in C3aR deficient mice the immune response upon epicutaneous introduction of antigen shifted towards TH2 responses (Kawamoto et al., 2004). In contrast, deficiency of C5 or C5aR inhibited TH2 immune responses in asthma, thus providing protection to antigen-challenged mice (Drouin et al., 2006; Köhl et al., 2006).

C3a and C5a can also up-regulate macrophage function to promote TH1 immune responses (for a review, see Zhou, 2012). Furthermore, recent evidence suggested that C5a can also promote the development/expansion of TH17 cells by the action on macrophages, thus contributing to the pathogenesis of experimental autoimmune diseases (Fang et al., 2009; Hashimoto et al., 2010).

Most important are the effects of C3aR and C5aR signaling on the induction of Tregs. DCs are able to expand antigen-specific CD4+CD25+ regulatory T cells (Yamazaki et al., 2003; Kretschmer et al., 2005) and Peng et al. (2006) have demonstrated that C3^{+/+} and C3^{-/-} DCs (which are unable to generate C3a or C5a) have different abilities to elicit the development of Tregs. Co-culturing of CD4+ T cells with allogeneic C3 deficient (C3^{-/-}) DCs for 9 days yielded a 4-fold higher level in T cells expressing the *foxp3* gene (used as marker for CD4+CD25+ regulatory T cells; Fontenot et al., 2003) as compared to those stimulated with C3^{+/+} DCs. In a very recent study, Strainic et al. (2013) have analyzed the phenotype of T cell responses when C3aR- and C5aR-mediated signals are absent on DCs, CD4+ T cells or both. In vitro activation of naive CD4+ T cells from mice deficient in both C3aR and C5aR resulted in a lower abundance of the proinflammatory cytokines IL-6 and IFN-gamma, but a greater abundance of the anti-inflammatory cytokines IL-10 and TGF-beta1. Furthermore, the frequency of Foxp3+ T cells proved to be much greater in cultures of *C3ar1*^{*-*/}*⁻C5ar1*^{*-*/*-*} T cells than in cultures derived from *C3ar1*^{*-*/*-*} mice, *C5ar1*^{*-*/*-*} mice or wild-type mice. These data indicate that signaling via the complement factors C3a and C5a regulates effector T cell responses and that the absence of local complement activation paves the way for a default pathway leading to Foxp3+ Tregs.

### Treg modulation by TNF-alpha-mediated pathways.

TNF-alpha is a pleiotropic cytokine with biphasic pro-inflammatory and anti-inflammatory effects. Upon activation of pro-inflammatory signals, TNF-alpha is produced by DCs, macrophages, B cells, T cells, and by other types of somatic cells such as endothelial cells, mast cells, and neuronal tissue cells. TNF-alpha is first produced as a transmembrane protein (mTNF-alpha) that can subsequently be cleaved by the TNF-alpha converting enzyme (TACE, which releases a soluble form (sTNF-alpha). Both mTNF-alpha and sTNF-alpha are biologically active. TNF-alpha acts by binding to two receptors, TNFR1 (CD120a, or p55) and TNFR2 (CD120b, or p75). TNFR1 is ubiquitously expressed by a broad array of cell types, contains a death domain in its intracellular portion and can induce apoptosis via a caspase 3-dependent pathway. TNFR2 does not contain a death domain and binds preferentially to mTNF-alpha.

TNFR2 is expressed by cells of the immune system, predominantly on Tregs. All human thymic CD4+CD25+ Tregs and immunosuppressive human CD4-CD8+CD25+ T cells constitutively express TNFR2, while thymic CD4+CD25- cells do not express this receptor. In human peripheral blood cells, CD4+CD25+ T cells express the highest level of TNFR2. Only a fraction of CD4+CD25- T cells (20-30%) express TNFR2 at a low level. After TCR stimulation, surface expression of TNFR2 is up-regulated on both CD4+CD25+ and CD4+CD25- T cells, but activated CD4+CD25+ T cells still express considerably higher levels of TNFR2. TNFR1 was not detected by FACS on either CD4+CD25+ or CD4+CD25- T cells (for a review, see Chen and Oppenheim, 2010).

Induction of the suppressive effect of human antigen-specific regulatory T cells by tolerogenic dendritic cells has been demonstrated to involve mTNF-alpha but not sTNF-alpha (Kleijwegt et al., 2010). In this study, anti-TNF-alpha antibodies (blocking both mTNF-alpha and sTNF-alpha) prevented Tregs from acquiring a suppressive effect on the proliferation of effector T cells (Teff), whereas soluble TNFR2 (blocking only sTNF-alpha) had no effect on the induction of a suppressive effect.

In the presence of interleukin 2 (IL-2), TNF-alpha-mediated activation of Tregs results in proliferation, up-regulation of FoxP3 expression and increase of their suppressive activity (for reviews, see Chen and Oppenheim, 2010; Biton et al., 2012). TNF-alpha has been shown to selectively up-regulate CD25 (alpha-chain of the IL-2 receptor) expression on Tregs (Chen et al., 2007), while IL-2 preferentially up-regulates TNFR2 expression on Tregs (Chen and Oppenheim, 2010). Thereby, TNF-alpha and IL-2 generate a powerful mechanism for receptor amplification and synergistically up-regulate Treg suppressive activity. Tregs are also capable to shed TNFR2, resulting in neutralization of sTNF-alpha (van Mierlo et al., 2008). Furthermore, in concert with a common gamma-chain cytokine (IL-2, IL-7, or IL-15) TNF-alpha also up-regulates the expression of other co-stimulatory members of the TNFR superfamily on Tregs including 4-1BB (CD137; TNFRSF9) and OX40 (CD134; TNFRSF4) (Hamano et al., 2011). Thus, TNF-alpha enhances multiple TNFRSF pathways by up-regulating a number of receptors that can cooperate to limit excessive inflammation and prevent self-destructive tissue damage.

*Treg modulation by vitamin D-mediated pathways.*

Vitamin D, in particular the biologically active metabolite 1α,25-dihydroxyvitamin D3 (1,25-(OH)₂D3), is a pleiotropic hormone exerting a variety of biological effects including the regulation of calcium, bone and mineral metabolism, as well as the modulation of immune responses by promoting both directly and indirectly regulatory T cell populations (for reviews, see Hart et al., 2011; Chambers and Hawrylowicz, 2011; Fletcher et al., 2012).

Vitamin D3, also known as cholecalciferol, is synthesized from 7-dehydrocholesterol in the skin upon exposure to ultraviolet light, then transported to the liver, where it is converted to 25-hydroxyvitamin D3 (25(OH)D3; calcidiol) by cytochrome P450 enzymes. 25(OH)D3 is a circulating metabolite that is converted to the active hormone 1,25-(OH)₂D3 (calcitriol) in the mitochondria of the kidney by 25-hydroxyvitamin lahydroxylase (CYP27B1). However, numerous other cell populations including keratinocytes in the epidermis possess the enzymes capable of processing vitamin D3 to active metabolites (Anderson et al., 2008). Macrophages, dendritic cells (DCs) as well as T and B cells express CYP27B1 when they are activated and, thereby, have the ability to produce the active hormone 1,25-(OH)₂D3 (Bikle, 2009).

1,25-(OH)₂D3 binds to the vitamin D receptor (VDR), a member of the superfamily of nuclear hormone receptors, followed by binding to the retinoic X receptor (RXR) and formation of a heterodimer. The heterodimer translocates to the nucleus where it can bind to specific DNA sequence elements, so-called vitamin D response elements (VDREs) identified as direct repeats of PuG(G/T)TCA motifs, thereby promoting transcription of vitamin D-responsive genes. The VDR-RXR heterodimer can also bind to a negative vitamin D response element, thereby preventing gene transcription. Alternatively, the VDR-RXR heterodimer can bind to transcription factors present in the nucleus which prevents binding of these transcription factors to their target gene promoters. VDR is expressed by many cells of the immune system including activated B and T cells, monocytes and DCs (for a review, see Chambers and Hawrylowicz, 2011).

Several studies have demonstrated that locally synthesized 1,25-(OH)₂D3 activates innate immune responses, but can also suppress adaptive immune responses (for reviews, see Bouillon et al., 2008; Hewison, 2010). Suppressive effects of 1,25-(OH)₂D3 on adaptive immune responses include antigen-presenting cell functions, effector T cell responses and the induction of regulatory T cells (Tregs). For example, 1,25-(OH)₂D3 treatment of human DCs in vitro resulted in reduced expression of the co-stimulatory molecules CD80 and CD86 as well as decreased expression of HLA-DR and the maturation marker CD83, all associated with an immature DC phenotype which has poor stimulatory function for the induction of effector T cell responses (Penna and Adorini, 2000). It is important to note that immature DCs often drive Treg responses. 1,25-(OH)₂D3 also affects DC maturation from monocytes by inhibition of IL-12p70 production and enhanced IL-10 secretion upon activation by CD40 ligation (Penna and Adorini, 2000). Furthermore, 1,25-(OH)₂D3 has been demonstrated to induce in vitro the expression of the inhibitory receptor ILT3 (immunoglobulin-like transcript 3) on DCs which appears to play a role in inhibiting T cell responses (Penna et al., 2005).

Vitamin D affects effector T cell responses also via direct inhibition of T cell responses. For example, 1,25-(OH)₂D3 has been shown a) to inhibit Th1 cytokine release with a large reduction in IFN-γ from human peripheral CD3+CD4+ T cells (Reichel et al., 1987), and b) to hinder cytokine production by Th17 cells (Tang et al., 2009). The effects of vitamin D on Th2 responses appear to be concentration-dependent. After the addition of a single very high dose of 1,25-(OH)₂D3 to human blood mononuclear cells, enhancement of Th2 cytokine production was observed (Jirapongsananuruk et al., 2000). In contrast, lower concentrations of 1,25-(OH)₂D3 inhibited the expression of IL-4 and other Th2 cytokines in human T cells (Pichler et al., 2002).

Concomitant with the inhibition of effector T cell responses by 1,25-(OH)₂D3 is the induction of Treg populations. For example, in bulk cultures of human CD4+CD25⁻ T cells and putative naive T cells, 1,25-(OH)₂D3 increased in the presence of IL-2 the frequency of activation-induced FoxP3+ T cells expressing high levels of the inhibitory receptor CTLA-4 (cytotoxic T lymphocyte antigen 4). Furthermore, a significant reduction in the pro-inflammatory cytokines IFN-γ and IL-17 was observed in this study (Jeffery et al., 2009). In another study, treatment of human peripheral blood CD4+ T cells with 1,25-(OH)₂D3 induced IL-10+ Tregs expressing Toll-like receptor (TLR)9 (Urry et al., 2009). The concomitant induction of TLR9 expression, employed as biomarker of vitamin D-induced IL-10+ Tregs, could be relevant for infections. Ligation of TLR9 with agonistic CPG oligonucleotides turned off IL-10 production suggesting a control mechanism whereby Treg function is transiently blocked to facilitate a more effective immune response for clearance of the pathogen. The capacity of 1,25-(OH)₂D3 to promote tolerogenic T cell functions in humans is further supported by the observation that vitamin D supplementation and pharmacologic treatment with biologically active vitamin D increased the level of serum- or T cell-associated TGF-β and IL-10 (Mahon et al., 2003; Urry et al., 2009).

In a recent study, the molecular and cellular events underlying the immunosuppressive effects of 1,25-(OH)₂D3 have been elucidated more in detail (van der Aar et al., 2011). 1,25-(OH)₂D3 promotes tolerogenic epidermal Langerhans cells (LCs) and dermal dendritic cells (DDCs). Both subsets are able to generate Treg cells with different effector functions. Treatment of epidermal LCs with 1,25-(OH)₂D3 generates functional Foxp3+ Tregs through a mechanism that is dependent on keratinocyte-derived TGF-β. In contrast, treatment of DDCs with 1,25-(OH)₂D3 generates functional IL-10+FoxP3⁻ T_{R}1 cells in an IL-10-dependent fashion.

### Treg modulation by IL-4/IL-13.

Among the cytokines contributing to the development of allergic inflammation, IL-4 and IL-13 play key roles in the pathogenesis. Both IL-4 and IL-13 represent pleiotropic cytokines and are associated with the induction of the IgE isotype switch, secretion of IgE by B lymphocytes, and enhancement of IgE-mediated responses by up-regulating IgE receptors on B lymphocytes, mast cells and basophils. IL-13 has also important effects on epithelial cell maturation, mucus production, generation of extracellular matrix proteins, enhancement of the contractility of airway smooth muscle cells, and the recruitment of eosinophils, macrophages and T cells.

Most important is the fact that IL-4 inhibits the expression of Foxp3, which is a requirement for inducible Treg (iTreg) differentiation. As a result, IL-4 is able to drive the differentiation of naive Th cells into Th2 lymphocytes, leading to the production of effector cytokines such as IL-4, IL-5, IL-9, and IL-13. Furthermore, IL-4 has been shown to be crucial for the priming of antigen-specific CD8+ T cells after vaccination with the Leishmania antigen HASPB-1 (Stäger et al., 2003). Based on this observation, IL-4 may be crucial also for the priming of autoreactive CD8+ cytotoxic T cells in autoimmune diseases such as type I diabetes and multiple sclerosis.

IL-4 exerts its activities by interacting with specific Type I and type II receptors on the cell surface (for a review, see Gessner et al., 2000). The type I receptor comprises a 140 kDa binding chain (Swiss Prot accession number P24394), IL-4Rα (also referred to as CD124), and the common γ-chain (γ_{c}) of the IL-2R, which is shared by multiple cytokine receptors. In the absence of the common γ-chain IL-4 can use the type II IL-4 receptor, comprising IL-4Rα and an IL-13 binding chain, IL-13Rα1. This receptor complex is also a functional receptor for IL-13, and this explains the overlap of the majority of biological effects of IL-4 and IL-13, although both cytokines exhibit only 25% homology. Type I receptor complexes can only be formed by IL-4 and appear to be responsible for mediating IL-4 responses in T cells which do not express IL-13Rα1. Type II receptor complexes can be formed and activated by either IL-4 or IL-13. The type I receptor complex predominates in hematopoietic cells, whereas the type II receptor complex is expressed on both hematopoietic cells and non-hematopoietic cells. The binding sequence of IL-4 and IL-13 to type II receptor complexes differs in that IL-4 first binds to IL-4Rα with high affinity before associating with the second subunit, whereas IL-13 first binds to IL-13Rα1 with low affinity and then this complex recruits IL-4Rα to form a high affinity binding site.

### Problems associated with therapeutic Treg modulation in combination with specific immunotherapy.

It is obvious that currently used treatment protocols for allergen- or antigen-specific immunotherapy need to be combined with additional Treg-modulating techniques to restore lasting clinical tolerance. For the establishment of suitable treatment modalities, however, several issues need to be addressed and solved. For example, the different Treg-modulating pathways described above offer several possibilities for therapeutic intervention, but suitable Treg-modulating therapeutics for a combination with allergen- or antigen-specific immunotherapy need to be defined.

Furthermore, the development of immunologic memory requires the engagement of the T cell receptor (TCR) over 12-48 h and long lasting memory may even require repetitive exposure. Accordingly, Treg-modulating therapeutics need to be in effect at least for the same period of time at the site of allergen or antigen presentation, most likely as long as allergens or antigens are released from the injected allergen-adjuvant complex.

As important is the need for high local concentrations of Treg-modulating therapeutics at the site of allergen or antigen presentation. Since activated complement factors, TNF-alpha and IL-4 can be shuttled in an antigen-specific manner in the immunological synapse that is formed between the antigen presenting cells and the T cells, effective inhibition of these mechanisms in the micro-environment of the immunological synapse requires high local inhibitor concentrations. High local concentrations of inhibitors, however, are extremely difficult to achieve via systemic administration and high doses of therapeutics are frequently associated with increased adverse effects.

There is also a need for Treg-modulating therapeutics with a relatively short plasma half-life to minimize potential adverse effects mediated by interaction of the therapeutics with targets away from the site of allergen or antigen presentation.

Most important is the need for effective combinations of Treg-modulating therapeutics capable of addressing the complex network of Treg induction at different target sites. Immune responses are the result of multi-factorial processes and mono-therapies targeting a single process are unlikely to be successful. It is a characteristic feature of networks that inhibition of an individual pathway can be compensated by activation of other network-associated pathways. The need for effective combinations of Treg-modulating therapeutics is further complicated by the requirement of individual combinations for the different allergic and autoimmune diseases. For example, several chronic inflammatory and autoimmune disorders such as chronic inflammatory arthritis and multiple sclerosis (MS) are associated with deregulated TNF over-expression. However, TNF-blocking agents have been shown to be beneficial for the treatment of rheumatoid arthritis, whereas anti-TNF therapy in patients with MS resulted in worsening of disease symptoms.

As of today, there is a need for suitable approaches for the treatment of different allergic and autoimmune diseases that enable efficient specific immunotherapy supported by individual combinations of Treg-modulating therapeutics.

The present invention solves these problems by disclosing methods for sustained local delivery at the site of allergen or antigen presentation of high concentrations of various low molecular weight therapeutics which a) are capable of addressing the complex network of Treg induction at different target sites, b) can be combined according to the individual requirements of allergic and autoimmune diseases, and c) provide all the characteristics required for efficient support of specific immunotherapy including high solubility in aqueous environments for fast diffusion into the immunological synapse and fast elimination from the circulation upon diffusion away from the site of allergen or antigen presentation.

### SUMMARY OF THE INVENTION

With a few exceptions, the therapeutic efficacy of current allergen- or antigen-specific immunotherapeutic protocols for the treatment of allergy, allergic asthma, type I diabetes, rheumatoid arthritis, and multiple sclerosis needs to be optimized. Control of the development, survival, and function of regulatory T cells (Tregs) is essential for therapeutic success. However, the complex network of Treg induction requires novel approaches capable of addressing this network at different target sites. The present invention provides methods for restoring lasting immunological tolerance by allergen- or antigen-specific immunotherapy in combination with individual combinations of Treg-modulating therapeutics for the treatment of allergy, allergic asthma, type I diabetes, rheumatoid arthritis, and multiple sclerosis. In the present invention, methods are disclosed for allergen- or antigen-specific immunotherapy in combination with sustained, locally restricted delivery of high concentrations of low molecular weight immune modulators capable of stimulating the induction of Tregs at the site of allergen or antigen presentation. Thereby, the therapeutic efficacy of allergen-or antigen-specific immunotherapy is significantly increased, and due to the locally restricted delivery of low molecular weight immune modulators at the site of allergen or antigen presentation, potential adverse side effects induced by systemic application of such immune modulators are minimized.

In one embodiment, the present invention discloses low molecular weight immune modulators capable of addressing the complex network of Treg induction at different target sites including low molecular weight immune modulators targeting complement-mediated pathways, TNF-alpha-mediated pathways, vitamin D3-mediated pathways, and IL-4/Il-13-mediated pathways.

In one specific embodiment, the present invention discloses low molecular weight complement inhibitors capable of modulating the induction of Tregs. In a preferred specific embodiment, the present invention discloses low molecular weight complement inhibitors targeting complement protein C3 and interactions of the anaphylatoxins C3a and C5a with their respective receptors.

In another specific embodiment, the present invention discloses low to moderate molecular weight inhibitors of TNFR1 or TNFR1-mediated functions capable of modulating the induction of Tregs. Suitable low to moderate molecular weight inhibitors of TNFR1 or TNFR1-mediated include but are not limited a) to molecules capable of specific inhibition of sTNF so that the mTNF-TNFR1 and mTNF-TNFR2 interactions remain intact such as dominant-negative sTNF mutants, b) molecules capable of specific inhibition of TNFR1 while leaving TNFR2 signaling intact such as antagonistic TNF mutants with specificity for TNFR1, antisense oligonucleotides with specificity for TNFR1, aptamers with specificity for TNFR1, and oligonucleotides capable of TNFR1 silencing via RNAi, and c) molecules capable of inhibiting TNFR1-mediated pathways including such as S-methylglutathione, pro-glutathione drugs (e.g., N-acetylcysteine), and salicylates.

In another specific embodiment, the present invention discloses low molecular weight molecules capable of modulating the induction of Tregs via vitamin D-mediated pathways including but not limited to a) active vitamin D3 (calcitriol; 1,25-(OH)₂D3), b) its inactive form (cholecaliferol; 25(OH)D3), c) vitamin D3 analogues such as 19-nor-vitamin D analogues, 24-hydroxy vitamin D derivatives, and 1α-hydroxyvitamin D3, and d) non-secosteroidal vitamin D receptor (VDR) modulators.

In still another specific embodiment, the present invention discloses low to moderate molecular weight molecules capable of modulating the induction of Tregs via inhibition of IL-4/IL-13-mediated pathways including but not limited to a) antagonistic IL-4 and IL-13 derivatives, b) aptamers with specificity for IL-4 and IL-13 capable of blocking their biologic activity, c) aptamers with specificity for IL-4 and IL-13 receptor complexes capable of blocking the interaction of IL-4 and IL-13 with their receptor subunits, and d) monoclonal antibodies fragments or other medium molecular weight proteinaceous constructs with specificity for IL-4 and IL-13 receptor complexes capable of blocking the interaction of IL-4 and IL-13 with their receptor subunits.

In another embodiment, the present invention discloses methods for restricting high local concentrations of low molecular weight immune modulators capable of stimulating the induction of Tregs mainly to the site of allergen presentation to reduce adverse effects due to interaction of the immune modulators with targets distal from the site of allergen or antigen presentation. In one specific embodiment, low molecular weight immune modulators are used which provide a relatively short serum half-life that is sufficient to be locally active upon their release from a depot at the site of allergen presentation, and which allows fast removal from circulation upon diffusion and transport away from the site of allergen presentation. Preferred therapeutics providing such characteristics include but are not limited to a) oligopeptide-based complement inhibitors, b) oligonucleotide-based therapeutics for the inhibition of TNF-alpha-, IL-4/IL-13- and complement-mediated pathways, c) glutathione- and salicylate-based therapeutics for the inhibition of TNFR1-mediated pathways, d) selected vitamin D3 analogs such as calipotriol, and e) low molecular weight proteins such as Il-4 muteins and TNF-alpha mutants.

In another embodiment the present invention discloses suitable matrices for sustained local delivery of low molecular weight immune modulators capable of stimulating the induction of Tregs at the site of allergen presentation along with a prolonged presentation of allergens or antigens. To achieve effective and prolonged inhibition of TNFR1 or TNFR1-mediated functions at the site of allergen or antigen presentation, the present invention discloses methods for a sustained release of such immune modulators or combinations thereof from a depot at the site of allergen presentation. Preferred matrices include but are not limited to biodegradable polymers which are suitable as depot for substantial quantities of such immune modulators or combinations thereof, which allow the release of sufficient quantities of such immune modulators or combinations thereof for efficient local induction of Treg cells over a prolonged period of time, and which are chemically and physically compatible with the adjuvant and allergen (s) or antigen(s) used for the induction of tolerance according to the method of the present invention. In a preferred specific embodiment, injectable in situ-forming gel systems which are biodegradable, are used for controlled delivery of low molecular weight immune modulators capable of stimulating the induction of Tregs or combinations thereof. Such in situ-forming gel systems (hydrogels) undergo a sol-gel-sol transition, which is free flowing sol at room temperature and a non-flowing gel at body temperature. Compared to other biodegradable polymers, the injectable thermo-gelling polymers possess several advantages including easy preparation, high encapsulation efficiency of bioactive molecules such as low molecular weight immune modulators or combinations thereof, and free of harmful organic solvents in the formulation process.

In another embodiment, the present invention discloses adjuvants for allergen or antigen presentation according to the method of the present invention. If IL-4/IL-13 inhibitors are included in the treatment protocols, adjuvants are suitable which elicit Th2-type immune responses including but are not limited to aluminum salts, Montanide emulsions (squalene-based water-in-oil emulsions) and polyphosphazenes. If vitamin D3 or analogs thereof are included in the treatment protocols, adjuvants are also suitable which elicit Th1-type immune responses including but are not limited to monophosphoryl lipid A (MPL) and CpG oligonucleotides. Adjuvants eliciting a combined Th1- and Th2-type immune response may also be used for the method of the present invention if inhibitors of Il-4- and IL-13-mediated pathways and vitamin D3 or analogs thereof are included in the treatment protocols. Suitable adjuvants eliciting a combined Th1-type and Th2-type immune response include but are not limited to squalene-based oil-in-water emulsions, granulocyte-macrophage colony stimulating factor (GM-CSF), and adjuvants based on inulin and virosomes.

In another embodiment, the present invention discloses compositions comprising a) one or more low or medium molecular weight immune modulators capable of stimulating the induction of Tregs, b) one or more allergens or antigens, c) optionally one or more adjuvants, and d) one or more matrices mediating sustained delivery of the components. In a preferred specific embodiment, compositions comprise immune modulators which are selected from low molecular weight complement inhibitors, low or medium molecular weight inhibitors of TNFR1 or TNFR1-mediated functions, low or medium molecular weight inhibitors of IL-4/IL-13-mediated pathways, and low molecular weight molecules capable of modulating the induction of Tregs via vitamin D-mediated pathways. In another preferred specific embodiment, compositions are used which comprise combinations of such immune modulators addressing the network of Treg induction at different target sites. In still another preferred embodiment, matrices mediating sustained delivery of the components are selected from injectable in situ-forming gel systems which are biodegradable.

In another embodiment, the present invention discloses compositions for separate local administration of a) allergens or antigens (composition A) and b) low to moderate molecular weight immune modulators capable of stimulating the induction of Tregs (composition B). In a preferred embodiment, composition A comprises one or more allergens or antigens, optionally in the presence of one or more adjuvants, and one or more matrices mediating sustained delivery of the components. Composition B comprises combinations of low to moderate molecular weight immune modulators capable of stimulating the induction of Tregs, and one or more matrices mediating sustained delivery of the components at the site of allergen or antigen presentation. Preferred matrices mediating sustained delivery of a) allergens or antigens and, optionally, adjuvants in composition A and b) immune modulators in composition B are selected from injectable in situ-forming gel systems which are biodegradable. Preferred combinations of low or medium molecular weight immune modulators comprise therapeutic molecules which are selected from low molecular weight complement inhibitors, low to moderate molecular weight inhibitors of TNFR1 or TNFR1-mediated functions, low to moderate molecular weight inhibitors of IL-4/IL-13-mediated pathways, and low molecular weight molecules capable of modulating the induction of Tregs via vitamin D-mediated pathways.

In another embodiment, the present invention discloses methods for incorporating compositions into pharmaceutical formulations suitable for administration.

In another embodiment, the present invention discloses the application of compositions providing sustained local delivery of low or medium molecular weight immune modulators capable of stimulating the induction of Tregs, in combination with antigen- or allergen-specific immunotherapy for the treatment of patients suffering from allergy, allergic asthma or various autoimmune diseases including but not limited to type 1 diabetes, rheumatoid arthritis, juvenile idiopathic arthritis, and multiple sclerosis.

In a preferred embodiment, the present invention discloses individual combinations of low to moderate molecular weight immune modulators, capable of stimulating the induction of Tregs, in compositions designed for the treatment of individual allergic and autoimmune diseases in combination with antigen- or allergen-specific immunotherapy. Compositions comprising low molecular weight complement inhibitors are applied preferably for the treatment of patients having a complement-associated condition and/or for which inhibition of complement at the C3 level or inhibition of C3a and C5a signaling is beneficial. Compositions comprising low to medium molecular weight inhibitors of TNFR1 or TNFR1-mediated functions are applied preferably for the treatment of patients having a TNF-alpha-related condition and/or for which enhanced mTNF-TNFR2 signaling by specific inhibition of TNFR1-mediated signaling is beneficial. Compositions comprising low molecular weight molecules capable of modulating the induction of Tregs via vitamin D-mediated pathways are applied preferably for the treatment of patients having an autoimmune disease related to vitamin D deficiency and/or for which vitamin D, analogs thereof or low molecular weight VDR modulators are beneficial. Compositions comprising low or medium molecular weight inhibitors of IL-4/IL-13-mediated pathways are applied preferably for the treatment of patients having a Th2-related condition and/or which are treated with therapeutics including adjuvants promoting Th2 immune responses.

In yet another embodiment, the present invention discloses therapeutic methods including therapeutic applications of suitable compositions, the determination of therapeutically effective doses, and modes of administration for the induction of allergen or autoantigen tolerance using the compositions of the present invention.

Specific preferred embodiments of the present invention will become evident from the following more detailed description and the claims.

### BRIEF DESCRIPTION OF THE DRAWINGS

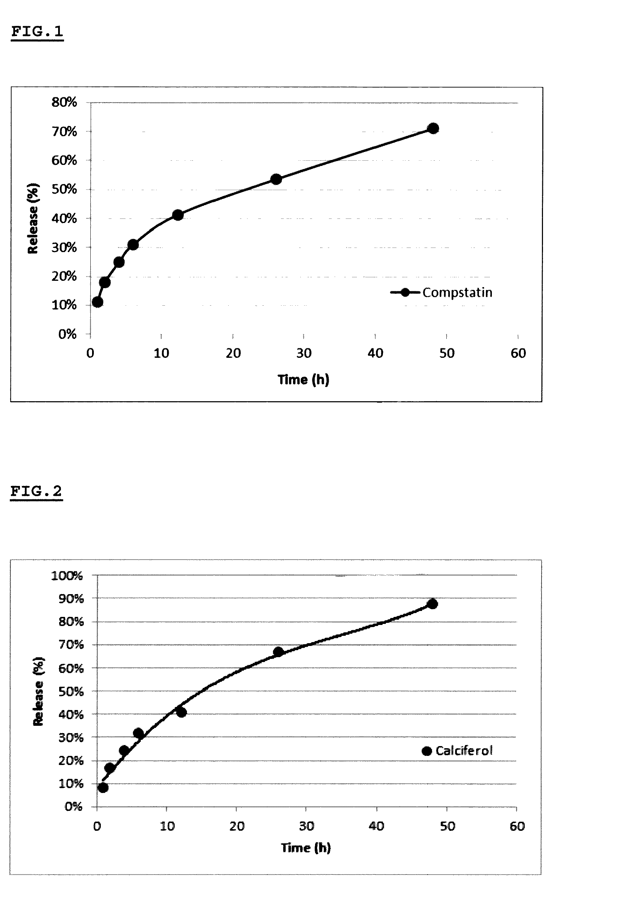

Figure 1 shows the *in vitro* release of the 13-residue cyclic peptide (H-I[CVVQDWGHHRC]T-NH₂) (termed compstatin, see SEQ ID 1) from the hydrogel of Example 2.1. A stock solution of 2 mg/ml in 30% acetonitrile of compstatin (Tocris Bioscience, UK) is prepared. 20 µl of stock solution are combined with 160 µl of 25% gel solution from Example 2.1. and 20 µl of 10x PBS. The mixture was incubated for 2 minutes at 37°C to induce gelling and overlayed with 200 µl of 1x PBS. At frequent timepoints the supernatant was removed by pipetting and stored at 4°C. The removed supernatant was replaced by fresh 200 µl of 1x PBS. After 48 hours the samples were measured spectrometrically at 280 nm and the amount of released compstatin was calculated as percentage of a reference sample containing a concentration of compstatin equalling 100% release.
Figure 2 shows the *in vitro* release of calcitriol (1α,25-dihydroxyvitamin D3; 1,25-(OH)₂D3) from the hydrogel of Example 2.1. A stock solution of 2 mg/ml in absolute ethanol of calcitriol (Cayman/Biomol GmbH, Hamburg) is prepared. 20 µl of stock solution are combined with 160 µl of 25% gel solution from Example 2.1. and 20 µl of 10x PBS. The mixture was incubated for 2 minutes at 37°C to induce gelling and overlayed with 200 µl of 1x PBS. At frequent timepoints the supernatant was removed by pipetting and stored at 4°C. The removed supernatant was replaced by fresh 200 µl of 1x PBS. Controls of the same concentration of calciferol in 1x PBS without gel are incubated and sampled in parallel. After 48 hours the samples and controls are analysed using an Vitamin D ELISA kit, according to the manual of the manufacturer (Euroimmun AG, Luebeck). In brief, the samples are diluted ?. 20 µl of the sample a mixed with 230 µl of sample buffer containing a biotin-labed 25-OH vitamin D derivative. 200 µl of the mixture are transferred to an ELISA well of a 8-well strip and incubated at room temperature for 2 hours. The wells are washed with 3x 300 µl washing buffer. 100 µl of peroxidase labelled steptavidin-conjugate is added and incubated for a further hour. The well is again washed 3x with 300 ml washing buffer. 100 µl of TMB (tetramethyl-benzidine) substrate solution is added and developed until sufficient coloring. The reaction is stopped by addition of 100 µl 0.5 M sulphuric acid and measured spectrometrically at 420 nm with reference 650 nm.

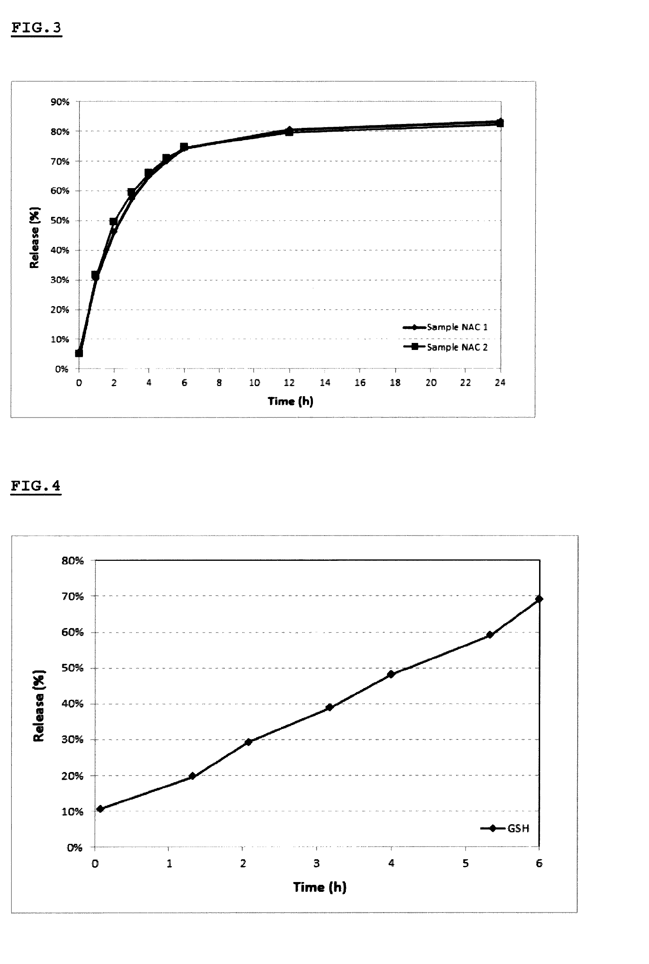

Figure 3 shows the *in vitro* release of N-acetyl-L-csyteine (NAC) from gelled polymer at 37°C. The polymers (25% concentration in PBS pH 7.4) were synthesized according to Example 2.1. Varying volumes (10 µl, 20 µl, 40 µl, 80 µl) of NAC (50 mg/ml PBS (0.3 M) at pH 7.4,) were added to 200 µl gel solution and incubated at 37°C for gelation. Gels were covered with 1.8 ml PBS and incubated at 37°C. At indicated times samples of the supernatant of the gels were taken and analysed for NAC using the reaction of 4,4'-dithiopyridine (4-PDS) with thiols, which gives the corresponding 4-thiopyridone (4-TP).
Figure 4 shows the *in vitro* release of reduced glutathione (GSH) from gelled polymer at 37°C. The polymers (25% concentration in PBS pH 7.4) were synthesized according to Example 2.1. Varying volumes (10 µl, 20 µl, 40 µl, 80 µl) of 10 GSH in PBS, pH 7.4, were added to 200 µl gel solution and incubated at 37°C for gelation. Gels were covered with 1.8 ml PBS and incubated at 37°C. At indicated times samples of the supernatant of the gels were taken and analysed for GSH using the reaction of 4,4'-dithiopyridine (4-PDS) with thiols, which gives the corresponding 4-thiopyridone (4-TP).

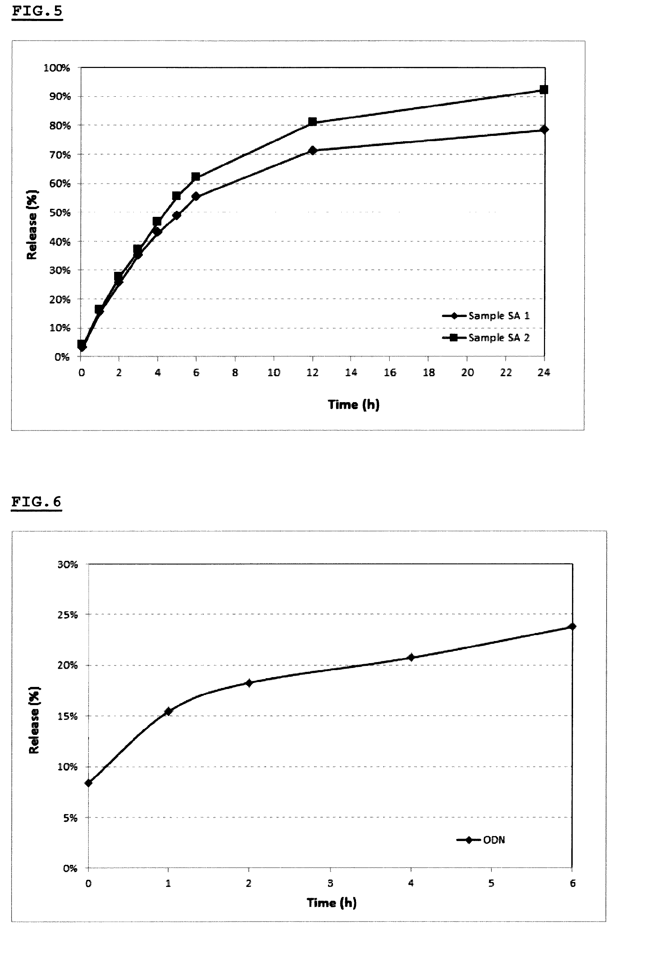

Figure 5 shows the *in vitro* release of sodium salicylate (SA) from gelled polymer at 37°C. The polymers (25% concentration in PBS pH 7.4) were synthesized according to Example 2.1. Varying volumes (10 µl, 20 µl, 40 µl, 80 µl) of 10 mM SA in PBS, pH 7.4, were added to 200 µl gel solution and incubated at 37°C for gelation. Gels were covered with 1.8 ml PBS and incubated at 37°C. At indicated times samples of the supernatant of the gels were taken and analysed for SA by derivatization with Fe(III) and quantification of the violet coloured tetraaquosalicylatroiron (III) complex at 530 nm.
Figure 6 shows the *in vitro* release of oligodeoxynucleotides (ODNs) complexed with cationic liposomes (Cellfectin) from gelled polymer at 37°C. The polymers (25% concentration in PBS pH 7.4) were synthesized according to Example 2.1. Varying volumes (10 µl, 20 µl, 40 µl, 80 µl) of ODN/Cellfection complexes prepared with a 22-mer ODN according to Example 3.5.2., were added to 200 µl gel solution and incubated at 37°C for gelation. Gels were covered with 1.8 ml PBS and incubated at 37°C. At indicated times samples of the supernatant of the gels were taken and analysed for released ODNs by absorption at 260 nm.

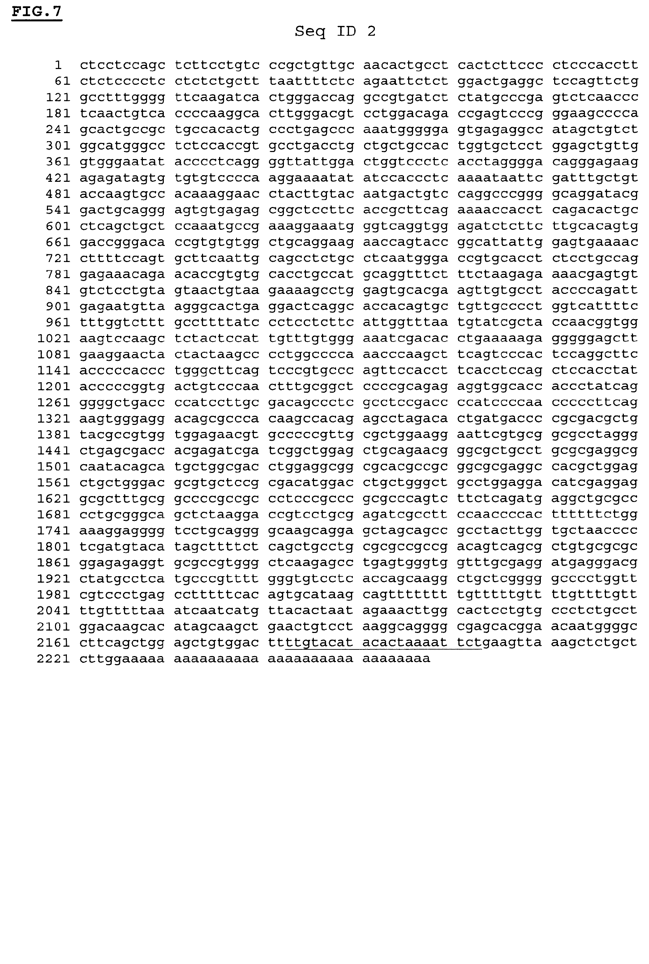

Figure 7 shows the DNA sequence coding for human TNF-alpha receptor 1 (TNFR1; Database no.: NM_001065.3; mRNA with 5'-and 3'-untranslated regions; 2258 base pairs; CDS: 304-1671, Signal peptide: 304-366, Mature peptide: 367-1668, see SEQ ID 2).
   The underlined sequence marks position of TNFR1-specific 22mer antisense pPT ODN (Ojwang and Rando 1999).
   Preferred TNFR1-specific anti-sense oligodeoxynucleotides (anti-TNFR1-ODNs) are 15-mer to 25-mer ODNs selected randomly from the TNFR1-coding sequence.

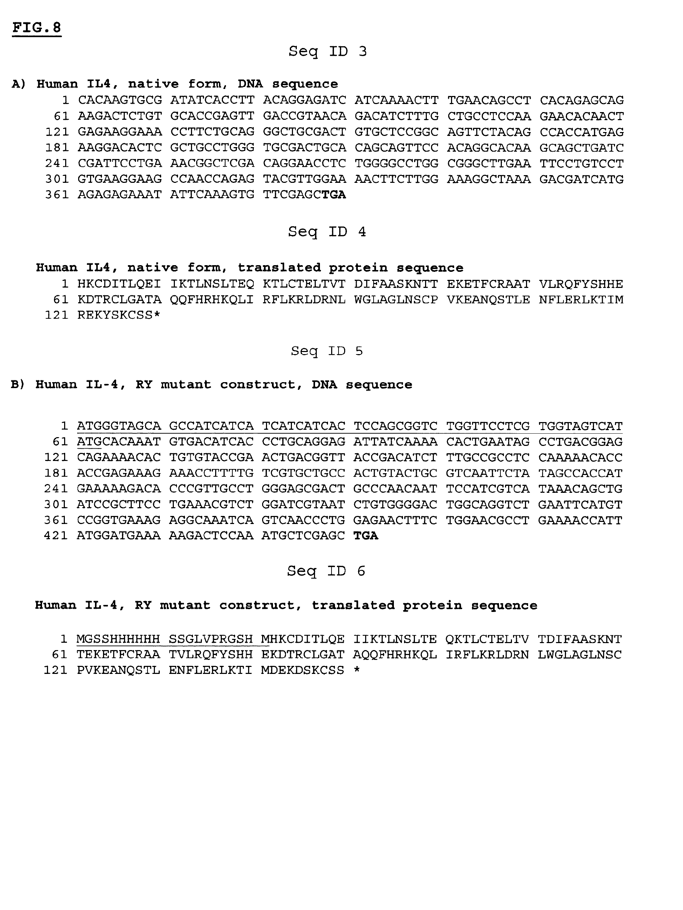

Figure 8 shows the amino acid sequence of human interleukin-4 (IL-4). A) The DNA sequence (without signal peptide sequence; 390 base pairs; Stop codon marked in bold, SEQ ID 3) and protein sequence (without signal peptide; 129 amino acids; Asterix is marking stop codon position, SEQ ID 4) of the native form. B) The DNA sequence (codon optimized for E. coli expression; 453 base pairs; N-terminal 6xHis- and protease cleavage-Tag; Stop codon marked in bold, SEQ ID 5) and protein sequence (150 amino acids, approx. 17.2 kDa; N-terminal tag underlined; Asterix is marking stop codon position, SEQ ID 6) of the human IL-4 RY mutant. The introduced mutations are R142D and Y145D.

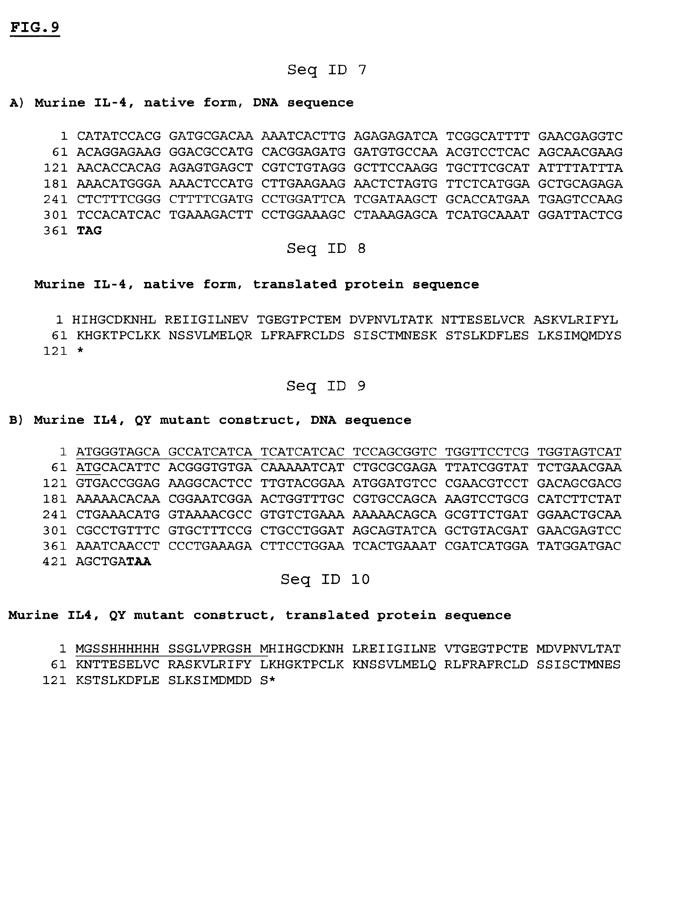

Figure 9 shows the amino acid sequence of murine interleukin-4 (IL-4).
   A) The DNA sequence (without signal peptide sequence; 363 base pairs; Stop codon marked in bold, SEQ ID 7) and protein sequence (without signal peptide; 120 amino acids; Asterix is marking stop codon position, SEQ ID 8) of the native form.
   B) The DNA sequence (codon optimized for *E. coli* expression; 429 base pairs; N-terminal 6xHis- and protease cleavage-Tag; Stop codon marked in bold, SEQ ID 9) and protein sequence (141 amino acids, approx. 15.8 kDa; N-terminal tag underlined; Asterix is marking stop codon position, SEQ ID 10) of the murine IL-4 QY mutant. The introduced mutations are Q137D and Y140D.

### DETAILED DESCRIPTION OF THE INVENTION

The functions of Tregs in allergic and autoimmune diseases indicate that control of the development, survival, and function of Tregs is instrumental for effective control of immune responses. The present invention provides methods for restoring lasting immunological tolerance by allergen- or antigen-specific immunotherapy in combination with individual combinations of Treg-modulating therapeutics for the treatment of allergy, allergic asthma, type I diabetes, rheumatoid arthritis, and multiple sclerosis.

### I. REGULATORY T CELL-MODULATING THERAPEUTICS

In one embodiment of the present invention, low molecular weight therapeutics are disclosed which a) are capable of addressing the complex network of Treg induction at different target sites, b) can be combined according to the individual requirements of allergic and autoimmune diseases, and c) provide all the characteristics required for efficient support of allergen- or antigen-specific immunotherapy including high solubility in aqueous environments for fast diffusion into the immunological synapse and fast elimination from the circulation upon diffusion away from the site of allergen or antigen presentation.

### 1.1. LOW MOLECULAR WEIGHT COMPLEMENT INHIBITORS

In one embodiment, low molecular weight inhibitors of the complement system are disclosed which are suitable for the method the present invention. Suitable low molecular weight inhibitors of the complement system include but are not limited to inhibitors targeting a) complement protein-protein interactions such as C1q- and C3-specific inhibitors, b) serine proteases such as inhibitors of C1s, factor B and C2, and c) anaphylatoxin receptors such as antagonists of C3aR and C5aR (for reviews, see Qu et al., 2009; Wagner and Frank, 2010). Suitable for the method of the present invention are low molecular weight structures derived from peptides (including peptidomimetics), nucleic acid-based aptamers, peptide nucleic acid-based aptamers as well as structures selected from compound libraries and optimized derivatives thereof (for a review, see Qu et al., 2009).

More preferred for the method of the present invention are low molecular weight inhibitors targeting complement component C3 and the anaphylatoxin receptors C3aR and C5aR. The complement system comprises three known anaphylatoxin receptors, C3aR, C5aR and C5L2, all of which belong to the family of G protein-coupled receptors. However, it is still a matter of discussion whether C5L2 is a decoy receptor or functional receptor.

### I.1.1. Inhibition of locally synthesized C3

In one preferred embodiment, C3-specific inhibitors are employed for the method of the present invention. Intervention at the central level of C3 is an attractive strategy because this approach can effectively modulate the production of all the critical complement mediators. More than half of the naturally occurring complement regulatory proteins including factor H, complement receptor 1 (CR1; CD35), complement receptor Ig (CRIg), decay accelerating factor (DAF; CD55) and membrane cofactor protein (MCP; CD46) inhibit at the C3 level.

Important for the method of the present invention is inhibition of locally synthesized complement component C3. The liver is the primary source for the synthesis of C3. However, many other specialized cells have the capacity to synthesize C3 including activated macrophages, dendritic cells and epithelial cells (Pratt et al., 2002; Verschoor et al., 2003; Caroll, 2004; Peng et al., 2006; Strainic et al., 2008; Raedler et al., 2009). These cells synthesize C3 spontaneously or in response to cytokine stimulation. Extrahepatic synthesis of C3 is thought to serve important physiological and pathological functions, including regulation of the inflammatory response, host defense, and allograft rejection.

Macrophages secrete all components of the complement system and antigens or inflammatory stimuli activate macrophages to produce substantial amounts of C3 (Morgan and Gasque, 1997). In particular, pro-inflammatory cytokines such as IL-6, tumor necrosis factor and interferon-γ stimulate macrophages to express the individual components. Macrophage-derived C3 has been shown to locally opsonize various particles and pathogens leading to their more efficient phagocytosis (Ezekowitz et al., 1984). Furthermore, locally produced macrophage-derived C3-fragments can modulate the functions of dendritic cells (DCs). For example, C3b bound to dendritic cell receptors has been demonstrated to increase the capacity of DCs in stimulating allogeneic T cells (Sandor et al., 2009).

Dendritic cells also synthesize and secrete C3 (Peng et al., 2006). However, in the absence of inflammation or 'danger' signals DCs appear to produce only a small amount of C3, which is in accordance with the limited ability of DCs to stimulate an immune response under these conditions. In contrast, in some pathological conditions DC synthesis of C3 may be up-regulated by microbial factors and by non-specific inflammatory stimuli. Based on the observation that DC synthesis of C3 is essential for full T cell activation (Peng et al., 2006), DC synthesis of C3 appears to be an important characteristic of DC maturation and up-regulation of DC synthesis of C3 during inflammation and infection could enhance the antigen-presenting capacity of DCs.

### Cyclic peptide-based C3 inhibitors

One preferred low molecular weight inhibitor targeting complement component C3 is the 13-residue cyclic peptide (H-I[CVVQDWGHHRC]T-NH₂) which is able to bind selectively to primate C3 and its C3b and C3c fragments, and to inhibit cleavage of C3 by C3 convertases of both the classical and the alternative pathway (Sahu et al., 1996; Ricklin and Lambris, 2008). The cyclic peptide, named compstatin, is more efficacious in inhibiting the alternative pathway than the classical pathway which is due to the fact that it binds to both C3 and C3b in the alternative pathway C3 convertase, whereas it cannot bind to the classical pathway C3 convertase that involves C4b.

Another preferred low molecular weight inhibitors targeting complement component C3 are derivatives of the 13-residue cyclic peptide (H-I[CVVQDWGHHRC]T-NH₂). As revealed by analysis of alanine substitution analogues, substitution of valine⁴, histidine⁹, histidine¹⁰, and arginine¹¹ result in minimal change in the activity (Morikis et al., 1998). Substitution of valine⁴ for tryptophan has been shown to establish stronger hydrophobic interactions between the peptide and C3c. Methylation of the tryptophan indole nitrogen further strengthened this hydrophobic interaction (Katragadda et al., 2006).

Compstatin and its derivatives have been shown to be safe and effective in a series of ex vivo and in vivo experiments in which the effect of complement inhibition under different conditions was studied including transplantation (Fiane et al., 1999; Tjernberg et al., 2008), bioincompatibility (Nilsson et al., 1998; Schmidt et al., 2003; Lappegard et al., 2005; Lappegard et al., 2008), and inflammation (Soulika et al., 2000: Mollnes et al., 2002). Recently, a compstatin derivative successfully completed a phase I clinical study under the name POT-4 (Potentia Pharmaceuticals, Inc.) for the treatment of age-related macular degeneration (Francois et al., 2009).

### I.1.2. Inhibition of C3a-receptor signaling.

In another preferred embodiment, C3aR-specific inhibitors are employed for the method of the present invention. Therapeutic intervention of C3a/C3aR interactions is attractive, since C3a has been linked to various pro-inflammatory processes including contraction of smooth muscle, chemotaxis, increases of vascular permeability, release of vasoactive amines, and activation of leukocytes such as macrophages, mast cells and eosinophils (for a review, see Gerad and Gerad, 2002). Furthermore, C3aR knock-out mice are characterized by decreased airway hyperresponsiveness (Drouin et al., 2002) and KO guinea pigs show decreased allergic responses (Bautsch et al., 2000). In humans, asthmatic patients have elevated levels of C3a in the broncho-alveolar fluid (Humbles et al., 2000) and in the plasma (Nakano et al., 2003). As demonstrated in a very recent study, signaling via C3a and C5a regulates effector T cell responses (Strainic et al., 2013).

Preferred low molecular weight C3aR antagonists include but are not limited to those with scaffolds featuring a) an arginine moiety (arginine derivatives) or b) an amino-piperidine linker and a pyridine moiety (amino-piperidine derivatives). Arginine derivatives include but are not limited to N²-[(2,2-dipheneylethoxy)acetyl]-L-arginine (SB290157; Ames et al., 2001), and those described by Denonne et al. (2007a). Amino-piperidine derivatives include but are not limited to those described by Denonne et al. (2007b).

In a preferred embodiment, the arginine derivative SB290157 is used for the method of the present invention. SB290157 has been shown to inhibit C3a-induced calcium mobilization in human neutrophils (IC₅₀ of 28 nM) and C3a-induced receptor internalization in human neutrophils. The in vivo therapeutic efficacy of SB290157 has been demonstrated in several disease models including lung inflammation in guinea pigs (Ames et al., 2001), arthritis in rats (Ames et al., 2001), intestinal ischaemia/reperfusion injury in rats (Proctor et al., 2004), lupus nephritis in mice (Bao et al., 2005a), and allergic asthma in mice (Baelder et al., 2005). SB290157 has a short in vivo half-life which is favorable for the method of the present invention.

In a more preferred embodiment, the arginine derivative 'compound 5' described by Denonne et al. (2007b) is used for the method of the present invention. Compound 5 is a modification of SB290157 (aryl substitution in the linker moiety) and provides a higher affinity due to its more rigid linker. The in vivo therapeutic efficacy of compound 5 has been demonstrated in a murine model of ovalbumin-triggered airway inflammation (Denonne et al., 2007b). The short in vivo half-life of compound 5 is comparable to that of SB290157.

### I.1.3. Inhibition of C5a-receptor signaling.

In another preferred embodiment, C5aR-specific inhibitors are employed for the method of the present invention. Therapeutic intervention of C5a/C5aR interactions can be beneficial for various pathological conditions including inflammatory, autoimmune, and neurodegenerative disorders (for a review, see Lee et al., 2008).

Suitable low molecular weight C5aR antagonists include but are not limited to a) peptidomimetics such as linear C089 (Konteatis et al., 1994), cyclic PMX53 (Finch et al., 1999), cyclic PMX205 (March et al., 2004), and linear JPE1375 (Schnatbaum et al., 2006), b) low molecular weight antagonists that are not based on peptides such as W-54011 (Sumichika et al., 2002), NDT9520492 (Waters et al., 2005), NGD2000-1 (Lee et al., 2008), CP-447,697 (Blagg et al., 2008), and NDT9513727 (Brodbeck et al., 2008), and c) nucleic acid-based aptamers such as those described by Biesicker et al. (1999).

In a preferred embodiment, peptidomimetic C5aR antagonists including C089, PMX53, PMX205 and JPE1357 are employed for the method of the present invention. PMX53, PMX205 and JPE1375 are analogs of the linear hexapeptide C089 (NMeFKPdChaWdR) which was derived from the C-terminus of C5a. Residues in position 1, 4 and 6 appear to be important for binding, while position 5 is responsible for the antagonist activity. The sequence P-dXaa is critical for the reverse turn structure of these peptidometic C5aR antagonists, which is further stabilized by a lactam ring in the cyclic analogs PMX53 and PMX205. Hydrophobic substitutions at the C-terminus led to JPE1375 which provides increased receptor selectivity as compared to PMX53.

The in vivo therapeutic efficacy of these peptidomimetic C5aR antagonists has been demonstrated in various disease models (for a review, see Qu et al, 2009). For example, the in vivo therapeutic efficacy of C089 has been demonstrated in disease models of allergic asthma in rats (Abe et al., 2001) and thrombotic glomerulonephritis in rats (Kondo et al., 2001). The in vivo therapeutic efficacy of PMX53 has been demonstrated in several disease models including sepsis in mice (Huber-Lang et al., 2002), renal ischaemia/reperfusion injury in rats (Arumugam et al., 2003), hepatic ischaemia/reperfusion injury in rats (Arumugam et al., 2004), intestinal ischaemia/reperfusion injury in rats (Proctor et al., 2004), ruptured abdominal aortic aneurysm in rats (Harkin et al., 2004), inflammatory bowel disease in rats (Woodruff et al., 2005), lupus nephritis in mice (Bao et al., 2005b), 3-nitropropionic acid-induced Huntington's disease in rats (Woodruff et al., 2006), abdominal pain in mice and rats (Ting et al., 2008), and tumor growth in mice (Markiewski et al., 2008). Furthermore, PMX53 has been shown to be safe and well tolerated in Phase 1 clinical studies for rheumatoid arthritis and psoriasis (Kohl, 2006).

### I.2. VITAMIN D3, DERIVATIVES THEREOF, AND VDR MODULATORS

In another embodiment, low molecular weight molecules capable of modulating the induction of Tregs via vitamin D-mediated pathways are disclosed which are suitable for the method the present invention. Suitable low molecular weight molecules include but are not limited to 25-OH-D3, its biologically active metabolite 1α,25-dihydroxyvitamin D3 (1,25-(OH)₂D3), analogues of vitamin D3 and non-secosteroidal vitamin D receptor (VDR) modulators (for reviews, see Plum and DeLuca, 2010; Fletcher et al., 2012).

### I.2.1. 25-OH-D3 and 1,25-(OH)₂D3

In one specific embodiment, the active vitamin D3 metabolite 1,25-(OH)₂D3 (calcitriol) is used for the method of the present invention. Calcitriol has been shown to directly promote the development of regulatory T cells (van der Aar et al., 2011). 1,25-(OH)₂D3 promotes tolerogenic epidermal Langerhans cells (LCs) and dermal dendritic cells (DDCs), both of which are able to generate Treg cells with different effector functions. Treatment of epidermal LCs with 1,25-(OH)₂D3 generates functional Foxp3+ Tregs through a mechanism that is dependent on keratinocyte-derived TGF-β. In contrast, treatment of DDCs with 1,25-(OH)₂D3 generates functional IL-10+FoxP3⁻ T_{R}1 cells in an IL-10-dependent fashion (van der Aar et al., 2011).

In another specific embodiment, the relatively biologically inactive 25-OH-D3 (calcidiol) is used for the method of the present invention. Calcidiol has similar effects on the immune system, since various immune cells including macrophages, Dcs, and even B and T cells are cable of converting inactive 25-OH-D3 to active 1,25-(OH)₂D3.

Current indications for clinical treatment with 25-OH-D3 or 1,25-(OH)₂D3 include renal osteodystrophy and osteoporosis. Systemic application of these molecules, however, is obstructed by toxicity issues, since the supraphysiological doses needed to modulate immune responses elicit hypercalcemia which can cause mineralization of the kidneys, heart, aorta and other blood vessels as well as cutaneous tissues. Toxicity occurs when serum levels of 25-OH-D3 rise to 500 ng/ml or above (Shephard and DeLuca, 1980). Based on experiments in the rat, serum levels of 25-OH-D3 should be kept below 250 ng/ml to avoid toxicity (Shephard and DeLuca, 1980). However, doses needed for achieving the immune effects exceed this level. One solution could be topical application of 1,25-(OH)₂D3, but even this administration has been demonstrated to lead to hypercalcemia and bone decalcification (Mortensen et al., 1996). It should be noted, however, that topical applications include relatively large surface areas of the skin, which implies administration of substantial amounts of 1,25-(OH)₂D3. In contrast, local administration of 1,25-(OH)₂D3 according to the present invention allows application of lower doses, since according to the disclosed method 1,25-(OH)₂D3 is embedded in a hydrogel together with the allergen or antigen. Thereby, 1,25-(OH)₂D3 is in close proximity during presentation of the peptides by APCs to T cells and does not have to reach its targets via transdermal diffusion.

### I.2.2. Vitamin D3 analogues

In another specific embodiment, vitamin D analogues with modifications in the side chain are used for the method of the present invention. On one hand, such modifications may affect their binding to the vitamin D binding protein (DBP) by reducing their affinity. Reduced binding to DBP has the effect of reducing longevity of the analogue in the circulation, which appears to reduce calcemic side effects. On the other hand, modifications in the side chain may also modify the rate of metabolism. The 24-hydroxylase catalyses cleavage and hydroxylation of the side chain, thereby producing metabolites with reduced VDR (vitamin D receptor) activity, and side chain modification can alter their susceptibility to 24-hydroxylation (for reviews, see Plum and DeLuca, 2010; Fletcher et al., 2012).

Suitable vitamin D analogues for the method of the present invention include but are not limited to those reviewed by Plum and DeLuca (2010) such as alfacalcidol, paricalcitol, oxacalcitriol, doxercalciferol, falecalcitriol, calcipotriol, and tacalcitol, as well as those reviewed by Fletcher et al. (2012).

In a preferred specific embodiment, calcipotriol is used for the method of the present invention. As compared to 1,25-(OH)₂D3, calcipotriol has 100-200 times less effect on calcium metabolism including activation of calcium absorption and bone calcium mobilization (Kissmeyer and Binderup, 1991). An important advantage for the method of the present invention is the short half-life of calcipotriol in circulation which is measured in minutes (Kragballe, 1995). After i.v. injection of 10 µg/kg calcipotriol in rats, calcipotriol was detectable in serum by HPLC analysis only up to 5 min, and after i.v. injection of 50 µg/kg up to 10 min (Kissmeyer and Binderup, 1991). The rate of clearance (half-life of 4 min) was approximately 140 times higher for calcipotriol than for 1,25-(OH)₂D3. Furthermore, calcipotriol is rapidly metabolized and effects of the metabolites have been demonstrated to be 100 times weaker than those of the parent compound (Kissmeyer and Binderup, 1991). Due to these characteristics, calcipotriol has been used clinically for more than 10 years for topical treatment of psoriasis without systemic toxicity (for a review, see Plum and DeLuca, 2010).

Most important, calcipotriol has been demonstrated to promote antigen-specific tolerance. In a recent study in mice, topical pretreatment with calcipotriol for 1 day followed by transcutaneous immunization within the treated skin area (stratum corneum was removed by tape stripping) with ovalbumin in the presence of a CpG oligonucleotide adjuvant (TLR9 agonist), priming of antigen-specific CD8+ T cells was abolished and CD4+CD25+ Tregs were induced (Ghoreishi et al., 2009). The suppressive effect of calcipotriol was found to be a local effect, as application of antigen to an area untreated with calcipotriol did not result in suppression.

### I.2.3. Non-secosteroidal vitamin D receptor (VDR) modulators

In another specific embodiment, non-secosteroidal VDR modulators are used for the method of the present invention. VDR modulators are currently used as therapeutic agents for the treatment of psoriasis, osteoporosis and secondary hyperparathyroidism and have the potential for the treatment of autoimmune diseases (for a review, see Fletcher et al., 2012). However, like vitamin D3 analogues, VDR modulators can also cause hypercalcemia. Useful VDR modulator for the method of the present invention exert reduced hypercalcemia-inducing activity including but not limited to those described by Bunel et al. (2009) and Lu et al. (2011).

### I.3. LOW TO MODERATE MOLECULAR WEIGHT INHIBITORS OF TNFR1-MEDIATED EFFECTS

In one embodiment of the present invention, low molecular weight inhibitors of TNFR1-mediated functions are disclosed which are suitable for the method the present invention, including but are not limited to N-acetyl-L-cysteine (NAC), glutathione (GSH) and derivatives thereof, salicylates, TNFR1-specific aptamers, and TNFR1-specific antisense oligo-nucleotides.

In another embodiment of the present invention, moderate molecular weight inhibitors of TNFR1-mediated functions are disclosed which are suitable for the method the present invention, including but are not limited to TNFR1-selective antagonistic TNF mutants and dominant-negative sTNF mutants, which leave the mTNF-TNFR1 and mTNF-TNFR2 interactions intact (for a review, see Van Hauwermeiren et al., 2011).

### I.3.1. N-acetylcysteine, glutathione and derivatives thereof

In one specific embodiment, glutathione (GSH), derivatives of GSH such as S-methylglutathione (GSM), precursors of glutathione such as N-acetyl-L-cysteine (NAC, ACC), alpha-lipoic acid, S-adenosylmethionine (SAMe), lipoate and its analogue lipoamide, L-2-oxothiazalidine-4-carboxylate (OTC), reduced cysteine and oxidized cystine disulfide, or combinations of these agents are used as inhibitors of TNFR1-mediated effects for the method of the present invention.

In a preferred specific embodiment, NAC is used for the method of the present invention. In vivo, NAC is readily deacetylated to form cysteine which efficiently supports intracellular glutathione (GSH) biosynthesis. In vitro, NAC and GSH have been shown to act on T cells by increasing interleukin-2 (IL-2) production, synthesis and turnover of IL-2 receptors (Liang et al., 1989), proliferation (Eylar et al., 1993), cytotoxic properties (Liang et al., 1991), and resistance to oxidative stress-mediated apoptosis (Sandstrom et al., 1994). Subsequent analyses revealed also a decrease of human IL-4 production by stimulated peripheral blood T cells and T helper cells after treatment with NAC and GSH in a dose-dependent manner, associated with a decrease of IL-4-induced IgE and IgG4 production by human peripheral blood mononuclear cells (Jeannin et al., 1995). In mice, oral administration of NAC (water containing 0.5-2.0 g/l NAC) for 8 days decreased both IgE and IgG1 antibody responses to intraperitoneally injected ovalbumin (1 µg) adsorbed onto aluminium hydroxide (Jeannin et al., 1995).

Recent studies have reported that glutathione deficiency might be causally linked to exacerbated TNF-alpha activation in several chronic diseases including neurodegeneration (Andersen, 2004), rheumatoid arthritis (Feldmann and Maini, 2003), and chronic lung diseases (Tirouvanziam et al., 2006). The well being of the patients affected with those diseases has been improved by treatment with the glutathione precursor NAC (e.g., Demedts et al., 2005).

NAC has also been demonstrated to prevent the deleterious effects of TNF-alpha on calcium transients and contraction in rat cardiac myocytes (Cailleret et al., 2004), and to hinder the progression of cardiac injury in hypertensive rats (Bourraindeloup et al., 2004). TNF-alpha is not expressed in the normal heart, but is up-regulated in the failing heart and the cardiac glutathione status determines the severity of the adverse effects of TNF-alpha on heart contraction (Cailleret et al., 2004). Interaction of sTNF-alpha with TNFR1 mediates several processes including oxidative stress and activation of the Mg-dependent neutral sphingomyelinase (N-SMase) which hydrolyzes sphingomyelin into ceramide, thereby controlling the sphingolipid signaling cascade and mediating TNF-alpha apoptotic and negative inotropic effects in cardiomyocytes. Downstream ceramide generation, the N-SMase pathway comprises down-regulation of the pro-survival factor Bcl-2, resulting in a shift of the pro-apoptotic Bax/pro-survial Bcl-2 protein ratio which in turn elicits activation of caspase-3. Sphingosine, produced by downstream ceramide production after activation of N-SMase by arachidonic acid (Amadou et al., 2002), mediates the immediate negative inotropic effects of TNF-alpha in mammalian cardiac myocytes (Oral et al., 1997). Both TNFR1-mediated pathways, oxidative stress and N-SMase activation, are inhibited by glutathione which serves as natural inhibitor of N-SMase (Liu and Hannun, 1997) and as scavenger of reactive oxygen species (ROS). In addition, high cellular glutathione concentrations inhibit also nuclear factor kappa B activation (Staal et al., 1990), thereby down-regulating TNF-alpha expression (Von Haehling et al., 2004). NAC has also been reported to selectively block TNF-alpha-induced signaling by lowering the affinity of TNFR1 to TNF-alpha (Hayakawa et al., 2003). However, in cardiac myocytes isolated from NAC-treated rats, TNF-alpha enhanced in the absence of NAC calcium transients and cell fractional shortening via TNFR2-dependent activation of several proteins including ERK, MSK1, cPLA2, and CaMKII (Defer et al., 2007). Consequently, direct inhibition of TNF-alpha binding to TNFR1 by NAC or direct antioxidant effect of NAC can be ruled out.

In vitro and in vivo experiments have demonstrated that glutathione and its precursor NAC selectively neutralize TNFR1-mediated effects of TNF-alpha while releasing TNFR2 pathways. For example, glutathione repletion by oral NAC treatment of post-myocardial infarction rats has been shown to induce cardiac function and tissue recovery (Adamy et al., 2007). TNFR2-dependent activation of the cytosolic Phospholipase A2 has been identified as mechanism for the positive effect of TNF-alpha on the cardiomyocytes (Defer et al., 2007). With regard to allergen-specific immunotherapy or vaccination with self-antigens, glutathione and its precursor NAC provide important signals for the induction of tolerance via activation and proliferation of Tregs. As shown by Liang et al. (1989), NAC and GSH act on T cells by increasing interleukin-2 (IL-2) production, synthesis and turnover of IL-2 receptors. Up-regulation of IL-2 and its receptor is important for the induction of tolerance since in the presence of IL-2 TNF-alpha-mediated activation of Tregs via interaction with TNFR2 results in proliferation, up-regulation of FoxP3 expression and increase of their suppressive activity. Furthermore, the decreased production of human IL-4 in stimulated peripheral blood T cells and T helper cells after treatment with NAC and GSH (Jeannin et al., 1995) is helpful since IL-4 plays a key role in the development of allergic inflammation.

Analyis of the pharmacokinetics, pharmacodynamics and toxicity of NAC in a Phase I clinical study (Pendyala and Creaven, 1995) revealed that in most of the subjects the highest non-toxic dose was 800 mg/m²/day. NAC was readily absorbed (median tₘₐₓ: 1.0 h) and the plasma decay proved to be monoexponential (t_{1/2}: 2.4 h). Pharmacokinetics proved to be linear with dose.

In a more preferred specific embodiment, S-methylglutathione (GSM) is used for the method of the present invention. Although NAC is capable of selectively blocking TNFR1-mediated effets, effective inhibition requires an extended pretreatment period with NAC. For example, experiments with post-myocardial infarction rats have demonstrated that oral NAC treatment for an extended period of time is required to benefit from this therapy. After 3-day NAC treatment (120 mg/kg/day) of 2-month post-myocardial infarction rats, the GSH content of the left ventricle was partly replenished to 75% of its control level in sham rats, N-SMase activation was blunted, the Bcl-2 content replenished and caspase-3 inhibited, but changes of the cardiac phenotype required 1-month NAC treatment (Adamy et al., 2007). S-Methylglutathione (GSM) provides an attractive alternative to NAC, since a short incubation of isolated cardiomyocytes with GSM under in vitro conditions has been demonstrated to reproduce the effects of in vivo NAC treatment (Cailleret et al., 2004). Glutathione repletion and unmasking of TNFR2-mediated effects by TNF-alpha in the isolated cardiomyocytes was achieved by incubation for 2-3 hours in the presence of 50 µmol/L GSM. In contrast, incubation of the isolated cardiomyocytes under identical conditions with 50 µmol/L NAC or 10 mmol/L GSH could not reproduce the effects of in vivo NAC treatment. It should be noted that the concentration of GSH was 200-fold higher than that of GSM since GSH is not effectively transported into cells (for a review, see Sen, 1997).

### I.3.2. Salicylates

In another specific embodiment, salicylates including but not limited to aspirin (acetylsalicylic acid; ASA) and salicylic acid (SA) are used as inhibitors of TNFR1-mediated effects for the methods of the present invention.

In a preferred specific embodiment, ASA is used as inhibitors of TNFR1-mediated effects for the methods of the present invention. ASA is a widely used non-steroidal anti-inflammatory drug (NSAID). The anti-inflammatory actions are mediated by inhibition of the inducible COX (cyclo-oxygenase) isoform COX-2, whereas the detrimental effects on gastric mucosa viability and platelet function are due mostly to inhibition of COX-1. However, several studies have demonstrated that ASA and related salicylates have a spectrum of biochemical and pharmacological effects that are not related to COX inhibition. Lower concentrations of ASA and AS can enhance (by means of inhibition of monocyte prostaglandin generation) the expression of some Th1 cytokines and, thereby, counteract Th2 responses (e.g., Tsuboi et al., 1995). High doses of ASA and SA, however, have been shown to interfere with the activation of critical transcription factors such as NF-kappa B (Koop and Ghosh, 1994) and activator protein 1 (AP-1; Dong et al., 1997). NF-kappa B is found in the cytoplasm of cells in an inactive form in association with the inhibitor I-kappa B-alpha. Stimulation triggers the release of NF-kappa B from I-kappa B, resulting in the translocation of NF-kappa B from the cytoplasm to the nucleus where NF-kappa B binds to DNA and regulates transcription of specific genes. Most of the genes known to be activated by NF-kappa B are involved in the immune and inflammatory response. These include cytokines such as IL-1, IL-6, IL-8, interferon-beta, and TNF-alpha, and the cell adhesion molecules endothelial leukocyte adhesion molecule-1 (ELAM-1), intercellular adhesion molecule-1 (ICAM-1), and vascular cell adhesion molecule-1 (VCAM-1). The inhibitory effect of salicylates is caused by activation of the p38 mitogen-activated kinase which leads to inhibition of TNF-alpha-induced I-kappa B-alpha phosphorylation and degradation. As a result of NF-kappa B inhibition, high doses of salicylates can interfere with Th1 cell differentiation and effector responses (e.g., Mazzeo et al., 1998).

An important finding was the observation that TNFR1-mediated activation of NF-kappa B is inhibited by high concentrations of sodium salicylate, but not TNFR2-mediated activation of NF-kappa B (Thommesen and Laegreid, 2005). Apparently, TNFR2 signaling involved in NF-kappa B activation proceeds independently of salicylate-sensitive signaling components, indicating distinct signaling pathways not shared with TNFR1. Inhibition of TNFR1-mediated activation of NF-kappa B requires high doses of salicylate (10-20 mM), but the inhibitory effect is highly reproducible (Thommesen and Laegreid, 2005).

Another major finding was the discovery that ASA and AS reduce IL-4 secretion and RNA expression in human CD4+ T cells (Cianferoni et al., 2001). IL-4 inhibition is important for the induction of Tregs since GATA3 induced by IL-4 inhibits the expression of FOXP3, which is a requirement for inducible Treg differentiation (Mantel *et al*., 2007). The inhibitory effect on IL-4 expression occurs at the transcriptional level and is due to interference with the binding of a calcium-inducible factor to a proximal IL-4 promoter element upstream of, but not overlapping, the NF-kappa B-binding P1 element. IL-4 gene inhibition by ASA is not associated with reduced NF-kappa B activation (Cianferoni et al., 2001). As important is the observation that salicylate-mediated inhibition of IL-4 expression does not affect IL-2 expression (Cianferoni et al., 2001), an essential cytokine for up-regulating TNFR2 expression on Tregs (Chen and Oppenheim, 2010). Since TNF-alpha has been shown to selectively up-regulate the expression of the IL-2 receptor alpha-chain (CD25) on Tregs (Chen et al., 2007), both TNF-alpha and IL-2 generate a powerful mechanism for receptor amplification and synergistically up-regulate Treg suppressive activity.

The various inhibitory effects of ASA and related salicylates are concentration-dependent. A 50% inhibition by ASA requires a concentration of approx. 2 x 10⁻⁶ M for COX-1, appprox. 3 x 10⁻⁴ M for COX-2, approx. 1 x 10⁻³ M for IL-4 gene transcription, and approx. 3 x 10⁻³ M for NF-kappa B translocation (Cianferoni et al., 2001). Selective inhibition of TNFR1-mediated activation of NF-kappa B requires even higher doses of salicylates in the range of 1-2 x 10⁻² M (Thommesen and Laegreid, 2005). However, the therapeutic range for ASA and SA has been restricted to 0.8-1.7 10⁻³ M, since salicylate-related toxicity (e.g., tinnitus) can occur with salicylate plasma levels as low as 1.2 x 10⁻³ M (Furst et al., 1987). Therefore, exploitation of COX-independent effects of salicylates for the induction of regulatory T cells requires novel application techniques that allow for high local concentrations of ASA and related salicylates well above the therapeutic range of systemically administered salicylates without the risk of increased side effects.

The present invention solves this problem by disclosing suitable matrices for sustained local delivery of ASA and related salicylates. Thereby, selective inhibition of TNFR1-mediated activation of NF-kappa B and effective inhibition of IL-4 gene transcription can be achieved at the site of antigen or allergen presentation, while upon diffusion away from the delivery site the salicylate concentration is rapidly decreased to systemically tolerable levels.

In another preferred specific embodiment, sodium salicylate, salicylamide or choline magnesium trisalicylate are used as inhibitors of TNFR1-mediated effects according to the methods of the present invention for patients suffering from aspirin-induced asthma (also known as aspirin-triad or aspirin-intolerant asthma, AIA). In these patients application of ASA for selective inhibition of TNFR1-mediated activation of NF-kappa B and inhibition of IL-4 gene transcription is contra-indicated. AIA refers to the development of acute bronchoconstriction, profuse rhinorrhea and skin flushing in asthmatic individuals following the ingestion of aspirin. AIA is likely to be mediated by a deviation of the arachidonic acid metabolic pathway toward excessive leukotriene production. Both cyclo-oxygenase and lipoxygenase are involved in the arachidonic acid metabolic pathway. The prevalence of AIA is 4.3% in Poland, 8.8% in Finland and 10.5% in Australia (for a review, see Gohil et al., 2010). AIA appears after puberty and is present in 10-20% of adult asthma. Approximately 50% of the patients with AIA have chronic, severe, corticosteroid-dependent asthma, approximately 30% have moderate asthma that can be controlled with inhaled steroids, and approximately 20% have mild to intermittent asthma. Atopy is present in on third of the AIA patients (for a review, see Gohil et al., 2010). Challenge tests with aspirin are the cornerstones of AIA diagnosis. The nasal challenge is quick and free of life-threatening systemic or bronchial side effects.

While ASA is contra-indicated for patients with ASA, these patients can safely take sodium salicylate, salicylamide and choline magnesium trisalisylate, since these drugs are weak inhibitors of COX.

### I.3.3. TNFR1-specific antisense oligonucleotides

In another specific embodiment antisense oligonucleotides with specificity for TNFR1, are used as inhibitors of TNFR1-mediated effects for the methods of the present invention.

TNFR1-specific antisense oligonucleotides have been applied to prevent apoptotic signaling through TNFR1 after ionizing radiation treatment (Huang et al., 2006). Pretreatment of mice with 2'-O-(2-methoxy)ethyl-modified antisense oligonucleotides (25 mg/kg i.p. every other day for a total of four times) protected the mice successfully against radiation-induced liver damage.

However, a major limitation to the use of TNFR1-specific antisense oligonucleotides is the limited cellular uptake of oligonucleotides. In order to overcome this problem cationic lipids have been used as one of the major components in making non-viral delivery systems such as liposomes (Ross et al., 1998). Since cationic lipids have positive charges, they can be complexed with negatively charged oligonucleotides and then formulated into cationic liposomes. Such liposomes have been demonstrated to enhance the transfection efficiency of plasmid DNA significantly (Helbling-Leclerc et al., 1999).

Another major limitation to the use of TNFR1-specific antisense oligonucleotides is the necessity for efficient local inhibition of TNFR1-mediated effects without the risk of severe systemic side effects. This problem is solved in the present invention by disclosing suitable matrices for sustained local delivery of cationic liposome-embedded TNFR1-specific antisense oligonucleotides at the site of antigen or allergen presentation. Suitable matrices are listed in section II. Different liposome-hydrogel formulations for topical application of liposomes are described in the literature (e.g., Nie et al., 2011). The present invention discloses liposome-doped hydrogels for sustained local delivery of cationic liposome-embedded TNFR1-specific antisense oligonucleotides. In a preferred embodiment, thermosensitive hydrogels doped with cationic liposome-embedded TNFR1-specific antisense oligonucleotides are used.

### I.3.4. Antagonistic TNF mutants with specificity for TNFR1

In another preferred embodiment, TNFR1-selective antagonistic TNF mutants are used for the method of the present invention. Useful TNF mutants include but are not limited to R1antTNF developed by Shibata et al. (2008).

For systemic applications the TNFR1-selective antagonistic TNF mutant R1antTNF is not suitable due to its very short half-life (approx. 10 min) in plasma (Shibata et al., 2009), but for the method of the present invention TNFR1-selective antagonistic TNF mutants with a short plasma half-life are advantageous. Utilizing matrices for sustained local delivery of such mutants, high local concentrations of the TNF mutants can be achieved at the site of antigen or allergen presentation, whereas upon diffusion away from the delivery site the TNF mutant concentration is rapidly decreased to therapeutically ineffective levels. Thereby, potential systemic side effects are minimized. If necessary, however, the plasma half-life of TNFR1-selective antagonistic TNF mutants can be extended by site-specific PEGylation as described for the TNF mutant R1antTNF (Shibata et al., 2009).

### I.3.5. TNFR1-neutralizing aptamers

In another specific embodiment, TNFR1-neutralizing aptamers are used for the method of the present invention. Aptamers are nucleic acid binding species generated by iterative rounds of in vitro selection (for reviews, see Famulok et al., 1999; Yan et al., 2005). Typically, selected aptamers, bind very tightly (often in the low nanomolar range) and specifically to their targets. Aptamers can also discriminate between closely related protein targets. Aptamers have several key features that make them particularly well suited as reagents for the method of the present invention. First, aptamers are relatively small and can readily access sites which are difficult to target with large molecules such as the immunological synapse that is formed between the antigen presenting cells and the T cells. Another advantage for the present invention is the possibility to engineer the stability of aptamers towards degrading nucleases, thereby determining the period of their inhibitory activity. Unmodified RNA- and DNA-based aptamers can begin degrading in serum within minutes, whereas aptamers containing modified bases and phosphorothioate linkages display a significantly increased stability towards degrading nucleases. For example, 2'-aminopyrimidine-modified RNA has been shown to remain stable for days. Furthermore, modifications can also be applied to the ends of aptamers to confer greater stability. For example, a 3'-3' linkage can be added to prevent 3'-exonuclease degradation. Stable aptamers can be generated by the Spiegelmer-technology (for a review, see Famulok et al., 1999) or by utilizing peptide nucleic acids (EP 1 785 490 B1). Peptide nucleic acids (PNA) represents a class of nucleic acid analogues where the charged deoxyribose-phosphodiester backbone has been replaced by a peptide strand comprising N-(2-aminoethyl)glycine monomers with the nucleobases adenine, thymine, guanine and cytosine attached to their alpha-carbon. Due to its artificial character PNA provides complete resistance against nucleases and proteases.

In another specific embodiment of the invention, aptamers are used which provide limited resistance against enzymatic degradation that is sufficient for effective inhibition of TNFR1 at the site of allergen presentation and in its close proximity, but guarantees rapid degradation of the aptamer while diffusing away from this site. In a specific embodiment, the stability of an inhibitory aptamer is adjusted according to the requirement of the method of the present invention by modification of its structure including but not limited to the introduction of phosphorothioate linkages, modification of bases (e.g., by modification with 2'-aminopyrimidine), and addition of a 3'-3' linkage to the end of the aptamer.

In another specific embodiment of the invention, the physiological clearance of an inhibitory aptamer is adjusted according to the requirement of the method of the present invention. In cases of an extremely fast clearance, the plasma life-time of an inhibitory aptamer can be extended by attachment of lipids or high molecular weight compounds such as 40 kD polyethylene glycol moieties.

### I.3.6. Dominant-negative sTNF mutants

In another specific embodiment, variant TNF proteins are used for the method of the present invention which rapidly form inactive heterotimers with native sTNF-alpha to give complexes that neither bind to nor stimulate signaling through TNF receptor. By sequestration of sTNF-alpha these variant TNF proteins selectively inhibit the pro-inflammatory action mediated by TNFR1. Useful variant TNF proteins with these characteristics, named dominant-negative TNF variants (DN-TNF), include but are not limited to those described by Steed et al. (2003). One of the most potent DN-TNF molecules, the TNF-alpha double mutant A145R/Y87H, has been demonstrated to efficiently protect mice from TNF-alpha-induced damage. Lethal doses of native TNF-alpha (30 µg/kg) mixed before injection with varying ratios of A145R/Y87H produced no TNF-induced damage. This protection was even observed at native:variant ratios as low as 1:1 and with a superlethal dosis of TNF-alpha (Steed et al., 2003). Furthermore, A145R/Y87H proved to be non-toxic. Dosed as high as 30 mg/kg of mouse body weight (along with 30 µg/kg native TNF-alpha) A145R/Y87H did not induce mortality or hepatotoxicity. In a murine model of collagen-induced arthritis, variant A145R/Y87H reduced joint swelling when dosed once daily at 2 mg/kg subcutaneously with an intravenous loading dose of 2 mg/kg on the first treatment day (Steed et al., 2003).

### I.4. LOW TO MODERATE MOLECULAR WEIGHT INHIBITORS OF IL-4/IL-13-MEDIATED EFFECTS

In one embodiment of the present invention, low molecular weight inhibitors of IL-4/IL-13-mediated effects are disclosed which are suitable for the method the present invention, including but are not limited to aptamers capable of inhibiting the binding of IL-4 or IL-13 to an IL-4 receptor or Il-13 receptor, respectively, and antisense oligo-nucleotides with specificity for the IL-4 receptor or Il-13 receptor.

In another embodiment of the present invention, moderate molecular weight inhibitors of IL-4/IL-13-mediated effects are disclosed which are suitable for the method the present invention, including but are not limited to interleukin variants capable of antagonizing the activity of IL-4 and/or IL-13 are used as inhibitors.

### I.4.1. IL-4/IL-13-neutralizing aptamers

In a specific embodiment of the invention, one or more antagonistic aptamers with specificity of the IL-4 receptor and/or Il-13 receptor are used to inhibit IL-4- and IL-13-mediated pathways. Generation of such aptamers as well as useful modifications of their features such as resistance against enzymatic degradation and adjustment of plasma life-time are performed as described in section I.3.5.

### I.4.2. Antagonistic interleukin variants

In a preferred embodiment of the invention, interleukin variants capable of antagonizing the activity of IL-4 and IL-13 are used as inhibitors. Any interleukin variant that functions as an IL-4, IL-13 or IL-4/IL-13 antagonist and is suitable for administration in accordance with the method of the present invention may be employed. Interleukin-based antagonists do not need to completely abolish IL-4- or IL-13-induced biological activity to be useful. Rather, a given antagonist may reduce a biological activity of IL-4 and/or IL-13.

Antagonistic IL-4 mutants have been described (Kruse et al., 1992; Muller et al., 1994; US patent 6,028,176; US patent 5,723,118). Within the four helix bundle of human IL-4, tyrosine (Y124) on the D helix forms an important contact with the common γ-chain (γ_{c}). When Y124 is mutated to aspartic acid (Y124D), the resulting molecule binds to IL-4Rα with the same affinity as the wild-type IL-4, but has only 0.5% of the agonistic activity (Letzelter et al., 1998). Similarly, the arginine at position 121 of human IL-4 interacts with IL-13Rα1. Mutations of R121 generates mutants that can bind to IL-4Rα, but are antagonists of both IL-4- and IL-13-induced responses (Kruse et al., 1993). However, other or additional mutations of IL-4 may also be considered for generating IL-4 variants suitable for the method of the present invention. For example, by mutation of serine at position 125 of IL-4 mutants have been generated that can bind to IL-4Rα, but are antagonists of both IL-4- and IL-13-induced responses (Kruse et al., 1993). Suitable for the method of the present invention are antagonistic interleukin-4 variants with single, double and triple mutations, single mutations including but not limited to amino acid positions R121, Y124, and S125; double and triple mutations including but not limited to combinations of the above listed amino acid positions. Preferred are antagonistic IL-4 variants with a relatively short half-life to limit adverse effects distant from the site of allergen presentation.

IL-13 mutants useful for the method of the present invention include but are not limited to the Glutamate at position 13 in IL-13 associates with IL-4Rα and a mutation to lysine decreases its binding to IL-4Rα. Although IL-13E13K prevents binding of IL-4Rα to IL-13Rα1, full inhibition of IL-4 responses cannot be achieved due to the availability of the alternative IL-4R complex, IL-4Rα:γ_{c}.

In a specific embodiment, the human IL-13 mutant E13K is used for the method of the present invention. IL-13 mutant E13K is a powerful antagonist of human IL-13 and capable of partially inhibiting the responses of human IL-4 as well (Kioi et al., 2004).

In a preferred embodiment, the human IL-4 double mutant R121D/Y124D is used for inhibition of IL-4- and IL-13-mediated pathways in accordance with the method of the present invention. The human IL-4 double mutant R121D/Y124D is able to antagonize the activity of IL-4 as well as that of IL-13 (Grunewald et al., 1998; Tony et al., 1994). The double mutant of human IL-4 (R121D/Y124D) has been shown to protect allergic cynomolgus monkeys from allergen-induced airways hyper-responsiveness when administered either subcutaneously or via nebulisation. Furthermore, the effect of the double mutant of IL-4 (R121D/Y124D) on late phase asthmatic response to allergen challenge in asthmatic patients has been evaluated recently in clinical studies (Wenzel et al., 2007). The studies have demonstrated that dual inhibition of IL-4 and IL-13 can positively affect the course of late asthmatic response after experimental allergen challenge.

### II. SUSTAINED LOCAL DELIVERY OF LOW MOLECULAR WEIGHT

### INHIBITORS

Effective local targeting of the complex network of Treg-inducing mechanisms at the site of allergen or antigen presentation requires a sustained local delivery of Treg-modulating therapeutics from a depot-mediating matrix for a period of time that is sufficient for the purpose of the particular medical treatment, at least for the period of allergen or antigen presentation.

In one embodiment, matrices are used for local delivery of suitable Treg-modulating therapeutics that a) can serve as depot for substantial quantities of one or more suitable Treg-modulating therapeutics, b) allow the release of sufficient quantities of the embedded Treg-modulating therapeutics over a prolonged period of time (optimally for a few days), and c) are chemically and physically compatible with the Treg-modulating therapeutics and other compounds necessary for modulation of effector T cell and regulator T cell responses according to the method of the present invention.

### II.1. Biodegradable polymers

In one specific embodiment of the invention, biodegradable polymers are used for controlled delivery of suitable Treg-modulating therapeutics. Preferred biodegradable polymers approved by FDA and used in a clinical trial, include but are not limited to poly(D,L-lactic acid), poly(lactic-co-glycolic acid) (PLGA), and copolymers of L-lactide and D,L-lactide. An important characteristic of such polymers is their ability to be applied locally. All FDA approved polymers have been studied extensively for their biocompatibility, toxicology, and degradation kinetics. Furthermore, these polymers have been shown to release embedded therapeutics for several hours up to 40 weeks in vitro and several weeks in vivo.

### II.2. Biodegradable thermo-gelling hydrogels

In a preferred specific embodiment, injectable in situ-forming gel systems which are biodegradable, are used for controlled delivery of suitable Treg-modulating therapeutics (for a review, see Ruel-Gariepy and Leroux, 2004). Preferred in situ-forming gel systems (hydrogels) undergo a sol-gel-sol transition, which is a free flowing sol at room temperature and a non-flowing gel at body temperature. Compared to other biodegradable polymers, the injectable thermo-gelling polymers possess several advantages including easy preparation, high encapsulation efficiency of bioactive molecules including therapeutic proteins, and free of harmful organic solvents in the formulation process (Qiao et al. 2005).

Useful for the method of the present invention are biodegradable thermogelling block polymers which are based on monomethoxy poly(ethylene glycol) (MPEG) including but not limited to a) diblock copolymers consisting of MPEG and poly(ε-caprolactone) (PCL) (Hyun et al., 2007), b) MPEG-b-(PCL-ran-PLLA) diblock copolymers (Kang *et al.,* 2010), and c) diblock copolymers consisting of MPEG and PLGA (Peng et al., 2010). MPEG copolymers containing PCL provide the advantage that they do not create an acidic environment upon biodegradation in contrast to MPEG copolymers containing PLLA and PLGA (Hyun et al., 2007).

Useful for the method of the present invention are also biodegradable thermogelling triblock polymers including but not limited to a) PLGA-PEG-PLGA (Qiao et al., 2005), b) PEG-PLGA-PEG (Zhang et al., 2006), and c) PEG-PCL-PEG (PECE) (Gong et al., 2009 a). Various biodegradable thermogelling triblock polymers made up of PLGA and PEG are disclosed in patent application WO 99/18142. At lower temperatures, hydrogen bonding between hydrophilic PEG segments of the copolymer chains and water molecules dominate in aqueous solutions, resulting in the dissolution of these copolymers in water. As the temperature increases, the hydrogen bonding becomes weaker, while hydrophobic forces of the hydrophobic segments such as PLGA segments are getting stronger, leading to sol-gel transition. PEG, PLGA and PCL are well-known FDA-approved biodegradable and biocompatible materials which have been widely used in the biomedical field.

Useful for the method of the present invention are also biodegradable thermo-gelling diblock and triblock copolymers which consist of polyethylene oxide (PEO) and a biodegradable polyester such as poly-L-lactic acid (PLLA) (Jeong et al., 1997). These block copolymers, however, are a free flowing sol at a higher temperature and form a gel at a lower temperature. For example, a 23% aqueous solution of PEO-PLLA-PEO (Mᵣ 5,000-2,040-5,000) is a sol at 45°C and becomes a gel at 37⁰C By changing the biodegradable block length, the sol-gel transition temperature can be manipulated, e.g., increasing the PLLA block length increases the aggregation tendency of a block copolymer in water, resulting in a steepening of the gel-sol transition curve slopes and the onset of gelation at lower concentrations. The sol-gel transition temperature is a function of concentration as well as composition of a block polymer.

### II.3. Non-biodegradable thermo-gelling hydrogels

In another specific embodiment, poloxamers (trade name Pluronics) are used. Poloxamers are nonionic triblock copolymers composed of a central hydrophobic chain of poly(propylene oxide) (PPO) flanked by two hydrophilic chains of poly (ethylene oxide) (PEO) (Gilbert et al., 1987). Poloxamers exhibit a sol-gel transition behavior in aqueous solutions and have been used for sustained delivery of several therapeutic agents. However, poloxamers are not biodegradable and can be accumulated in the body which may lead to toxic side effects. Thus, the application of poloxamers in biomedical fields has been greatly restricted. In a recent study, Pluronic F127 (100-unit PEO chain surrounding one 65-unit PPO) has been used to form composite thermo-sensitive hydrogels with PECE (Gong et al., 2009 b). Based on the results of this study Pluronic F127/PECE composite hydrogels are biocompatible with low cell cytotoxicity and, therefore, may also be suitable for the method of the present invention.

### II.4. Trimethylated chitosan-based hydrogels

In another specific embodiment, thermal-sensitive hydrogels formulated on the basis of trimethylated chitosan derivatives (Wu et al., 2012) are used for the sustained delivery of suitable active substances and one or more antigens. In addition to its application as a vaccine delivery system, the cationic polysaccharide has shown promising results as an adjuvant. For example, application of a chitosan solution for subcutaneous vaccination has been demonstrated to enhance both humoral and cell-mediated immune responses (Zaharoff et al., 2007). However, the unfavorable pH-dependent solubility and charge density of chitosan is a limiting factor. In contrast, trimethylated chitosan is well soluble in aqueous solution at neutral pH and provides excellent biocompatibility and mucoadhesive nature. Trimethylated chitosan derivatives are best characterized by the degree of quarternization, the degree of O-methylation and the degree of acetylation (Hagenaars et al., 2010).

Trimethylated chitosan derivatives are especially suited for nasal delivery of vaccines and are frequently formulated into particles or spray powder (Alhalaweh et al., 2009). Very recently, however, the trimethylated chitosan derivative N[(2-hydroxy-3-trimethylammonium) propyl] chitosan chloride has also been formulated together with α,β-glycerophosphate into a thermal-sensitive hydrogel (Wu et al., 2012). This hydrogel was shown a) to significantly prolong the antigen residence time in the nasal cavity, b) to enhance the transepithelial transport via the paracellular routes, and c) to induce in mice a high mucosal immunity (sIgA) and systemic immune response (IgG1 and IgG2a).

### II.5. Stability of thermo-gelling polymers

The various biodegradable thermo-gelling polymers provide different stability characteristics. For example, poloxamer triblock polymers provide excellent thermo-sensitivity, but due to weak hydrophobicity of the PPO block such copolymers form fast eroding gels which have been reported to persist in vivo a few hours at most. Exposure of poloxamer gels to phosphate-buffered saline under in vitro conditions demonstrated gel erosion within 2 days (Hyun et al., 2007). Similar results were observed when the polymer solutions were subcutaneously injected into rats. Poloxamer gels could not be observed after 2 days (Hyun et al., 2007). Different results were obtained with MPEG-PCL gels. Under in vitro conditions MPEG-PCL gels maintained their structural integrity for more than 28 days and after subcutaneous injection into rats MPEG-PCL gels maintained their structural integrity longer than 30 days. The stability of MPEG-PCL gels, however, may also create problems since the rate of degradation of PCL *in vivo* is rather slow (2-3 years) compared to that of PLA, PGA or PLGA (for a review, see Sinha et al., 2004). Thus, after serving the function in delivering suitable Treg-modulating therapeutics, MPEG-PCL copolymers may remain in the body under physiological conditions for an uncertain period. Therefore, most preferred biodegradable thermo-gelling polymers for the method of the present invention are those which maintain their structural integrity for a few days but do not remain in the body for more than a month.

In a preferred embodiment of the present invention, biodegradable thermo-gelling polymers are used which allow to modify their degradation kinetics. For example, PLLA segments can be incorporated into the PCL segment of MPEG-PCL copolymers, since PLLA provides better accessibility of water to the ester bonds of PLLA which enhances the hydrolytic degradation of the copolymer (Kang et al., 2010). The resulting MPEG-b-(PCL-ran-PLLA) diblock copolymers offer a therapeutic window that is adjustable from a few weeks to a few months by varying the amount of PLLA in the PCL segment (Kang et al., 2010). In another example, the rate of PLGA-PEG-PLGA hydrogel erosion can be modified by altering the molar ratio of DL-lactide/glycolide in the PLGA segment. The DL-lactide moiety is more hydrophobic than the glycolide moiety. Therefore, by increasing the molar ratio of DL-lactide/glycolide in the PLGA segment of PLGA-PEG-PLGA triblöck copolymers, more stable hydrogels are formed due to stronger hydrophobic interactions among the copolymer molecules (Qiao et al. 2005).

### II.6. Release characteristics of thermo-gelling polymers

Several of the biodegradable thermo-gelling polymers have been analyzed for their ability to mediate sustained release of proteins. Although different proteins are likely to affect the release behavior of each copolymer in individual ways, characterization of the release of model proteins such as bovine serum albumin (BSA) provides important information. Using composite hydrogels containing different percentages of PECE and Pluronic F127, the in vitro release behavior of BSA proved to be dependent on the hydrogel composition, initial BSA loading amount, and hydrogel concentration (Gong et al., 2009b). Sustained release of BSA above 15 days was achieved with a composite hydrogel containing 60% PECE and 40% Pluronic F127, loaded with 4 mg BSA in the presence of 30wt% hydrogel. Using MPEG-PCL copolymers, sustained in vitro release of BSA proved to be above 20 days, and under in vivo conditions (after subcutaneous injection into rats) sustained release lasted for more than 30 days (Hyun et al., 2007).

While for most clinical applications a sustained release of therapeutic drugs from biodegradable thermo-gelling polymers over a period of several weeks is desirable, the method of the present invention does not require such an extended sustained release of active substance since the development of immunologic memory requires the engagement of the T cell receptor (TCR) for a period of only 1 to 2 days. Therefore, preferred are biodegradable thermo-gelling polymers which deliver TNFR1 inhibitors for a limited period only which does not exceed a week.

PLGA-PEG-PLGA triblock copolymers represent one example of hydrogels providing advantageous degradation and release kinetics for the method of the present invention. As demonstrated in a recent study, the release of insulin from PLGA-PEG-PLGA triblock copolymers lasted for approximately 4 days with an initial burst effect during the first day (Choi et al, 2003). The initial burst effect may be advantageous for effective inhibition of TNFR1 in the initial phase. However, the release kinetics may be modified by reducing the solubility of Treg-modulating therapeutics via complexation with zink as shown for zink-insulin complexes (Choi et al, 2003).

Although Poloxamer gels are not biodegradable, Poloxamer 407 (trade name Pluronic F127) gels represent also an example of thermo-gelling polymers providing advantageous degradation and release kinetics for the method of the present invention. Although under in vitro conditions the release of BSA from Poloxamer 407 gels proved to be almost complete within 1 day, the sustained release of BSA under in vivo conditions (after subcutaneous injection into rats) lasted for 3 days (Hyun et al., 2007).

### II.7.Liposome-hydrogel formulations

For sustained local delivery of liposome-embedded TNFR1-specific antisense oligonucleotides at the site of antigen or allergen presentation, different liposome-hydrogel formulations are suitable (e.g., Nie et al., 2011). In a preferred embodiment, hydrogels doped with cationic liposome-embedded TNFR1-specific antisense oligonucleotides are used. In a more preferred embodiment, thermo-sensitive hydrogels doped with cationic liposome-embedded TNFR1-specific antisense oligonucleotides are used.

### II.8. Safety aspects of PEG/PLGA-based hydrogels

Preferred biodegradable polymers include but are not limited to poly(ethylene glycol) (PEG), poly(D,L-lactic acid), copolymers of L-lactic acid and D,L-lactic acid, poly(glycolic acid), and poly(lactic-co-glycolic acid) (PLGA). The use of these polymers as sustained-release protein delivery systems has been studied extensively (for a review, see Pai et al., 2009) and they represent the most compelling biodegradable polymers for depot systems due their inclusion in the FDA's General Recognized as Safe (GRAS) list for use in medical devices and drug formulations (FDA, http://vm.cfsan.fda.gov/%7Edms/eafus.html. Accessed various dates).

### III. ADJUVANTS

The time of allergen or antigen exposure is extremely important since T cell differentiation and thus the development of immunologic memory requires the engagement of the T cell receptor (TCR) over 12-48 h and long lasting memory may even require repetitive exposure. Adjuvants such as oils and alum provide a depot effect that extends the lifetime of allergens and antigens and thus prolong antigen-stimulation of T cells. However, in addition to the depot effect currently available adjuvants trigger either Th1-type or Th2-type immune responses or both (for reviews, see Leroux-Roels, 2010; Nicholls et al., 2010; Brunner et al., 2010).

Based on the assumption that the generation of Tregs represents a default pathway which requires T-cell receptor activation but the absence of decision signals for effector T cells, currently available adjuvants are not optimal for the purpose of the present invention. For the method of the present invention adjuvants are needed that promote allergen uptake and prolonged stimulation of T cells without providing decision signals, thereby inducing Treg expansion. Unfortunately, adjuvants providing all of the desired properties are not available.

Liposomes, synthetic nanospheres consisting of lipid layers, represent a classic adjuvant that is not active per se. Their main mode of action is through allergen presentation. Liposomes can encapsulate allergens and act as allergen delivery vehicles. However, liposomes are not suitable for a slow release of allergens, which appears to be essential for a prolonged immune stimulation.

### III.1. Adjuvants eliciting a Th2-type immune response

In one specific embodiment, aluminum-containing adjuvants, typically aluminum phosphate or aluminum hydroxide gels (generally referred to as alum) are used for the method of the present invention. One mechanism of action is due to its retention of immunogenic molecules at specific sites in the body, allowing for their slow release and consequently a prolonged immune response, the so-called depot effect. Alum also appears to exert its adjuvant activity by keeping antigens in a particulate (rather than soluble) form, thereby enhancing phagocytosis of antigens by APCs. Dendritic cells (DC) are capable of taking up both soluble antigens and antigens adsorbed onto aluminum-containing adjuvants. Furthermore, it has become evident that alum is capable of directly activating immune cells. Alum can induce maturation of monocytes/macrophages into DC-like cells. This process is dependent on the activation of NLRP3 (NOD-like receptor family, pyrin domain containing 3), part of the inflammasome which causes production of pro-inflammatory cytokines such as IL-1β and IL-18. Primarily, alum elicits a Th2 response including IL-4 and IL-5 production as well as IgG1 and IgE production by B cells (for a review, see Nicholls et al., 2010).

Upon incubation of allergens with aluminum-containing adjuvants, the antigens or allergens are partially or completely adsorbed onto the adjuvant via electrostatic interactions and exchange between phosphate and hydroxyl groups. Several studies have demonstrated that adsorption onto aluminum-containing adjuvants prevents rapid enzymatic degradation of antigens and allergens, but the adsorption process may also induce structural changes resulting in a decreased thermal stability (for a review, see Clapp et al., 2011). One approach to improving the thermal stability of vaccines is to use a combination of buffers to alter the surface chemistry of aluminum-containing adjuvants and to control the ionization state of the antigen's or allergen's amino acid side chains. Another approach is to identify conditions that stabilizes antigens and allergens in the solution state and to use this as guidance on minimizing the extent of adsorption-induced thermal destabilization of the allergen(s). For practical purposes, however, stabilization procedures may be omitted since the importance of antigen (including allergen) adsorption to aluminum-containing adjuvants for the immunological response has been questioned by recent studies (for a review, see Clapp et al., 2011). For example, the immune response elicited in rabbits against hen egg white lysozyme was irrespective of the fraction of antigen adsorbed onto the aluminum-containing adjuvant (Chang et al., 2001). For maximizing the immune response, however, some degree of adsorption or at least the proximity of the allergen and the adjuvant appears to be important.

In another specific embodiment, polyphosphazenes (watersoluble polymers) are used for the method of the present invention. Poly [di(carboxylatophenoxy) phosphazene] (PCPP) has been shown to enhance antibody responses while being negligibly reactogenic (for a review, see Nicholls et al., 2010).

In still another specific embodiment, montanides (squalene-based water-in-oil emulsions) are used for the method of the present invention. Montanide emulsions are similar to incomplete Freund's adjuvant, but they are biodegradable and therefore significantly less toxic and have been used in several clinical trials (for a review, see Nicholls et al., 2010).

In a preferred embodiment of the present invention, local administration of Treg-modulating therapeutics in support of antigen- or allergen-specific immunotherapy includes local inhibition of IL-4/IL-13. Coincident blockade of IL-4/IL-13-mediated pathways provides an important benefit for the use of adjuvants eliciting Th2-type immune responses including but not limited to aluminum salts, Montanide emulsions (squalene-based water-in-oil emulsions) and polyphosphazenes. Although adjuvants eliciting primarily a Th2-type immune response are potentially interfering with the induction of Tregs, in the presence of inhibitors of Il-4- and IL-13-mediated pathways such adjuvants are better suited for the therapeutic aims of the present invention than those eliciting primarily a Th1-type immune response. In the presence of inhibitors of Il-4-and IL-13-mediated pathways the contra-productive activity of adjuvants eliciting Th2-type immune responses is significantly reduced or even abolished.

### III.2. Adjuvants eliciting a Th1-type immune response

In another embodiment, adjuvants eliciting a Th1-type immune response including but not limited to monophosphoryl lipid A (MPL) and CpG oligonucleotides are used for the method of the present invention.

In one specific embodiment, monophosphoryl lipid A is used as adjuvant for the method of the present invention. Monophosphoryl lipid A (MPL) is a derivative of *Salmonella Minnesota* R595 lipopolysaccharide (LPS). MPL is made by removing a phosphate group and an acyl chain from the PLS molecule. Like LPS, MPL activates TLR4, but is over 100 times less toxic (for a review, see Nicholls et al., 2010).

In another specific embodiment, unmethylated CpG oligodeoxynucleotides (CpG ODNs) are used as adjuvant for the method of the present invention. CpG ODNs activate immune cells through TLR9, resulting in the production of pro-inflammatory cytokines. Studies in various animal models have demonstrated that CpG ODNs improve immune responses when administered in combination with currently used vaccine-adjuvant combinations (for a review, see Mutwiri et al., 2009).

In a preferred embodiment of the present invention, local administration of Treg-modulating therapeutics in support of antigen- or allergen-specific immunotherapy includes local administration of vitamin D3 or analogs thereof. Coincident administration of low molecular weight therapeutics capable of modulating the induction of Tregs via vitamin D-mediated pathways provides an important benefit for the use of adjuvants eliciting Th1-type immune responses including but not limited to CpG oligonucleotides and monophosphoryl lipid A (MPL). A recent study in mice has demonstrated that topical pretreatment with the vitamin D3 analog calcipotriol for 1 day followed by transcutaneous immunization within the treated skin area with ovalbumin in the presence of a CpG oligonucleotide adjuvant (TLR9 agonist) abolished antigen-specific CD8+ T cell priming and induced CD4+CD25+ Tregs, thereby promoting antigen-specific tolerance (Ghoreishi et al., 2009). Based on this study, the contra-productive activity of adjuvants eliciting Th1-type immune responses can be assumed to be significantly reduced or even abolished in the presence of vitamin D3 or analogs thereof.

### III.3. Adjuvants eliciting Th1- and Th2-type immune response

In another embodiment, adjuvants eliciting a combined Th1-type and Th2-type immune response including but not limited to squalene-based oil-in-water emulsions (MF59), saponins, granulocyte-macrophage colony stimulating factor (GM-CSF), and adjuvants based on inulin and virosomes are used for the method of the present invention.

In one specific embodiment, squalene-based oil-in-water emulsions (MF59) are used as adjuvant for the method of the present invention. MF59 consists of 5% squalene, 0.5% Tween 80 and 0.5% sorbitan trioleate. MF59 interacts with APCs at the injection site and disperses slowly to the draining lymph nodes where it is endocytosed. In mice MF59 has been shown to be capable of inducing high IgE and moderate IgG antibody titers, but in general MF59 elicits a predominant TH1 response which promotes efficient generation of of cytotoxic T cells (for a review, see Brunner et al., 2010).

In another specific embodiment, Quil-A is used as adjuvant for the method of the present invention. Quil-A is a natural saponin, extracted from the bark of the South American tree *Quillaja saponaria.* A purified fraction of Quil-A (QS21) has shown potent adjuvant abilities, elicting both TH1 and TH2 cytokine secretion in animals, but predominantly inducing cytotoxic T cell production (for a review, see Leroux-Roels, 2010). QS21 has been incorporated into several adjuvant formulations including AS01 (contains liposomes, MPL and QS21) and AS02 (oil-in-water emulsion containing MPL) (Tagliabue and Rappuoli, 2008).

In a preferred embodiment of the present invention, local administration of Treg-modulating therapeutics in support of antigen- or allergen-specific immunotherapy includes both local inhibition of IL-4/IL-13 and local administration of vitamin D3 or analogs thereof. Thereby, adjuvants eliciting a combined Th1- and Th2-type immune response including but are not limited to squalene-based oil-in-water emulsions, granulocyte-macrophage colony stimulating factor (GM-CSF), and adjuvants based on inulin and virosomes (for a review, see Nicholls et al., 2010) can be used for the method of the present invention.

### IV. FIELDS OF APPLICATION

In one embodiment, the present invention discloses allergic and autoimmune diseases for which the method of the present invention is beneficial. Allergic diseases include but are not limited to allergic conjunctivitis, allergic rhinitis, and allergic asthma. Autoimmune diseases include but are not limited to type I diabetes, rheumatoid arthritis, and multiple sclerosis. For such diseases, the present invention discloses methods for restoring lasting immunological tolerance by allergen- or antigen-specific immunotherapy in combination with individual combinations of low to moderate molecular weight Treg-modulating therapeutics selected from those listed in section I. Furthermore, the present invention discloses for each of theses diseases the rationale for the application of individual combinations of Treg-modulating therapeutics in support of allergen- or antigen-specific immunotherapy.

### IV.1. TREATMENT OF ALLERGY

Allergen-specific immunotherapy has been used for many decades as a desensitizing therapy for allergic diseases and represents the potentially curative method of treatment. The main principle of specific immunotherapy in atopic patients is to increase the number of Tregs capable of controlling allergen-specific effector T cells. Based on current knowledge, allergen tolerance is mediated by peripherally induced regulatory T cells as evidenced by a deficit of allergen-specific IL-10 producing T cells in the peripheral blood of allergic patients (for reviews, see Schmidt-Weber et al., 2005; Schmidt-Weber et al., 2006).

Subcutaneous immunotherapy (SCIT) involves the regular subcutaneous injection of allergen extracts or recombinant allergens using incremental regimes, with the induction of tolerance taking from several days to several months depending on the regime used. The usual approach is a build-up phase (consisting of weekly injections) followed by a maintenance phase (consisting of monthly injections). Once tolerance is induced it can last for several years without further treatment. The limiting factor in SCIT is anaphylactic side-effects, which vary in incidence from 0.1-5% of individuals depending on severity. Therefore, improved efficacy with decreased side-effects has been the aim of recent approaches to SCIT including chemically modified allergens (allergoids), T-cell-reactive peptide, and hypoallergenic recombinant allergens. However, further improvements are required to revitalize the acceptance of allergen-specific immunotherapy by patients and physicians.

The present invention aims for immune intervention by targeting of the molecular mechanisms of allergen tolerance and reciprocal regulation of effector and regulatory T cells. The locally restricted adjuvant therapy at the site of allergen presentation according to the method of the present invention is based on four concepts, each of which addresses the complex network of Treg induction at a different target site. Most important appears to be the combination of local inhibition of IL-4/IL-13-mediated effects and local inhibition of complement activation at the site of allergen presentation. However, local administration of vitamin D3 or analogs thereof as well as local inhibition of TNFR1-mediated functions at the site of allergen presentation represent also important supportive Treg-inducing strategies for the treatment of allergy as described below. The combination of two or more of these Treg-inducing strategies (including the combination of two or more Treg-modulating therapeutics suitable for a particular Treg-inducing strategy) along with allergen stimulation of T cells has the potential to improve the development of tolerance significantly and, thereby, to reverse the pathological processes in allergy.

### IV.1.1. Local inhibition of IL-4/IL-13-mediated effects as adjuvant therapy for allergy

In a preferred embodiment, local inhibition of IL-4/IL-13-mediated functions at the site of allergen presentation is an essential part of the supportive Treg-inducing therapy along with allergen-specific immunotherapy in the treatment of allergy according to the method of the present invention.

Inhibition of IL-4-mediated functions along with allergen-specific immunotherapy represents a particularly promising therapeutic approach, since IL-4 inhibits the expression of FOXP3, which is a requirement for inducible Treg (iTreg) differentiation and it is known that IL-4 is released locally upon allergen injection due to the activation of Th2 memory T cells. IL-13 is also known to be associated with the induction of the IgE isotype switch, secretion of IgE by B lymphocytes, and enhancement of IgE-mediated responses. Therefore, blockade of IL-13-mediated pathways along with allergen stimulation of T cells provides also important benefits for patients undergoing allergen-specific immunotherapy. Furthermore, coincident blockade of IL-4/IL-13-mediated pathways provides an additional benefit for the use of alum-adsorbed allergens for specific immunotherapy (see section III).

Animal experiments in mice have demonstrated that inhibition of the IL-4/Il-13 receptor system can completely abrogate humoral immune response to allergen and development of allergic symptoms in vivo (Grunewald et al., 1998). Immunization was performed by i.p. injection of 10 µg ovalbumin (OVA) in the presence of alum as adjuvant. At day 0, the murine IL-4 mutant Q116D/Y119D (the murine equivalent of the human IL-4 double mutant R121D/Y124D) was applied by i.p. injection 2 hours pre- and post-OVA-immunization as a 50 µg dose each. From day 1 to 8 treatment was continued with 30 µg mutant twice a day by i.p. injection. The data of this study show that treatment of mice with the murine IL-4 mutant Q116D/Y119D before and after immunization with OVA completely inhibited synthesis of OVA-specific IgE and IgG1. In addition, the synthesis of specific IgG2a, IgG2b and IgG3 was suppressed, which is indicative for the development of tolerance toward OVA.

Based on these data, local inhibition of IL-4/IL-13-mediated functions at the site of allergen presentation has the potential to support the efficacy of allergen-specific immunotherapy for the treatment of allergies significantly. Preferred low to moderate molecular weight inhibitors of IL-4 and IL-13 are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects mediated by interaction of the inhibitor with targets away from the site of allergen presentation. In a preferred embodiment of the invention, interleukin variants capable of antagonizing the activity of IL-4 and IL-13 such as the double mutant of human IL-4 (R121D/Y124D) are used as inhibitors.

### IV.1.2. Local inhibition of complement-mediated effects as adjuvant therapy for allergy

In another preferred embodiment, local inhibition of complement activation at the site of allergen presentation is also an essential part of the supportive Treg-inducing therapy along with allergen-specific immunotherapy for the treatment of allergy according to the method of the present invention. The impact of local inhibition of complement activation on the development of tolerance to allergens is evident from experiments with C3-deficient mice (Yalcindag et al., 2006). In order to investigate the role of C3 in inciting allergic skin inflammation and systemic immune responses after epicutaneous or intraperitoneal sensitization with allergens, C3-deficient (C3^{-/-}) mice and wild-type mice were subjected to epicutaneous or intraperitoneal immunization with ovalbumin (OVA) adsorbed onto alum. By comparison of the epicutaneous or intraperitoneal immunization procedure, the study addressed the local synthesis of C3 by keratinocytes and peritoneal macrophages, both of which are activated by the adjuvant alum. The study revealed that splenocytes from C3-deficient mice secreted less TH2-specific interleukins including IL-4, IL-5 and IL-13, and less TH1-specific IFN-γ in response to OVA stimulation than splenocytes from wild-type control mice. C3-deficient mice had impaired IgG1, IgG2a, and IgE antibody responses after both epicutaneous and intraperitoneal immunization. It is important to note that the defect was corrected by the addition of purified C3 protein (Yalcindag et al., 2006).

Another study has demonstrated that C3^{+/+} and C3^{-/-} dendritic cells (DCs) have different abilities to elicit the development of regulatory T cells (Peng et al., 2006). Co-culturing of CD4+ T cells with allogeneic C3 deficient (C3^{-/-}) dendritic cells (DCs) for 9 days yielded a 4-fold higher level in T cells expressing the *foxp3* gene as compared to those stimulated with C3^{+/+} DCs (Peng et al., 2006).

A very recent study has analyzed the phenotype of T cell responses when C3a receptor (C3aR)- and C5a receptor (C5aR)-mediated signals are absent on DCs, CD4+ T cells or both (Strainic et al., 2013). In vitro activation of naive CD4+ T cells from mice deficient in both C3aR and C5aR resulted in a lower abundance of the proinflammatory cytokines IL-6 and IFN-gamma, but a greater abundance of the anti-inflammatory cytokines IL-10 and TGF-beta1. Furthermore, the frequency of Foxp3+ T cells proved to be much greater in cultures of C3ar1^{-/-}C5ar1^{-/-} T cells than in cultures derived from C3ar1^{-l-} mice, C5ar1^{-/-} mice or wild-type mice. These data indicate that signaling via the complement factors C3a and C5a regulates effector T cell responses and that the absence of local complement activation paves the way for a default pathway leading to Foxp3+ Tregs.

Another potential benefit of complement inhibition during specific immunotherapy is due to the important role of complement in peanut-induced anaphylaxis. As shown by Khodoun et al. (2009), peanuts can activate complement and generate C3a-induced hypothermia in mice under conditions of high sensitivity to vasoactive mediators. Recently, it could be demonstrated in a murine model of peanut-induced anaphylaxis that peanut extracts activate complement by both the lectin and the classical pathways in vivo, thereby contributing to hypothermia shock in mice (Kodama et al., 2012). Most important, peanut-induced anaphylactic shock was prevented in C4-deficient mice in this study. Since complement protein C3 is the central component of all activation pathways, inhibition of complement at this level during immunotherapy may enable the development of an effective treatment for peanut allergy.

Based on the above listed studies, inhibition of locally synthesized C3 and/or inhibition of the interaction of locally generated C3a and C5a with their respective receptors on DCs and CD4+ T cells will support the efficacy of allergen-specific immunotherapy significantly. Preferred low molecular weight inhibitors of complement for the adjuvant treatment of allergy are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects.

In a preferred specific embodiment of the invention, low molecular weight agents capable of inhibiting locally synthesized complement component C3 are used for the method of the present invention. One preferred low molecular weight inhibitor targeting complement component C3 is the 13-residue cyclic peptide (H-I[CVVQDWGHHRC]T-NH₂) which is able to bind selectively to primate C3 and its C3b and C3c fragments, and to inhibit cleavage of C3 by C3 convertases of both the classical and the alternative pathway (Sahu et al., 1996; Ricklin and Lambris, 2008). Other preferred low molecular weight inhibitors targeting complement component C3 are derivatives of the 13-residue cyclic peptide (H-I[CVVQDWGHHRC]T-NH₂) as described by Morikis et al. (1998) and Katragadda et al. (2006).

In another preferred specific embodiment of the invention, low molecular weight agents capable of inhibiting the interaction of C3a and C5a with their respective receptors are used for the method of the present invention. Preferred low molecular weight C3aR antagonists include but are not limited to those with scaffolds featuring a) an arginine moiety (arginine derivatives) or b) an amino-piperidine linker and a pyridine moiety (amino-piperidine derivatives). Arginine derivatives include but are not limited to N²-[(2,2-dipheneylethoxy)acetyl]-L-arginine (SB290157; Ames et al., 2001), and those described by Denonne et al. (2007a). Amino-piperidine derivatives include but are not limited to those described by Denonne et al. (2007b). Preferred low molecular weight C5aR antagonists include but are not limited to peptidomimetic C5aR antagonists including C089, PMX53, PMX205 and JPE1357. PMX53, PMX205 and JPE1375 are analogs of the linear hexapeptide C089 (NMeFKPdChaWdR) which was derived from the C-terminus of C5a.

### IV.1.3. Local enhancement of vitamin D-mediated effects as adjuvant therapy for allergy

In another preferred embodiment, local administration of vitamin D3 or analogs thereof at the site of allergen presentation is an optional part of the supportive Treg-inducing therapy along with allergen-specific immunotherapy in the treatment of allergy according to the method of the present invention.

Several studies have demonstrated that locally synthesized 1,25-(OH)₂D3 has the capacity to induce Treg cells and to suppress adaptive immune responses including antigen-presenting cell functions and effector T cell responses (for reviews, see Bouillon et al., 2008; Hewison, 2010). As shown in a recent study, 1,25-(OH)₂D3 promotes tolerogenic epidermal Langerhans cells (LCs) and dermal dendritic cells (DDCs), both of which are able to generate Treg cells with different effector functions. Treatment of epidermal LCs with 1,25-(OH)₂D3 generates functional Foxp3+ Tregs through a mechanism that is dependent on keratinocyte-derived TGF-β. In contrast, treatment of DDCs with 1,25-(OH)₂D3 generates functional IL-10+FoxP3⁻ T_{R}1 cells in an IL-10-dependent fashion (van der Aar et al., 2011).

In accordance with these data, a recent study in mice has demonstrated that topical pretreatment with the vitamin D3 analogue calcipotriol for 1 day followed by transcutaneous immunization within the treated skin area (stratum corneum was removed by tape stripping) with ovalbumin in the presence of a CpG oligonucleotide adjuvant (TLR9 agonist) abolished antigen-specific CD8+ T cell priming and induced CD4+CD25+ Tregs, thereby promoting antigen-specific tolerance (Ghoreishi et al., 2009). The suppressive effect of calcipotriol was found to be a local effect, as application of antigen to an area untreated with calcipotriol did not result in suppression.

Based on the data of Ghoreishi et al. (2009), local administration of vitamin D3 or analogs thereof at the site of allergen presentation has the potential to support the efficacy of allergen-specific immunotherapy. Preferred low molecular weight molecules include but are not limited to 25-OH-D3, its biologically active metabolite 1α,25-dihydroxyvitamin D3 (1,25-(OH)₂D3), analogues of vitamin D3 and non-secosteroidal vitamin D receptor (VDR) modulators (for reviews, see Plum and DeLuca, 2010; Fletcher et al., 2012). In a preferred specific embodiment, the vitamin D3 analogue calcipotriol is used for the method of the present invention. Since topical calcipotriol has been used clinically for many years in the treatment of psoriasis without systemic toxicity (Kragballe, 1995) and has been demonstrated to effectively induce Tregs upon topical application, calcipotriol represents a preferred agent for the induction of Tregs at the site of allergen presentation according to the method of the present invention.

### IV.1.4. Local inhibition of TNFR1-mediated effects as adjuvant therapy for allergy

In another preferred embodiment, local inhibition of TNFR1 or TNFR1-mediated functions at the site of allergen presentation is an optional part of the supportive Treg-inducing therapy along with allergen-specific immunotherapy in the treatment of allergy according to the method of the present invention.

The rationale for concomitant TNFR1 inhibition along with allergen presentation is based on several lines of evidence. First, it has been demonstrated that antigen-specific activation of CD4+ effector T cells (Teff) strongly boosts the expansion of CD4+CD25+FoxP3+ Tregs and their suppressive function (Grinberg-Bleyer et al., 2010). TNF family members are involved in the Teff-mediated Treg boost, and the Treg boost has been demonstrated to be partially dependent on TNF-alpha via TNFR2-mediated signaling. Although this helper function of CD4+ T cells was observed in a murine model of type 1 diabetes (Grinberg-Bleyer et al., 2010), a Teff-mediated boost of Tregs represents most likely a general mechanism in immune diseases.

Second, by inhibition of TNFR1-mediated pro-inflammatory effects at the site of allergen presentation, TNFR2-mediated up-regulation of CD25 (alpha-chain of the IL-2 receptor) expression on Tregs is favored and, as a result of enhanced Il-2-mediated functions, up-regulation of TNFR2 expression on Tregs (for reviews, see Chen and Oppenheim, 2010; Biton et al., 2012). IL-2 has been shown to have a critical role in thymic development, expansion within peripheral blood, and the suppressive activity of CD4+CD25FoxP3+ Tregs. Furthermore, the selective availability of IL-2 has been demonstrated to represent a major determinant controlling the production of CD4+CD25+FoxP3+ T regulatory cells (Yu and Malek, 2006). While the present invention does not provide methods for increasing the local concentration of IL-2, the local synergistical up-regulation of CD25 and TNFR2 according to the method of the present invention favors a selective enhancement of IL-2-mediated functions on Tregs. Therefore, selective inhibition of TNFR1 represents an attractive adjuvant therapeutic approach for enhancing the proliferation, FoxP3 expression and the suppressive activity of Tregs in support of allergen-specific immunotherapy.

Third, the important role of TNF-alpha for the induction of allergen tolerance by specific immunotherapy is further documented by the observation that anti-TNF-alpha therapy during specific immunotherapy prevents the induction of allergen tolerance. As described in detail in the Examples of the present invention, discontinuation of anti-TNF-alpha therapy was required for a successful immunotherapy.

The important role of TNF family members in the development of allergen tolerance is further emphasized by the observation that the induction of Tregs in mice by topical treatment with calcipotriol prior to transcutaneous immunization with ovalbumin was associated with the induced expression of RANKL (receptor activator of NF-kappaB ligand) by keratinocytes, a TNF family member involved in modulation of skin dendritic cells (Ghoreishi et al., 2009).

Based on the data of above listed studies, local inhibition of TNFR1 and/or TNFR1-mediated pathways at the site of allergen presentation has the potential to support the efficacy of allergen-specific immunotherapy for the treatment of allergies. Preferred low to moderate molecular weight inhibitors of TNFR1 or TNFR1-mediated functions are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects mediated by interaction of the inhibitor with targets away from the site of allergen presentation.

In one specific embodiment, TNFR1-selective antagonistic TNF mutants are used for the method of the present invention. Preferred TNF mutants include but are not limited to R1antTNF developed by Shibata et al. (2008). In a preferred specific embodiment, TNFR1-specific antisense oligonucleotides are used for the method of the present invention. To allow for efficient cellular uptake of such oligonucleotides, thermosensitive hydrogels doped with cationic liposome-embedded TNFR1-specific antisense oligonucleotides are used. In a more preferred specific embodiment, S-methylglutathione (GSM) is used for the method of the present invention. GSM provides an attractive alternative to NAC, since a short incubation of isolated cardiomyocytes with GSM under in vitro conditions has been demonstrated to reproduce the effects of in vivo NAC treatment (Cailleret et al., 2004). In a most preferred specific embodiment, ASA and related salicylates are used for the method of the present invention. ASA and related salicylates allow selective inhibition of TNFR1-mediated activation of NF-kappa B and effective inhibition of IL-4 gene transcription. Although inhibition of TNFR1-mediated activation of NF-kappa B requires high doses of salicylates in the range of 1-2 x 10⁻² M (Thommesen and Laegreid, 2005), the risk of potential toxic side effects is minimized by application of matrices for sustained local delivery of ASA and related salicylates. Thereby, high local salicylate concentrations can be achieved at the site of allergen presentation, while upon diffusion away from the delivery site the salicylate concentration is rapidly decreased to systemically tolerable levels.

### IV.2. TREATMENT OF ALLERGIC ASTHMA

Combination inhalers are highly effective in asthma therapy and relatively inexpensive. However, even when taken regularly, inhaled corticosteroids (ICS) with or without long-acting beta-2-agonists (LABA) are not curative, as asthma symptoms and inflammation rapidly recur when the treatment is discontinued. In contrast, treatments that target the immune deviation in asthma might hold the prospect of long-term cure.

The majority of patients with asthma experience allergies, and treatments that target the underlying allergic inflammation are therefore a logical approach, particularly as such treatments might also treat associated allergic conditions. However, the efficacy and safety of SCIT in patients with allergic asthma is controversial. According to a review of 75 trials covering a total of 3,188 patients with asthma, SCIT led to a significant reduction in asthma symptom scores, medication use and airway hyper-responsiveness, with evidence of a dose-related effect (Abramson et al., 2003). Dose-response studies carried out for dog, cat, ragweed and grass allergies show consistent efficacy of SCIT using between 5 and 20 µg of the major allergen (for a review, see Holgate and Polosa, 2008). SCIT and SLIT (sublingual immunotherapy) also decrease the development of sensitization to new allergens and decrease the risk of new asthma in both adults and children with rhinitis. A recent retrospective cohort study of 322 subjects with allergic rhinitis showed that 53.1% of subjects who were not treated with SCIT developed asthma, whereas only 41.6% of subjects who received SCIT were diagnosed with asthma (for a review, see Holgate and Polosa, 2008).

Taken together, immunotherapy has been proven efficacious in treating mild asthma, as well as in preventing the progression to asthma in patients suffering from rhinoconjunctivitis (Moller et al., 2002; Jacobsen et al., 2007), but it is not yet recommended for the treatment of moderate to severe asthmatic patients (Rolland et al., 2009). Therefore, new strategies are required that are capable to reconstitute regulatory responses in combination with allergen-specific immunotherapy. Since Treg cells are key players in maintaining homeostasis within the lung, enhancement of the proliferation, FoxP3 expression and the suppressive activity of Tregs in support of allergen-specific immunotherapy by Treg-modulating therapeutics at the site of allergen presentation represents an attractive adjuvant therapeutic approach.

The locally restricted adjuvant therapy at the site of allergen presentation according to the method of the present invention is based on four concepts, each of which addresses the complex network of Treg induction at a different target site. Most important appears to be the combination of local inhibition of IL-4/IL-13-mediated effects, local inhibition of complement activation, in particular local inhibition of the interaction of C3a and C5a with their respective receptors, and local administration of vitamin D3 or analogs thereof at the site of allergen presentation. However, local inhibition of TNFR1-mediated functions at the site of allergen presentation represent also an important supportive Treg-inducing strategy for the treatment of allergic asthma as described below. The combination of two or more of these Treg-inducing strategies (including the combination of two or more Treg-modulating therapeutics suitable for a particular Treg-inducing strategy) along with allergen stimulation of T cells has the potential to improve the pathological processes in allergic asthma and represents a promising approach for preventing the progression to asthma in patients suffering from rhinoconjunctivitis as well as for treating mild and possibly moderate allergic asthma.

### IV.2.1. Local inhibition of IL-4/IL-13-mediated effects as adjuvant therapy for allergic asthma

In a preferred embodiment, local inhibition of IL-4/IL-13-mediated functions at the site of allergen presentation is an essential part of the supportive Treg-inducing therapy along with allergen-specific immunotherapy in the treatment of allergic asthma according to the method of the present invention.

Blockade of IL-13-mediated pathways along with allergen stimulation of T cells provides important benefits for patients undergoing allergen-specific immunotherapy since IL-4 inhibits the expression of FoxP3. FoxP3 is a requirement for inducible Treg (iTreg) differentiation and it is known that IL-4 is released locally upon allergen injection due to the activation of Th2 memory T cells. IL-13 is also known to be associated with the induction of the IgE isotype switch, secretion of IgE by B lymphocytes, and enhancement of IgE-mediated responses. Furthermore, coincident blockade of IL-4/IL-13-mediated pathways provides an additional benefit for the use of alum-adsorbed allergens for specific immunotherapy.

Based on the data of above listed studies, local inhibition of IL-4/IL-13-mediated effects at the site of allergen presentation has the potential to support the efficacy of allergen-specific immunotherapy in the treatment of allergic asthma significantly. Preferred low to moderate molecular weight inhibitors of IL-4 and IL-13 are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects mediated by interaction of the inhibitor with targets away from the site of allergen presentation.

In a preferred embodiment of the invention, interleukin variants capable of antagonizing the activity of IL-4 and IL-13 such as the double mutant of human IL-4 (R121D/Y124D) are used as inhibitors. The double mutant of human IL-4 (R121D/Y124D) has been shown to protect allergic cynomolgus monkeys from allergen-induced airways hyper-responsiveness when administered either subcutaneously or via nebulisation. Furthermore, subcutaneous administration of this double mutant in monkeys for 6 weeks or more also reduced the cutaneous wheal response and circulating concentrations of allergen-specific IgE. The effect of the double mutant of IL-4 (R121D/Y124D) on late phase asthmatic response to allergen challenge in asthmatic patients has been evaluated recently in clinical studies (Wenzel et al., 2007). The studies demonstrate that dual inhibition of IL-4 and Il-13 can positively affect the course of late asthmatic response after experimental allergen challenge. Treatment with the double mutant was associated with few adverse events, whether administered by subcutaneous injection (up to 30 mg for up to 13 weeks) or by inhalation (up to 60 mg for up to 4 weeks) in participants with atopic asthma or atopic eczema.

### IV.2.2. Local inhibition of complement-mediated effects as adjuvant therapy for allergic asthma

In another preferred embodiment, local inhibition of complement activation at the site of allergen presentation is also an essential part of the supportive Treg-inducing therapy along with allergen-specific immunotherapy for the treatment of allergic asthma according to the method of the present invention.

In the past decade, an important role for complement in the pathogenesis of allergic asthma has been uncovered. Several studies have demonstrated complement activation in human and experimental allergic asthma. Furthermore, blockade of complement activation with the mouse membrane complement inhibitor *Crry* (complement receptor-related gene y) and by targeting C3, C5, C3aR (C3a receptor) or C5aR (C5a receptor) during the allergic effector phase has been shown to decrease the alleric phenotype in different models of experimental allergic asthma (for a review, see Zhang and Köhl, 2010).

In a preferred specific embodiment, local inhibition of complement component C3 along with allergen stimulation of T cells is performed as adjuvant approach for the therapy of allergic asthma. The impact of local inhibition of complement component C3 on the pathological processes in allergic asthma is evident from experiments with C3-deficient mice. For example, using a mixture of *Aspergillus fumigatus* and OVA in a murine model of pulmonary allergy, C3 deficient mice were protected from airway reduction due to reduced airways responsiveness to inhaled methacholine (Drouin et al., 2001). Furthermore, in this study reduced levels of IL-4, antigen-specific IgE and IgG, and reduced eosinophil infiltration were observed.

In another preferred specific embodiment, local inhibition of C3a-C3aR and/or C5a-C5aR interactions along with allergen stimulation of T cells is performed as adjuvant approach for the therapy of allergic asthma. Different models of experimental allergic asthma suggest that the anaphylatoxins C3a and C5a not only promote proallergic effector functions during the allergic effector phase but regulate the development of Th2 immunity during allergen sensitization (for a review, see Zhang and Köhl, 2010).

Current data support a concept in which C5a is dominant during allergen sensitization and protects against the development of maladaptive Th2 immunity. In mouse models of allergic asthma, ablation of C5aR signaling prior to initial allergen sensitization leads to the induction of a Th2-biased immune response. By contrast, blockade of C5aR in already sensitized mice results in suppressed airway inflammation and airway hyper-responsiveness (AHR) (for a review, see Zhang and Köhl, 2010). Using a rat model, a synthetic hexapeptide C5a antagonist as well as inhibition of complement activation by a soluble CR1-derivative blocked the inflammatory response and AHR to methacholine (Abe et al., 2001). Furthermore, deficiency of C5 or C5aR has been shown to inhibit TH2 immune responses in asthma, thus providing protection to antigen-challenged mice (Drouin et al., 2006; Köhl et al., 2006). In a recent clinical trial conducted by Alexion Pharmaceuticals (CT, US) with the anti-C5 antibody eculizumab, even a single dose of eculizumab has been shown to attenuate allergen-induced airway responses in patients with mild asthma (Gail et al., 2009).

The role of C3a in the sensitization phase is less clear. Available data suggest that C3a/C3aR signaling contributes to the development of the allergic phenotype. During the effector phase, C3a and C5a appear to act synergistically and drive allergic inflammation (for a review, see Zhang and Köhl, 2010). Using the OVA model of antigen-induced airway hyper-responsiveness, near complete protection from the development of hyper-responsiveness to aerosolized methacholine was observed in C3aR deficient mice (Humbles et al., 2000). The same observation was made in a strain of guinea pigs with a natural C3aR defect (Bautsch et al., 2000). Using an OVA sensitization and challenge model of asthma, significant protection from airway broncho-constriction following antigen challenge (in contrast to airway hyper-responsiveness to methacholine) was noticed. In the absence of C3a-C3aR interactions, bone marrow-derived DCs exhibit also a less activated phenotype with reduced capacity for antigen uptake/presentation and lowered ability to stimulate TH1 responses (i.e. IL-12 production, generation of IFN-γ producing T cells) in vitro and in vivo (for a review, see Zhou, 2012).

As a result of the reduced ability of macrophages and DCs to stimulate TH1 responses in the absence of C3a-C3aR interactions, immune responses could shift potentially to TH2 responses as suggested by studies with C3aR deficient mice (Kawamoto et al., 2004). In C3aR deficient mice the immune response upon epicutaneous introduction of antigen has been demonstrated to shift towards TH2 responses, with significantly higher levels of serum IgG1 and lower levels of serum IgG2a, IgG3, and IgA compared with wild-type mice (Kawamoto et al., 2004). However, the potential shift towards TH2 responses upon local inhibition of C3a-C3aR interactions can be counteracted by local inhibition of IL-4- and IL-13-mediated effects along with allergen stimulation of T cells. Furthermore, inhibition of C5a-C5aR interactions will also counteract a potential shift towards TH2 responses since deficiency of C5 or C5aR has been shown to inhibit TH2 immune responses in asthma (Drouin et al., 2006; Köhl et al., 2006).

A very recent study has analyzed the phenotype of T cell responses when C3aR- and C5aR-mediated signals are absent on DCs, CD4+ T cells or both (Strainic et al., 2013). In vitro activation of naive CD4+ T cells from mice deficient in both C3aR and C5aR resulted in a lower abundance of the pro-inflammatory cytokines IL-6 and IFN-gamma, but a greater abundance of the anti-inflammatory cytokines IL-10 and TGF-beta1. Furthermore, the frequency of Foxp3+ T cells proved to be much greater in cultures of C3ar1^{-/-}C5ar1^{-/-} T cells than in cultures derived from C3ar1^{-/-} mice, C5ar1^{-/-} mice or wild-type mice. These data indicate that signaling via the complement factors C3a and C5a regulates effector T cell responses and that the absence of local complement activation paves the way for a default pathway leading to Foxp3+ Tregs.

Based on these studies, inhibition of locally synthesized C3 and/or inhibition of the interaction of locally generated C3a and C5a with their respective receptors on DCs and CD4+ T cells can be expected to support the efficacy of allergen-specific immunotherapy for the treatment of allergic asthma according to the method of the present invention significantly. Preferred low molecular weight inhibitors of complement for the adjuvant treatment of allergic asthma are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects.

In a preferred specific embodiment of the invention, low molecular weight agents capable of inhibiting locally synthesized complement component C3 are used for the method of the present invention. One preferred low molecular weight inhibitor targeting complement component C3 is the 13-residue cyclic peptide (H-I[CVVQDWGHHRC]T-NH₂) which is able to bind selectively to primate C3 and its C3b and C3c fragments, and to inhibit cleavage of C3 by C3 convertases of both the classical and the alternative pathway (Sahu et al., 1996; Ricklin and Lambris, 2008). Other preferred low molecular weight inhibitors targeting complement component C3 are derivatives of the 13-residue cyclic peptide (H-I[CVVQDWGHHRC]T-NH₂) as described by Morikis et al. (1998) and Katragadda et al. (2006).

In another preferred specific embodiment of the invention, low molecular weight agents capable of inhibiting the interaction of C3a and C5a with their respective receptors are used for the method of the present invention. Preferred low molecular weight C3aR antagonists include but are not limited to those with scaffolds featuring a) an arginine moiety (arginine derivatives) or b) an amino-piperidine linker and a pyridine moiety (amino-piperidine derivatives). Arginine derivatives include but are not limited to N²-[(2,2-dipheneylethoxy)acetyl]-L-arginine (SB290157; Ames et al., 2001), and those described by Denonne et al. (2007a). Amino-piperidine derivatives include but are not limited to those described by Denonne et al. (2007b). Preferred low molecular weight C5aR antagonists include but are not limited to peptidomimetic C5aR antagonists including C089, PMX53, PMX205 and JPE1357. PMX53, PMX205 and JPE1375 are analogs of the linear hexapeptide C089 (NMeFKPdChaWdR) which was derived from the C-terminus of C5a

### IV.2.3. Local enhancement of vitamin D-mediated effects as adjuvant therapy for allergic asthma

In another preferred embodiment, local administration of vitamin D3 or analogs thereof at the site of allergen presentation is an essential part of the supportive Treg-inducing therapy along with allergen-specific immunotherapy in the treatment of allergic asthma according to the method of the present invention.

Evidence for the beneficial effect of vitamin D on the symptoms and laboratory parameter associated with allergic asthma comes from in vitro and in vivo studies. For example, recent in vitro studies have demonstrated that treatment of human monocytic THP-1 cells and human primary monocytes with 1,25-(OH)₂D3 prior to stimulation with lipopolysaccharide significantly suppressed TNF-alpha and Th2-related chemokine IP-10, both of which play important roles in pathogenesis of severe refractory asthma (Kuo et al., 2010).

In another study, vitamin D receptor (VDR)-deficient mice (VDR-KO) failed to develop experimental allergic asthma (Wittke et al., 2004). VDR-KO mice did develop antigen-specific Th2 cell responses, but failed to develop lung inflammation or airway hyper-responsiveness. The absence of vitamin D signaling through VDRs protected these mice from developing experimental allergic asthma.

In a mouse model of ovalbumin (OVA)-induced allergic asthma, 1,25-(OH)₂D3 has been demonstrated to potentiate the beneficial effects of allergen immunotherapy (Taher et al., 2008). Coadministration of 10 ng 1,25-(OH)₂D3 with 100 µg of OVA immunotherapy significantly inhibited airway hyper-responsiveness (AHR) and potentiated the reduction of serum OVA-specific IgE levels, airway eosinophilia, and Th2-related cytokines concomitant with increased IL-10 levels in lung tissues and TGF-β and OVA-specific IgA levels in serum. Omission of 1,25-(OH)₂D3 during immunotherapy resulted in partial suppression of bronchoalveolar lavage eosinophilia, but no reduction of AHR (Taher et al., 2008).

Using also the classical mouse model of ovalbumin (OVA)-induced allergic asthma, a recent study has demonstrated that topical administration of 1,25-(OH)₂D3 enhances the regulatory capacity of CD4+CD25+ T cells from skin-draining lymph nodes to suppress Th2-mediated immune pathologies such as OVA-induced airway disease (Gorman et al., 2010).

Similar observations were made when the potential of human IL-10-secreting CD4+ Tregs from asthma patients to inhibit cytokine production by allergen-specific Th2 cells under in vitro conditions was analyzed (Xystrakis et al., 2006). The data of this study show that the impaired induction of IL-10 by glucocorticoids in human CD4+ Tregs from glucocorticoid-resistant asthma patients can be reversed by treatment with vitamin D3 (calcitriol) and IL-10, leading to levels of IL-10 production comparable to those observed in glucocorticoid-sensitive asthma patients (Xystrakis et al., 2006).

In a recent study, 1,25-(OH)₂D3 has been shown to affect the CD46 pathway in human T cells and, thereby, the production of IL-10 (Kickler et al., 2012). CD46 (also known as membrane cofactor protein, MCP) is a type 1 transmembrane regulator of complement activity that binds to C3b and C4b, allowing their cleavage by factor I. Moreover, CD46 is key in the regulation of the adaptive immune response (for a review, see Ni Choileain and Astier, 2012). Importantly, CD46 costimulation promotes Tr1-like Treg differentiation, characterized by secretion of large amounts of IL-10 and low levels of IFN-γ (Cardone et al., 2010). In CD4+ T cells from patients with asthma, CD46 activation has been demonstrated to be defective as evidenced by an impaired IL-10 production by CD46-activated T cells from these patients (Xu et al., 2010). Since IL-10 is capable of inhibiting synthesis of pro-inflammatory cytokines and to suppress the antigen-presentation capacity of antigen presenting cells, it is important that by the addition of 1,25-(OH)₂D3 to human CD4+ T cells with a CD46 defective pathway, a normal IL-10/IFN-γ ratio could be restored (Kickler et al., 2012). Although this observation was made with CD4+ T cells from patients with MS, it is likely that CD4+ T cells from patients with asthma will respond in a similar manner.

A recent prospective randomized, double-blind study including 48 children from 5 to 18 years of age with newly diagnosed asthma (only sensitive to house dust mites) has demonstrated that after six months of treatment with the glucocorticoid budesonide (800 µg/d, administered as dry powder) and cholecalciferol (500 IU; vitamin D3) a significantly lower number of children experienced asthma exacerbation (17%) as compared to the group receiving only glucocorticoids (46%) (Majak et al., 2011). It should be noted that these promising results which are in accordance with other studies (Brehm et al., 2010), were obtained with relatively low doses of vitamin D3 that are insufficient for an increase of 25(OH)D3 serum levels (Majak et al., 2011).

Based on the data of above listed studies, local administration of vitamin D3 or analogues thereof at the site of allergen presentation has the potential to support the efficacy of allergen-specific immunotherapy for the treatment of allergic asthma significantly. Preferred low molecular weight molecules include but are not limited to 25-OH-D3, its biologically active metabolite 1α,25-dihydroxyvitamin D3 (1,25-(OH)₂D3), analogues of vitamin D3 and non-secosteroidal vitamin D receptor (VDR) modulators (for reviews, see Plum and DeLuca, 2010; Fletcher et al., 2012).

In a preferred specific embodiment, the vitamin D3 analogue calcipotriol is used for the method of the present invention. Since topical calcipotriol has been used clinically for many years in the treatment of psoriasis without systemic toxicity (Kragballe, 1995) and has been demonstrated to effectively induce Tregs upon topical application, calcipotriol represents a preferred agent for the induction of Tregs at the site of allergen presentation according to the method of the present invention.

### IV.2.4. Local inhibition of TNFR1-mediated effects as adjuvant therapy for allergic asthma

In another preferred embodiment, local inhibition of TNFR1 or TNFR1-mediated pathways at the site of allergen presentation is an optional part of the supportive Treg-inducing therapy along with allergen-specific immunotherapy in the treatment of allergic asthma according to the method of the present invention.

Several small studies have suggested that anti-TNF-alpha therapy using the TNF-alpha-blocking antibody Infliximab or the soluble TNFR2 Etanercept, might be useful in reducing symptoms, exacerbations and airway hyper-responsiveness in patients with severe asthma (for a review, see Barnes, 2010), although a recent large multicenter trial with the fully human anti-TNF-alpha antibody Golimumab which also affects both TNFR1- and TNFR2-mediated signaling, did not show beneficial effects on lung function, symptoms and exacerbations (Wenzel et al., 2009). It should be noted, however, that specific local targeting of TNFR1-mediated functions in combination with allergen-specific immunotherapy as disclosed in the present invention represents a fundamentally different approach, since specific TNFR1 inhibition releases and favors the desired TNFR2-mediated functions at the site of allergen presentation. Furthermore, the enhancement of TNFR2-mediated functions is focused to the site of allergen presentation and, thereby, the reported Golimumab-induced side effects such as pneumonia and cancer (Wenzel et al., 2009) are avoided.

Selective inhibition of TNFR1-mediated signaling will also enhance an effector T cell (Teff)-mediated boost of Tregs. Although the strong boost of the expansion of CD4+CD25+FoxP3+ Tregs and their suppressive function by antigen-specific activation of CD4+ effector T cells (Teff) has been observed in a murine model of type 1 diabetes (Grinberg-Bleyer et al., 2010), this feed-back mechanism represents most likely a general process in immune responses. Most important, the Treg boost has been demonstrated to be partially dependent on TNF-alpha via TNFR2-mediated signaling. Therefore, selective inhibition of TNFR1-mediated signaling is a promising approach to support the induction of tolerance.

Based on the data of above listed studies, local inhibition of TNFR1 and/or TNFR1-mediated pathways at the site of allergen presentation has the potential to support the efficacy of allergen-specific immunotherapy for the treatment of allergic asthma. Preferred low to moderate molecular weight inhibitors of TNFR1 or TNFR1-mediated functions are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects mediated by interaction of the inhibitor with targets away from the site of allergen presentation.

In one specific embodiment, TNFR1-selective antagonistic TNF mutants are used for the method of the present invention. Preferred TNF mutants include but are not limited to R1antTNF developed by Shibata et al. (2008). In a preferred specific embodiment, TNFR1-specific antisense oligonucleotides are used for the method of the present invention. To allow for efficient cellular uptake of such oligonucleotides, thermo-sensitive hydrogels doped with cationic liposome-embedded TNFR1-specific antisense oligonucleotides are used. In a more preferred specific embodiment, S-methylglutathione (GSM) is used for the method of the present invention. GSM provides an attractive alternative to NAC, since a short incubation of isolated cardiomyocytes with GSM under in vitro conditions has been demonstrated to reproduce the effects of in vivo NAC treatment (Cailleret et al., 2004). In a most preferred specific embodiment, ASA and related salicylates are used for the method of the present invention. ASA and related salicylates allow selective inhibition of TNFR1-mediated activation of NF-kappa B and effective inhibition of IL-4 gene transcription. Although inhibition of TNFR1-mediated activation of NF-kappa B requires high doses of salicylates in the range of 1-2 x 10⁻² M (Thommesen and Laegreid, 2005), the risk of potential toxic side effects is minimized by application of matrices for sustained local delivery of ASA and related salicylates. Thereby, high local salicylate concentrations can be achieved at the site of allergen presentation, while upon diffusion away from the delivery site the salicylate concentration is rapidly decreased to systemically tolerable levels.

### IV.3. TREATMENT OF DIABETES TYPE 1

Type 1 diabetes (previously known as juvenile diabetes) is a T cell-dependent autoimmune disease in which islet antigens from insulin-producing beta-cells of the pancreas are presented by APCs to autoreactive T cells, breaking self-tolerance. Expansion of autoreactive CD4+ and CD8+ T cells, autoantibody-producing B cells and activation of the innate immune system cause beta-cell injury (Bluestone et al., 2010).

The appearance of diabetes-related autoantibodies has been shown to be able to predict the appearance of diabetes type 1 before any hyperglycemia arises, the main ones being islet cell autoantibodies, insulin autoantibodies, autoantibodies targeting the 65 kDa isoform of glutamic acid decarboxylase (GAD65) and autoantibodies targeting the phosphatase-related IA-2 molecule (Knip et al., 2005). The emergence of autoantibodies may be termed 'latent autoimmune diabetes', since not everyone with autoantibodies develops diabetes type 1. However, the risk increases with the number of autoantibody types and the presence of three to four antibody types is associated with a risk of progressing to type 1 diabetes of 60% to 100%. The time interval from emergence of autoantibodies to diabetes type 1 can be a few months in infants and young children, but in some people it may take years (Knip et al., 2005). Only after destruction of approximately 90% of the β-cells, type 1 diabetes becomes clinically manifest (for a review, see Homann and von Herrath, 2004). It has been proposed that diabetes Type 1 might be prevented at the latent autoimmune stage, before it starts destroying beta-cells (Bluestone et al., 2010).

Type 1 diabetes causes an estimated 5-10% of all diabetes cases or 11-22 million worldwide. The incidence of type 1 diabetes has been increasing by about 3% per year. In 2006, it affected 440,000 children under 14 years of age and was the primary cause of diabetes in those less than 10 years of age. However, the majority of new-onset type 1 diabetes is seen in adults. Adult-onset type 1 diabetes is two to three times more common than classic childhood-onset type 1 diabetes (http://en.wikipedia.org/wiki/Diabetes_mellitus_type_1).

Eventually, type 1 diabetes (autoimmune diabetes) is fatal unless continuously treated with insulin. Pancreatic islet cell transplantation is performed, but serious limitations are associated with this kind of treatment. Immunotherapy with anti-CD3 antibodies, including the humanized anti CD3 mAb Teplizumab (mutated to prevent binding to Fc receptors) and anti CD3 mAb Otelixizumab (similarly Fc-mutated), has provided evidence for preserved insulin production in newly diagnosed type 1 diabetes patients (Bluestone et al., 2010), but phase III studies with both antibodies failed to show clinical efficacy (Tolerx, 2011; MacroGenics, 2011), potentially due to an insufficient dosing schedule. The anti-CD20 antibody Retuximab inhibits B cells and has been shown to provoke C-peptide responses (evidence for insulin production) three months after diagnosis of type 1 diabetes, but long-term effects of this treatment have not been reported (Bluestone et al., 2010).

Adverse effects of the therapeutic approaches with broad immune-suppressive agents as well as the lack of permanent remission of disease with any agent tested to date have boosted interest in the development of antigen-specific interventions. Such an approach may require only a single beta-cell-derived antigen despite the polyclonal nature of the autoimmune response, reflected by the presence of multiple autoantibodies and multiple T cell epitopes that are recognized by peripheral blood cells. Immunological tolerance mechanisms, even by antigen-specific cells, are not restricted to a single antigen. Cytokines such as IL-10 and TGF-beta, produced by T cells in response to antigen or antigen-presenting cells, can modulate the function of effector T cells in the vicinity of the antigen. Thereby, activated regulatory T cells can exert their function at the site of the immune response without the need to recognize those antigen(s) recognized by effector T cells.' In addition, CD4+ CD25+ FoxP3+ and other regulatory T cells are able to modulate the function of effector T cells through contact-dependent mechanisms. It has been demonstrated that polyclonal diabetogenic T cells can be inhibited by antigen-specific Treg cells in vitro and in an adoptive transfer model of diabetes (for a review, see Bluestone et al., 2010).

A promising approach is the vaccination with the 65 kDa isoform of glutamic acid decarboxylase (GAD65). Immunization with alum-GAD65 was shown to attenuate the loss of C-peptide in individuals treated within six months of diagnosis (Ludvigsson et al., 2008). Furthermore, oral insulin administration yielded also promising results in a subset of patients with high levels of insulin autoantibodies (Skyler et al., 2005), an observation that is now being studied in detail. These data support the concept that an antigen-specific intervention may have broad immunological effects, provided the selected antigen is directly involved in the disease pathogenesis and/or regulation. Phase III trials with alum-GAD65 are underway in the USA and in Europe (Diamyd, 2011a and 2011b). In addition, prevention studies are underway in which this vaccine is given to persons who have not yet developed type 1 diabetes (Diamyd, 2011c).

Taken together, auto-antigen-specific immunotherapy has been proven efficacious for the treatment of patients with type 1 diabetes, provided the therapy is initiated shortly after diagnosis of the disease. There is no question, however, that current immunotherapeutic approaches need to be optimized. The present invention provides methods that are capable to reconstitute regulatory responses in combination with auto-antigen-specific immunotherapy.

The locally restricted adjuvant therapy at the site of auto-antigen presentation according to the method of the present invention is based on four concepts, each of which addresses the complex network of Treg induction at a different target site. Most important appears to be the combination of local inhibition of complement activation, in particular local inhibition of C3a-C3aR and C5a-C5aR interactions, local administration of vitamin D3 or analogs thereof and local inhibition of TNFR1 and TNFR1-mediated pathways. However, local inhibition of IL-4-mediated functions at the site of auto-antigen presentation represents also a useful supportive Treg-inducing strategy for the treatment of type I diabetes. The combination of two or more of these Treg-inducing strategies (including the combination of two or more Treg-modulating therapeutics suitable for a particular Treg-inducing strategy) along with auto-antigen stimulation of T cells has the potential to improve the pathological processes in type I diabetes.

### IV.3.1. Local inhibition of complement-mediated effects as adjuvant therapy for type 1 diabetes

In one preferred embodiment, local inhibition of complement at the site of auto-antigen presentation is an essential part of the supportive Treg-inducing therapy along with auto-antigen-specific immunotherapy for the treatment of type 1 diabetes according to the method of the present invention.

Based on recent evidence, autoimmune processes such as diabetes type 1 are composed not only of auto-aggressive T cell responses, but also of auto--reactive regulatory components. Therefore, successful approaches for immunotherapy of type 1 diabetes obviously require enhancement of regulatory T cell responses and inhibition of auto-reactive CD8+ T cells responses.

Animal studies have provided strong evidence that complement component C3 is required for priming and expansion of CD8+ T cells. For example, primed CD8+ T cell responses to allogeneic endothelial cells are controlled by local complement activation (Raedler et al., 2009), and activation as well as expansion of CD8+ T cells during a systemic viral infection was markedly reduced in C3-deficient mice (Suresh et al., 2003). Furthermore, priming of CD8+ T cells requires help from CD4+ T cells, and local complement has been shown to regulate the help of CD4+ cells for CD8+ T cells required for murine allograft rejection (Vieyra et al., 2011). Cognate interactions between CD4+ T cells and dendritic cells (DCs) induce C3a and C5a production and CD4-cell help is mediated, in part, by interaction of DC-derived C3a/C5a with CD8+ T cell-expressed C3aR/C5aR. This concept is supported by two observations. First, CD8+ T cells lacking C3aR and C5aR proliferate weakly to allogeneic DCs despite CD4 help. Second, augmented production of C3a/C5a by DCs bypasses the requirement for CD4- and CD40-dependent help to wild-type CD8+ T cells (Vieyra et al., 2011).

The impact of these observations for type I diabetes is evident from another recent animal study (Munegowda et al., 2011). The data of this study demonstrate that both CD4+TH17-cells and TH17-stimulated CD8+ cytotoxic T cells are involved in type I diabetes in mice, but induction of type I diabetes required only TH17-stimulated CD8+ T cells. This observation is in accordance with the recently described role of human islet auto-reactive CD8+ T cells in the pathogenesis of type I diabetes in humans (Coppieters et al., 2012). Although the role of local complement activation for TH17-mediated stimulation of CD8+ T cells has not been investigated in this study, the mechanisms of activation are most likely to be identical with those observed in other immune responses.

In animal studies, immune cell-derived C3, C3a receptors and C5a receptors have been demonstrated to be essential for the development of T cell-dependent type 1 diabetes induced by multiple low doses of streptozotocin (Lin et al., 2010). Although the results of this study have been questioned (Østergard et al., 2011), another recent study in mice has confirmed that complement C3 deficiency prevents against the onset of streptozotocin-induced autoimmune diabetes involving expansion of regulatory T cells (Gao et al., 2011). Higher numbers of CD4+CD25+ Treg cells with characteristics of expressing FoxP3 were observed in C3-/- mice. The central role of Tregs was further evidenced by that depleting these cells using anti-CD25 antibody dramatically abrogated the preventive effects of C3 deficiency on streptozotocin-induced autoimmune diabetes. Importantly, transforming growth factor beta (TGF-β) proved to be a key factor for Treg-mediated immune suppression (Gao et al., 2011).

Based on these studies, inhibition of locally synthesized C3 and/or inhibition of the interaction of locally generated C3a and C5a with their respective receptors (e.g., by interaction of DC-derived C3a/C5a with CD8+ T cell-expressed C3aR/C5aR) will support the efficacy of auto-antigen-specific immunotherapy significantly. Preferred low molecular weight inhibitors of complement for the adjuvant treatment of allergy are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects.

In a preferred specific embodiment of the invention, low molecular weight agents capable of inhibiting locally synthesized complement component C3 are used for the method of the present invention. One preferred low molecular weight inhibitor targeting complement component C3 is the 13-residue cyclic peptide (H-I[CVVQDWGHHRC]T-NH₂) which is able to bind selectively to primate C3 and its C3b and C3c fragments, and to inhibit cleavage of C3 by C3 convertases of both the classical and the alternative pathway (Sahu et al., 1996; Ricklin and Lambris, 2008). Other preferred low molecular weight inhibitors targeting complement component C3 are derivatives of the 13-residue cyclic peptide (H-I[CVVQDWGHHRC]T-NH₂) as described by Morikis et al. (1998) and Katragadda et al. (2006).

In another preferred specific embodiment of the invention, low molecular weight agents capable of inhibiting the interaction of C3a and C5a with their respective receptors are used for the method of the present invention. Preferred low molecular weight C3aR antagonists include but are not limited to those with scaffolds featuring a) an arginine moiety (arginine derivatives) or b) an amino-piperidine linker and a pyridine moiety (amino-piperidine derivatives). Arginine derivatives include but are not limited to N²-[(2,2-dipheneylethoxy)acetyl]-L-arginine (SB290157; Ames et al., 2001), and those described by Denonne et al. (2007a). Amino-piperidine derivatives include but are not limited to those described by Denonne et al. (2007b). Preferred low molecular weight C5aR antagonists include but are not limited to peptidomimetic C5aR antagonists including C089, PMX53, PMX205 and JPE1357. PMX53, PMX205 and JPE1375 are analogs of the linear hexapeptide C089 (NMeFKPdChaWdR) which was derived from the C-terminus of C5a.

### IV.3.2. Local enhancement of vitamin D-mediated effects as adjuvant therapy for type 1 diabetes

In another preferred embodiment, local administration of vitamin D3 or analogs thereof at the site of autoantigen presentation is an essential part of the supportive Treg-inducing therapy along with auto-antigen-specific immunotherapy for the treatment of type I diabetes according to the method of the present invention.

Strong evidence of a vitamin D3 effect on type I diabetes risk comes from experiments in the non-obese diabetic (NOD) mouse which exhibits a disease pathogenesis similar to that of humans including autoimmune destruction of beta cells. When 1,25-(OH)₂D3 was administered to NOD mice in pharmacological doses, it prevented the development of diabetes (Mathieu et al., 1994). The same observation was also made in a later animal study (Zella et al., 2003). Another recent study demonstrated that NOD mice raised in a vitamin D deficient state developed diabetes at an earlier age than non-deficient NOD controls (Giulietti et al., 2004).

Based on these animal data, several observational studies analyzing the effect of supplemental vitamin D3 on type I diabetes risk in humans have been performed. The first of these studies was a large case-control study conducted in seven European countries including the parents of 820 diabetic children and 2335 population-based controls (The EURODIAB Substudy 2 Study Group, 1999). The risk for type 1 diabetes by age 15 was reduced by about a third in children supplemented with vitamin D during the first year of life as compared to unsupplemented children. Another large case-control study was conducted in Norway including the parents of 545 diabetic children and 1668 population-based controls (Stene and Joner, 2003). A significant reduction in type 1 diabetes risk was observed when cod liver oil had been given to infants at least 5 times a week. A first prospective study included 12,055 pregnant women who lived in northern Finland (Hyppönen et al., 2001). Compared with children who had not been given vitamin D supplements, the relative risk of developing type 1 diabetes was only 0.12 among children given vitamin D supplements regularly and 0.16 among children given them irregularly.

Based on the data of above listed studies, local administration of vitamin D3 or analogs thereof at the site of auto-antigen presentation has the potential to support the efficacy of auto-antigen-specific immunotherapy for the treatment of type 1 diabetes significantly. Preferred low molecular weight molecules include but are not limited to 25-OH-D3, its biologically active metabolite 1α,25-dihydroxyvitamin D3 (1,25-(OH)₂D3), analogues of vitamin D3 and non-secosteroidal vitamin D receptor (VDR) modulators (for reviews, see Plum and DeLuca, 2010; Fletcher et al., 2012).

In a preferred specific embodiment, the vitamin D3 analog calcipotriol is used for the method of the present invention. Since topical calcipotriol has been used clinically for many years in the treatment of psoriasis without systemic toxicity (Kragballe, 1995) and has been demonstrated to effectively induce Tregs upon topical application, calcipotriol represents a preferred agent for the induction of Tregs at the site of auto-antigen presentation according to the method of the present invention.

### IV.3.3. Local inhibition of TNFR1-mediated effects as adjuvant therapy for type 1 diabetes

In another preferred embodiment, local inhibition of TNFR1 or TNFR1-mediated pathways at the site of auto-antigen presentation is an essential part of the supportive Treg-inducing therapy along with auto-antigen-specific immunotherapy in the treatment of type 1 diabetes according to the method of the present invention.

It is known for a long time that prolonged administration of antigens in the absence of pro-inflammatory stimuli could lead to the induction of tolerance and that part of this in vivo tolerance induction is mediated by CD4+CD25+FoxP3+ Tregs being induced from naive T cells (for a review, see Jaeckel et al., 2008). With regard to type 1 diabetes development, compelling evidence indicates that the loss of control of the autoimmune response in by FoxP3+ Tregs is a major contributing factor (for a review, see Li et al., 2012). There is evidence that human patients with diabetes type 1 have a reduced frequency or functionality of CD4+ CD25+ regulatory T cells (Kukreja et al., 2002). The necessity for increasing the number of CD4+ CD25⁺ regulatory T cells in patients with type I diabetes is supported by the observation that several therapeutic approaches which protect from diabetes, are correlated with an increase in CD4+ CD25+ regulatory T cells (for a review, see Juedes and von Herrath, 2004). For example, increased numbers of circulating CD4+ CD25+ regulatory T cells have been reported in patients receiving successful islet transplants and were treated with the anti-CD3 mAb hOKT3γ1(Ala-Ala) (Hering et al., 2004). Important for therapeutic approaches is the observation that human CD4+ CD25+ regulatory T cells may also be generated in the periphery after activation of CD4+ CD25⁻ T cells (Akbar et al., 2003; Walker et al., 2003). It has also been reported that adaptive Tregs elicited from murine CD4+ CD25⁻ T cells can persist as CD25- Treg cells despite expressing FoxP3 (Godebu et al., 2008).

Specific local inhibition of TNFR1 is likely to support the generation of CD4+ CD25+ regulatory T cells at the site of self-antigen presentation, since the resulting enhancement of TNFR2-mediated functions promotes Treg expansion (for reviews, see Chen and Oppenheim, 2010; Biton et al., 2012). This assumption is in agreement with the observation that mice deficient in TNF-alpha (Genetic Knock-Out (KO)) or treated with neutralizing antibody) developed various immune diseases including diabetes, whereas TNF-alpha administration has therapeutic effects in these diseases (Kassiotis and Kollias, 2001). The immunoregulatory role of TNF-alpha has been explained by its capacity to boost Treg activity.

Recently, it has been demonstrated that antigen-specific activation of CD4+ effector T cells (Teff) strongly boosts the expansion of CD4+CD25+FoxP3+ Tregs and their suppressive function (Grindberg-Bleyer et al., 2010). This helper function of CD4+ T cells was observed in the pancreas and draining lymph nodes in mouse recipients of islet-specific Teffs and Tregs. When islet-specific Teffs were transferred alone, they induced diabetes. When the same Teffs were co-transferred with islet-specific Tregs, they induced disease protection by boosting Treg expansion and suppressive function. Thus, diabetogenic Teffs help islet-specific Tregs to provide sustained protection from diabetes. RNA microarray analyses suggested that TNF family members are involved in the Teff-mediated Treg boost. In vivo experiments showed that this Treg boost is partially dependent on TNF-alpha but not on IL-2 (Grindberg-Bleyer et al., 2010). Again, the TNF-alpha-mediated effect has been explained by its capacity to boost Treg activity. In the Teff-mediated boost of Tregs, IL-2 was necessary to maintain Treg survival and was thus critical to preserving a high number of boosted Tregs, but was clearly not involved in the increased Treg proliferation mediated by Teffs. On the other hand, TNF-alpha is clearly involved, since blocking of TNF-alpha with soluble TNFR2 (Etanercept) or with anti-TNF-alpha monoclonal antibody XT3.11 significantly reduced the Teff-mediated Treg boost without inhibiting Teff activation (Grindberg-Bleyer et al., 2010).

Thus, during an autoimmune process such as type 1 diabetes, there is a local enrichment of both auto-reactive Teffs and Tregs. Since the efficacy of Treg-mediated suppression depends on the equilibrium between activated Teffs and activated Tregs, any factor that could tip this balance to one side or the other could then determine the outcome of the autoimmune process. By specific inhibition of TNFR1-mediated pro-inflammatory effects at the site of allergen presentation, TNFR2-mediated up-regulation of CD25 (alpha-chain of the IL-2 receptor) expression on Tregs is favored and, as a result of enhanced I1-2-mediated functions, up-regulation of TNFR2 expression on Tregs inducing a boost of Treg activity.

Based on the data of above listed studies, local inhibition of TNFR1 and/or TNFR1-mediated pathways at the site of autoantigen presentation has the potential to support the efficacy of auto-antigen-specific immunotherapy for the treatment of type I diabetes. Preferred low to moderate molecular weight inhibitors of TNFR1 or TNFR1-mediated functions are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of auto-antigen presentation, and d) have a relatively short plasma half-life to reduce potential adverse effects mediated by interaction of the inhibitor with targets away from the site of auto-antigen presentation.

In one specific embodiment, TNFR1-selective antagonistic TNF mutants are used for the method of the present invention. Preferred TNF mutants include but are not limited to R1antTNF developed by Shibata et al. (2008). In a preferred specific embodiment, TNFR1-specific antisense oligonucleotides are used for the method of the present invention. To allow for efficient cellular uptake of such oligonucleotides, thermosensitive hydrogels doped with cationic liposome-embedded TNFR1-specific antisense oligonucleotides are used. In a more preferred specific embodiment, S-methylglutathione (GSM) is used for the method of the present invention. GSM provides an attractive alternative to NAC, since a short incubation of isolated cardiomyocytes with GSM under in vitro conditions has been demonstrated to reproduce the effects of in vivo NAC treatment (Cailleret et al., 2004). In a most preferred specific embodiment, ASA and related salicylates are used for the method of the present invention. ASA and related salicylates allow selective inhibition of TNFR1-mediated activation of NF-kappa B and effective inhibition of IL-4 gene transcription. Although inhibition of TNFR1-mediated activation of NF-kappa B requires high doses of salicylates in the range of 1-2 x 10⁻² M (Thommesen and Laegreid, 2005), the risk of potential toxic side effects is minimized by application of matrices for sustained local delivery of ASA and related salicylates. Thereby, high local salicylate concentrations can be achieved at the site of allergen presentation, while upon diffusion away from the delivery site the salicylate concentration is rapidly decreased to systemically tolerable levels.

### IV.3.4. Local inhibition of IL-4-mediated effects as adjuvant therapy for type 1 diabetes

In another preferred embodiment, local inhibition of IL-4-mediated effects at the site of auto-antigen presentation is an optional part of the supportive Treg-inducing therapy along with auto-antigen-specific immunotherapy for the treatment of type 1 diabetes according to the method of the present invention.

Local inhibition of IL-4-mediated effects will contribute to the reduction of activated autoreactive CD8+ cytotoxic T cells by local inhibition of complement at the site of auto-antigen presentation, since IL-4 has been shown to be crucial for the priming of antigen-specific CD8+ T cells after vaccination with the Leishmania antigen HASPB-1 (Stäger et al., 2003). Immune complexes formed by reaction of natural antibodies with the Leishmania antigen initiate activation of complement, which in turn stimulates primary IL-4 secretion from CD11b+ CD11c+ phagocytes. In the presence of specific antibodies to ovalbumin (OVA), OVA injection induced IL-4 secretion comparable to that induced by the Leishmania antigen. Therefore, the authors concluded that immune complexes can, in general, provide a means of stimulating primary IL-4 secretion from CD11b+ CD11c+ phagocytes. Complement activation is essential for this process since in C1q-deficient mice neither IL-4 secretion nor priming of antigen-specific CD8+ T cell could be observed (Stäger et al., 2003).

In type 1 diabetes patients undergoing vaccination therapy, circulating auto-antibodies will also form immune complexes with injected auto-antigens. Therefore, local inhibition of IL-4-mediated effects in combination with local inhibition of complement represents a promising approach to reduce priming of harmful autoreactive CD8+ cytotoxic T cells and, thereby, to support the efficacy of auto-antigen-specific immunotherapy for the treatment of type 1 diabetes. Preferred low to moderate molecular weight inhibitors of IL-4 are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of auto-antigen presentation, and d) have a relatively short plasma half-life to reduce potential adverse effects mediated by interaction of the inhibitor with targets away from the site of auto-antigen presentation. In a preferred specific embodiment of the invention, interleukin variants capable of antagonizing the activity of IL-4 such as the double mutant of human IL-4 (R121D/Y124D) are used as inhibitors.

### IV.4. TREATMENT OF RHEUMATOID ARTHRITIS (RA)

RA is characterized by inflammatory reactions in the synovial membranes and articular structures of joints. Approximately 1% of the general population is believed to be affected with peak incidence of onset during the 4^{th} and 5^{th} decades of life. Despite new treatment modalities and more intensive therapeutic strategies, in most cases RA remains a chronic and progressive disease. The challenge for novel treatments is to specifically inhibit immune cells initiating and perpetuating RA-related inflammation and tissue destruction and to change the disease without causing severe side effects and/or general immunosuppression.

Anti-TNF therapy with TNF-alpha blockers (e.g., Infliximab, Adalimumab, Etanercept) has been shown to ameliorate clinical symptoms and laboratory parameters of inflammation in the majority of RA patients. However, despite the beneficial therapeutic effects of TNF-alpha blockers for RA patients, long-term use of these inhibitors can cause serious side effects including an increased incidence of infections. For example, in a retrospective analysis of 709 patients treated with at least one TNF-alpha blocker, 34.5% of patients reported infectious complications during the treatment period, with 6.2% falling under the category of serious infections (for a review, see Ichim et al., 2008).

Furthermore, clinical inhibition of inflammatory cytokines does not lead to a long-term cure. Therefore, various approaches for the induction of auto-antigen-specific tolerance have been tested (for a review, see Ichim et al., 2008). In animal models, immune suppressive epitopes of collagen II have been identified that induce protection from disease. However, the majority of clinical work has been performed targeting the auto-antigen hsp 60. For example, a clinical trial using hsp 60 peptides demonstrated clinical remission in patients with juvenile RA (Kamphuis et al., 2005). Recently, a multicentre, double-blind, placebo-controlled, auto-antigen-specific immunotherapy study was performed using the human cartilage glycoprotein-39 (HC gp-39) (Landewe et al., 2010). HC gp-39 was administered via the intranasal route since the nasal mucosa is currently regarded as the most powerful route for induction of immunological tolerance (Higuchi et al., 2000). However, the used treatment protocol did not result in more clinical improvement than in placebo-controlled patients, although HC gp-39 has been identified as a key auto-antigen in RA. Based on these examples it becomes clear that auto-antigen-specific immunotherapy appears to be feasible, but in its present state the antigen-specific suppression is too weak for widespread clinical implementation. In order to develop more potent protocols, there is a need for concomitant inhibition of effector T cells and the induction of regulatory T cells along with auto-antigen presentation. The present invention provides methods that are capable to reconstitute regulatory responses in combination with auto-antigen-specific immunotherapy.

In one embodiment, the present invention provides methods for locally restricted adjuvant therapy at the site of auto-antigen presentation that is based on four concepts, each of which addresses the complex network of Treg induction at a different target site. Most important appears to be the combination of local inhibition of TNFR1 and TNFR1-mediated pathways and local administration of vitamin D3 or analogs thereof. However, local inhibition of complement activation, in particular local inhibition of C3a-C3aR and C5a-C5aR interactions, and local inhibition of IL-4-mediated functions at the site of auto-antigen presentation represent also useful supportive Treg-inducing strategies for the treatment of RA. The combination of two or more of these Treg-inducing strategies (including the combination of two or more Treg-modulating therapeutics suitable for a particular Treg-inducing strategy) along with auto-antigen stimulation of T cells has the potential to improve the pathological processes in rheumatoid arthritis.

### IV.4.1. Local inhibition of TNFR1-mediated effects as adjuvant therapy for rheumatoid arthritis (RA)

Important evidence for the involvement of TNF-alpha in RA came from studies with mice over-expressing human TNF-alpha and spontaneously developing polyarthritis. Successful treatment of these mice with an anti-TNF-alpha antibody demonstrated the detrimental role of TNF-alpha in this model. Currently, different mouse models are used to study arthritis. One model of TNF-alpha-induced arthritis is the TNFdARE mouse. In this mouse, deletion of the ARE region in the 3'UTR of the TNF-alpha mRNA stabilizes the mRNA and, thereby, causes continuous production of TNF-alpha. These animals develop spontaneous polyarthritis and small bowel inflammation resembling Crohn's disease. When crossed with TNFR1 deficient mice, TNFdARE mice do not develop arthritis, but crossing with TNFR2 KO mice leads to exacerbation of disease progression. This indicates that TNFR1-mediated signaling is pathologic, whereas TNFR2-mediated signaling induces anti-inflammatory effects.

In another arthritis model, collagen-induced arthritis (CIA), TNF-alpha plays an important but not a dominant role. However, anti-TNF-alpha therapy is also effective in these mice. Therapeutic inhibition of TNFR1 has shown some success in the CIA model. For example, specific inhibition of TNFR1 with the TNF-alpha mutant R1antTNF was effective in reducing the clinical symptoms (Shibata et al., 2009).

To understand the role of sTNF-alpha in arthritis, mice expressing an uncleavable transmembrane TNF-alpha (mTNT-alpha) were tested in a TNF-alpha-dependent model of arthritis (Alexopoulou et al., 2006). The mice expressing only mTNF-alpha were resistant to arthritis, indicating that sTNF-alpha is essential for the development of this disease. This observation was confirmed by specific therapeutic inhibition of sTNF-alpha in experimental arthritis by a dominant negative TNF-alpha mutant (Zalevsky et al., 2007).

Treatment with total TNF-alpha blockers (e.g., Infliximab, Adalimumab, Etanercept) has been shown to attenuate TNFR1-mediated inflammatory responses and at the same time to inhibit TNFR2-mediated anti-inflammatory effects. A recent study demonstrated that anti-TNF-alpha therapy in experimental arthritis expanded pathogenic Th1 and Th17 cells in the lymph nodes, mediated by up-regulation IL-12/IL-23p40 expression. However, anti-TNF-alpha therapy blocked the accumulation of Th1/Th17 cells in the synovium, thereby providing an explanation for the paradox that anti-TNF-alpha therapy ameliorates experimental arthritis despite increasing the number of pathogenic T cells in lymph nodes (Notley et al., 2008).

Different effects of TNF-alpha on Tregs and Teffs may also contribute to the beneficial therapeutic effects of anti-TNF-alpha therapy. Tregs are often increased at the site of RA-related inflammation, presumably resulting from active recruitment and in situ proliferative expansion. High levels of TNFR2 expression by Tregs mediated by chronic inflammatory responses may enable Tregs to outcompete other TNFR2-expressing cells for TNF-alpha and, thereby, to accumulate. However, despite the increased presence of suppressive Tregs the inflammation in joints of RA patients is ongoing, possibly due to the interaction of TNF-alpha with TNFR2 expressed by effector T cells (Teffs). Expression of TNFR2 by Teffs is also up-regulated upon TCR stimulation (Chen et al., 2007), which may allow the TNF-alpha signal mediated by TNFR2 to effectively stimulate Teffs and, thereby, render Teffs more resistant to Treg-mediated inhibition. The observation that higher concentrations of TNF-alpha down-regulated Treg suppressive activity (Valencia et al., 2006) is likely to reflect a dose-dependent stimulating effect of TNF-alpha on Teffs. Thus, the increasing resistance of Teffs to Treg-mediated inhibition may be the reason for pro-inflammatory effects of TNF-alpha. In view of different effects of TNF-alpha on Tregs and Teffs, the therapeutic effect of anti-TNF-alpha therapy is probably achieved by eliminating the TNF-alpha/TNFR2 costimulation pathway on activated pathogenic Teffs which most likely express elevated levels of TNFR2.

It should be mentioned, however, that anti-TNF therapy of RA patients has also been demonstrated to result in the generation of a distinct population of suppressor cells derived from naive CD4+ cells. The suppressive activity of these induced cells which lack CD62L expression, proved to be dependent on IL-10 and TGF-beta (Nadkarni et al., 2007). Therefore, these induced suppressor cells are assumed to be Tr1 and/or Th3 cells.

However, despite the beneficial therapeutic effects of total TNF-alpha blockers for RA patients, long-term use of these inhibitors can cause serious side effects including an increased incidence of infections. The present invention avoids these risks by disclosing methods for selective inhibition of TNFR1-mediated functions and matrices for local delivery of TNFR1 specific inhibitors. Thereby, beneficial mTNF-alpha/TNFR2 interactions are retained and potential systemic side effects are minimized.

Although synovial fibroblasts have been identified as the primary targets for TNF-alpha in the development of arthritis, the site for selective inhibition of TNFR1-mediated functions does not appear to be critical for the therapeutic effect. As demonstrated in a recent study, systemic treatment with an adenoviral vector containing a short hairpin RNA directed against TNFR1 ameliorated collagen-induced arthritis (CIA) in mice almost to the same extent as local treatment (Arntz et al., 2010). Furthermore, TNFR1 silencing in knee joints also protected the ipsilateral ankle joints in CIA mice. Based on these observations, local inhibition of TNFR1-mediated functions at the site of auto-antigen presentation has the potential to induce systemic tolerance.

Preferred low to moderate molecular weight inhibitors of TNFR1 or TNFR1-mediated functions are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of auto-antigen presentation, and d) have a relatively short plasma half-life to reduce potential adverse effects mediated by interaction of the inhibitor with targets away from the site of auto-antigen presentation.

In one specific embodiment, TNFR1-selective antagonistic TNF mutants are used for the method of the present invention. Preferred TNF mutants include but are not limited to R1antTNF developed by Shibata et al. (2008). In a preferred specific embodiment, TNFR1-specific antisense oligonucleotides are used for the method of the present invention. To allow for efficient cellular uptake of such oligonucleotides, thermosensitive hydrogels doped with cationic liposome-embedded TNFR1-specific antisense oligonucleotides are used. In a more preferred specific embodiment, S-methylglutathione (GSM) is used for the method of the present invention. GSM provides an attractive alternative to NAC, since a short incubation of isolated cardiomyocytes with GSM under in vitro conditions has been demonstrated to reproduce the effects of in vivo NAC treatment (Cailleret et al., 2004). In a most preferred specific embodiment, ASA and related salicylates are used for the method of the present invention. ASA and related salicylates allow selective inhibition of TNFR1-mediated activation of NF-kappa B and effective inhibition of IL-4 gene transcription. Although inhibition of TNFR1-mediated activation of NF-kappa B requires high doses of salicylates in the range of 1-2 x 10⁻² M (Thommesen and Laegreid, 2005), the risk of potential toxic side effects is minimized by application of matrices for sustained local delivery of ASA and related salicylates. Thereby, high local salicylate concentrations can be achieved at the site of allergen presentation, while upon diffusion away from the delivery site the salicylate concentration is rapidly decreased to systemically tolerable levels.

### IV.4.2. Local enhancement of vitamin D-mediated effects as adjuvant therapy for rheumatoid arthritis (RA)

In another preferred embodiment, local administration of vitamin D3 or analogs thereof at the site of auto-antigen presentation is an essential part of the supportive Treg-inducing therapy along with auto-antigen-specific immunotherapy for the treatment of RA according to the method of the present invention.

Strong evidence for an inhibitory effect of vitamin D3 on the progression of autoimmune arthritis comes from two different animal models of arthritis, namely murine Lyme arthritis and collagen-induced arthritis (Cantorna et al., 1998). Infection of mice with *Borrelia burgdoferi,* the causative agent of human Lyme arthritis, produced arthritic lesions including footpad and ankle swelling. Supplementation with 1,25-dihydroxycholecalciferol of an adequate diet fed to mice infected with *B. burgdorferi* minimized or prevented these symptoms. Mice immunized with type II collagen also developed arthritis. The symptoms of this disease were also prevented by dietary supplementation with 1,25-dihydroxycholecalciferol (calcitriol).

Based on these animal data, several studies evaluating the association of vitamin D deficiency and disease activity in RA were conducted (for a review, see Gatenby et al., 2013). Although these studies point to a relationship between a low dietary intake of vitamin D and increased susceptibility to RA, the results are mixed and non conclusive.

Only few studies have measured the effects of vitamin D supplementation on disease activity in RA in a prospective fashion. However, in one open label study, 19 patients with RA on methotrexate therapy were treated with 2 µg/day of alfacalcidiol (1(OH)D3) for a period of 3 months. Disease activity was monitored by electron spin resonance (ESR) and the Ritchie articular index (RAI). After three months of therapy, 89% of patients experienced an improvement of disease activity, with 45% (9/19) going into remission (Andjelkovic et al., 1999).

Since anti-TNF therapy has been shown to ameliorate clinical symptoms and laboratory parameters of inflammation in the majority of RA patients, it is also noteworthy that in a recent study treatment of human monocytic THP-1 cells and human primary monocytes with 1,25-(OH)₂D3 prior to stimulation with lipopolysaccharide has been demonstrated to significantly suppress TNF-alpha (Kuo et al., 2010).

Recently, 1,25-(OH)₂D3 has been shown to affect also the CD46 pathway in human T cells and, thereby, to promote anti-inflammatory responses mediated by CD46 (Kickler et al., 2012). CD46 is a type 1 transmembrane regulator of complement activity that binds to C3b and C4b, allowing their cleavage by factor I. Moreover, CD46 is key in the regulation of the adaptive immune response (for a review, see Ni Choileain and Astier, 2012). Importantly, CD46 costimulation promotes Tr1-like Treg differentiation, characterized by secretion of large amounts of IL-10 and low levels of IFN-γ (Cardone et al., 2010). In T cells from patients with RA, CD46 activation has been demonstrated to be defective as evidenced by an impaired IL-10 production by CD46-activated T cells from these patients (Cardone et al., 2010). Since IL-10 is capable of inhibiting synthesis of pro-inflammatory cytokines such as IFN-γ, IL-2, IL-3, TNF-α and GMCSF, and to suppress the antigen-presentation capacity of antigen presenting cells, it is important that by the addition of 1,25-(OH)₂D3 to human CD4+ T cells with a CD46 defective pathway, a normal IL-10/IFN-γ ratio could be restored (Kickler et al., 2012). Although this observation was made with CD4+ T cells from patients with MS, it is likely that CD4+ T cells from patients with RA will respond in a similar manner.

Based on the data of above listed studies, local administration of vitamin D3 or analogues thereof at the site of auto-antigen presentation has the potential to support the efficacy of auto-antigen-specific immunotherapy for the treatment of RA. Preferred low molecular weight molecules include but are not limited to 25-OH-D3, its biologically active metabolite 1α,25-dihydroxyvitamin D3 (1,25-(OH)₂D3), analogues of vitamin D3 and non-secosteroidal vitamin D receptor (VDR) modulators (for reviews, see Plum and DeLuca, 2010; Fletcher et al., 2012).

In a preferred specific embodiment, the vitamin D3 analogue calcipotriol is used for the method of the present invention. Since topical calcipotriol has been used clinically for many years in the treatment of psoriasis without systemic toxicity (Kragballe, 1995) and has been demonstrated to effectively induce Tregs upon topical application, calcipotriol represents a preferred agent for the induction of Tregs at the site of auto-antigen presentation according to the method of the present invention.

### IV.4.3. Local inhibition of complement-mediated effects as adjuvant therapy for rheumatoid arthritis (RA)

In another preferred embodiment, local inhibition of complement activation at the site of auto-antigen presentation is also an essential part of the supportive Treg-inducing therapy along with auto-antigen-specific immunotherapy for the treatment of RA according to the method of the present invention.

Given the central role of dendritic cells (DCs) in stimulating naive T cell responses and also generating T cells with regulatory functions, means of manipulating DCs towards a tolerogenic phenotype appears to be a promising approach. Local inhibition of complement component C3 provides a way to reduce the functions of immune stimulatory DCs and, thereby, to tip the balance more to a tolerogenic phenotype.

Several human dendritic cell subsets (e.g., monocyte derived, dermal, Langerhans, myeloid, plasmacytoid) express C3a and/or C5a receptors (C3aR; C5aR, CD88), and both C3a and C5a, which are present in the DC environment, can up-regulate TH1 responses by modulating DC activation and function (for a review, see Zhou, 2012). In the absence of C3a-C3aR interactions, the functions of bone marrow-derived DCs were impaired, resulting in a less activated phenotype with reduced capacity for antigen uptake/presentation and lowered ability to stimulate TH1 responses (i.e. IL-12 production, generation of IFN-γ producing T cells) in vitro and in vivo. Accordingly, stimulation of C3aR with C3a increased the capacity of DCs for antigen presentation and enhanced their ability to stimulate allospecific T cell responses (Peng et al., 2008; Li et al., 2008). Similar observations have been made for C5a-C5aR interactions (for a review, see Zhou, 2012).

Furthermore, a recent study has demonstrated that the interaction of serum-derived C5a with immune cell-expressed C5aR is an essential mediator in an IL-17-dependent model of autoimmune arthritis (Hashimoto et al., 2010).

Collectively, these data demonstrate that activation of C3 and C5 plays also an important role in the regulation of auto-reactive T cells (for a review, see Heeger and Kemper, 2012). Accordingly, complement deficiency or blockade effects attenuation of T cell-mediated autoimmunity (for a review, see Kwan et al., 2012).

Based on these studies, inhibition of locally synthesized C3 and/or inhibition of the interaction of locally generated C3a and C5a with their respective receptors are likely to support the efficacy of auto-antigen-specific immunotherapy. Preferred low molecular weight inhibitors of complement for the adjuvant treatment of allergy are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects.

In a preferred specific embodiment of the invention, low molecular weight agents capable of inhibiting locally synthesized complement component C3 are used for the method of the present invention. One preferred low molecular weight inhibitor targeting complement component C3 is the 13-residue cyclic peptide (H-I[CVVQDWGHHRC]T-NH₂) which is able to bind selectively to primate C3 and its C3b and C3c fragments, and to inhibit cleavage of C3 by C3 convertases of both the classical and the alternative pathway (Sahu et al., 1996; Ricklin and Lambris, 2008). Other preferred low molecular weight inhibitors targeting complement component C3 are derivatives of the 13-residue cyclic peptide (H-I[CVVQDWGHHRC]T-NH₂) as described by Morikis et al. (1998) and Katragadda et al. (2006).

In another preferred specific embodiment of the invention, low molecular weight agents capable of inhibiting the interaction of C3a and C5a with their respective receptors are used for the method of the present invention. Preferred low molecular weight C3aR antagonists include but are not limited to those with scaffolds featuring a) an arginine moiety (arginine derivatives) or b) an amino-piperidine linker and a pyridine moiety (amino-piperidine derivatives). Arginine derivatives include but are not limited to N²-[(2,2-dipheneylethoxy)acetyl]-L-arginine (SB290157; Ames et al., 2001), and those described by Denonne et al. (2007a). Amino-piperidine derivatives include but are not limited to those described by Denonne et al. (2007b). Preferred low molecular weight C5aR antagonists include but are not limited to peptidomimetic C5aR antagonists including C089, PMX53, PMX205 and JPE1357. PMX53, PMX205 and JPE1375 are analogs of the linear hexapeptide C089 (NMeFKPdChaWdR) which was derived from the C-terminus of C5a.

### IV.4.4. Local inhibition of IL-4-mediated effects as adjuvant therapy for rheumatoid arthritis (RA)

In another preferred embodiment, local inhibition of IL-4-mediated effects at the site of auto-antigen presentation is an optional part of the supportive Treg-inducing therapy along with auto-antigen-specific immunotherapy for the treatment of RA according to the method of the present invention.

Local inhibition of IL-4-mediated functions is likely to provide benefits for RNA patients, since IL-4 inhibits the expression of FOXP3, which is a requirement for inducible Treg (iTreg) differentiation.

Furthermore, local inhibition of IL-4-mediated effects will contribute to the reduction of activated CD8+ cytotoxic T cells by local inhibition of complement at the site of self-antigen presentation, since IL-4 has been shown to be crucial for the priming of antigen-specific CD8+ T cells after vaccination with the Leishmania antigen HASPB-1 (Stäger et al., 2003). Immune complexes formed by reaction of natural antibodies with the Leishmania antigen initiate activation of complement, which in turn stimulates primary IL-4 secretion from CD11b+ CD11c+ phagocytes. In the presence of specific antibodies to ovalbumin (OVA), OVA injection induced IL-4 secretion comparable to that induced by the Leishmania antigen. Therefore, the authors concluded that immune complexes can, in general, provide a means of stimulating primary IL-4 secretion from CD11b+ CD11c+ phagocytes. Complement activation is essential for this process since in C1q-deficient mice neither IL-4 secretion nor priming of antigen-specific CD8+ T cell could be observed (Stäger et al., 2003). In RA patients undergoing vaccination therapy, circulating auto-antibodies will also form immune complexes with injected auto-antigens. Therefore, local inhibition of IL-4-mediated effects in combination with local inhibition of complement represents a promising approach to reduce priming of harmful auto-reactive CD8+ cytotoxic T cells and, thereby, to support the efficacy of auto-antigen-specific immunotherapy for the treatment of RA.

Preferred low to moderate molecular weight inhibitors of IL-4 are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of auto-antigen presentation, and d) have a relatively short plasma half-life to reduce potential adverse effects mediated by interaction of the inhibitor with targets away from the site of auto-antigen presentation. In a preferred specific embodiment of the invention, interleukin variants capable of antagonizing the activity of IL-4 such as the double mutant of human IL-4 (R121D/Y124D) are used as inhibitors.

### IV.5. TREATMENT OF MULTIPLE SCLEROSIS (MS)

Worldwide, MS may affect 2.5 million individuals and it is the most common disease affecting young adults. MS is thought to occur in genetically predisposed individuals following exposure to an environmental trigger that activates myelin-specific T cells. To initiate central nervous system (CNS) inflammation, myelin-specific T cells must gain access to the CNS after activation in the periphery. Tight junctions between the endothelial cells of the blood-brain barrier and the epithelial cells of the blood-cerebrospinal fluid barrier limit access to the CNS by circulating cells. However, activated and memory T cells can carry out immune surveillance of the CNS because they express adhesion molecules, chemokine receptors and integrins that allow them to cross the barrier. Reactivation of the T cells by CNS-resident antigen presenting cells (APCs) that present myelin antigens, triggers the recruitment of innate immune cells, which have important roles in mediating demyelination and axonal damage.

MS has been widely studied using the animal model of experimental autoimmune encephalomyelitis (EAE) which is induced by immunization with myelin-derived antigens in adjuvant or by the adoptive transfer of activated myelin-specific T cells. The inflammatory infiltrates and demyelination seen in EAE have many similarities to the pathology of MS. Myelin basic protein (MBP) and the myelin-derived proteolipid protein (PLP) are the most abundant proteins in the myelin sheath in the CNS and were the first to be identified as CD4+ T cell targets in the initial subsets of mouse strains that exhibited susceptibility to EAE. Susceptibility to EAE requires not only the expression of an MHC class II molecule that can bind at least one peptide derived from a myelin antigen, but also that some myelin-specific T cells escape central tolerance so that they are available for activation in the periphery. Central tolerance is the process in which T cells that exhibit high avidity interactions with APCs presenting self-antigen-MHC complexes in the thymus are deleted. High avidity, however, is a function of both the affinity of the T cell receptor (TCR) for the self-antigen-MHC complex and the abundance of these complexes on the surface of the APC. The observation that many myelin proteins mediate central tolerance suggests that myelin-specific T cells which escape to the periphery are mostly T cells with low avidity for self-antigens (for a review, see Goverman, 2009). The low avidity of these T cells for self-antigen normally prevents them from engaging self-antigen in the periphery and allows them to circulate without inducing damage. However, the avidity of T cells for self-antigen can be increased under certain circumstances. For example, APCs responding to an immunogenic stimulus such as an infection increase their expression of co-stimulatory molecules and MHC complexes on the cell surface. These changes can result in an increase in the avidity of the interaction between APC and self-reactive T cells. Thereby, T cells with low avidity for self-antigens can now respond to the self-antigen that they previously ignored.

Several studies have described TCRs that recognize both myelin antigens and pathogen-derived epitopes (for a review, see Goverman, 2009). According to the molecular mimicry hypothesis, the more T cells with this type of degenerate recognition an individual has, the greater the chance that T cells activated in the response to a specific pathogen will initiate an autoimmune response against a myelin antigen. Support for this hypothesis is based on the observation that the incidence of spontaneous EAE which develops in one model of MBP-specific TCR-transgenic mice increases as the level of microbial exposure increases.

MS and its animal model EAE have long been regarded as primarily T helper cell type 1-mediated diseases. However, recent evidence suggests that CD4+Th17 cells may be even more pathogenic than CD4+Th1 cells. In the EAE model, this cell type is crucial for the recruitment of leukocytes into the CNS and for triggering parenchymal inflammation. In humans, Th17 cells are found in acutely active and on the borders of chronically active lesions. Furthermore, recent studies point also to the potential importance of CD8+ T cells in CND autoimmunity. One compelling reason for this is that depletion of CD4+ T cells in patients with MS resulted in no improvement in disease, whereas treatment with a monoclonal antibody which depletes several leukocyte populations including CD4+ and CD8+ T cells seemed to be beneficial. CD8+ T cells also outnumber CD4+ T cells in multiple sclerosis lesions (for a review, see Goverman, 2009).

The function of CD4+FoxP3+ Tregs in preventing inflammation within the CNS is controversial. In myelin-specific CD4+TCR transgenic mouse models, FoxP3+ regulatory T cells prevent spontaneous EAE by suppressing the activation of myelin-specific CD4+ T cells in the periphery. Furthermore, a correlation between the presence of IL-10-producing FoxP3+ Treg cells in the CNS and disease recovery has been reported (McGeachy et al., 2005). However, Tregs seem ineffective in suppressing effector T cells in the CNS until the local levels of IL-6 and TNF-alpha decrease (for a review, see Goverman, 2009).

Regulatory CD8+ T cells have also been reported to be present in EAE and patients with MS (for a review, see Zozulya and Wiendl, 2008). Such regulatory CD8+ T cells include naturally occurring CD8+CD122+ T cells that inhibit CD8+ effector T cells by secreting IL-10, and induced regulatory CD8+ T cells that are thought to function in the periphery. CD8+ T cells were detected in patients with MS that lysed CD4+ myelin-specific T cells and, importantly, their numbers were decreased during exacerbation of the disease. Since there is also evidence that human patients with multiple sclerosis have a reduced frequency or functionality of CD4+ CD25+ regulatory T cells (Viglietta et al., 2004), strategies for enhancing CD8+ as well as CD4+ regulatory T cells in vivo may be beneficial of the treatment of patients with MS. The present invention discloses methods that support the enhancement of Treg activity in combination with self-antigen vaccination.

Several attempts have been made to apply self-antigen-specific immunotherapy in MS patients including DNA vaccines encoding myelin proteins (e.g., Garren et al., 2008), myelin proteins and myelin protein-derived peptides (for a review, see Steinman and Zamvil, 2010). Key autoantigens in MS include myelin basic protein (MBP), proteolipid protein (PLP) and myelin oligodendrocyte glycoprotein (MOG). In clinical trials, oral application of myelin proteins (Faria and Weiner, 2005) and intravenous application of MBP (Warren et al., 2006) showed no or only modest effects on MS disease course. However, a recent clinical trial in 30 relapsing-remitting MS patients with transdermally applied peptides derived from three myelin proteins (MBP. PLP, MOG) induced immune tolerogenic effects (Jurynczyk et al., 2010). The transdermally applied peptides activated dendritic Langerhans cells in the skin at the site of immunization, induced a unique population of granular dendritic cells in local lymph nodes, and generated type 1, IL-10-producing Tregs in the periphery. Repeated transdermal application of these peptides (weekly in the first month and then monthly for the remainder of the year) resulted in suppression of specific autoreactive proliferative responses and suppression of interferon-gamma and TGF-beta production.

A random copolymer of alanine, lysine, glutamic acid and tyrosine (glatiramer acetate, known also as Copaxone or Copolymer 1) has also been shown to slow the progression of disability and to reduce relapse rate (for reviews, see Arnon and Aharoni, 2004; Arnon, 1996). The mode of action of glatiramer is by strong promiscuous binding to MHC molecules and consequent competition with various myelin antigens for their presentation to T cells. A further aspect of its action is potent induction of specific suppressor cells of the Th2 type that migrate to the brain and lead to in situ bystander suppression. Furthermore, the glatiramer-specific cells in the brain express the anti-inflammatory cytokines IL-10 and TGF-beta, whereas they do not express IFN-gamma (for a review, see Arnon and Aharoni, 2004). Glatiramer acetate is an approved drug for the treatment of MS.

Application of fusion proteins containing GM-CSF coupled to myelin auto-antigens for immunotherapy of EAE in mice (Abbott et al., 2011) have demonstrated that auto-antigen-based vaccination approaches can be optimized by combining auto-antigen presentation with immune response-modulating agents. GM-CSF is recognized as a potent regulatory cytokine able to ameliorate disease in several mouse models of autoimmunity. For example, GM-CSF drives differentiation of dendritic cells (DCs), including tolerogenic DC subsets that in turn facilitate Treg differentiation and antigen-specific tolerance. In mice with EAE, a fusion protein of GM-CSF and the encephalitogenic MOG35-55 peptide facilitated tolerance rather than immunity to dominant self-epitopes of myelin (Abbott et al., 2011). In patients, however, therapeutic applications of GM-CSF can be associated with serious side effects such as the vascular leak syndrome. Therefore, alternative approaches are needed that employ more suitable enhancers of self-antigen-specific immunotherapy. The present invention discloses methods that are capable to reconstitute regulatory responses in combination with auto-antigen-specific immunotherapy.

In one embodiment, the present invention provides methods for locally restricted adjuvant therapy at the site of auto-antigen presentation that is based on four concepts, each of which addresses the complex network of Treg induction at a different target site. Most important appears to be the combination of local administration of vitamin D3 or analogs thereof and local inhibition of TNFR1 and TNFR1-mediated pathways. However, local inhibition of complement activation, in particular local inhibition of C3a-C3aR and C5a-C5aR interactions, and local inhibition of IL-4-mediated functions at the site of auto-antigen presentation represent also useful supportive Treg-inducing strategies for the treatment of MS. The combination of two or more of these Treg-inducing strategies (including the combination of two or more Treg-modulating therapeutics suitable for a particular Treg-inducing strategy) along with auto-antigen stimulation of T cells has the potential to improve the pathological processes in rheumatoid arthritis.

### IV.5.1. Local enhancement of vitamin D-mediated effects as adjuvant therapy for MS

In one preferred embodiment, local enhancement of vitamin D-mediated effects at the site of auto-antigen presentation is an essential part of the supportive Treg-inducing therapy along with auto-antigen-specific immunotherapy for the treatment of MS according to the method of the present invention.

More than 30 years ago an inverse relationship between exposure to ultraviolet light and the incidence of multiple sclerosis (MS) has been noted. A recent study has provided convincing evidence that overall there is a significant association between latitude and age standardized MS prevalence globally (Simpson Jr et al., 2011). However, the latitude gradient applied only to regions of largely European descent which have a relatively high frequency of risk alleles including the MS associated DR2 haplotype. The DR2 haplotype contains a highly conserved vitamin D responsive element which is functionally important since expression of the multiple sclerosis-associated MHC class II allele HLA-DRB1*1501 has been found to be regulated by vitamin D (Ramagopalan et al., 2009).

The role of vitamin D in experimental autoimmune encephalomyelitis (EAE) has been investigated in several studies (for a review, see Fletcher et al., 2012). The data indicate that the role of vitamin D is complex and point to a dual role of vitamin D at different concentrations in vivo. A basic level appears to be necessary for normal immune activation, whereas administration of higher levels of vitamin D can exert immunomodulatory functions. For example, prophylactic feeding of mice with relatively high doses of 1,25-(OH)₂D3 has been demonstrated to suppress the development of EAE (for a review, see Plum and DeLuca, 2010). However, high doses of 1,25-(OH)₂D3 are associated with adverse side effects and the required dose of 1,25(OH)₂D3 for complete prevention of EAE has been shown to induce hypercalcemia.

It should be noted, however, that in contrast to systemic administrations of vitamin D3 via feeding, the present invention allows application of lower doses to achieve suppressive immunomodulatory functions. According to the method of the present invention, vitamin D3 or analogs thereof are embedded in a hydrogel together with the auto-antigen. Upon release from the hydrogel, even lower doses of vitamin D3 generate relatively high local concentration of vitamin D3 or analogs in close proximity of the auto-antigen during presentation by APCs to T cells. Upon diffusion away from the site of auto-antigen presentation, the concentration of vitamin D3 or analogs thereof decreases rapidly, thereby minimizing potential side effects.

Recently, 1,25-(OH)₂D3 has been shown to affect the CD46 pathway in human T cells and, thereby, to promote anti-inflammatory responses mediated by CD46 (Kickler et al., 2012). CD46 is a type 1 transmembrane regulator of complement activity that binds to C3b and C4b, allowing their cleavage by factor I. Moreover, CD46 is key in the regulation of the adaptive immune response (for a review, see Ni Choileain and Astier, 2012). Importantly, CD46 costimulation promotes Tr1-like Treg differentiation, characterized by secretion of large amounts of IL-10 and low levels of IFN-γ (Cardone et al., 2010). In T cells from patients with MS, CD46 activation has been demonstrated to be defective as evidenced by an impaired IL-10 production by CD46-activated T cells from these patients (Astier et al., 2006; Martinez-Forero et al., 2008). Since IL-10 is capable of inhibiting synthesis of pro-inflammatory cytokines such as IFN-γ, IL-2, IL-3, TNF-α and GMCSF, and to suppress the antigen-presentation capacity of antigen presenting cells, it is important that by the addition of 1,25-(OH)₂D3 to CD4+ T cells from patients with MS in the relapsing-remitting stage, a normal IL-10/IFN-γ ratio could be restored (Kickler et al., 2012).

Until 2012 seven vitamin D intervention studies have been performed in MS patients, six of them being performed with inactive 25-OH-D3 and one with active 1,25-(OH)₂D3 (for a review, see Fletcher et al., 2012). Some of these explorative trials did show a trend to improvement, but significant clinical effects were not reported. For example, after oral administration of 4,000-40,000 IU/day of 25-OH-D3 for 28 weeks, a significant decrease in the number of new lesions as assessed by magnetic resonance imaging (MRI) was observed, but disease progression and relapse activity were not affected (Kimball et al., 2007). As mentioned above, however, this outcome is likely to be a result of the limited dose of vitamin D3 to avoid adverse side effects such as hypercalcemia.

Despite the limited clinical efficacy of published vitamin D intervention studies, the studies indicate that systemic vitamin D therapy is well tolerated since the trials (most of which administered doses of 1,000 to 40,000 IU/day of 25-OH-D3) recorded no adverse effects (for a review, see Fletcher et al., 2012). Only the study performed with active 1,25-(OH)₂D3 reported hypercalcaemia in those individuals who did not adhere to the recommended dietary calcium restriction of 2.5 µg/day (Wingerchuk et al., 2005).

Based on the data of above listed studies, local administration of vitamin D3 or analogues thereof at the site of auto-antigen presentation has the potential to support the efficacy of auto-antigen-specific immunotherapy for the treatment of MS. Preferred low molecular weight molecules include but are not limited to 25-OH-D3, its biologically active metabolite 1α,25-dihydroxyvitamin D3 (1,25-(OH)₂D3), analogues of vitamin D3 and non-secosteroidal vitamin D receptor (VDR) modulators (for reviews, see Plum and DeLuca, 2010; Fletcher et al., 2012).

In a preferred specific embodiment, the vitamin D3 analog calcipotriol is used for the method of the present invention. Since topical calcipotriol has been used clinically for many years in the treatment of psoriasis without systemic toxicity (Kragballe, 1995) and has been demonstrated to effectively induce Tregs upon topical application, calcipotriol represents a preferred agent for the induction of Tregs at the site of auto-antigen presentation according to the method of the present invention.

### IV.5.2. Local inhibition of TNFR1-mediated effects as adjuvant therapy for MS

In another preferred embodiment, local inhibition of TNFR1 or TNFR1-mediated pathways at the site of auto-antigen presentation is also an essential part of the supportive Treg-inducing therapy along with auto-antigen-specific immunotherapy for the treatment of MS according to the method of the present invention.

Although TNF-alpha is involved in the initiation of MS, its presence in later stages is essential for resolving the inflammation and initiating repair. Mice deficient in TNF-alpha (KO or treated with neutralizing antibody) develop EAE, whereas TNF-alpha administration has been demonstrated to provide therapeutic effects (Kassiotis and Kollias, 2001). Also, TNF-alpha neutralization in patients with MS exacerbated the disease (The Lenercept Multiple Sclerosis Study Group and The University of British Columbia MS/MRI Analysis Group; 1999).

Of interest for therapeutic design is that the two opposing functions of TNF-alpha diverge at the level of TNF receptors. In the murine EAE model, TNFR1 was found to be responsible for the detrimental signals, whereas TNFR2 was found to be essential for resolving inflammation and initiating repair (Kassiotis and Kollias, 2001). Accordingly, EAE was exacerbated in TNFR2-KO mice and reduced in TNFR1-KO mice (Suvannavejh et al., 2000). Interestingly, mutant mice expressing uncleavable transmembrane TNF-alpha (mTNF-alpha) were protected against autoimmunity, indicating that sTNF-alpha/TNR1 interactions are responsible for the pathology (Alexopoulou et al., 2006). In agreement with these data, specific inhibition of TNFR1 by a pegylated mutant TNF molecule (PEG-R1antTNF) was shown to ameliorate the clinical symptoms in the EAE model (Nomura et al., 2011).

Evidence that the beneficial immune-regulatory function of TNF-alpha/TNFR2 interaction results from its capacity to boost Treg activity (Chen et al., 2007) is provided by the observation that spontaneous disease remission in EAE after high Treg expansion can be abolished by depleting Tregs (McGeachy et al., 2005). Accordingly, TNF-alpha/TNFR2 interactions are also essential for the recently described boost of the expansion of CD4+CD25+FoxP3+ Tregs and their suppressive function by CD4+ effector T cells (Teff) after antigen-specific activation (Grinberg-Bleyer et al., 2010). Although this helper function of CD4+ T cells was observed in a murine model of type 1 diabetes, a Teff-mediated boost of Tregs most likely occurs also in other autoimmune diseases including MS and EAE. Furthermore, the dynamic interaction between Teffs and Tregs may explain the relapsing/remitting status of autoimmune diseases such as MS and EAE. During EAE Teffs first colonize the CNS, where they extensively proliferate during the acute phase of the disease. This is followed by a secondary active Treg proliferation and the disease remission, due to the presence of Tregs (McGeachy et al., 2005; Korn et al., 2007; O'Connor et al., 2007). Possibly, the primary Teff activation has promoted a Teff-mediated Treg boost, favoring remission. Then, because of the low level of Teff activation during remission, the Teff-mediated Treg boost is weakened, leading to lower Treg suppressive activity, opening a window for disease relapse when a new wave of Teffs colonizes the target tissue (Grinberg-Bleyer et al., 2010).

Specific local inhibition of TNFR1 represents a promising approach for supporting the generation of regulatory T cells in the periphery at the site of self-antigen presentation, since the resulting enhancement of TNFR2-mediated functions promotes Treg expansion (for reviews, see Chen and Oppenheim, 2010; Biton et al., 2012). The sustained presence and close proximity of TNFR1 inhibitors and auto-antigens creates a favorable environment for the expansion of Tregs and their suppressive function which in turn will decrease the number of activated myelin-specific T cells capable of gaining access to the CNS. Therefore, specific inhibition of TNFR1 at the site of auto-antigen presentation allows more effective treatment strategies for MS in which mTNF/TNFR2 signaling is beneficial.

Preferred low to moderate molecular weight inhibitors of TNFR1 or TNFR1-mediated functions are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of auto-antigen presentation, and d) have a relatively short plasma half-life to reduce potential adverse effects mediated by interaction of the inhibitor with targets away from the site of auto-antigen presentation.

In one specific embodiment, TNFR1-selective antagonistic TNF mutants are used for the method of the present invention. Preferred TNF mutants include but are not limited to R1antTNF developed by Shibata et al. (2008). In a preferred specific embodiment, TNFR1-specific antisense oligonucleotides are used for the method of the present invention. To allow for efficient cellular uptake of such oligonucleotides, thermosensitive hydrogels doped with cationic liposome-embedded TNFR1-specific antisense oligonucleotides are used. In a more preferred specific embodiment, S-methylglutathione (GSM) is used for the method of the present invention. GSM provides an attractive alternative to NAC, since a short incubation of isolated cardiomyocytes with GSM under in vitro conditions has been demonstrated to reproduce the effects of in vivo NAC treatment (Cailleret et al., 2004). In a most preferred specific embodiment, ASA and related salicylates are used for the method of the present invention. ASA and related salicylates allow selective inhibition of TNFR1-mediated activation of NF-kappa B and effective inhibition of IL-4 gene transcription. Although inhibition of TNFR1-mediated activation of NF-kappa B requires high doses of salicylates in the range of 1-2 x 10⁻² M (Thommesen and Laegreid, 2005), the risk of potential toxic side effects is minimized by application of matrices for sustained local delivery of ASA and related salicylates. Thereby, high local salicylate concentrations can be achieved at the site of allergen presentation, while upon diffusion away from the delivery site the salicylate concentration is rapidly decreased to systemically tolerable levels.

### IV.5.3. Local inhibition of complement-mediated effects as adjuvant therapy for MS

In another preferred embodiment, local inhibition of complement activation at the site of auto-antigen presentation is an optional part of the supportive Treg-inducing therapy along with auto-antigen-specific immunotherapy for the treatment of MS according to the method of the present invention.

Using a murine model of experimental allergic encephalomyelitis (T cell-mediated autoimmunity that mimics aspects of human multiple sclerosis), DAF^{-/-} mice developed more severe paralysis (associated with enhanced IL-17 production) upon immunization with myelin oligodendrocyte glycoprotein than wild-type mice (Liu et al., 2008). The stronger auto-reactive T cell response in DAF^{-/-} mice is C3aR-and C5aR-dependent since mice deficient in either or both of these receptors develop weaker auto-reactive T cell responses and are resistant to the development of experimental allergic encephalomyelitis, regardless of DAF expression (Strainic et al., 2008).

As mentioned earlier, animal studies have also provided strong evidence that complement component C3 is required for priming and expansion of CD8+ T cells which play an important role in MS. Furthermore, priming of CD8+ T cells requires help from CD4+ T cells, and local complement has been shown to regulate the help of CD4+ cells for CD8+ T cells required for murine allograft rejection (Vieyra et al., 2011). Cognate interactions between CD4+ T cells and dendritic cells (DCs) induce C3a and C5a production and CD4-cell help is mediated, in part, by interaction of DC-derived C3a/C5a with CD8+ T cell-expressed C3aR/C5aR (Vieyra et al., 2011).

Based on these studies, inhibition of locally synthesized C3 and/or inhibition of the interaction of locally generated C3a and C5a with their respective receptors are likely to support the efficacy of auto-antigen-specific immunotherapy. Preferred low molecular weight inhibitors of complement for the adjuvant treatment of allergy are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects.

In a preferred specific embodiment of the invention, low molecular weight agents capable of inhibiting locally synthesized complement component C3 are used for the method of the present invention. One preferred low molecular weight inhibitor targeting complement component C3 is the 13-residue cyclic peptide (H-I[CVVQDWGHHRC]T-NH₂) which is able to bind selectively to primate C3 and its C3b and C3c fragments, and to inhibit cleavage of C3 by C3 convertases of both the classical and the alternative pathway (Sahu et al., 1996; Ricklin and Lambris, 2008). Other preferred low molecular weight inhibitors targeting complement component C3 are derivatives of the 13-residue cyclic peptide (H-I[CVVQDWGHHRC]T-NH₂) as described by Morikis et al. (1998) and Katragadda et al. (2006).

In another preferred specific embodiment of the invention, low molecular weight agents capable of inhibiting the interaction of C3a and C5a with their respective receptors are used for the method of the present invention. Preferred low molecular weight C3aR antagonists include but are not limited to those with scaffolds featuring a) an arginine moiety (arginine derivatives) or b) an amino-piperidine linker and a pyridine moiety (amino-piperidine derivatives). Arginine derivatives include but are not limited to N²-[(2,2-dipheneylethoxy)acetyl]-L-arginine (SB290157; Ames et al., 2001), and those described by Denonne et al. (2007a). Amino-piperidine derivatives include but are not limited to those described by Denonne et al. (2007b). Preferred low molecular weight C5aR antagonists include but are not limited to peptidomimetic C5aR antagonists including C089, PMX53, PMX205 and JPE1357. PMX53, PMX205 and JPE1375 are analogs of the linear hexapeptide C089 (NMeFKPdChaWdR) which was derived from the C-terminus of C5a.

### IV.5.4. Local inhibition of IL-4/IL-13-mediated effects as adjuvant therapy for MS

In another preferred embodiment, local inhibition of IL-4-mediated effects at the site of auto-antigen presentation is also an optional part of the supportive Treg-inducing therapy along with auto-antigen-specific immunotherapy for the treatment of MS according to the method of the present invention.

First, local inhibition of IL-4-mediated functions is likely to provide benefits for MS patients, since IL-4 inhibits the expression of FOXP3, which is a requirement for inducible Treg (iTreg) differentiation.

Second, local inhibition of IL-4-mediated effects will contribute to the reduction of activated CD8+ cytotoxic T cells by local inhibition of complement at the site of auto-antigen presentation, since IL-4 has been shown to be crucial for the priming of antigen-specific CD8+ T cells after vaccination with the Leishmania antigen HASPB-1 (Stäger et al., 2003). Immune complexes formed by reaction of natural antibodies with the Leishmania antigen initiate activation of complement, which in turn stimulates primary IL-4 secretion from CD11b+ CD11c+ phagocytes. In the presence of specific antibodies to ovalbumin (OVA), OVA injection induced IL-4 secretion comparable to that induced by the Leishmania antigen. Therefore, the authors concluded that immune complexes can, in general, provide a means of stimulating primary IL-4 secretion from CD11b+ CD11c+ phagocytes. Complement activation is essential for this process since in C1q-deficient mice neither IL-4 secretion nor priming of antigen-specific CD8+ T cell could be observed (Stäger et al., 2003).

In MS patients undergoing vaccination therapy, circulating auto-antibodies will also form immune complexes with injected auto-antigens. Therefore, local inhibition of IL-4-mediated effects in combination with local inhibition of complement represents a promising approach to reduce priming of harmful auto-reactive CD8+ cytotoxic T cells and, thereby, to support the efficacy of auto-antigen-specific immunotherapy for the treatment of MS.

Preferred low to moderate molecular weight inhibitors of IL-4 are those which a) provide high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of auto-antigen presentation, and d) have a relatively short plasma half-life to reduce potential adverse effects mediated by interaction of the inhibitor with targets away from the site of auto-antigen presentation. In a preferred specific embodiment of the invention, interleukin variants capable of antagonizing the activity of IL-4 such as the double mutant of human IL-4 (R121D/Y124D) are used as inhibitors.

### V. COMPOSITIONS FOR THE TREATMENT OF ALLERGIC AND AUTOIMMUNE DISEASES

In one embodiment, the present invention discloses compositions for the treatment of allergic and autoimmune diseases suitable for one-step and two-step immunotherapeutic procedures.

### V.1. COMPOSITIONS FOR A ONE-STEP ADMINISTRATION PROCEDURE

In one embodiment, the present invention discloses compositions for a one-step administration procedure comprising two or more low molecular weight immune modulators selected from those listed in section I, one or more antigens or allergens, optionally one or more adjuvants selected from those listed in section III, and one or more matrices selected from those listed in section II, suitable for mediating sustained delivery of the components.

In one preferred specific embodiment, a biodegradable thermogelling polymer solution is used for sustained delivery of the components. The quantity of each component in the biodegradable thermogelling polymer is balanced in a way that a) upon injection into the body the polymer forms a non-flowing gel in which the other components are embedded, and b) upon gelation of the polymer composit at body temperature the amount of released components is sufficient for the therapeutic aims of the method of the present invention.

In another preferred specific embodiment, aluminum-containing adjuvants, typically aluminum phosphate or aluminum hydroxide gels (generally referred to as alum) are used for prolonged allergen or antigen presentation at the site of the gelled polymer composit.

### V.2. COMPOSITIONS FOR A TWO-STEP ADMINISTRATION PROCEDURE

In another embodiment, the present invention discloses compositions for a two-step administration procedure including administration of composition A comprising one or more allergens or antigens, and optionally one or more adjuvants selected from those listed in section III, followed by administration of composition B at the site of administered composition A, comprising two or more low molecular weight immune modulators selected from those listed in section I, and a matrix selected from those listed in section II, suitable for mediating sustained delivery of the components.

In one preferred specific embodiment, composition A comprises aluminum-containing adjuvants, typically aluminum phosphate or aluminum hydroxide gels (generally referred to as alum) for prolonged allergen or antigen presentation.

In another preferred specific embodiment, composition B comprises a biodegradable thermogelling polymer solution for sustained delivery of the low molecular weight immune modulators. The quantity of each immune modulator in the biodegradable thermogelling polymer is balanced in a way that a) upon injection into the body the polymer forms a non-flowing ge1 in which the other components are embedded, and b) upon gelation of the polymer composit at body temperature the amount of released immune modulators is sufficient for the therapeutic aims of the method of the present invention.

### V.3. SPECIFIC COMBINATIONS OF IMMUNE MODULATORS

In another embodiment, the present invention discloses combinations of two or more low to moderate weight immune modulators selected from those listed in section I, in compositions suitable for the treatment of individual diseases in combination with allergen- or auto-antigen-specific immunotherapy including allergy, allergic asthma, type 1 diabetes, rheumatoid arthritis and multiple sclerosis.

### V.3.1. Combinations for adjuvant treatment of allergy

*Combinations of two classes of immune modulators.* In one preferred specific embodiment, combinations of low to moderate weight immune modulators for adjuvant therapy of patients with allergy comprise regulatory T cell-modulating therapeutics capable of addressing the network of Treg induction at two different target sites. Preferred combinations comprise at least one immune modulator selected from inhibitors of IL-4/IL-13-mediated effects listed in section I.4., and at least one immune modulator selected from inhibitors of complement listed in section I.1. Preferred inhibitors of IL-4/IL-13-mediated effects include antagonistic interleukin variants listed in section I.4.2. Most preferred inhibitors of IL-4/IL-13-mediated effects include the human IL-4 double mutant R121D/Y124D. Preferred inhibitors of complement include cyclic peptide-based C3 inhibitors listed in section I.1.1., low molecular weight C3aR antagonists listed in section I.1.2., low molecular weight C5aR antagonists listed in section I.1.3., or combinations of such inhibitors of complement. Most preferred inhibitors of complement include the cyclic peptide (H-I[CVVQDWGHHRC]T-NH₂) for C3 inhibition, N²-[(2,2-dipheneylethoxy)acetyl]-L-arginine (SB290157) for C3aR inhibition, and cyclic Ac-Phe-[Orn-Pro-DCha-Trp-Arg] (PMX53) for C5aR inhibition.

*Combinations of three classes of immune modulators.* In a more preferred specific embodiment, combinations of low to moderate weight immune modulators for adjuvant therapy of patients with allergy comprise regulatory T cell-modulating therapeutics capable of addressing the network of Treg induction at three different target sites. Preferred combinations comprise at least one immune modulator selected from inhibitors of IL-4/IL-13-mediated effects listed in section I.4., at least one immune modulator selected from inhibitors of complement listed in section I.1., and at least one immune modulator selected from vitamin D3 molecules and analogs thereof listed in section I.2. Preferred inhibitors of IL-4/IL-13-mediated effects and preferred inhibitors of complement are those defined for combinations of two classes of immune modulators. Preferred vitamin D3 molecules or analogs thereof are selected from those listed in sections II.1. and II.2., respectively. Most preferred are the active vitamin D3 metabolite 1,25-(OH)₂D3 (calcitriol) and the vitamin D3 analog calcipotriol.

*Combinations of four classes of immune modulators.* In another specific embodiment, combinations of low to moderate weight immune modulators for adjuvant therapy of patients with allergy comprise regulatory T cell-modulating therapeutics capable of addressing the network of Treg induction at four different target sites. Such combinations comprise at least one immune modulator selected from inhibitors of IL-4/IL-13-mediated effects listed in section I.4., at least one immune modulator selected from inhibitors of complement listed in section I.1., at least one immune modulator selected from vitamin D3 or analogs thereof listed in section I.2., and at least one immune modulator selected from inhibitors of TNFR1 or TNFR1-mediated pathways listed in section I.3. Preferred inhibitors of IL-4/IL-13-mediated effects and preferred inhibitors of complement are those defined for combinations of two classes of immune modulators. Preferred vitamin D3 molecules or analogs thereof are those defined for combinations of three classes of immune modulators. Preferred inhibitors of TNFR1-mediated functions include glutathione derivatives listed in section I.3.1., salicylates listed in section I.3.2., antisense oligonucleotides with specificity for TNFR1 listed in section I.3.3., and TNFR1-selective antagonistic TNF mutants listed in section I.3.4. Most preferred inhibitors of TNFR1-mediated functions include S-methylglutathione (GSM), salicylic acid, and antisense oligonucleotides with specificity for TNFR1.

### V.3.2. Combinations for adjuvant treatment of allergic asthma

*Combinations of two classes of immune modulators.* In one preferred specific embodiment, combinations of low to moderate weight immune modulators for adjuvant therapy of patients with allergic asthma comprise regulatory T cell-modulating therapeutics capable of addressing the network of Treg induction at two different target sites. Preferred combinations comprise at least one immune modulator selected from inhibitors of IL-4/IL-13-mediated effects listed in section I.4., and at least one immune modulator selected from inhibitors of complement listed in section I.1. Preferred inhibitors of IL-4/IL-13-mediated effects include antagonistic interleukin variants listed in section I.4.2. Most preferred inhibitors of IL-4/IL-13-mediated effects include the human IL-4 double mutant R121D/Y124D. Preferred inhibitors of complement include cyclic peptide-based C3 inhibitors listed in section I.1.1., low molecular weight C3aR antagonists listed in section I.1.2., low molecular weight C5aR antagonists listed in section I.1.3., or combinations of such inhibitors of complement. Most preferred inhibitors of complement include the cyclic peptide (H-I[CVVQDWGHHRC]T-NH₂) for C3 inhibition, N²-[(2,2-dipheneylethoxy)acetyl]-L-arginine (SB290157) for C3aR inhibition, and cyclic Ac-Phe-[Orn-Pro-DCha-Trp-Arg] (PMX53) for C5aR inhibition.

*Combinations of three classes of immune modulators.* In a more preferred specific embodiment, combinations of low to moderate weight immune modulators for adjuvant therapy of patients with allergic asthma comprise regulatory T cell-modulating therapeutics capable of addressing the network of Treg induction at three different target sites. Preferred combinations comprise at least one immune modulator selected from inhibitors of IL-4/IL-13-mediated effects listed in section I.4., at least one immune modulator selected from inhibitors of complement listed in section I.1., and at least one immune modulator selected from vitamin D3 molecules and analogs thereof listed in section I.2. Preferred inhibitors of IL-4/IL-13-mediated effects and preferred inhibitors of complement are those defined for combinations of two classes of immune modulators. Preferred vitamin D3 molecules or analogs thereof are selected from those listed in sections II.1. and II.2., respectively. Most preferred are the active vitamin D3 metabolite 1,25-(OH)₂D3 (calcitriol) and the vitamin D3 analog calcipotriol.

*Combinations of four classes of immune modulators.* In another specific embodiment, combinations of low to moderate weight immune modulators for adjuvant therapy of patients with allergic asthma comprise regulatory T cell-modulating therapeutics capable of addressing the network of Treg induction at four different target sites. Such combinations comprise at least one immune modulator selected from inhibitors of IL-4/IL-13-mediated effects listed in section I.4., at least one immune modulator selected from inhibitors of complement listed in section I.1., at least one immune modulator selected from vitamin D3 molecules or analogs thereof listed in section I.2., and at least one immune modulator selected from inhibitors of TNFR1 or TNFR1-mediated pathways listed in section I.3. Preferred inhibitors of IL-4/IL-13-mediated effects and preferred inhibitors of complement are those defined for combinations of two classes of immune modulators. Preferred vitamin D3 molecules or analogs thereof are those defined for combinations of three classes of immune modulators. Preferred inhibitors of TNFR1-mediated functions include glutathione derivatives listed in section I.3.1., salicylates listed in section I.3.2., antisense oligonucleotides with specificity for TNFR1 listed in section I.3.3., and TNFR1-selective antagonistic TNF mutants listed in section I.3.4. Most preferred inhibitors of TNFR1-mediated functions include S-methylglutathione (GSM), salicylic acid, and antisense oligonucleotides with specificity for TNFR1.

### V.3.3. Combinations for adjuvant treatment of type 1 diabetes

*Combinations of two classes of immune modulators.* In one preferred specific embodiment, combinations of low to moderate weight immune modulators for adjuvant therapy of patients with type I diabetes comprise regulatory T cell-modulating therapeutics capable of addressing the network of Treg induction at two different target sites. Preferred combinations comprise at least one immune modulator selected from inhibitors of complement listed in section I.1., and at least one immune modulator selected from vitamin D3 molecules and analogs thereof listed in section I.2. Preferred inhibitors of complement include cyclic peptide-based C3 inhibitors listed in section I.1.1., low molecular weight C3aR antagonists listed in section I.1.2., low molecular weight C5aR antagonists listed in section I.1.3., or combinations of such inhibitors of complement. Most preferred inhibitors of complement include the cyclic peptide (H-I[CVVQDWGHHRC]T-NH₂) for C3 inhibition, N²-[(2,2-dipheneylethoxy)acetyl]-L-arginine (SB290157) for C3aR inhibition, and cyclic Ac-Phe-[Orn-Pro-DCha-Trp-Arg] (PMX53) for C5aR inhibition. Preferred vitamin D3 molecules or analogs thereof are selected from those listed in sections II.1. and II.2., respectively. Most preferred are the active vitamin D3 metabolite 1,25-(OH)₂D3 (calcitriol) and the vitamin D3 analog calcipotriol.

*Combinations of three classes of immune modulators.* In a more preferred specific embodiment, combinations of low to moderate weight immune modulators for adjuvant therapy of patients with type 1 diabetes comprise regulatory T cell-modulating therapeutics capable of addressing the network of Treg induction at three different target sites. Preferred combinations comprise at least one immune modulator selected from inhibitors of complement listed in section I.1., at least one immune modulator selected from vitamin D3 molecules and analogs thereof listed in section I.2., and at least one immune modulator selected from inhibitors of TNFR1 or TNFR1-mediated pathways listed in section I.3. Preferred inhibitors of complement and preferred vitamin D3 molecules or analogs thereof are those defined for combinations of two classes of immune modulators. Preferred inhibitors of TNFR1-mediated functions include glutathione derivatives listed in section I.3.1., salicylates listed in section I.3.2., antisense oligonucleotides with specificity for TNFR1 listed in section I.3.3., and TNFR1-selective antagonistic TNF mutants listed in section I.3.4. Most preferred inhibitors of TNFR1-mediated functions include S-methylglutathione (GSM), salicylic acid, and antisense oligonucleotides with specificity for TNFR1.

*Combinations of four classes of immune modulators.* In another specific embodiment, combinations of low to moderate weight immune modulators for adjuvant therapy of patients with type 1 diabetes comprise regulatory T cell-modulating therapeutics capable of addressing the network of Treg induction at four different target sites. Such combinations comprise at least one immune modulator selected from inhibitors of complement listed in section I.1., at least one immune modulator selected from vitamin D3 molecules or analogs thereof listed in section I.2., at least one immune modulator selected from inhibitors of TNFR1 or TNFR1-mediated pathways listed in section I.3., and at least one immune modulator selected from inhibitors of IL-4-mediated effects listed in section I.4. Preferred inhibitors of complement, preferred vitamin D3 molecules or analogs thereof, and preferred inhibitors of TNFR1-mediated functions are those defined for combinations of three classes of immune modulators. Preferred inhibitors of IL-4-mediated effects include antagonistic interleukin variants listed in section I.4.2. Most preferred inhibitors of IL-4-mediated effects include the human IL-4 double mutant R121D/Y124D.

### V.3.4. Combinations for adjuvant treatment of RA

*Combinations of two classes of immune modulators.* In one preferred specific embodiment, combinations of low to moderate weight immune modulators for adjuvant therapy of patients with rheumatoid arthritis (RA) comprise regulatory T cell-modulating therapeutics capable of addressing the network of Treg induction at two different target sites. Preferred combinations comprise at least one immune modulator selected from inhibitors of TNFR1 or TNFR1-mediated pathways listed in section I.3., and at least one immune modulator selected from vitamin D3 molecules and analogs thereof listed in section I.2. Preferred inhibitors of TNFR1-mediated functions include glutathione derivatives listed in section I.3.1., salicylates listed in section I.3.2., antisense oligonucleotides with specificity for TNFR1 listed in section I.3.3., and TNFR1-selective antagonistic TNF mutants listed in section I.3.4. Most preferred inhibitors of TNFR1-mediated functions include S-methylglutathione (GSM), salicylic acid, and antisense oligonucleotides with specificity for TNFR1. Preferred vitamin D3 molecules or analogs thereof are selected from those listed in sections II.1. and II.2., respectively. Most preferred are the active vitamin D3 metabolite 1,25-(OH)₂D3 (calcitriol) and the vitamin D3 analog calcipotriol.

*Combinations of three classes of immune modulators.* In a more preferred specific embodiment, combinations of low to moderate weight immune modulators for adjuvant therapy of patients with RA comprise regulatory T cell-modulating therapeutics capable of addressing the network of Treg induction at three different target sites. Preferred combinations comprise at least one immune modulator selected from inhibitors of TNFR1 or TNFR1-mediated pathways listed in section I.3., at least one immune modulator selected from vitamin D3 molecules and analogs thereof listed in section I.2., and at least one immune modulator selected from inhibitors of complement listed in section I.1. Preferred inhibitors of TNFR1 or TNFR1-mediated pathways and preferred vitamin D3 molecules or analogs thereof are those defined for combinations of two classes of immune modulators. Preferred inhibitors of complement include cyclic peptide-based C3 inhibitors listed in section I.1.1., low molecular weight C3aR antagonists listed in section I.1.2., low molecular weight C5aR antagonists listed in section I.1.3., or combinations of such inhibitors of complement. Most preferred inhibitors of complement include the cyclic peptide (H-I[CVVQDWGHHRC]T-NH2) for C3 inhibition, N²-[(2,2-dipheneylethoxy)acetyl]-L-arginine (SB290157) for C3aR inhibition, and cyclic Ac-Phe-[Orn-Pro-DCha-Trp-Arg] (PMX53) for C5aR inhibition.

*Combinations of four classes of immune modulators.* In another specific embodiment, combinations of low to moderate weight immune modulators for adjuvant therapy of patients with RA comprise regulatory T cell-modulating therapeutics capable of addressing the network of Treg induction at four different target sites. Such combinations comprise at least one immune modulator selected from inhibitors of TNFR1 or TNFR1-mediated pathways listed in section I.3., at least one immune modulator selected from vitamin D3 molecules or analogs thereof listed in section I.2., at least one immune modulator selected from inhibitors of complement listed in section I.1., and at least one immune modulator selected from inhibitors of IL-4-mediated effects listed in section I.4. Preferred inhibitors of TNFR1-mediated functions, preferred vitamin D3 molecules or analogs thereof, and preferred inhibitors of complement are those defined for combinations of three classes of immune modulators. Preferred inhibitors of IL-4-mediated effects include antagonistic interleukin variants listed in section I.4.2. Most preferred inhibitors of IL-4-mediated effects include the human IL-4 double mutant R121D/Y124D.

### V.3.5. Combinations for adjuvant treatment of MS

*Combinations of two classes of immune modulators.* In one preferred specific embodiment, combinations of low to moderate weight immune modulators for adjuvant therapy of patients with multiple sclerosis (MS) comprise regulatory T cell-modulating therapeutics capable of addressing the network of Treg induction at two different target sites. Preferred combinations comprise at least one immune modulator selected from vitamin D3 molecules and analogs thereof listed in section I.2. and at least one immune modulator selected from inhibitors of TNFR1 or TNFR1-mediated pathways listed in section I.3. Preferred vitamin D3 molecules or analogs thereof are selected from those listed in sections II.1. and II.2., respectively. Most preferred are the active vitamin D3 metabolite 1,25-(OH)₂D3 (calcitriol) and the vitamin D3 analog calcipotriol. Preferred inhibitors of TNFR1-mediated functions include glutathione derivatives listed in section I.3.1., salicylates listed in section I.3.2., antisense oligonucleotides with specificity for TNFR1 listed in section I.3.3., and TNFR1-selective antagonistic TNF mutants listed in section I.3.4. Most preferred inhibitors of TNFR1-mediated functions include S-methylglutathione (GSM), salicylic acid, and antisense oligonucleotides with specificity for TNFR1.

*Combinations of three classes of immune modulators.* In a more preferred specific embodiment, combinations of low to moderate weight immune modulators for adjuvant therapy of patients with MS comprise regulatory T cell-modulating therapeutics capable of addressing the network of Treg induction at three different target sites. Preferred combinations comprise at least one immune modulator selected from vitamin D3 molecules and analogs thereof listed in section I.2., at least one immune modulator selected from inhibitors of TNFR1 or TNFR1-mediated pathways listed in section I.3., and at least one immune modulator selected from inhibitors of complement listed in section I.1. Preferred vitamin D3 molecules or analogs thereof and preferred inhibitors of TNFR1 or TNFR1-mediated pathways are those defined for combinations of two classes of immune modulators. Preferred inhibitors of complement include cyclic peptide-based C3 inhibitors listed in section I.1.1., low molecular weight C3aR antagonists listed in section I.1.2., low molecular weight C5aR antagonists listed in section I.1.3., or combinations of such inhibitors of complement. Most preferred inhibitors of complement include the cyclic peptide (H-I[CVVQDWGHHRC]T-NH2) for C3 inhibition, N²-[(2,2-dipheneylethoxy)acetyl]-L-arginine (SB290157) for C3aR inhibition, and cyclic Ac-Phe-[Orn-Pro-DCha-Trp-Arg] (PMX53) for C5aR inhibition.

*Combinations of four classes of immune modulators.* In another specific embodiment, combinations of low to moderate weight immune modulators for adjuvant therapy of patients with MS comprise regulatory T cell-modulating therapeutics capable of addressing the network of Treg induction at four different target sites. Such combinations comprise at least one immune modulator selected from vitamin D3 molecules or analogs thereof listed in section I.2., at least one immune modulator selected from inhibitors of TNFR1 or TNFR1-mediated pathways listed in section I.3., at least one immune modulator selected from inhibitors of complement listed in section I.1., and at least one immune modulator selected from inhibitors of IL-4-mediated effects listed in section I.4. Preferred vitamin D3 molecules or analogs thereof, preferred inhibitors of TNFR1-mediated functions, and preferred inhibitors of complement are those defined for combinations of three classes of immune modulators. Preferred inhibitors of IL-4-mediated effects include antagonistic interleukin variants listed in section I.4.2. Most preferred inhibitors of IL-4-mediated effects include the human IL-4 double mutant R121D/Y124D.

### VI. PHARMACEUTICAL FORMULATIONS

In one embodiment, the therapeutic compositions of the present invention are incorporated into pharmaceutical compositions suitable for administration. Such compositions typically comprise the therapeutic compositions of the present invention and a pharmaceutically acceptable carrier. As used herein, a 'pharmaceutically acceptable carrier' is intended to include any and all solvents, dispersion media, coatings, antibacterial and antifungal agents, isotonic systems, and the like, compatible with the components of the therapeutic compositions of the present invention and pharmaceutical administration. The use of such media and agents for pharmaceutically active substances is well known in the art. Supplementary active compounds can also be incorporated into the composition.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents, antioxidants such as ascorbic acid or sodium bisulfite, chelating agents such as ethylenediaminetetraacetic acid, buffers such as acetates, citrates or phosphates and agents for the adjustment of toxicity such as sodium chloride or dextrose. The pH can be adjusted with acids or bases, such as hydrochloric acid or sodium hydroxide. In many cases, it will be preferable to include isotonic agents, for example, sugars, polyalcohols such as mannitol, sorbitol, sodium chloride in the composition. The composition should be fluid to the extent that easy syringability exists. The proper fluidity can be maintained, for example, by the use of a coating such as lecithin, by the maintenance of the required particle size in the case dispersion and by use of surfactants. The composition must be stable under the conditions of manufacture and storage and must be preserved against the contaminating action of microorganisms such as bacteria and fungi. Prevention of the action of microorganisms can be achieved by various antibacterial and antifungal agents such as parabens, chlorobutanol, phenol, ascorbic acid, thimoseral, and the like. In all cases, the composition must be sterile. Sterile injectable solutions can be prepared by filtered sterilization. The preparation can be enclosed in ampoules, disposable syringes or multiple dose vials made of glass or plastic. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof. For practical purposes it should be kept in mind that aluminum-adsorbed vaccines are frost sensitive and therefore not lyophilizable.

### VII. THERAPEUTIC METHODS

In one embodiment, the present invention discloses therapeutic methods including information about suitable therapeutically effective doses of low molecular weight immune modulators listed in section I., and modes of administration for the induction of allergen or auto-antigen tolerance using the compositions of the present invention.

### VII.1. THERAPEUTIC EFFECTIVE DOSES OF LOW MOLECULAR WEIGHT IMMUNE MODULATORS

Determination of a therapeutically effective dose is well within the capability of those skilled in the art. The therapeutically effective dose can be estimated initially in animal models, usually mice, rats, rabbits, dogs, pigs, or non-human primates. The animal model also can be used to determine the appropriate concentration range and route of administration. Such information can then be used to determine useful doses and routes for administration in humans.

### VII.1.1. Therapeutically effective doses of complement inhibitors

Low molecular weight inhibitors of complement protein C3 and the receptors of C3a and c5a have been studied in a variety of animal models in the recent past. Results obtained from these studies provide useful information for the application of these inhibitors in humans. Furthermore, some of these inhibitors have entered clinical trials.

### Inhibitors of complement protein C3

One preferred low molecular weight inhibitor targeting complement component C3 is the 13-residue cyclic peptide (H-I[CVVQDWGHHRC]T-NH₂) (termed compstatin) which is able to bind selectively to primate C3 and its C3b and C3c fragments, and to inhibit cleavage of C3 by C3 convertases of both the classical and the alternative pathway (Sahu et al., 1996; Ricklin and Lambris, 2008). compstatin exhibits exclusive specificity for primate C3 proteins and does not bind either to C3 proteins from lower mammalian species or to the structural C3 homologs C4 and C5 (Sahu et al., 2003). The concentration of compstatin causing 50% inhibition (IC₅₀) of the haemolytic activity (lysis of rabbit erythrocytes in serum by the alternative pathway) is 12 µM for human sera and 10 µM for sera from cynomolgus monkeys, whereas the IC₅₀ values for sera from mice, rats, and guinea pigs is >600 µM (Sahu et al., 2003).

In a recent animal study, administration of compstatin as a 10 mg/kg intravenous bolus injection followed by 60 µg/kg/min continuous infusion has been demonstrated to decrease the procoagulant response and to confer organ protection in a baboon model of *Escherichia coli* sepsis (Silasi-Mansat et al., 2010).

Recently, a phase I clinical study with the compstatin derivative POT-4 (Potentia Pharmaceuticals, Inc.) for the treatment of age-related macular degeneration has been completed successfully (Francois et al., 2009).

### Inhibitors of C5a-C5aR interactions

One preferred low molecular weight inhibitor targeting the C5a receptor (C5aR) is the peptidomimetic C5aR antagonist PMX53, a cyclic analog of the linear NMeFKPdChaWdR. PMX53 has a short half-life of 70 min and is susceptible to proteolytic cleavage (for a review, see Wagner and Frank, 2010), which is favorable for the method of the present invention.

The in vivo therapeutic efficacy of PMX53 has been demonstrated in several disease models (for reviews, see Qu et al, 2009; Monk et al., 2007). Relevant studies include antigen-induced monoarticular arthritis in rats (Woodruff et al., 2002), sepsis in mice (Huber-Lang et al., 2002), renal ischaemia/reperfusion injury in rats (Arumugam et al., 2003), hepatic ischaemia/reperfusion injury in rats (Arumugam et al., 2004), intestinal ischaemia/reperfusion injury in rats (Proctor et al., 2004), ruptured abdominal aortic aneurysm in rats (Harkin et al., 2004), inflammatory bowel disease in rats (Woodruff et al., 2005), lupus nephritis in mice (Bao et al., 2005b), 3-nitropropionic acid-induced Huntington's disease in rats (Woodruff et al., 2006), abdominal pain in mice and rats (Ting et al., 2008), and tumor growth in mice (Markiewski et al., 2008). The dosis of PMX53 in theses studies varied from 1-3 mg/kg/day p.o. in arthritis models, 1 mg/kg i.v and 10 mg/kg p.o. in reperfusion injury models, and up to 3 mg/kg i.v. in sepsis models (for a review, see Monk et al., 2007).

In humans, PMX53 has been shown to be safe and well tolerated in Phase 1 clinical studies for rheumatoid arthritis and psoriasis (Kohl, 2006).

### Inhibitors of C3a-C3aR interactions

One preferred low molecular weight inhibitor targeting the C3a receptor (C3aR) is the arginine derivative SB290157. SB290157 has a short in vivo half-life which is favorable for the method of the present invention.

The in vivo therapeutic efficacy of SB290157 has been demonstrated in several disease models including lung inflammation in guinea pigs (Ames et al., 2001), arthritis in rats (Ames et al., 2001), intestinal ischaemia/reperfusion injury in rats (Proctor et al., 2004), lupus nephritis in mice (Bao et al., 2005a), and allergic asthma in mice (Baelder et al., 2005). The dosis of SB290157 in theses studies varied from up to 30 mg/kg i.p. twice a day in the guinea pig neutrophilia model and in the adjuvant-induced arthritis model (Ames et al., 2001), and 0.1 to 1 mg/kg i.v. in the intestinal ischaemia/reperfusion injury model (Proctor et al., 2004). In the latter study, 10 mg/kg i.v. caused a rapid and transient hypertension. For treatment of allergic asthma in mice, 0.2 mg of SB290157 was administered intra nasal and 0.5 mg i.p. (Baelder et al., 2005).

### VII.1.2. Therapeutically effective doses of vitamin D3

Preferred vitamin D3 molecules include 25-OH-D3 (calcidiol), its biologically active metabolite 1,25-(OH)₂D3 (calcitriol), and vitamin D3 analog calcipotriol. These molecules have been studied in a variety of animal models and evaluated in clinical trials (for reviews, see Plum and DeLuca, 2010; Fletcher et al., 2012).

Current indications for clinical treatment with 25-OH-D3 or 1,25-(OH)₂D3 include renal osteodystrophy and osteoporosis. Toxicity occurs when serum levels of 25-OH-D3 rise to 500 ng/ml or above (Shephard and DeLuca, 1980). Based on experiments in the rat, serum levels of 25-OH-D3 should be kept below 250 ng/ml to avoid toxicity (Shephard and DeLuca, 1980).

The vitamin D3 analog calcipotriol has a very short half-life in circulation (Kragballe, 1995) and due to this property calcipotriol has been used clinically for more than 10 years for topical treatment of psoriasis without systemic toxicity (for a review, see Plum and DeLuca, 2010).

In animal studies, vitamin D molecules and analogs thereof have been used for the treatment of OVA-induced allergy in mice (Ghoreishi et al., 2009), OVA-induced allergic asthma in mice (Taher et al., 2008), insulin-dependent diabetes mellitus in NOD mice (Zella et al., 2003), Lyme arthritis and collagen-induced arthritis in mice (Cantorna et al., 1998), and experimental autoimmune encephalomyelitis (EAE) in mice (Branisteanu et al., 1995). In the allergy model, mice were treated on their shaved dorsal skin with 30 mg/day of calcipotriol ointment (50 µg/g) (Donovex, Leo Pharma) for three days followed by transcutaneous immunization with OVA in the presence of CpG adjuvant. This treatment abolished antigen-specific CD8+ T cell priming and induced CD4+CD5+ Tregs, thereby promoting antigen-specific tolerance (Ghoreishi et al., 2009). In the allergic asthma mode, OVA-sensitized mice were subjected to allergen-specific immunotherapy by three s.c. injections of 100 µg OVA in the presence of 10 ng 1,25-(OH)₂D3. This treatment significantly inhibited airway hyper-responsiveness and caused a significant reduction of serum OVA-specific IgE levels (Taher et al., 2008). In the murine model of type 1 diabetes, a diet containing 50 ng 1,25-(OH)₂D3/mouse/day was administered three times/week. This treatment prevented diabetes onset in NOD mice as of 200 days (Zella et al., 2003). In the arthritis models, mice received a daily diet supplemented with 20 ng 1,25-(OH)₂D3/mouse/day. This dose was found to be effective in inhibiting the progression of arthritis without producing hypercalcemia (Cantorna et al., 1998). In the EAE model, i.p. injection of 5 µg of 1,25-(OH)₂D3/kg body weight every 2 days prevented the appearance of paralysis in 70% of the treated mice (Branisteanu et al., 1995).

Evaluation of vitamin D supplementation in clinical trials support some observations made in animal models. It should be noted, however, that only low doses of vitamin D3 or analogs thereof were administered to avoid adverse side effects such as hypercalcemia.

A recent prospective randomized, double-blind study including 48 children from 5 to 18 years of age with newly diagnosed asthma (only sensitive to house dust mites) has demonstrated that after six months of treatment with the glucocorticoid budesonide (800 µg/d, administered as dry powder) and cholecalciferol (500 IU) a significantly lower number of children experienced asthma exacerbation (17%) as compared to the group receiving only glucocorticoids (46%) (Majak et al., 2011).

For the prevention of type 1 diabetes, vitamin D supplementation in infants with 10 µg/day (400 IU/d) does not appear to be sufficient, but doses of 50 µg/day (2000 IU/d) and higher may have a strong protective effect (for a review, see Harris, 2005). A prospective study of vitamin D supplementation in infants and type 1 diabetes including 12,055 pregnant women in Northern Finland showed that regular vitamin D supplements during infancy at doses of over 50 µg/day (2000 IU/d) reduced the relative risk of type 1 diabetes over the subsequent 30 years to 0.14, and at doses of exactly 50 µg/day to 0.22 (Hyppönen et al., 2001). Current U.S. recommendations are in the range of 5-25 µg/day (200-1000 IU/d).

In one open label study, 19 patients with rheumatoid arthritis on methotrexate therapy were treated with 2 µg/day of alfacalcidiol (1(OH)D3) for a period of 3 months. After three months of therapy, 89% of patients experienced an improvement of disease activity, with 45% (9/19) going into remission (Andjelkovic et al., 1999).

Until 2012 seven vitamin D intervention studies have been performed in patients with multiple sclerosis, six of them being performed with inactive 25-OH-D3 and one with active 1,25-(OH)₂D3 (for a review, see Fletcher et al., 2012). Some of these explorative trials did show a trend to improvement, but significant clinical effects were not reported. For example, after oral administration of 4,000-40,000 IU/day of 25-OH-D3 for 28 weeks, a significant decrease in the number of new lesions as assessed by magnetic resonance imaging (MRI) was observed, but disease progression and relapse activity were not affected (Kimball et al., 2007). However, despite the limited clinical efficacy of published vitamin D intervention studies, the studies indicate that systemic vitamin D therapy is well tolerated since the trials (most of which administered doses of 1,000 to 40,000 IU/day of 25-OH-D3) recorded no adverse effects (for a review, see Fletcher et al., 2012). Only the study performed with active 1,25-(OH)₂D3 reported hypercalcemia in those individuals who did not adhere to the recommended dietary restriction of 2.5 µg/day (Wingerchuk et al., 2005).

### VII.1.3. Therapeutically effective doses of IL-4/IL-13 inhibitors

Preferred inhibitors of IL-4/IL-13-mediated effects include the human IL-4 double mutant R121D/Y124D (Grunewald et al., 1998; Tony et al., 1994). This double mutant has been evaluated in clinical trials (Wenzel et al., 2007). For animal experiments, an analogous murine double mutant (Q116D/Y119D) has been developed capable of inhibiting IL-4/IL-13-mediated effects in mice.

In a murine model of allergy, the analogous double mutant (Q116D/Y119D) has been administered 2 hours pre- and post-OVA immunization as a 50 µg dose each. Thereafter, treatment was continued from day 1 to 8 with 30 µg double mutant Q116D/Y119D per injection twice a day. This treatment completely abrogated humoral immune responses to OVA and development of allergic symptoms (Grunewald et al., 1998).

In a murine model of OVA-induced allergic airway inflammation, immunotherapeutic treatment (SIT) of the mice was performed by intranasal administration of 10 µg of the double mutant (Q116D/Y119D) together with increasing amounts of OVA (1 µg-1 mg) every fourth day over a 3-week period. However, mice treated with the IL-4/IL-13 inhibitor during SIT did not produce significantly different results as compared to those treated with increasing amounts of OVA only (Gogishvili et al., 2006).

### VII.1.4. Therapeutically effective doses of TNFR1-inhibitors

Preferred low molecular weight inhibitors of TNFR1-mediated functions include N-acetyl-L-cysteine (NAC) and other glutathione (GSH) prodrugs, salicylates, and TNFR1-specific antisense oligo-nucleotides. Preferred moderate molecular weight inhibitors of TNFR1-mediated functions include TNFR1-selective antagonistic TNF mutants and dominant-negative sTNF mutants, which leave the mTNF-TNFR1 and mTNF-TNFR2 interactions intact (for a review, see Van Hauwermeiren et al., 2011). Such molecules have been studied in varies animal models and some of them have been evaluated in clinical studies.

### N-acetyl-L-cysteine (NAC) and other glutathione prodrugs

S-Methylglutathione (GSM; MW 321.35) is a glutathione donor and NAC (MW 163.20) a glutathione precursor. NAC is readily deacetylated in the body to form cysteine which efficiently supports glutathione (GSH) synthesis.

In cultures of peripheral blood T cells, 10 mM NAC proved to be an effective enhancer of T cell function and enhancer of growth (Eylar et al., 1993). Cytotoxicity studies with resting T cells from seven donors over a 3 day period in the absence and presence of NAC ranging from 1 mM (163.2 µg/ml) to 20 mM (3.26 mg/ml) revealed that NAC is nontoxic even at 20 mM (Eylar et al., 1993).

In isolated rat cardiomyocytes, exogenously added S-methylglutathione (GSM) has been demonstrated to reproduce the effects of in vivo NAC treatment (Cailleret et al., 2004). After preincubation for 2-3 hours in a medium containing 50 µM GSM, cardiomyocytes isolated from control rats were protected against the deleterious effects of TNF-alpha on contraction to the same extent as cardiomyocytes isolated from rats treated by two i.p. injections of 100 mg NAC, 48 and 24 hours before anesthesia. Cardiomyocytes isolated from control rats were not protected by exogenously added NAC under these experimental conditions, pointing to the necessity of a long-term preincubation (at least 2-3 days) in case of NAC administration and a short-term pre-incubation (at least 2-3 hours) in case of GSM administration.

Mice sensitized to ovalbumin have been treated for 8 days with drinking water containing 0.5 g/liter up to 2 g/liter NAC adjusted to pH 7.5 and changed every 2 days (Jeannine et al., 1995). Using 1.0 g/liter NAC, both ovalbumin-specific IgE and IgG1 (the murine isotype equivalent to human IgG4) responses were decreased by more than 70%. Even at 0.5 g/liter NAC the decrease of ovalbumin-specific IgE and IgG1 was still in the range of 30%. On the basis of the volume of water drunk in one day, the quantity of NAC swallowed by a mouse could be estimated at 50 mg/kg/day, comparable to 9 mg/kg per day given in humans as a mucolytic or 300 mg/kg per day as an antidote against acetaminophen-induced hepatotoxicity (Jeannine et al., 1995). This suggests that NAC acts in the range of doses that are already used in human therapeutics.

In patients, NAC has been administered orally at high doses in clinical studies for the treatment of idiopathic pulmonary fibrosis (Demedts et al., 2005) and cystic fibrosis (Tirouvanziam et al., 2006). Evaluation of NAC for the treatment of idiopathic pulmonary fibrosis was performed over one year in a double-blind, randomized, placebo-controlled multicenter study with a total of 182 patients. In this study, NAC was administered at a total oral dose of 1800 mg/day in addition to the standard therapy with prednisone and azathioprine. Although this NAC dose is three to nine times the usual approved dose of NAC when it is administered as an antioxidant and mucolytic agent in chronic obstructive pulmonary disease, the NAC and placebo groups had similar overall rates of side effects. The primary end points including vital capacity proved to be better in the group receiving NAC in addition to the standard therapy (Demedts et al., 2005).

For the treatment of cystic fibrosis, high NAC doses in excess of 1800 mg/day (600 mg to 1000 mg NAC three times daily) were administered orally for 4 weeks in a phase 1 study with 18 cystic fibrosis patients (Tirouvanziam et al., 2006). The high doses proved to be safe and effective in augmenting the GSH level in blood neutrophils and decreasing the airway neutrophil count and elastase activity.

The data of both studies confirm the proven safety record of long-term use of high doses of NAC (Kelly, 1998).

### Salicylates

The various inhibitory effects of acetylsalicylic acid (ASA; MW 180.2) and related salicylates (e.g., sodium salicylate; SA; MW 160.11) are concentration dependent. A 50% inhibition by ASA requires a concentration of approx. 2 x 10⁻⁶ M for COX-1, appprox. 3 x 10⁻⁴ M for COX-2, approx. 1 x 10⁻³ M for IL-4 gene transcription, and approx. 3 x 10⁻³ M for NF-kappa B translocation (Cianferoni et al., 2001). Selective inhibition of TNFR1-mediated activation of NF-kappa B requires even higher doses of salicylates in the range of 1-2 x 10⁻² M (Thommesen and Laegreid, 2005).

The therapeutic range for ASA and SA has been restricted to 0.8-1.7 x 10⁻³ M, corresponding to a serum salicylate concentration of 150-300 mg/liter. Restriction of the therapeutic range is necessary to avoid salicylate-related toxicity such as tinnitus. It should be mentioned, however, that tinnitus may occur also occasionally at levels as low as 95 mg/liter (Furst et al., 1987).

Salicylate kinetics are complex, involving 2 capacity limited and 3 first order processes (Furst et al., 1987). Capacity limited processes mean that the enzyme(s) acting to metabolize a drug become saturated and are working at their maximum capacity, even at relatively low drug doses. Therefore, small increases in dose can result in a disproportionate increase in serum levels. First order processes are those which metabolize increasing amounts of drug as more drug is presented to them. Therefore, an increase in dose results in a directly proportional increase in serum concentration. Further complications of salicylate kinetics include the facts that protein binding is not linear, that urinary excretion of un-metabolized salicylate is urine pH dependent, and that there is inherent genetic variability.

Using choline magnesium trisalicylate (CMS) for the treatment of patients with rheumatoid arthritis (RA), a strategy for reaching therapeutic salicylate levels has been developed (Furst et al., 1987). Despite the complex salicylate kinetics, the authors used a simplified weight adjusted dose of 45 mg CMS/kg/day as an initial dose. After one to two weeks of 45 mg CMS/kg/day in two divided doses, 51 of 71 patients with RA had steady state serum salicylate levels between 150 and 300 mg/liter (mean value: 213 ± 10 mg/L). Seventeen patients required dose adjustment using the formula: dosing rate = total clearance x concentration. Since all salicylate preparations are metabolized similarly once they are adsorbed and broken down to the parent salicylate molecule, the results of this study apply to all salicylates.

### TNFR1-specific antisense oligonucleotides

In mice, TNR1-specific antisense oligonucleotides have been administered as 2'-O-(2-methoxy)ethyl-modified antisense oligonucleotides (ASO). After i.p. injection of 25 mg/kg ASO every other day for a total of four times, mice were protected against subsequent radiation-induced liver damage (Huang et al., 2006).

In a preferred embodiment of the present invention, non-modified TNR1-specific antisense oligonucleotides are complexed with cationic liposomes to enhance cellular uptake and protect the oligonucleotides against enzymatic degradation in serum.

In one specific embodiment of the present invention, ultradeformable cationic liposomes (UCL) containing sodium cholate in addition to DOTAP (Kim et al., 2004) are used for complexing non-modified TNR1-specific antisense oligonucleotides. Sodium cholate is an edge activator and responsible for rendering flexibility in liposomes. The addition of sodium cholate provides an additional advantage in that instead of toxic organic solvents such as chloroform phosphate-buffered saline (PBS) can be used for the preparation of UCLs. PBS is not capable of dissolving DOTAP, but as soon as sodium cholate is added at a ratio of 1:6 (w/w) to the mixture of DOTAP and PBS (10 mg DOTAB/ml PBS), DOTAB is dissolved. Furthermore, sodium cholate inhibits the tendency of DOTAP for aggregation. The average particle size of this formulation is approximately 80 nm. Assessment of the in vitro transfection efficiency of plasmid DNA revealed an optimal ratio of 1:14 DNA/UCL (Kim et al., 2004). Recently, such liposomes have been used for transdermal delivery of non-modified IL-13-specific antisense oligonucleotides (IL-13 ASO) for the treatment of mice with atopic dermatitis (Kim et al., 2009). At a ratio of 6:1 (IL-13 ASO/UCL), maximum inhibition of IL-13 secretion was observed (Kim et al., 2004).

In a preferred specific embodiment of the present invention, multilammelar vesicles (MLVs) containing the cationic lipid 1,2-dioleoyl-3-trimethylammonium-propane (DOTAP) are used for complexing non-modified TNR1-specific antisense oligonucleotides. Such MLV lipoplexes have been shown to mediate efficient cellular association and uptake (Ross et al., 1998). The cellular uptake is believed to occur mainly through endocytosis with escape of the DNA into the cytoplasm of the cell by fusion of the liposomes with, or rupture of the endosomal membrane. In the presence of serum, multilammelar vesicles (>300 nm) are more efficient vectors for DNA transfer than small unilammelar vesicles (SUVs; <300 nm), which interact to higher extent with serum proteins (Ross et al., 1998). Serum proteins are thought to destabilize the bilayer membranes of liposomes, which may alter the lipoplex and prevent its ability to associate with cells, thus decreasing the uptake of DNA. A number of variables have been identified to influence the effectiveness of lipoplexes as vector including the charge ratio of cationic lipid to DNA. Using DOTAP-containing MLVs, optimal charge ratios of cationic lipid to DNA are below and up to about 1:1 (Ross et al., 1998). For example, for the preparation of lipoplexes with a ratio of 1:2 (cationic lipid to DNA) according to the method of Ross et al. (1998), 12 µg of DNA is added to 300 µl of a DOTAP-containing MLV suspension with a concentration of 0.133 µmole/ml.

In another preferred embodiment of the present invention, non-modified TNR1-specific antisense oligonucleotides complexed with cationic liposomes are embedded into hydrogels. Preferred hydrogels include those listed in section II.

### Dominant-negative sTNF mutants

Several sTNF-alpha variants have been designed by exchange of one or two amino acid residues, which are capable of sequestering native TNF-alpha homotrimers from TNF receptors by formation of inactive native:variant heterotrimers (Steed et al., 2003). At concentrations as low as two-fold that of native TNF-alpha, variants A145R/Y87H and A145R/I97T attenuated TNF-induced caspase activity by 50% and at 20-fold excess, activity was reduced to baseline. At 10-fold excess over native TNF-alpha, variant A145R/Y87H blocked TNF-induced translocation of the NF-kappa B p-65-RelA subunit in HeLa cells. Several dominant-negative sTNF-alpha variants neutralize TNF-induced caspase and NF-kappa B-mediated transcriptional activity over a wide range of native TNF-alpha concentrations, including the clinically relevant range of 100 to 200 pg/ml found in the synovial fluid of rheumatoid arthritis patients (Steed et al., 2003).

Furthermore, variant A145R/Y87H proved to be non-toxic. Dosed as high as 30 mg/kg of mouse body weight (along with 30 µg/kg native TNF-alpha), A145R/Y87H did not induce mortality or hepatotoxicity. In a murine model of collagen-induced arthritis, variant A145R/Y87H reduced joint swelling when dosed once daily at 2 mg/kg subcutaneously with an intravenous loading dose of 2 mg/kg on the first treatment day (Steed et al., 2003).

### TNFR1-specific TNF mutants

Useful TNFR1-specific sTNF-alpha mutants include but are not limited to R1antTNF containing mutations A84S, V85T, S86T, Y87H, Q88N, and T89Q (Shibata et al., 2008). R1antTNF neutralizes TNFR1-mediated responses without affecting TNFR2-mediated bioactivity. Like wild-type TNF-alpha, R1antTNF has a very short half-life of approximately 10 min in plasma of mice. By site-specific PEGylation of R1antTNF at its N-terminus, the plasma half-life of R1antTNF could be increased significantly (Shibata et al., 2009).

Administration of R1antTNF or PEG-RlantTNF at 1 µg or 3 µg/mouse twice a day for 3 weeks in mice with collagen-induced arthritis (the treatment was started 2 days after the second immunization) significantly decreased the severity of arthritis (Shibata et al., 2009). Only R1antTNF at 1 µg/mouse had no therapeutic effect, most likely due to the short plasma half-life of R1antTNF, since 1 µg/mouse of PEG-R1antTNF was effective.

### VII.1.5. General Considerations.

Dosage regimens may be adjusted to provide the optimum therapeutic response. The quantity of the polymer-embedded components depends on the release kinetics of the biodegradable thermo-gelling polymer and is adjusted to a level that guarantees the continuous release of therapeutically effective doses over a period of 3 to 5 days. The quantity of embedded components will vary according to factors such as the weight and the age of the individual, and the ability of the composition to induce an effective immune response in the individual.

In summary, these data provide useful guidelines for the determination of a therapeutically effective dose for those skilled in the art.

### VII.2. THERAPEUTIC APPLICATIONS OF SUITABLE COMPOSITIONS

In one embodiment of the present invention, methods for local enhancement of the suppressive functions of regulatory T cells at the site of allergen or auto-antigen presentation as adjuvant therapy prior and during allergen- or antigen-specific immunotherapy in patients with allergy, allergic asthma or autoimmune diseases are disclosed using suitable compositions of the present invention.

In a specific embodiment of the present invention, a suitable combination of low to moderate weight immune-modulators is administered subcutaneously or intradermally in a depot-mediating matrix for sustained delivery of the immune-modulators. After a period of time, sufficient for exerting their biological effects, one or more allergens or auto-antigens, optionally in the presence of alum or another adjuvant, are injected at the same site. Dependent on the properties of the immune-modulators embedded in the depot-mediating matrix, additional injection(s) of embedded immune-modulators at the site of allergen presentation may be performed to support the local enhancement of the suppressive functions of regulatory T cells within the period of allergen or auto-antigen presentation to T cells.

In a preferred specific embodiment of the present invention, a suitable combination of low to moderate weight immune-modulators and one or more allergens or auto-antigens, optionally in the presence of alum or another adjuvant, all of them embedded in a depot-mediating matrix for sustained delivery, are administered subcutaneously or intradermally. Dependent on the properties of the immune-modulators embedded in the depot-mediating matrix, additional injection(s) of embedded immune-modulators at the site of allergen or auto-antigen presentation may be performed to support local enhancement of the suppressive functions of regulatory T cells within the period of allergen or auto-antigen presentation to T cells

In another preferred specific embodiment of the present invention, a suitable combination of low to moderate weight immune-modulators is administered subcutaneously or intradermally in a depot-mediating matrix for sustained delivery of the immune-modulators. After a period of time, sufficient for exerting their biological effects, the same combination of low to moderate weight immune-modulators and one or more allergens or auto-antigens, optionally in the presence of alum or another adjuvant, all of them embedded in a depot-mediating matrix for sustained delivery, are administered subcutaneously or intradermally. Dependent on the properties of the immune-modulators embedded in the depot-mediating matrix, additional injection(s) of embedded immune-modulators at the site of allergen or auto-antigen presentation may be performed to support local enhancement of the suppressive functions of regulatory T cells within the period of allergen or auto-antigen presentation to T cells.

### VII.3. MODES OF ADMINISTRATION OF THERAPEUTIC COMPOSITIONS

Routes of administration of the compositions of the present invention by injection or by implantation include but are not limited to subcutaneous, intradermal, intramuscular, nasal, transbucal, transmucosal, sublingual, rectal, vaginal, intraocular, or topical administration. Preferred examples of routes of administration of the compositions of the present invention include but are not limited to intradermal, subcutaneous, and intramuscular administration.

For mucosal immunization, the physicochemical characteristics of the administered components and the delivery vehicle have to be adjusted to stimulate their uptake through the various mucosal routes. For example, alum salts are ineffective when administered by the oral or nasal route. In contrast, cationic chitosan derivatives are of special interest in nasal delivery because of their excellent biocompatibility and mucoadhesive nature (Hagenaars et al., 2010). For example, a thermal-sensitive hydrogel which was formulated as intranasal vaccine with N[(2-hydroxy-3-trimethylammonium)propyl] chitosan chloride (HTCC) and α,β-glycerophosphate, was shown to significantly prolong the antigen residence time in the nasal cavity and to enhance the transepithelial transport via the paracellular routes (Wu et al., 2012).

### EXAMPLES

The following examples are intended to illustrate but not limit the present invention.

### EXAMPLE 1. PREPARATION OF IMMUNE MODULATORS

This example describes the synthesis of C5aR inhibitor PMX53, murine Il-4 antagonist OY, TNFR1-specific antisense oligonucleotides and mutant TNF-alpha R1antTNF.

### 1.1. SYNTHESIS OF C5aR ANTAGONIST PMX53

The C5aR antagonist PMX53 (AcF-(OPdChaWR)) is synthesized as described (Finch et al., 1999). First, a linear peptide is synthesized on a peptide synthesizer using Fmoc chemistry (433A; Applied Biosystems, Foster City, CA), followed by formation of a lactam bridge in solution as described (Finch et al., 1999). The peptide is purified using reversed-phase high-performance liquid chromatography. The mass is confirmed by matrix-assisted laser desorption ionization/time-of-flight mass spectrometry.

### 1.2. EXPRESSION OF RECOMBINANT MURINE IL-4 ANTAGONIST QY

The murine IL-4 mutant QY (Q116D/Y119D) is analogous to the R121D/Y124D double mutant of human IL-4, and an excess of the murine QY mutant has been shown to completely inhibit responses toward wild-type murine IL-4 (Grunewald et al., 1997).

### 1.2.1. Cloning and expression of murine IL-4 antagonist QY.

First, the sequence of the native mature murine IL-4 protein is modified to include a thrombin cleavable N-terminal 6xHistag and the QY (Q116D/Y119D) mutations, then the protein sequence is translated into DNA using codon optimization for expression in prokaryontic *E. coli* cells. The resulting sequence is synthesized and amplified using PCR with Pfu polymerase according to the manufacture and annealing temperatures rising from initial 5 cycles at 50°C to 60°C and 30 cycles total. The resulting PCF fragment is gel-purified in 1% agarose (GeneJET Gel Extraction Kit, Fermentas) and transferred to a restriction enzyme double digest containing *Nde I* and *Xho I* (Fermentas). The restriction digest is again gel-purified and the fragment is ligated into *Nde I* and *Xho I* cut pET-22b (Novagen) expression vector.

*Expression of the murine IL-4 antagonist QY.* Recombinant His-tagged murine IL-4 antagonist QY (Q116D/Y119D) is expressed in *E. coli* (BL21(DE3)pLysS; Novagen) at 37°C. Cultures are grown in 1-L batches in minimal medium containing 100 µg/ml ampicillin and 34 µg/ml chloramphenicol. The minimal medium contains the following components: 0.1 M phosphate buffer, pH 7.0, 2 mM MgSO₄, 17 mM NaCl, 0.1 mM CaCl₂, 5 mg/L FeSO₄, 0.2 mg/L (NH₄)MO₇O₂₄, 1 mg/L H₃BO₃, 0.2 mg/L CoCl₂, 0.2 mg/L CuSO₄, 2 mg/L MnSO₄, and 2 mg/L ZnSO₄, supplemented with thymidine, biotin, folic acid, pantothenic acid, niacinamide, pyroxidine phosphate, and thiamine (1 mg/L each). The expression is induced by the addition of isopropyl-D-thiogalactopyranoside to a final concentration of 1 mM after the culture reaches an OD₆₀₀ of approximately 0.6. The cells are allowed to grow for 4h and then harvested by centrifugation at 5000 x g for 10 min at 4°C. The pellet is stored at -20°C.

*Refolding of the murine IL-4 antagonist QY from inclusion bodies.* The insoluble His-tagged murine IL-4 antagonist QY (Q116D/Y119D) is refolded according to patent application EP 11075261.5. The *E. coli* pellet containing the recombinant IL-4 antagonist QY is resuspended in 15 ml of 50 mM Tris-HCl, pH 8.0, and 5 mM EDTA (lysis buffer) containing 2 mg lysozyme and incubated at room temperature for 20 min. The cell lysate is diluted with 60 ml lysis buffer and sonicated. It is then centrifuged at 11,000 rpm for 30 min at 4°C to collect the pellet containing inclusion bodies. The pellet is washed twice with 60 ml with 2 M urea in lysis buffer and collected by centrifugation as described above. Finally, the pellet is resuspended in 25 ml of 6 M GdnHCl, 50 mM potassium phosphate, pH 8.0, and 1 mM reduced glutathione (extraction buffer) and stirred at room temperature for 10 min. The solution is centrifuged to remove the insoluble portion. The guanidine-extracted IL-4 antagonist QY is loaded onto 15 ml of Ni-charged HisTrap™ FF columns (GE Healthcare) equilibrated with the extraction buffer. The beads are washed with 8 column volumes of 7 M urea and 50 mM potassium phosphate, pH 6.8, to remove the cellular proteins. The His-tagged IL-4 antagonist QY is refolded on the column by the gradual removal of urea through a linear gradient expanding from 100% unfolding buffer to 100% refolding buffer (380 ml at a flow rate of 1 ml/min) using a fast protein liquid chromatography ÄKTA™ system (Pharmacia). The unfolding buffer is composed of 7 M urea, 100 mM NaCl, 50 mM potassium phosphate, pH 6.8, 1 mM reduced glutathione, and 1 mM oxidized glutathione; the refolding buffer has the same composition but without urea. After washing the column with 40 ml refolding buffer, the refolded His-tagged IL-4 antagonist QY is eluted from the column with 100 mM EDTA and 10 mM Tris-HCl, pH 8.0. EDTA is used to chelate divalent cations that can potentially cause protein aggregation during dialysis. The His-tagged IL-4 antagonist QY is then dialyzed against 50 mM TrisHCl, pH 8.0, 5 mM CaCl₂, and 100 mM NaCl to remove EDTA. The protein is concentrated to approximately 2 mg/ml.

*Removal of the His-tag.* The His-tag is cleaved overnight by incubation of each mg fusion protein with 10 U thrombin (Sigma) in PBS buffer. The mix is incubated at room temperature overnight. The reaction can be stopped with 1 mM PMSF. Cleavage products are separated by chromatography. Residual thrombin can be removed with pAminonezamidine-Agarose (Sigma) either by batch or chromatographic methods. The cleavage specificity is confirmed by N-terminus sequencing of blotted IL-4 antagonist QY. The His-tag is removed by passing the cleaved protein through Ni-fractogel beads. The purified samples are dialyzed in a desired buffer and kept at 4°C for short term storage or stored at -80°C in the presence of 25% glycerol.

### 1.2.2. Structural and functional in vitro assays.

These assays include an ELISA-type assay and cellular assays. The ELISA-type assay provides information about the integrity of the murine IL-4 epitope recognized by the anti-murine IL-4 antibody. Information about the structural integrity of the murine IL-4 antagonist QY is obtained from a cellular binding assay analyzing binding of murine IL-4 and the murine IL-4 antagonist QY to the murine IL-4 receptor. The functionality of murine IL-4 and the murine IL-4 antagonist QY is determined in a T cell proliferation assay.

*ELISA analysis.* The concentration of murine IL-4 or murine IL-4 mutants is determined by ELISA (Mouse IL-4 ELISA MAX™ Deluxe, BioLegend GmbH, Fell) according to the manufacturers instructions.

*Cellular binding assay.* Binding of murine IL-4 or the murine IL-4 antagonist QY to IL-4 receptor molecules on the surface of murine CTLL-2 cells is detected by FACScan analysis using a biotinylated anti-IL-4 antibody and fluorescent-labeled streptavidin. Murine CTLL-2 cells are IL-2-dependent and typically express 2000-5000 Il-4 receptors per cell.

Murine CTLL-2 cells, obtained from ATCC (ATCC TIB 214), are cultured in RPMI 1640 containing 8% fetal calf serum, 100 units/ml penicillin and 100 µg/ml streptomycin, supplemented with 100 ng/ml IL-2. For the cellular binding assay 5 x 10⁶ cells are incubated for 30 min at 37°C in 150 µl of PBS (pH 7.4) containing 1% (w/v) BSA, 1 x 10⁻⁹ M recombinant murine (rmu) IL-4 or the murine IL-4 antagonist QY. The mixture is chilled to 4°C, washed once in a large volume of PBS (pH 7.4) containing 1% (w/v) BSA, resuspended in 150 µl of PBS (pH 7.4) and subjected to FACScan analysis.

*T cell proliferation assay.* IL-4 induced proliferation of CTLL-2 cells is determined by incorporation of [³H] thymidine. The inhibitory effect of the murine IL-4 antagonist QY is determined by adding increasing amounts of the antagonist to IL-4.

CTLL-2 cells are incubated in 0.2 ml aliquots at a density of 5 x 10⁵/ml with log 2 dilutions of murine IL-4 for 3 days, before the amount of [³H] thymidine incorporated during the final 4 hr is determined. The concentration of IL-4 yielding half-maximal response is used for determining the inhibitory activity of the murine IL-4 antagonist QY. In these experiments, log 2 dilutions of the murine IL-4 antagonist QY are added to wild-type murine IL-4.

### 1.3. SYNTHESIS OF LIPOSOME-EMBEDDED TNFR1-SPECIFIC ANTISENSE OLIGONUCLEOTIDES

In this experiment, non-modified TNFR1-specific antisense oligodeoxynucleotides (TNFR1-ODN), targeting the 3'-polyadenylation [poly(A)] signal loci on the TNFR1 mRNA (Ojwang et al., 1997a; Ojwang et al., 1997b), are complexed with a cationic liposomal preparation (Cellfectin; Life Technologies, USA) containing the cationic lipid tetramethyltetra-palmitylspermine and the phospholipid dioleoylphosphatidyl-ethanolamine.

*Synthesis of a TNFR1-specific antisense ODN.* The synthesis is performed as described (Ojwang et al., 1997a).
*Sequence of the TNFR1-specific antisense ODN*
a g a a t t t t t a g t g t a t g t a c a a

*Complexation of TNFR1-ODN with Cellfectin.* TNFR1-specific antisense ODN (1.0 µM) in 200 µl PBS, pH 7.4, are mixed with 20 µl of Cellfectin in PBS, pH 7.4 (1 mg/ml) and incubated for 15 min at room temperature.

### 1.4. SYNTHESIS OF LIPOSOME-EMBEDDED pPT-MODIFIED TNFR1-SPECIFIC ANTISENSE OLIGONUCLEOTIDES

In this experiment, pPT-modified TNFR1-specific antisense oligodeoxynucleotides (TNFR1-pPT ODN), targeting the 3'-polyadenylation [poly(A)] signal loci on the TNFR1 mRNA (Ojwang et al., 1997a; Ojwang et al., 1997b), are complexed with a cationic liposomal preparation (Cellfectin; Life Technologies, USA) containing the cationic lipid tetramethyltetra-palmitylspermine and the phospholipid dioleoylphosphatidyl-ethanolamine.

*Synthesis of a pPT TNFR1-specific antisense ODN.* The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.
*Sequence of the 22mer pPT TNFR1-specific* antisense *ODN*
a* g* a* a* t t t t t a* g* t* g* t* a t g t a* c* a* a
(asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*Formulation of the 22mer pPT TNFR1-specific antisense ODN with Cellfectin.* TNFR1-specific pPT antisense ODN (1.0 µM) in 35 µl PBS, 7.4, are mixed with 3.5 µl of Cellfectin in PBS, pH 7.4 (1 mg/ml) and incubated for 15 min at room temperature.

### 1.5. EXPRESSION OF MUTANT TNF-alpha R1antTNF

After transfection of *E. coli* BL21(DE3) with a plasmid encoding the R1antTNF gene under control of the T7 promotor, R1antTNF is overexpressed, recovered from the inclusion bodies, washed with 2.5% Triton X-100 and 0.5 M NaCl in TES buffer, and the solubilized in 6 M guanidine-HCl, 0.1 M Tris-HCl, pH 8.0, and 3 mM EDTA (Shibata et al., 2008). The solubilized protein is reduced with 10 mg/ml dithioerythritol for 4 h at room temperature, and then refolded by 100-fold dilution in a refolding buffer (0.1 M Tris-HCl, pH 7.4, 2 mM EDTA, 0.5 M arginine, and 551 mg/liter oxidized glutathione). After dialyzing against against 20 mM Tris-HCl, pH 7.4, containing 100 mM urea, the active trimeric proteins are purified by Q-Sepharose chromatography (GE Healthcare Bioscience, USA) and additional gel filtration on Superose 12 (GE Healthcare Bioscience, USA).

### EXAMPLE 2. SYNTHESIS OF THERMOGELLING PLGA-PEG-PLGA HYDROGELS

This example describes the synthesis and chacterization of thermogelling PLGA-PEG-PLGA hydrogels.

### 2.1. SYNTHESIS OF THERMOGELLING PLGA-PEG-PLGA HYDROGELS

The biodegradable triblock polymer described in this example has a PLG/PEG weight ratio of 2.3 (70/30), and a lactide/glycolide molar ratio of approx. 15/1. Synthesis of the triblock copolymer is performed according to published protocols (Qiao et al., 2005).

### 2.1.1. Copolymer synthesis

Polyethylene glycol (PEG 1500) was purchased from Fluka, poly(DL-lactide) from Sigma, glycolide (1,4-Dioxane-2,5-dione) from Sigma, and stannous 2-ethylhexanoate from Aldrich.

A total of 25 g of DL-lactide, glycolide and PEG are used for polymerization (16.6 g DL-lactide, 0.9 g glycolide, 7.5 g PEG 1500). Under nitrogen atmosphere, PEG 1500 is dried under vacuum and stirring at 120°C for 2 h in a vigorously dried Erlenmeyer reaction flask. Then the reaction flask is filled with dry argon. DL-lactide and gycolide monomers are added under stirring followed by the addition of Stannous 2-ethylhexanoate (0.2% w/w). Then the tube is sealed under argon. The sealed flask was immersed and kept in an oil bath thermostated at 130°C. After 16 h the flask was cooled to room temperature, and the product was dissolved in cold water. After completely dissolved, the copolymer solution is heated to 80°C to precipitate the copolymer and to remove the watersoluble low molecular weight copolymers and unreacted monomers. The supernatant is decanted, the precipitated copolymer is again dissolved in cold water followed by heating to induce precipitation. This process of dissolution followed by precipitation is repeated three times. Finally, the copolymer is dried under vacuum at room temperature until constant weight.

### 2.1.2. Molecular weight determination

The molecular weight of the copolymer is determined by gel permeation chromatography using polystyrene standards as described by Qiao et al. (2005).

### 2.1.3. Measurement of gelation temperature

The gelation temperature is determined as described by Qiao et al. (2005). A 2 ml transparent vial is filled with 200 µl water solution of the copolymer (20% w/w and 25% w/w), is placed in a water bath. The solution is heated in 1°C steps beginning at 26°C in a thermomixing device (Eppendorf). At each temperature step the gelation is checked by careful inversion of the tube. When the solution is not free-flowing, gelation of the solution occurred, the temperature read from the thermometer is determined as gelation temperature.

### 2.2. IN VITRO DEGRADATION OF PLGA-PEG-PLGA HYDROGELS

The *in vitro* degradation behavior of the copolymer of Example 2.1 is evaluated by the mass loss and the molecular weight reduction with time upon incubation in phosphate-buffered saline.

Samples (0.5 ml) are incubated in phosphate-buffered saline pH 7.4 at 37°C under mild agitation in a water bath. The solid residues are removed from the incubation medium at scheduled time intervals and lyophilized. The samples are weighted and the weight loss is calculated. Then the solid residues are solved in cold water and analyzed by gel permeation chromatography using polystyrene standards as described by Qiao et *al.* (2005).

### 2.3. BIODEGRADATION OF PLGA-PEG-PLGA HYDROGELS

To test the *in vivo* gel formation behavior and the degradation characteristics of PLGA-PEG-PLGA hydrogels of Example 2.1., the hydrogel is injected subcutaneously into mice that have been anesthetized with ethyl ether. The resulting gel implants are then allowed to develop *in vivo* over the experimental period. At each of the post-injection sampling points, the mice are sacrificed, the gel implants are removed from the subcutaneous injection site, and the removed gel implants are analyzed as described in Example 2.2.

Two groups of wild-type BALB/c mice (each group: 16 mice) are analyzed for the *in vivo* gel formation behavior and the degradation characteristics: group 1: 20% (w/w) polymer; group 2: 25% (w/w) polymer. The analysis is performed according to following schedule: implantation of 200 µl polymer on day 0, analysis of size in two mice on days 1,2,3,4,5,10,20 and 30.

### EXAMPLE 3. RELEASE OF IMMUNE MODULATORS FROM

### HYDROGELS

This example describes the release charcteristics of selected embedded low to moderate weight immune-modulators from thermogelling PLGA-PEG-PLGA hydrogels.

### 3.1. RELEASE OF COMPLEMENT INHIBITOR COMPSTATIN FROM PLGA-PEG-PLGA HYDROGELS

This example describes the release of the 13-residue cyclic peptide (H-I[CVVQDWGHHRC]T-NH₂) (termed compstatin) from the hydrogel of Example 2.1. Compstatin is purchased from Tocris Bioscience (Bristol, UK).

The PLGA-PEG-PLGA triblock copolymer of Example 2.1. is dissolved at room temperature in 200 µl PBS pH 7.4 containing different concentrations of compstatin (0.5 mg up to 2 mg/ml) to make a 20% w/w or 25% w/w solution. Then the formulation is placed in a 2 ml vial, incubated at 37°C for 2 min until gelling, and 0.2 ml of PBS pH 7.4 is added. The vial is incubated at 37°C. At specified sample collection times, a sample is withdrawn and replaced by an identical volume of PBS pH 7.4 to maintain release conditions.

The amount of released compstatin is determined by bicinchoninc acid (BCA) assay using BCA^{™} Protein Assay Kit (Pierce, USA). The structural and functional integrity of released compstatin is determined by inhibition of the alternative pathway of complement activation using rabbit erythrocytes.

The inhibitory effect of released compstatin on the alternative pathway is determined by measuring the lysis of rabbit erythrocytes (Er) in normal human serum (NHS). A sample of 80 µl containing either released compstatin or different standard concentrations of compstatin is mixed with 7.5 µl NHS, 7.5 µl MgEGTA (0.1 M MgCl₂ and 0.1 M EGTA), 15 µl of Er (1 x 10⁹/ml), and 40 µl GVB (5 mM barbital and 145 mM NaCl, pH 7.4). The reaction mixture is incubated at 37°C for 20 min and stopped by adding 200 µl GVBE (GVB with 10 mM EDTA). After centrifugation, hemolysis is determined at 414 nm. The percentage of lysis is normalized by considering 100% lysis to be equal to lysis occurring in the absence of compstatin. The concentration of compstatin for 50% inhibition of the lysis of rabbit erythrocytes in normal human serum under these conditions is 12 µM (Sahu et al., 1996).

### 3.2. RELEASE OF CALCITRIOL FROM PLGA-PEG-PLGA HYDROGELS

This example describes the release of calcitriol (1α,25-dihydroxyvitamin D3; 1,25-(OH)₂D3) from the hydrogel of Example 2.1. Calcitriol is purchased from Sigma-Aldrich (Munich, Germany)

The PLGA-PEG-PLGA triblock copolymer of Example 2.1. is dissolved at room temperature in 200 µl PBS pH 7.4 containing different concentrations of calcitriol (up to 15 µg/ml) to make a 20% w/w or 25% w/w solution. Then the formulation is placed in a 2 ml vial, incubated at 37°C for 2 min until gelling, and 1.8 ml of PBS pH 7.4 is added. The vial is incubated at 37°C. At specified sample collection times, a sample is withdrawn and replaced by an identical volume of PBS pH 7.4 to maintain release conditions.

The amount of released calcitriol is determined by ELISA (25-OH Vitamin D ELISA, Euroimmun AG, Lübeck), according to the instructions of the manufacturer.

### 3.3. RELEASE OF IL-4 ANTAGONIST QY FROM PLGA-PEG-PLGA HYDROGELS

This example describes the release of murine IL-4 antagonist QY (Q116D/Y119D) of Example 1.2. from the hydrogel of Example 2.1.

The PLGA-PEG-PLGA triblock copolymer of Example 2.1. is dissolved at room temperature in PBS pH 7.4 containing different concentrations of the murine IL-4 antagonist QY (0.5 mg/ml up to 1.0 mg/ml) to make a 20% w/w or 25% w/w solution. Then 200 µl of the formulation is placed in a 2 ml vial, incubated at 37°C for 2 min until gelling, and 1.8 ml of PBS pH 7.4 is added. The vial is incubated at 37°C. At specified sample collection times, a sample is withdrawn and replaced by an identical volume of PBS pH 7.4 to maintain release conditions.

The amount of released murine IL-4 antagonist QY is determined by bicinchoninc acid (BCA) assay using BCA^{™} Protein Assay Kit (Pierce, USA) and by an ELISA-type assay as described above. The structural integrity of released murine IL-4 antagonist QY is determined by a cellular binding assay, and the functionality of released murine IL-4 antagonist QY is determined by inhibition of IL-4-induced T cell proliferation as described in Example 1.2.

### 3.4. RELEASE OF TNFR1 INHIBITORS FROM PLGA-PEG-PLGA HYDROGELS

This example describes the release of selected TNFR1 inhibitors from the hydrogel of Example 2.1.

### 3.4.1. Release of NAC from PLGA-PEG-PLGA hydrogels

The PLGA-PEG-PLGA triblock copolymer of Example 2.1 is dissolved at room temperature in phosphate buffered saline (PBS) at pH 7.4, containing 25% or 20% w/v polymer. Varying volumes (10 µl, 20 µl, 40 µl, 80 µl) of N-acetyl-L-cysteine (NAC, ACC) (50 mg/ml PBS (0.3 M) at pH 7.4,) are added to 200 µl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released NAC is determined by an assay for sulfhydryl groups using the reaction of 4,4'-dithiopyridine (4-PDS) with thiols, which gives the corresponding 4-thiopyridone (4-TP) (Grassetti and Murray, 1967). Using this assay, 15 nmol-0.5 µmol NAC is dissolved in 1.5 ml PBS pH 7.2 and mixed with 1.5 ml 2 mM 4-PDS in PBS pH 7.2. UV absorbance of 4-TP is determined at 324 nm (molar extinction coefficient 1.198 x 10⁴) immediately after mixing. The method permits determination of less then 0.5 µg SH (Grassetti and Murray, 1967).

For the determination of NAC concentrations below 15 nmol/1.5 ml (10 µM), a more sensitive spectrophotometric assay is used which is based on a coupled two-step redox and complexation procedure (Kukoc-Modun and Radic, 2011). In the first step, Fe(III) is reduced by the sulfhydryl group of NAC to Fe(II). Subsequently, Fe(II) is complexed with 2,4,6-tripyridyl-S-triazine (TPTZ). Acetate buffer (0.5 M), pH 3.6 (2.0 ml) is mixed with 125 µl 10 mM FeCl₃, 125 µl 10 mM TPTZ in 0.12 M HCl, and 100 µl NAC solution. After incubation for 60 min at room temperature, the absorbance of the produced Fe(II)-TPTZ complex, which remains constant for 24 hours, is measured at 593 nm. The detection limit of this method for NAC is 0.14 µM.

### 3.4.2. Release of GSH from PLGA-PEG-PLGA composits

In this example, non-methylated glutathione (GSH) is used to study the release characteristics of glutathione and derivatives thereof including S-methylglutathione (GSM) from PLGA-PEG-PLGA hydrogels.

The PLGA-PEG-PLGA triblock copolymer of Example 1.1 is dissolved at room temperature in phosphate buffered saline (PBS) at pH 7.4, containing 25% or 20% w/v polymer. Varying volumes (20 µl, 40 µl, 80 µl) of 10 mM GSH in PBS, pH 7.4, are added to 200 µl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released GSH is determined by an assay for sulfhydryl groups using the reaction of 4,4'-dithiopyridine (4-PDS) with thiols, which gives the corresponding 4-thiopyridone (4-TP). Using this assay, 15 nmol-0.5 µmol GSH is dissolved in 1.5 ml PBS pH 7.2 and mixed with 1.5 ml 2 mM 4-PDS in PBS pH 7.2. UV absorbance of 4-TP is determined at 324 nm (molar extinction coefficient 1.198 x 10⁴) immediately after mixing. The method permits determination of less then 0.5 µg SH (Grassetti and Murray, 1967).

For the determination of GSH concentrations below 15 nmol/1.5 ml (10 µM), a more sensitive spectrophotometric assay is used which is based on a coupled two-step redox and complexation procedure (Kukoc-Modun and Radic, 2011). In the first step, Fe(III) is reduced by the sulfhydryl group of GSH to Fe(II). Subsequently, Fe(II) is complexed with 2,4,6-tripyridyl-S-triazine (TPTZ). Acetate buffer (0.5 M), pH 3.6 (2.0 ml) is mixed with 125 µl 10 mM FeCl₃, 125 µl 10 mM TPTZ in 0.12 M HCl, and 100 µl GSH solution. After incubation for 60 min at room temperature, the absorbance of the produced Fe(II)-TPTZ complex, which remains constant for 24 hours, is measured at 593 nm.

### 3.4.3. Release of SA from PLGA-PEG-PLGA composits

The PLGA-PEG-PLGA triblock copolymer of Example 1.1 is dissolved at room temperature in phosphate buffered saline (PBS) at pH 7.4, containing 25% or 20% w/v polymer. Varying volumes (10 µl, 20 µl, 40 µl, 80 µl) of 10 mM sodium salicylate (SA) in PBS, pH 7.4, are added to 200 µl gel solution. The solubility of SA in water is 2g/liter at 20°C (14.5 mM). Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released SA is determined by derivatization with Fe(III) and quantification of the violet coloured tetraaquosalicylatroiron (III) complex at 530 nm (www.jenway.com (Application note A09-009A; The quantitative determination of the aspirin content of tablets using UV and visible wavelength spectroscopy).

### 3.4.4. Release of liposome-embedded TNFR1-specific antisense oligonucleotides from PLGA-PEG-PLGA composits

The PLGA-PEG-PLGA triblock copolymer of Example 2.1 is dissolved at room temperature in PBS at pH 7.4, containing 25% or 20% w/v polymer. Varying volumes (10 µl, 20 µl, 40 µl, 80 µl) of TNFR1-ODN/Cellfectin complexes of Example 1.3. are added to 200 µl gel solution. The formulation is placed in a 2 ml vial, incubated at 37°C for 2 min until gelling, and 1.8 ml of PBS, pH 7.4, is added. The vial is incubated at 37°C. At specified sample collection times, the supernatant is withdrawn and replaced by an identical volume of PBS pH 7.4 to maintain release conditions.

Released TNFR1-ODNs are liberated from the liposomal complex by the addition of 200 µl 100 mM Tris-HCl, pH 8.0, 5 mM EDTA, 10% Triton X-100 to the supernatant (1.8 ml). Thereafter, the amount of released TNFR1-ODN is determined in 100 µl aliquots of the supernatants by a one-step hybridization ELISA (Wei et al., 2006). Capture and detection of TNFR1-ODN is accomplished on a one-strand capture probe, which is complementary to the TNFR1-ODN without an overhang and contains biotin at the 3'-end and digoxigenin at the 5'-end. For TNFR1-ODN capture probe hybridization 200 nM capture probe in hybridization buffer solution (capture ODN concentration of 200 nM ensures highest hybridization with analytes) is first heated to 95°C for 5 min in a heating block to disrupt possible secondary structures. Then 100 µl of the capture probe solution is added to 100 µl of the TNFR1-ODN solution in a polystyrene 96-well plate. The mixture is incubated at 42°C for 2.5 hours. After TNFR1-ODN capture probe hybridization, 150 µl of the solution is transferred to 96-well Reacti-Bind NeutrAvidin-coated polystyrene plates (Pierce, USA) and incubated for 30 min at 37°C to allow attachment of the biotin-labeled duplex to the coated plates. The plates are washed 6 times with washing buffer, followed by the addition of 150 µl S1 nuclease (60 U in 100 mM NaCl), After incubation for 2 hours at 37°C, the plates are washed 6 times with washing buffer, followed by the addition of 150 µl anti-digoxigenin-alkaline phosphatase (AP) (Roche, USA) diluted 1:2500 with bovine serum albumin blocking buffer (Superblock buffer in Tris-buffered saline, TBS; Pierce, USA) and subsequent incubation for further 30 min at 37°C with gentle shaking. Thereafter, the plates are washed again 6 times with washing buffer, followed by the addition of 150 µl Attophos (Promega, USA) substrate solution (36 mg Attophos in 60 ml diethanolamine buffer). After 30 min at 37°C the generated fluorescence is measured at Exitation 430 nm/Emission 560 nm (filter 550 nm) using a fluorescence microtiter plate reader.

Buffers used for the determination of TNFR1-ODNs include the hybridization buffer (60 mM Na phosphate, pH 7.4, 1.0 M NaCl, 5 mM EDTA, and 0.2% Tween 20), the washing buffer (25 mM Tris-HCl, pH 7.2, 0.15 M NaCl, and 0.2% Tween 20), and the dilution buffer for ODN standards (10 mM Tris-HCl, pH 8.0, 1 mM EDTA).

According to Wei et al. (2006) the one-step hybridization ELISA is linear from 0.025 to 50 nM ODN including phosphorothioate oligonucleotides. Using 60 U S1 nuclease per well, a cutting efficiency of 99.3-99.7% is achieved.

### 3.4.5. Release of mutant TNF-α from PLGA-PEG-PLGA composits

The PLGA-PEG-PLGA triblock copolymer of Example 2.1 is dissolved at room temperature in phosphate buffered saline (PBS) at pH 7.4, containing 25% or 20% w/v polymer. Varying volumes (10 µl, 20 µl, 40 µl, 80 µl) of mutant TNF-alpha R1antTNF of Example 1.5. at a concentration of 1 mg R1antTNF/ml PBS, pH 7.4, are added to 200 µl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released R1antTNF is determined by bicinchoninc acid (BCA) assay using BCA^{™} Protein Assay Kit (Pierce, USA).

### EXAMPLE 4. IMMUNOTHERAPY OF OVA-ALLERGIC MICE

This example shows the efficacy of allergen-specific immunotherapy of OVA-allergic mice with increasing doses of subcutaneously injected alum-adsorbed OVA and co-injected polymer-embedded combinations of immune-modulator at the site of OVA presentation.

### Animals and materials.

Female wild-type C57BL/6 mice 6 to 10 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen. C3aR antagonist N²-[(2,2-dipheneylethoxy)acetyl]-L-arginine (SB290157; MW 526.5; solubility: 5 mg/ml DMSO or at least 10 mg/ml of a saline solution containing 5% ethanol and 10% Cremaphor-EL) is purchased from Santa Cruz Biotechnology, Inc. Calcipotriol (synonyms: MC 903, calcipotriene; MW 412,62; solubility: approx. 50 mg/ml of ethanol or approx. 0.15 mg/ml in a 1:5 solution of ethanol:PBS, pH 7.2) is purchased from Cayman Chemical Company. The C5aR antagonist PMX53 (AcF-(OPdChaWR)) is synthesized as described in Example 1.1. The murine IL-4 mutant Q116D/Y119D (the murine equivalent of the human IL-4 double mutant R121D/Y124D) is prepared as described in Example 1.2.

### Preparation of alum-adsorbed ovalbumin (OVA).

For immunization, 100 µg of OVA are solved in 0.3 ml of PBS, pH 7.4, and mixed with 0.70 ml Imject Alum (Pierce/KMF). For immunotherapy, 10 mg of OVA are solved in 3.0 ml of PBS, pH 7.4, and mixed with 7.0 ml Imject Alum (Pierce/KMF). From this mixture containing 100 µg alum-adsorbed OVA in 0.1 ml, dilutions are prepared containing in 0.1 ml 90 µg, 60 µg, 30 µg, 15 µg, and 7.5 µg alum-adsorbed OVA.

### Allergic sensitization procedure.

Mice are immunized three times by i.p. injection of 100 µl alum-adsorbed OVA (10 µg OVA in 100 µl of PBS/Imject Alum). The second i.p. injection is performed 7 days after the first injection and the third injection 14 days after the first injection.

### Immunotherapy.

One week after the third immunization with OVA, mice are subjected to two different modalities of immunotherapy, one based on conventional treatment with increasing doses of alum-adsorbed OVA, the other based on treatment with increasing doses of alum-adsorbed OVA in the presence of polymer-embedded immune-modulators including the murine IL-4/IL-13 antagonist QY, calcipotriol, the C3aR antagonist SB290157, and optionally the C5aR antagonist PMX53.

Subcutaneous administration of increasing doses of alum-adsorbed OVA is performed at 9-day intervals. The first subcutaneous administration of polymer-embedded immune-modulators (total volume: 300 µl; 150 µl are injected s.c. at two different sites) is performed 1 day before immunotherapy with alum-adsorbed OVA, at day 0 one hour before administration of alum-adsorbed OVA, and 24 hours after administration of alum-adsorbed OVA within the 9-day intervals. Increasing doses of OVA (7.5, 15, 30, 60, 90 µg) in 100 µl of PBS/Imject Alum are injected subcutaneously at the site of the gelled polymer composit (50 µl at each side of the gelled polymer). The injection procedure is repeated at a different subcutaneous site until the highest dose of alum-adsorbed OVA is injected.

For immunotherapy of mice with alum-adsorbed OVA in the presence of polymer-embedded immune-modulators, 150 µl of the PLGA-PEG-PLGA triblock copolymer of Example 1.1. (25% w/v polymer) in PBS, pH 7.4, are mixed with different amounts of immune modulators including 50 µl of murine IL-4/IL-13 antagonist QY in PBS, pH 7.4 (up to 50 µg, corresponding to 1.0 mg/ml), 30 µl of calcipotriol in a 1:5 solution of ethanol:PBS, pH 7.4 (up to 4.5 µg, corresponding to 150 µg/ml), 30 µl of C3aR antagonist SB290157 in 5% ethanol, 10% Cremaphor-EL, 85% saline (up to 300 µg SB290157, corresponding to 10 mg/ml), and 40 µl of C5aR antagonist PMX53 in PBS, pH 7.4, containing 10% ethanol (up to 200 µg, corresponding to 5 mg/ml). Optionally, 40 µl of C5aR antagonist PMX53 are replaced by 40 µl of PBS, pH 7.4.

For immunotherapy of mice with alum-adsorbed OVA only, 100 µl of PBS/Imject Alum containing increasing doses of OVA (7.5, 15, 30, 60, 90 µg) are injected subcutaneously at 9-day intervals. The injection procedure is repeated at a different subcutaneous site until the highest dose of alum-adsorbed OVA is injected.

Three groups of wild-type C57BL/6 mice (each group: 10 mice) are analyzed: group 1: immunization (3 x alum-OVA), therapy with non-loaded polymer and PBS; group 2: immunization (3 x alum-OVA), therapy with loaded polymer and increasing doses of alum-OVA; and group 3: immunization (3 x alum-OVA), therapy with increasing doses of alum-OVA only.

Mice are bled at day 0 before immunization with OVA, at day 7 after immunization with OVA (before starting immunotherapy), and at day 9 after the last immunotherapeutic treatment. Analyses include the determination of serum levels of OVA-specific antibodies, immediate hypersensitivity in skin responses upon challenge via intradermal injection of OVA and the development of anaphylactic shock upon i.v. injection of OVA.

### Analysis of serum levels of OVA-specific antibodies.

Murine anti-OVA IgE and IgG subclasses are determined by ELISA. Plates are coated with 10 µg OVA in 100 µl 0.1 M NaHCO₃ for 6 h at 37°C, followed by blocking with 200 µl 3% BSA in PBS, pH 7.4, for 2 h at 37°C. After washing, 100 µl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3). Analysis is performed at 405 nm in a microplate autoreader.

### Analysis of serum levels of cytokines.

The cytokine supernatant is profiled using a panel of 27 cytokines including the Th2 cytokines IL-4, IL-5 and IL-13.

### Analysis of FOXP3 and GATA3 mRNA expression.

The read-out for T cell differentiation is FOXP3 and GATA3 mRNA expression, which are inversely regulated. In addition the release is followed in realtime using STAT6 responsive Luciferase systems reporter systems.

### Analysis of immediate cutaneous hypersensitivity.

Active cutaneous anaphylaxis is tested by skin test after i.v. injection of 200 µl of 0.5% Evans Blue dye in PBS, pH 7.4. Thereafter, the skin of the belly is shaved and four injection sites are marked with a felt tip pen on the skin. Two of the marked sites are injected intradermally with 50 µl PBS, pH 7.4, containing 50 µg OVA, and the other two sites with protein-free PBS, pH 7.4. After 15 min, the mice were killed by cervical dislocation and the skin is stripped off for inspection of the injection sites. The intensity of blue patch formation on the dorsal side of the skin, resulting from fluid extravasation into the injection site upon mast cell degranulation, is scored by two independent observers. Reactions are rated as positive when the diameter of the blue patch exceeds 5 mm, which is pre-marked on the mouse skin. The intensity of bluing is rated in the following manner: 0 = no blue patch formation; 1 = slight bluing; 2 = marked bluing; 3 = strong bluing.

### Analysis of anaphylactic shock symptoms.

Mice are injected i.v. 500 µg OVA in 200 µl 0.5% Evans Blue solution. After 15 min, symptoms of an anaphylactic shock are assessed including bluing (no = 0; slight = 1; strong = 2), pilo erection (no = 0; slight = 1; strong = 2), spontaneous activity (running around = 0; sitting passively = 1; lying = 2), and responsiveness to external stimuli (running away upon touching = 0; slight reaction = 1; no reaction = 2). The animals are considered to be in a state of shock if at least three of the four indicated symptoms are observed by two independent observers who are unaware of the sensitization status of each animal.

### Analysis of T cell phenotype in inguinal lymphnodes.

Right and left inguinal lymph nodes are removed and T cell phenotype is tested by *in vitro* re-stimulation with allergen (ELISPOT/Luminex).

### EXAMPLE 5: IMMUNOTHERAPY OF OVA-INDUCED ALLERGIC AIRWAY INFLAMMATION IN MICE

This example shows the efficacy of immunotherapy of OVA-sensitized mice exhibiting allergic airway inflammation after inhaled OVA administration, using increasing doses of subcutaneously injected alum-adsorbed OVA and co-injected polymer-embedded combinations of immune-modulator at the site of OVA presentation.

### Animals and materials.

Female wild-type C57BL/6 mice 6 to 10 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen. C3aR antagonist N²-[(2,2-dipheneylethoxy)acetyl]-L-arginine (SB290157; MW 526.5; solubility: 5 mg/ml DMSO or at least 10 mg/ml of a saline solution containing 5% ethanol and 10% Cremaphor-EL) is purchased from Santa Cruz Biotechnology, Inc. Calcipotriol (synonyms: MC 903, calcipotriene; MW 412,62; solubility: approx. 50 mg/ml of ethanol or approx. 0.15 mg/ml in a 1:5 solution of ethanol:PBS, pH 7.2) is purchased from Cayman Chemical Company.

The C5aR antagonist PMX53 (AcF-(OPdChaWR)) is synthesized as described (Finch et al., 1999). First, a linear peptide is synthesized on a peptide synthesizer using Fmoc chemistry (433A; Applied Biosystems, Foster City, CA), followed by formation of a lactam bridge in solution as described (Finch et al., 1999). The peptide is purified using reversed-phase high-performance liquid chromatography. The mass is confirmed by matrix-assisted laser desorption ionization/time-of-flight mass spectrometry.

The murine IL-4 mutant Q116D/Y119D (the murine equivalent of the human IL-4 double mutant R121D/Y124D) is prepared as described in Example 1.6.1.

### Preparation of alum-adsorbed ovalbumin (OVA).

For immunization, 100 µg of OVA are solved in 0.3 ml of PBS, pH 7.4, and mixed with 0.70 ml Imject Alum (Pierce/KMF). For immunotherapy, 10 mg of OVA are solved in 3.0 ml of PBS, pH 7.4, and mixed with 7.0 ml Imject Alum (Pierce/KMF). From this mixture containing 100 µg alum-adsorbed OVA in 0.1 ml, dilutions are prepared containing in 0.1 ml 90 µg, 60 µg, 30 µg, 15 µg, and 7.5 µg alum-adsorbed OVA.

### Allergic sensitization procedure.

Mice are immunized three times by i.p. injection of 100 µl alum-adsorbed OVA (10 µg OVA in 100 µl of PBS/Imject Alum). The second i.p. injection is performed 7 days after the first injection and the third injection 14 days after the first injection.

One week after the last injection the mice are exposed in a Plexiglas chamber (approx. 10 x 15 x 25 cm) to 1% (wt/vol) aerosolized OVA in 0.9% saline (using a nebulizer with an airflow rate of 10L/min), 30 min/day for 10 days.

### Immunotherapy.

One week after the final aerosol exposure, mice are subjected to two different modalities of immunotherapy, one based on conventional treatment with increasing doses of alum-adsorbed OVA, the other based on treatment with increasing doses of alum-adsorbed OVA in the presence of polymer-embedded immune-modulators including the murine IL-4/IL-13 antagonist QY, calcipotriol, the C3aR antagonist SB290157, and optionally the C5aR antagonist PMX53.

Subcutaneous administration of increasing doses of alum-adsorbed OVA is performed at 9-day intervals. The first subcutaneous administration of polymer-embedded immune-modulators (total volume: 300 µl; 150 µl are injected s.c. at two different sites) is performed 1 day before immunotherapy with alum-adsorbed OVA, at day 0 one hour before administration of alum-adsorbed OVA, and 24 hours after administration of alum-adsorbed OVA within the 9-day intervals. Increasing doses of OVA (7.5, 15, 30, 60, 90 µg) in 100 µl of PBS/Imject Alum are injected subcutaneously at the site of the gelled polymer composit (50 µl at each side of the gelled polymer). The injection procedure is repeated at a different subcutaneous site until the highest dose of alum-adsorbed OVA is injected.

For immunotherapy of mice with alum-adsorbed OVA in the presence of polymer-embedded immune-modulators, 150 µl of the PLGA-PEG-PLGA triblock copolymer of Example 1.1. (25% w/v polymer) in PBS, pH 7.4, are mixed with different amounts of immune modulators including 50 µl of murine IL-4/IL-13 antagonist QY in PBS, pH 7.4 (up to 50 µg, corresponding to 1.0 mg/ml), 30 µl of calcipotriol in a 1:5 solution of ethanol:PBS, pH 7.4 (up to 4.5 µg, corresponding to 150 µg/ml), 30 µl of C3aR antagonist SB290157 in 5% ethanol, 10% Cremaphor-EL, 85% saline (up to 300 µg SB290157, corresponding to 10 mg/ml), and 40 µl of C5aR antagonist PMX53 in PBS, pH 7.4, containing 10% ethanol (up to 200 µg, corresponding to 5 mg/ml). Optionally, 40 µl of C5aR antagonist PMX53 are replaced by 40 µl of PBS, pH 7.4.

For immunotherapy of mice with alum-adsorbed OVA only, 100 µl of PBS/Imject Alum containing increasing doses of OVA (7.5, 15, 30, 60, 90 µg) are injected subcutaneously at 9-day intervals. The injection procedure is repeated at a different subcutaneous site until the highest dose of alum-adsorbed OVA is injected.

### Experimental animal groups.

Three groups of wild-type C57BL/6 mice (each group: 10 mice) are analyzed: group 1: immunization (3 x alum-OVA), therapy with non-loaded polymer and PBS; group 2: immunization (3 x alum-OVA), therapy with increasing doses of alum-OVA only; group 3: immunization (3 x alum-OVA), therapy with loaded polymer and increasing doses of alum-OVA.

### Challenge procedure.

One week after completed immune-therapeutic treatment, mice are challenged with 1% aerosolized OVA (as described above), 30 min/day for 3 consecutive days.

### Measurement of AHR: Lung resistance.

Measurement of airway hyperreactivity (AHR), defined as bronchoconstriction in response to methacholine, is performed in two animals of each group. Three days after the final aerosol exposure, the mice are sacrificed by exsanguination after i.p. injection of a ketamine/xylazine overdose, and analyses of bronchoalveolar lavage (BAL), lung tissue, and blood samples are performed.

Measurements of pulmonary resistance (R_{L}) are performed in anesthetized, mechanically ventilated mice (Yiamouyiannis et al., 1999). After anesthetizing the animals with pentobarbital (75 mg/kg i.p. injection), the abdominal inferior vena cava is cannulated, and a tracheostomy catheter is placed. The chest is opened by a small anterior incision, and the animal is placed in a whole-body plethysmograph. Mechanical ventilation is established with a small rodent respirator delivering a 10 ml/kg tidal volume at 140 breaths/minute, with a positive end-expiratory pressure (PEEP) of 3 cm H₂O. Values for R_{L} are calculated by analysis of electrical signals proportional to lung volume, airflow, and transpulmonary pressure. Changes in lung volume are determined from the measured changes in plethysmographic pressure and are differentiated over time to obtain flow measurements. Transpulmonary pressure is obtained from the difference between measured pressures at the airway opening and within the plethysmograph. After the establishment of baseline lung function, the animal receives sequentially increasing intravenous doses of methacholine (Sigma; 3 to 3000 µg/ml in 1 ml/kg body weight increments). Maximal R_{L} responses are determined from measurements averaged over 6-second intervals. Pulmonary function is allowed to return to baseline values before each subsequent dose.

### Bronchoalveolar lavage (BAL) analysis.

The lungs from five animals of each group are lavaged *in situ* with five 1 ml aliquots of sterile saline, with 3 to 4 ml BAL fluid recovered from each animal. The BAL is centrifuged and resulting cell pellets are suspended in 250 µl saline. BAL protein concentrations are measured in the supernatants by the bicinchoninc acid (BCA) assay using the BCA^{™} Protein Assay Kit (Pierce, USA) and bovine serum albumin as standard. Total leukocytes are counted in a hemocytometer using trypan blue dye exclusion as a measure of viability. Cytospin slides are made and stained with May-Grunwald/Giemsa to determine the BAL cell differential. The remaining cells are analyzed by fluorescence flow cytometry. For these analyses, BAL samples are washed in phosphate-buffered saline (PBS) containing 0.2% bovine serum albumin and 0.1% NaN₃. Aliquots containing 10⁴ to 10⁵ cells are incubated with 100 µl of appropriately diluted antibodies for 30 min at 4°C. After staining, the cells are washed twice with the above PBS solution, and relative fluorescence intensities are determined on a 4-decade log scale by flow cytometric analysis using a FACScan (Becton Dickinson). Fluorescent monoclonal antibodies used for the fluorescence flow cytometric analyses are directed against B cell and T cell antigens including CD3ε, TCRβ, TCRδ, CD4, CD8, and CD45.

### Analysis of serum levels of OVA-specific antibodies.

Mice are bled at day 0 before immunization with OVA, one week after immunization with OVA (before starting immunotherapy), and two weeks after the last immunotherapeutic treatment. Analyses include the determination of serum levels of OVA-specific antibodies and cytokines.

Murine anti-OVA IgE and IgG subclasses are determined by ELISA. Plates are coated with 10 µg OVA in 100 µl 0.1 M NaHCO₃ for 6 h at 37°C, followed by blocking with 200 µl 3% BSA in PBS, pH 7.4, for 2 h at 37°C. After washing, 100 µl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3). Analysis is performed at 405 nm in a microplate autoreader.

### Analysis of serum levels of cytokines.

The cytokine supernatant is profiled using a panel of 27 cytokines including the TH2 cytokines IL-4, IL-5 and IL-13.

### Tissue analysis.

For tissue immunofluorescence, unmanipulated lungs (not exposed to BAL or methacholine) are excised, cut into small pieces, and are rapidly frozen in optimal cutting temperature embedding media. The pieces are then cut into 5 µm frozen sections using a Hacker cryostat, mounted onto microscope slides, and stored at -20°C. For immunofluorescence staining, the slides are fixed in acetone (-20°C) for 5 minutes, dried, and blocked with 1% ChromPure IgG solution (Jackson ImmunoResearch) for 30 min at room temperature. After two washes with PBS containing 1% NaN₃, specific fluorescent monoclonal antibodies are added to the tissue and incubated for 60 min in a humidity chamber. Slides are then washed twice with PBS containing 1% NaN₃, and then analyzed by fluorescence microscopy.

For staining with hematoxylin and eosin (H&E), unmanipulated lungs (not exposed to BAL or methacholine) are excised, fixed with 10% buffered formalin, and stained with H&E according to standard protocols.

### EXAMPLE 6: IMMUNOTHERAPY OF AUTOIMMUNE DIABETES IN MICE

This example shows the efficacy of peptide immunotherapy of spontaneous autoimmune diabetes (IDDM; insulin-dependent diabetes mellitus) in non-obese diabetic (NOD/Lt) mice using repetive subcutaneous injections of alum-adsorbed or polymer-embedded MT-hsp65-derived peptide and co-injected polymer-embedded combinations of immune-modulator at the presentation site of MT-hsp65-derived peptide p277.

The 65 kDa heat shock protein of *Mycobacterium tuberculosis* (MT-hsp65) contains a peptide sequence that is cross-reactive with a beta-cell target antigen (termed hsp65 cross-reactive; hsp65-CR) in non- obese diabetic (NOD/Lt) mice (Elias et al., 1990). The onset of beta-cell destruction is associated with the spontaneous development of anti-hsp65 T lymphocytes. Subsequently hsp65-CR becomes detectable in the sera of prediabetic mice (72.5 ± 8.3 days before IDDM) and some weeks later anti-hsp65 antibodies (44 ± 4.3 days before IDDM), anti-insulin antibodies (29 ± 5.5 days before IDDM), and anti-idiotypic antibodies to insulin (19 ± 2.7 days before IDDM) become detectable. The hsp65-CR, the autoantibodies, and the T cell reactivity then decline with the development of overt insulin-dependent diabetes mellitus. The mean age of onset of spontaneous IDDM for 14 out of 18 female NOD/Lt mice (77.8%) was 150 ± 8.3 days (Elias et al., 1990).

In young prediabetic NOD/Lt mice, MT-hsp65 can be used either to induce diabetes or to vaccinate against diabetes, depending on its form of administration. Four- to 5-week old female NOD/Lt mice treated with a single intraperitoneal (i.p.) injection of MT-hsp65 (50 µg) emulsified in incomplete Freund's adjuvans (IFA), developed within 30 days after the treatment diabetes, although the induced diabetes was transient and mice recovered at the age of 6-8 months. In contrast, treatment with a single i.p. injection of MT-hsp65 (50 µg) in PBS (non-immunogenic treatment) did not cause the development of diabetes within the period of 30 days and reduced the developmet of IDDM at later ages (6-8 months) to a significantly extent (Elias et al., 1990).

A 23-mer peptide (VLGGG CALLRC IPALD SLTPA NED; termed p277) derived from MT-hsp65 was also shown to be protective (Elias et al., 1991). None of 10 one month old female NOD/Lt mice treated with a single i.p. injection of 50 µg of p277 emulsified in IFA, followed two weeks later by another i.p. injection of MT-hsp65 (50 µg) emulsified in IFA, developed diabetes within a period of 30 days. Furthermore, the incidence of spontaneous IDDM in these mice at the age of 8 months was reduced to approximately 30% (Elias et al., 1991).

Important for immunotherapeutic purposes is the fact that p277 is not diabetogenic. None of 7 one month old female prediabetic NOD/Lt mice treated with a single i.p. injection of 50 µg of p277 emulsified in IFA, developed blood glucose levels higher than 130 mg/dl within a period of 3 weeks (Elias et al., 1991). Due to the non-diabetogenic nature of p277, immunotherapy does not require the application of increasing doses of p277. Instead, the immunotherapy is performed with repetitive doses of 100 µg of p277.

### Animals.

Female NOD/Lt mice 4 weeks of age are purchased from Jackson Laboratory. The animals are kept under specific pathogen-free conditions.

NOD mice have polymorphisms in the Idd3 locus which are linked to IL-2 production. IL-2 promotes either immunity or tolerance in a concentration dependent fashion by acting on T helper cells, CTL and NK cells. Low amounts of IL-2 may be needed to promote survival of Treg in mice. Loss of IL-2 can thereby contribute to the development of autoimmunity in NOD mice. Furthermore, NOD mice have a mutation in exon 2 of the CTLA-4 gene, which causes it to be spliced incorrectly. CTLA-4 plays a major role in suppressing the T-cell immune response. Without the proper functioning of CTLA-4 T-cells attack the insulin producing cells. This results in Type 1 Diabetes.

Diabetes development in NOD mice is characterized by insulitis, a leukocytic infiltrate of the pancreatic islets. Marked decreases in pancreatic insulin content occur in females at about 12 weeks of age (Gaskins et al., 1992) and several weeks later in males. Onset of diabetes is marked by moderate glycosuria (1+ reading on Lilly Tes-TapeTM) and a non-fasting plasma glucose higher than 250 mg/dl. It is best to begin monitoring for development of glycosuria at weekly intervals starting at 10 weeks of age.

### Materials.

Imject Alum is obtained from Pierce/KMF. S-methylglutathione (GSM; solubility in water: 2.61 g/liter (8.1 mM)) and salicylic acid (SA; solubility in water: 14 mM, in ethanol/water (10:90 vol) 21 mM, in ethanol/water (20:80 vol) 35 mM, in ethanol/water (30:70 vol) 81 mM, in ethanol/water (40:60 vol) 277 mM, in ethanol/water (50:50 vol) 526 mM) are obtained from Sigma. C3aR antagonist N²-[(2,2-dipheneylethoxy)acetyl]-L-arginine (SB290157; MW 526.5; solubility: 5 mg/ml DMSO or at least 10 mg/ml of a saline solution containing 5% ethanol and 10% Cremaphor-EL) is purchased from Santa Cruz Biotechnology, Inc. Calcipotriol (synonyms: MC 903, calcipotriene; MW 412,62; solubility: approx. 50 mg/ml of ethanol or approx. 0.15 mg/ml in a 1:5 solution of ethanol:PBS, pH 7.2) is purchased from Cayman Chemical Company. The C5aR antagonist PMX53 (AcF-(OPdChaWR)) is synthesized as described in Example 1.1. TNFR1-specific antisense ODN comprising partial phosphorothioate linkages (pPT) are synthesized and formulated with Cellfectin as described in Example 1.4.

The MT-hsp65-derived 23-mer peptide (VLGGG CALLRC IPALD SLTPA NED; termed p277) is synthesized by solid-phase peptide synthesis and purified by HPLC according to standard procedures.

### Cloning, expression and purification of MT-hsp65.

The 65 kDa heat shock protein of *Mycobacterium tuberculosis* (MT-hsp65) and control antigen from E. coli transfected without the MT-hsp65 gene are prepared as described (Thole et al., 1987; van Eden et al., 1988).

### Preparation of alum-adsorbed peptide p277.

For immunotherapy with alum-asdorbed p277, 10 mg of p277 are solved in 3.0 ml of PBS, pH 7.4, and added to 7.0 ml Imject Alum (Pierce/KMF), giving a concentration of 100 µg alum-adsorbed p277 in 0.1 ml alum suspension.

### Preparation of polymer-embedded peptide p277.

For immunotherapy with polymer-embedded p277, 10 mg of p277 is solved in 3.0 ml of PBS, pH 7.4, and added to 7.0 ml of the PLGA-PEG-PLGA triblock copolymer of Example 1.1. (25% w/v polymer) in PBS, pH 7.4, giving a concentration of of 100 µg p277 in 0.1 ml hydrogel solution.

### Methods to identify autoimmune diabetes (IDDM).

Detection of serum anti-hsp65 antibodies (44 ± 4.3 days before overt IDDM) and serum anti-insulin antibodies (29 ± 5.5 days before overt IDDM) are used to analyze the development of autoimmune diabetes. Overt IDDM is identified by elevated blood glucose levels of approx. 200 mg/dl or greater.

For the analysis of serum anti-hsp65 antibodies and serum anti-insulin antibodies, microtiter plates are coated with 5 µg MT-hsp65 in 100 µl 0.1 M NaHCO₃ or 5 µg insulin in 100 µl 0.1 M NaHCO₃ for 6 h at 37°C, followed by blocking with 200 µl 3% BSA in PBS, pH 7.4, for 2 h at 37°C. After washing, 100 µl of 1:50 serum dilutions with PBS, pH 7.4, containing 1% BSA are incubated overnight at 4°C. The amount of bound antibody is analyzed using horseradish peroxidise-conjugated goat antimouse immunoglobulin (Becton Dickinson). Analysis is performed at 405 nm in a microplate autoreader.

For the determination of blood glucose, blood is removed from the tail vein of individual mice and the concentration of glucose is measured using a glucose meter (Bayer Diagnostics). A mouse is considered to be diabetic if the blood glucose is >3 standard deviations above the mean of that measured in nondiabetic or prediabetic control mice without insulitis, approx. 200 mg/dl or greater.

### Immunotherapy.

Three sets of female NOD/Lt mice are subjected to immunotherapy, the first at the age of 110-120 days characterized the detection of serum anti-hsp65 antibodies (approx. 6 weeks before overt IDDM), the second at the age of 130-140 days characterized the detection of serum anti-hsp65 antibodies and serum anti-insulin antibodies (approx. 3 weeks before overt IDDM), the third at the age of 180-200 days with overt IDDM characterized by blood glucose levels of approx. 200 mg/dl or greater.

In each set of female NOD/Lt mice the immunotherapeutic treatment procedure is repeated eight times at 5-day intervals.

Each set of mice is subjected to four different modalities of immunotherapy, one based on conventional treatment with of alum-adsorbed MT-hsp65-derived 23-mer peptide (VLGGG CALLRC IPALD SLTPA NED; termed p277) using 100 µg of p277 per injection, another based on treatment with alum-adsorbed p277 using 100 µg of p277 per injection in the presence of polymer-embedded immune-modulators including calcipotriol, the C3aR antagonist SB290157, optionally the C5aR antagonist PMX53, and a combination of TNFR1-specific inhibitors (salicylic acid, 22mer pPT antisense ODN, and optionally GSM), another being identical with the second approach but using 100 µg of polymer-embedded p277 instead of alum-adsorbed p277, and the residual one serving as control (injection of PBS, pH 7.4, only).

Subcutaneous administration of alum-adsorbed p277 or polymer-embedded p277 is performed at 5-day intervals. The first subcutaneous administration of polymer-embedded immune-modulators (total volume: 300 µl; 150 µl are injected s.c. at two different sites) is performed 1 day before immunotherapy with alum-adsorbed p277 or polymer-embedded p277, concomitantly with the administration of alum-adsorbed p277 or polymer-embedded p277, and 24 hours after administration of alum-adsorbed p277 or polymer-embedded p277 within the 5-day intervals.

Doses of 100 µg p277 in 100 µl of PBS/Imject Alum or in 100 µl of a PBS/hydrogel solution (PLGA-PEG-PLGA triblock copolymer of Example 1.1.; 25% w/v polymer) are injected subcutaneously at the site of the gelled polymer composit containing the immune-modulators (50 µl at each side of the gelled polymer).

For immunotherapy of mice with alum-adsorbed p277 or polymer-embedded p277 in the presence of polymer-embedded immune-modulators, 150 µl of the PLGA-PEG-PLGA triblock copolymer of Example 1.1. (25% w/v polymer) in PBS, pH 7.4, are mixed with different amounts of immune modulators including 30 µl of calcipotriol in a 1:5 solution of ethanol:PBS, pH 7.4 (up to 150 µg/ml; final concentration in 300 µl hydrogel compostion: up to 4.5 µg), 30 µl of C3aR antagonist SB290157 in 5% ethanol, 10% Cremaphor-EL, 85% saline (up to 10 mg/ml; final concentration in 300 µl hydrogel compostion: up to 300 µg SB290157), 40 µl of C5aR antagonist PMX53 in PBS, pH 7.4, containing 10% ethanol (up to 5 mg/ml; final concentration in 300 µl hydrogel compostion: up to 200 µg), 10 µl of sodium salicylate (SA) in ethanol/water, 50/50 vol (up to 375 mM; final concentration in 300 µl hydrogel compostion: up to 12.5 mM SA and 1.7% ethanol), 27.5 µl of TNFR1-ODN/Cellfectin complexes (comprising a mixture of 25 µl of up to 1.0 µM TNFR1-specific pPT antisense ODN in PBS, 7.4, and 2.5 µl of 1 mg/ml Cellfectin in PBS, pH 7.4), and 12.5 µl of S-methylglutathione (GSM) in PBS, pH 7.4 (up to 5 mM; final concentration in 300 µl hydrogel compostion: up to 0.2 mM GSM).

Alternatively, 40 µl of C5aR antagonist PMX53 are replaced by 40 µl of murine IL-4/IL-13 antagonist QY in PBS, pH 7.4 (up to 1.0 mg/ml; final concentration in 300 µl hydrogel compostion: up to 40 µg), or by 40 µl PBS, pH 7.4.

### Analyses during and after immunotherapy.

Analyses include the determination of serum anti-hsp65 antibodies, serum anti-insulin antibodies, and blood glucose levels by methods described above. Analyes are performed at day 8, 18, 28 and 38 after the start of immunotherapy and two weeks after completion of immunotherapy (49 days after the start of immunotherapy). A final analysis is performed five weeks after completion of immunotherapy (corresponding to 70 days after the start of immunotherapy) to evaluate the incidence of IDDM.

### EXAMPLE 7: IMMUNOTHERAPY OF ADJUVANT ARTHRITIS IN LEWIS RATS

This example shows the efficacy of peptide immunotherapy of adjuvant arthritis in rats induced by intradermal injection of a mycobacterial 65 kDa heat shock protein (hsp65) emulsified in incomplete Freund's adjuvans, using repetive subcutaneous injections of alum-adsorbed or polymer-embedded MT-hsp65-derived peptide and co-injected polymer-embedded combinations of immune-modulator at the presentation site of MT-hsp65-derived peptide.

Adjuvant arthritis is an animal model for rheumatoid arthtitis and is induced in Lewis rats by immunization with heat-killed *Mycobacterium tuberculosis* in Freund's incomplete adjuvant (Van Eden et al., 1988). Evidence for a role of Hsp65 in adjuvant arthritis was obtained by characterization of arthritogenic and protective T cell clones generated from arthritic rats (Cohen et al., 1985). The epitope recognized by thes clones was first identified as a nine amino acid sequence (180-188) of mycobacterial Hsp65 (Van Eden et al., 1988) and later shortened to amino acid sequence 180-186 (Van der Zee et al., 1989). Vaccination with the nonapeptide 180-188 has been shown to induce protection against adjuvant arthritis (Yang et al., 1990). In separate experiments, 11 out of 16 Lewis rats immunized three times with 100 µg of the nonapeptide emulsified in Freund's incomplete adjuvant (FIA) prior to induction of adjuvant arthritis were completely protected against subsequently induced adjuvant arthritis.

Important for therapeutic purposes is the fact that the nonapeptide is not arthritogenic. As demonstrated in the study of Yang et al. (1990), none of the rats treated 35 days before the induction of adjuvant arthritis by i.p. injection of 100 µg of the nonapeptide emulsified in FIA, showed any clinical symptoms of adjuvant arthritis during the pretreatment period. Due to the non-arthritogenic nature of the nonapeptide, immunotherapy does not require the application of increasing doses of the nonapeptide. Instead, the immunotherapy is performed with repetitive doses of 100 µg of the nonapeptide.

### Animals.

Inbred male Lewis rats (6-8 weeks of age) are obtained from Charles River Laboratories.

### Materials.

Imject Alum is obtained from Pierce/KMF. S-methylglutathione (GSM; solubility in water: 2.61 g/liter (8.1 mM)) and salicylic acid (SA; solubility in water: 14 mM, in ethanol/water (10:90 vol) 21 mM, in ethanol/water (20:80 vol) 35 mM, in ethanol/water (30:70 vol) 81 mM, in ethanol/water (40:60 vol) 277 mM, in ethanol/water (50:50 vol) 526 mM) are obtained from Sigma. C3aR antagonist N²-[(2,2-dipheneylethoxy)acetyl]-L-arginine (SB290157; MW 526.5; solubility: 5 mg/ml DMSO or at least 10 mg/ml of a saline solution containing 5% ethanol and 10% Cremaphor-EL) is purchased from Santa Cruz Biotechnology, Inc. Calcipotriol (synonyms: MC 903, calcipotriene; MW 412,62; solubility: approx. 50 mg/ml of ethanol or approx. 0.15 mg/ml in a 1:5 solution of ethanol:PBS, pH 7.2) is purchased from Cayman Chemical Company. The C5aR antagonist PMX53 (AcF-(OPdChaWR)) is synthesized as described in Example 1.1. TNFR1-specific antisense ODN comprising partial phosphorothioate linkages (pPT) are synthesized and formulated with Cellfectin as described in Example 1.4.

### Synthesis of the nonapeptide 180-188.

The nonapeptide Thr-Phe-Gly-Leu-Gln-Leu-Glu-Leu-Thr representing sequence 180-188 of Hsp65 from *Mycobacterium bovis* BCG (van Eden et al., 1988) is synthesized by solid-phase peptide synthesis according to standard procedures.

### Cloning, expression and purification of mycobacterial hsp65.

The 65 kDa heat shock protein of *Mycobacterium bovis BCG* (MB-hsp65) and control antigen from E. coli transfected without the MB-hsp65 gene are prepared as described (Thole et al., 1987; van Eden et al., 1988).

### Preparation of alum-adsorbed nonapeptide 180-188.

For immunotherapy with alum-adsorbed nonapeptide 180-188, 10 mg of nonapeptide 180-188 are solved in 3.0 ml of PBS, pH 7.4, and added to 7.0 ml Imject Alum (Pierce/KMF) giving a concentration of 100 µg nonapeptide 180-188 in 0.1 ml PBS/alum suspension.

### Preparation of polymer-embedded nonapeptide 180-188.

For immunotherapy with polymer-embedded nonapeptide 180-188, 10 mg of nonapeptide 180-188 is solved in 3.0 ml of PBS, pH 7.4, and added to 7.0 ml of the PLGA-PEG-PLGA triblock copolymer of Example 1.1. (25% w/v polymer) in PBS, pH 7.4, giving a concentration of 100 µg nonapeptide 180-188 in 0.1 ml PBS/hydrogel solution.

### Arhritis induction with Mycobacterium tuberculosis (MT).

Rats are immunized intradermally at the base of the tail with 100 µl of 10 mg/ml heat-killed MT H37Ra (Difco) in mineral oil (incomplete Freund's adjuvant; IFA) as described (Hogervorst et al., 1992) .

Inducing arthritis as described above, clinical symptoms of adjuvant arthritis become evident at day 10, reach a peak at day 20 (highest arthritis score), and decline continuously thereafter until symptoms disappear around day 50 (Hogervorst et al., 1992).

### Immunotherapy.

Three sets of male Lewis rats are subjected to immunotherapy, the first 1 week after arthritis induction (approx. 3 days before clinical symptoms of adjuvant arthritis become evident), the second 2 weeks after arthritis induction (approx. 4 days after appearance of clinical symptoms of adjuvant arthritis), and the third 3 weeks after arthritis induction (approx. at the peak of clinical symptoms of adjuvant arthritis). The day of arthritis induction is designated as day 0.

In each set of male Lewis rats the immunotherapeutic treatment procedure is repeated eight times at 5-day intervals.

Each set of mice is subjected to four different modalities of immunotherapy, one based on conventional treatment with of alum-adsorbed nonapeptide 180-188 using 100 µg of nonapeptide 180-188 per injection, another based on treatment with alum-adsorbed nonapeptide 180-188 using 100 µg of nonapeptide 180-188 per injection in the presence of polymer-embedded immune-modulators including calcipotriol, the C3aR antagonist SB290157, optionally the C5aR antagonist PMX53, and a combination of TNFR1-specific inhibitors (salicylic acid, 22mer pPT antisense ODN, and optionally GSM), another being identical with the second approach but using 100 µg of polymer-embedded nonapeptide 180-188 instead of alum-adsorbed nonapeptide 180-188, and the residual one serving as control (injection of PBS, pH 7.4, only).

Subcutaneous administration of alum-adsorbed nonapeptide 180-188 or polymer-embedded nonapeptide 180-188 is performed at 5-day intervals. Subcutaneous administrations of polymer-embedded immune-modulators (total volume: 300 µl; 150 µl are injected s.c. at two different sites) are performed 1 day before immunotherapy with alum-adsorbed nonapeptide 180-188 or polymer-embedded nonapeptide 180-188, concomitantly with the administration of alum-adsorbed nonapeptide 180-188 or polymer-embedded nonapeptide 180-188, and 24 hours after administration of alum-adsorbed nonapeptide 180-188 or polymer-embedded nonapeptide 180-188 within the 5-day intervals.

Nonapeptide 180-188 (100 pg) in 100 µl of PBS/Imject Alum or in 100 µl of a PBS/hydrogel solution (PLGA-PEG-PLGA triblock copolymer of Example 1.1.; 25% w/v polymer) is injected subcutaneously at the site of the gelled polymer composit (50 µl at each side of the gelled polymer).

For immunotherapy with alum-adsorbed nonapeptide 180-188 or polymer-embedded nonapeptide 180-188 in the presence of polymer-embedded immune-modulators, 150 µl of the PLGA-PEG-PLGA triblock copolymer of Example 1.1. (25% w/v polymer) in PBS, pH 7.4, are mixed with different amounts of immune modulators including 30 µl of calcipotriol in a 1:5 solution of ethanol:PBS, pH 7.4 (up to 150 µg/ml; final concentration in 300 µl hydrogel compostion: up to 4.5 µg), 30 µl of C3aR antagonist SB290157 in 5% ethanol, 10% Cremaphor-EL, 85% saline (up to 10 mg/ml; final concentration in 300 µl hydrogel compostion: up to 300 µg SB290157), 40 µl of C5aR antagonist PMX53 in PBS, pH 7.4, containing 10% ethanol (up to 5 mg/ml; final concentration in 300 µl hydrogel compostion: up to 200 µg), 10 µl of sodium salicylate (SA) in ethanol/water, 50/50 vol (up to 375 mM; final concentration in 300 µl hydrogel compostion: up to 12.5 mM SA and 1.7% ethanol), 27.5 µl of TNFR1-ODN/Cellfectin complexes (comprising a mixture of 25 µl of up to 1.0 µM TNFR1-specific pPT antisense ODN in PBS, 7.4, and 2.5 µl of 1 mg/ml Cellfectin in PBS, pH 7.4), and 12.5 µl of S-methylglutathione (GSM) in PBS, pH 7.4 (up to 5 mM; final concentration in 300 µl hydrogel compostion: up to 0.2 mM GSM).

Alternatively, 40 µl of C5aR antagonist PMX53 are replaced by 40 µl of murine IL-4/IL-13 antagonist QY in PBS, pH 7.4 (up to 1.0 mg/ml; final concentration in 300 µl hydrogel compostion: up to 40 µg), or by 40 µl PBS, pH 7.4.

### Evaluation of clinical signs of arthritis.

Rats are examined daily for clinical signs of arthritis (erythema, swelling and deformity) and an arthritis index is assigned as described (Taurog et al., 1988): 0, no evidence of disease; 1, inflammation of small digital joints; 2, inflammation of ankle and joints; 3 inflammation of foot-pad; and 4, inflammation of entire foot. Each paw is given a score between 0 and 4, the highest score being 16 per rat.

Arthritis can also be confirmed and evaluated by examination of radiographs (Taurog et al., 1988). Rats are anaesthetized with Evipan-sodium (100 mg/kg, Bayer) and radiographs are taken for fore and hind limbs of rats. Scores of 1-4 are assigned to each joint for peri-osteal reaction, bony erosions, joint space narrowing and joint space destruction.

### Challenge with Mycobacterium tuberculosis (MT) after immunotherypy.

10 weeks after induction of adjuvant arthritis, rats are challenged intradermally at the base of the tail with 100 µl of 10 mg/ml heat-killed MT H37Ra (Difco) in incomplete Freund's adjuvant. The potential relapse of adjuvant arthritis is observed for another 5 weeks.

### EXAMPLE 8: IMMUNOTHERAPY OF EXPERIMENTAL AUTOIMMUNE ENCEPHALOMYELITIS (EAE) IN MICE

This example shows the efficacy of peptide immunotherapy of remitting-relapsing EAE in SJL mice induced by proteolipid protein fragment 139-151 (PLP₁₃₉₋₁₅₁) emulsified in complete Freund's adjuvant (CFA), using increasing doses of subcutaneously injected alum-adsorbed or polymer-embedded PLP₁₃₉₋₁₅₁ and co-injected polymer-embedded combinations of immune-modulator at the site of PLP₁₃₉₋₁₅₁ presentation. This model is used for testing the efficacy of therapy on the development of EAE relapses.

PLP₁₃₉₋₁₅₁/CFA-induced remitting-relapsing EAE in SJL mice most strongly resembles the remitting-relapsing form of MS, the most common form of MS. Mice develop a first episode of paralysis 11-15 days after immunization. The first wave of EAE lasts several days (peak of disease: 1-3 days) and, similar to most MS patients, most mice fully or almost fully recover from this first wave of paralysis within 7-10 days. After a disease-free period, which can last from one day to several months, 50-80% of the mice develop a second wave of paralysis (relapse) during the first 5-7 weeks after immunization. After several months, almost all mice will have relapsed. As mice are cobserved longer, more relapses will occur and less recovery will occur. Each individual mouse will have a different course of EAE after the the first relapse. Concomitant injection of pertussis toxin (PTX) in PBS on the day of immunization enhances the development of EAE (first episode 9-13 days after immunization) and increases the severity of the first wave, but reduces the incidence (40-60% during the first 5-7 weeks after immunization) and severity of relapses in SJL mice.

The ability of T cell epitope-containing peptides to down-regulate the disease course of EAE is well documented (for a review, see Badawi and Siahaan, 2012). For example, EAE induced with a combination of myelin basic protein (MBP) and myelin oligodendrocyte glycoprotein (MOG) has been ameliorated by i.v. administration of a combination of MBP- and MOG-derived peptides (125 nmol each) every other day from day 7 to day 19 after EAE induction (Leadbetter et al., 1998).

### Materials.

PLP₁₃₉₋₁₅₁ peptide emulsified in complete Freund's adjuvant is obtained from Hooke Laboratories, Inc., USA (Hooke Kit EK-0120), Imject Alum from Pierce/KMF. S-methylglutathione (GSM; solubility in water: 2.61 g/liter (8.1 mM)) and salicylic acid (SA; solubility in water: 14 mM, in ethanol/water (10:90 vol) 21 mM, in ethanol/water (20:80 vol) 35 mM, in ethanol/water (30:70 vol) 81 mM, in ethanol/water (40:60 vol) 277 mM, in ethanol/water (50:50 vol) 526 mM) are obtained from Sigma. C3aR antagonist N²-[(2,2-dipheneylethoxy)acetyl]-L-arginine (SB290157; MW 526.5; solubility: 5 mg/ml DMSO or at least 10 mg/ml of a saline solution containing 5% ethanol and 10% Cremaphor-EL) is purchased from Santa Cruz Biotechnology, Inc. Calcipotriol (synonyms: MC 903, calcipotriene; MW 412,62; solubility: approx. 50 mg/ml of ethanol or approx. 0.15 mg/ml in a 1:5 solution of ethanol:PBS, pH 7.2) is purchased from Cayman Chemical Company. The C5aR antagonist PMX53 (AcF-(OPdChaWR)) is synthesized as described in Example 1.1. TNFR1-specific antisense ODN comprising partial phosphorothioate linkages (pPT) are synthesized and formulated with Cellfectin as described in Example 1.4.

### Synthesis of PLP₁₃₉₋₁₅₁ peptide.

The 13-mer peptide HSLGKWLGHPDKF (MW 1521.73) corresponding to amino acid residues 139-151 of proteolipid protein (PLP) is synthesized by solid-phase peptide synthesis and purified by HPLC according to standard procedures.

### Preparation of alum-adsorbed PLP₁₃₉₋₁₅₁ peptide.

For immunotherapy with alum-adsorbed PLP₁₃₉₋₁₅₁ peptide, 20 mg of PLP₁₃₉₋₁₅₁ peptide is solved in 3.0 ml of PBS, pH 7.4, and added to 7.0 ml Imject Alum (Pierce/KMF), giving a mixture containing 200 µg (approx. 130 nmol) alum-adsorbed PLP₁₃₉₋₁₅₁ peptide in 0.1 ml suspension.

### Preparation of polymer-embedded PLP₁₃₉₋₁₅₁ peptide.

For immunotherapy, 20 mg of PLP₁₃₉₋₁₅₁ peptide is solved in 3.0 ml of PBS, pH 7.4, and added to 7.0 ml of the PLGA-PEG-PLGA triblock copolymer of Example 1.1. (25% w/v polymer) in PBS, pH 7.4, giving a mixture containing 200 µg (approx. 130 nmol) PLP₁₃₉₋₁₅₁ peptide in 0.1 ml gel solution.

### Animals.

Female SJL/J mice (9-10 weeks of age) are obtained from the Jackson Laboratory. For most uniform EAE development, all mice are the same age. Mice are acclimated for at least 2 weeks before immunization.

### EAE induction.

Mouse stress induced any time after approximately 3 days before immunization (e.g., by excessive handling of mice or excessive noise strongly) reduces both EAE incidence and severity in SJL mice. To reduce stress effects induced by subcutaneous injection of PLP₁₃₉₋₁₅₁ in CFA and by subsequent therapeutic injections, sham dosing is performed 7 and 4 days before immunization of the mice. Unlike other EAE models, the stress of treatment and administration of therapeutics continues to affect the disease in SJL mice even after clinical signs of EAE appear.

In addition, a stress minimizing injection technique is applied including a) gripping the mouse tail with finger and thumb of the right hand, b) restraining the mouse against the cage with three remaining fingers of the right hand, c) restraining the mouse with two fingers of the left hand behind its head, and d) using the right hand for subcutaneous injection at 4 sites on the back of the mouse, with 0.05 mL of emulsion at each site: on the upper back, on the upper back, approximately 1 cm away from the initial injection, on the lower back, and on the lower back, approximately 1 cm away from the previous injection.

Using this technique, EAE is consistently induced in 90-100% of the mice, with onset of paralysis between 11 to 15 days after immunization.

All mice develop obvious bumps of emulsion at the injection sites 2 to 4 days after injection. In most mice, the emulsion remains at the site of injection for the duration of the experiment. Approximately 10% to 40% of the mice clear the emulsion by developing skin ulcers at injection sites. Most of the time, these ulcers do not require treatment. They usually heal in a few days with scar formation. In some mice, alopecia will develop at the site of injection 5 to 7 days after injection.

As soon as the first signs of paralysis occur, mice are provided with food pellets and wet food on the floor of the cage, and easily accessible water. HydroGel (ClearH2O, Portland ME) may be used as a source of water during the most severe paralysis. Even mice which develop very severe first wave EAE (score 4) show almost always some recovery within 2 or 3 days. If mice are unable to reach water due to temporary paralysis, Ringer's solution is administered subcutaneously.

The first wave of paralysis is associated with a loss of body weight which is mostly regained on recovery from the first wave. In each subsequent wave there is body weight loss at onset, but not as dramatic as in the first wave. Most of the body weight is regained again at recovery.

### EAE scoring.

Mice are checked for signs of EAE daily, starting on day 7 after the immunization. Following scoring guidelines are used:
score 0 No obvious changes in motor functions of the mouse in comparison to non-immunized mice. When picked up by the tail, the tail has tension and is erect. Hind legs are usually spread apart. When the mouse is walking, there is no gait or head tilting.
score 1 Limp tail. When the mouse is picked up by the tail, instead of being erect, the whole tail drapes over your finger.
score 2 Limp tail and weakness of hind legs. When mouse is picked up by tail, legs are not spread apart, but held closer together. When the mouse is observed when walking, it has a clearly apparent wobbly walk.
Score 3 Limp tail and complete paralysis of hind legs (most common), or limp tail with paralysis of one front and one hind leg, or all of a) severe head tilting, b) walking only along the edges of the cage, c) pushing against the cage wall, and d) spinning when picked up by the tail.
Score 4 Limp tail, complete hind leg and partial front leg paralysis. Mouse is minimally moving around the cage but appears alert and feeding. Usually, euthanasia is recommended after the mouse scores level 4 for 2 days. When the mouse is euthanized because of severe paralysis, score of 5 is entered for that mouse for the rest of the experiment.
Score 5 Complete hind and complete front leg paralysis, no movement around the cage. Mouse is spontaneously rolling in the cage. Mouse is found dead due to paralysis. If mouse is alive, euthanize the mouse immediately if it scores 5. Once mouse is scored 5, the same score is entered for all the days for the rest of the experiment.

In-between scores (i.e. 0.5, 1.5, 2.5, 3.5) are used when the clinical picture lies between two defined scores.

The mean maximum score (MMS) of the first paralytic episode ranges usually between 2.5 and 3.5, and the MMS of the second paralytic episode between 1.5 and 2.5.

### Immunotherapy.

To evaluate the therapeutic efficacy of peptide immunotherapy, treatment is initiated in one set of SJL mice at the onset of clinical signs of EAE and in another set of SJL mice at the start of recovery from the first wave of EAE (late therapeutic approach). The day of EAE induction is designated as day 0.

In each set of mice the immunotherapeutic treatment procedure is repeated eight times at 5-day intervals.

Each set of mice is subjected to four different modalities of immunotherapy one based on conventional treatment with of alum-adsorbed PLP₁₃₉₋₁₅₁ peptide using 200 µg of PLP₁₃₉₋₁₅₁ peptide per injection, another based on treatment with alum-adsorbed PLP₁₃₉₋₁₅₁ peptide using 200 µg of PLP₁₃₉₋₁₅₁ peptide per injection in the presence of polymer-embedded immune-modulators including calcipotriol, the C3aR antagonist SB290157, optionally the C5aR antagonist PMX53, and a combination of TNFR1-specific inhibitors (salicylic acid, 22mer pPT antisense ODN, and optionally GSM), another being identical with the second approach but using 200 µg of polymer-embedded PLP₁₃₉₋₁₅₁ peptide instead of alum-adsorbed PLP₁₃₉₋₁₅₁ peptide, and the residual one serving as control (injection of PBS, pH 7.4, only). Each group consists of 15 to 20 mice to ensure that enough mice relapse for a reliable statistical analysis.

Subcutaneous administration of alum-adsorbed PLP₁₃₉₋₁₅₁ peptide or polymer-embedded PLP₁₃₉₋₁₅₁ peptide is performed at 5-day intervals. The first subcutaneous administration of polymer-embedded immune-modulators (total volume: 300 µl; 150 µl are injected s.c. at two different sites) is performed 1 day before immunotherapy with alum-adsorbed PLP₁₃₉₋₁₅₁ peptide or polymer-embedded PLP₁₃₉₋₁₅₁ peptide, concomitantly with the administration of alum-adsorbed PLP₁₃₉₋₁₅₁ peptide or polymer-embedded PLP₁₃₉₋₁₅₁ peptide, and 24 hours after administration of alum-adsorbed PLP₁₃₉₋₁₅₁ peptide or polymer-embedded PLP₁₃₉₋₁₅₁ peptide within the 5-day intervals.

PLP₁₃₉₋₁₅₁ peptide (200 µg) in 100 µl of PBS/Imject Alum or in 100 µl hydrogel solution is injected subcutaneously at the site of the gelled polymer composit (50 µl at each side of the gelled polymer).

For immunotherapy with alum-adsorbed PLP₁₃₉₋₁₅₁ peptide or polymer-embedded PLP₁₃₉₋₁₅₁ peptide in the presence of polymer-embedded immune-modulators, 150 µl of the PLGA-PEG-PLGA triblock copolymer of Example 1.1. (25% w/v polymer) in PBS, pH 7.4, are mixed with different amounts of immune modulators including 30 µl of calcipotriol in a 1:5 solution of ethanol:PBS, pH 7.4 (up to 150 µg/ml; final concentration in 300 µl hydrogel compostion: up to 4.5 µg), 30 µl of C3aR antagonist SB290157 in 5% ethanol, 10% Cremaphor-EL, 85% saline (up to 10 mg/ml; final concentration in 300 µl hydrogel compostion: up to 300 µg SB290157), 40 µl of C5aR antagonist PMX53 in PBS, pH 7.4, containing 10% ethanol (up to 5 mg/ml; final concentration in 300 µl hydrogel compostion: up to 200 µg), 10 µl of sodium salicylate (SA) in ethanol/water, 50/50 vol (up to 375 mM; final concentration in 300 µl hydrogel compostion: up to 12.5 mM SA and 1.7% ethanol), 27.5 µl of TNFR1-ODN/Cellfectin complexes (comprising a mixture of 25 µl of up to 1.0 µM TNFR1-specific pPT antisense ODN in PBS, 7.4, and 2.5 µl of 1 mg/ml Cellfectin in PBS, pH 7.4), and 12.5 µl of S-methylglutathione (GSM) in PBS, pH 7.4 (up to 5 mM; final concentration in 300 µl hydrogel compostion: up to 0.2 mM GSM).

Alternatively, 40 µl of C5aR antagonist PMX53 are replaced by 40 µl of murine IL-4/IL-13 antagonist QY in PBS, pH 7.4 (up to 1.0 mg/ml; final concentration in 300 µl hydrogel compostion: up to 40 µg), or by 40 µl PBS, pH 7.4.

### Readouts of immunotherapy.

The most important readouts in this EAE model are a) incidence of relapse and b) MMS of the relapse period, calculated for all mice. The MMS of only those mice which relapse rarely shows significant differences between groups because only a fraction of mice relapse and relapse severity tends to be variable even within a single group. The MMS of the relapse period is more sensitive because it is based on all mice in the group and reflects both relapse severity and residual EAE severity in those mice which do not relapse.

### LITERATURE

Abbott D.J., et al., BMC Immunol. 12: 72; 2011.
Abdulamir A.S., et al, J. Invest. Allergol. Clin. Immunol. 19: 218-224; 2009.
Abe M., et al., J. Immunol. 167: 4651-4660; 2001.
Abramson M.J., et al. Cochrane Database Syst. Rev. CD001186; 2003.
Adamy C., et al., J. Mol. Cell. Cardiology 43: 344-353; 2007.
Akbar A.N., et al., Immunology 109: 319-325; 2003.
Akdis C.A., Akdis M, J. Allergy Clin. Immunol. 127: 18-27; 2011.
Alexopoulou L., et al., Eur. J. Immunol. 36: 2768-2780; 2006.
Alhalaweh A., et al., Eur. J. Pharm. Sci. 38: 206-214; 2009.
Amadou A., et al., Am. J. Physiol. 282: C1339-C1347; 2002.
Ames R.S., et al., J. Immunol. 166: 6341-6348; 2001.
Andersen J.K., Nature med. 10(Suppl.): S18-S25; 2004.
Anderson P.H., et al., Mol. Cell. Endocrinol. 285: 1-9; 2008.
Andjelkovic Z., et al., Clin. Exp. Rheumatol. 17: 453-456; 1999.
Arnon R., Immunol. Lett. 50: 1-125; 1996.
Arnon R., Aharoni R., Proc. Natl. Acad. Sci. USA 101, suppl. 2: 14593-14598; 2004.
Arntz O.J., et al., Arthritis Res. Therapy 12: R61; 2010.
Arumugam T.V., et al., Kidney Int. 63:134-142; 2003.
Arumugam T.V., et al., J. Hepatol. 40: 934-941; 2004.
Astier A.L., et al., J. Clin. Invest. 116: 3252-3257; 2006.
Badawi A.H., Siahaan T.J., Clin. Immunol. 144: 127-138; 2012.
Baelder R., et al., J. Immunol. 174: 783-789; 2005.
Bao L., et al., J. Immunol. 175: 1947-1955; 2005a.
Bao L., et al., Eur. J. Immunol. 35: 2496-2506; 2005b.
Barnes P.J., Trends Pharmacol. Sci. 31: 335-343; 2010.
Bautsch W., et al., J. Immunol. 165: 5401-5405; 2000.
Biesecker G., et al., Immunopharmacol. 42: 219-320; 1999.
Bikle D.D., Curr. Osteoporos. Rep. 7: 58-63; 2009.
Biton J., et al., Joint Bone Spine 79: 119-123; 2012.
Blagg J., et al., Bioorg. Med. Chem. Lett. 18: 5601-5604; 2008.
Bluestone J.A., et al., Nature 464: 1293-1300; 2010.
Bouillon R., et al., Endocrinol. Rev. 29: 726-775; 2008.
Bourraindeloup M., et al., Circulation 110: 2003-2009; 2004.
Branisteanu D.D., et al., J. Neuroimmunol. 61: 151-160; 1995.
Brehm J.M., et al., J. Allergy Clin. Immunol. 126: 52-58; 2010.
Brodbeck R.M., et al., J. Pharmacol. Exp. Ther.327 : 898-909 ; 2008.
Brunner R., et al., Immunol. Lett. 128: 29-35; 2010.
Bunel E.E., et al., US7595345; 2009.
Cailleret M., et al., Circulation 109: 406-411; 2004.
Cantorna M.T., et al., J. Nutr. 128: 68-72; 1998.
Cardone J., et al., Nat. Immunol.11: 862-871; 2010.
Carrol M.C., Nat. Immunol. 5: 981-986 ; 2004.
Chambers E.S., Hawrylowicz C.M., Curr. Allergy Asthma Rep. 11: 29-36; 2011.
Chang M., et al., Vaccine 19: 2884-2889; 2001.
Chen X., et al., J. Immunl. 179: 154-161; 2007.
Chen X., et al., J. Immunol. 185: 174-182; 2010.
Chen X., Oppenheim J.J. In: TNF pathophysiology. Molecular and cellular mechanisms. Curr. Dir. Autoimmun., pp. 119-134, Karger, Basel; 2010.
Choi S., et al., Pharmaceut. Res. 20: 2008-2010; 2003.
Cianferoni A., et al., Blood 97: 1742-1749; 2001.
Clapp T., et al., J. Pharm. Sci. 100: 388-401; 2011.
Cohen I.R., et al., Arthritis Rheum. 8: 841-845; 1985.
Coppieters K.T., et al. ; J. Exp. Med. 209: 51-60; 2012.
Defer N., et al., J. Biol. Chem. 282: 35564-35573; 2007.
Demedts M., et al., N. Engl. J. Med. 353: 2229-2242; 2005.
Denonne F., et al., Bioorg. Med. Chem. Lett. 17: 3258-3261; 2007a.
Denonne F., et al., Bioorg. Med. Chem. Lett. 17: 3262-3265; 2007b.
Diamyd (2011)
   a) Diamyd US phase III trial:
      http://clinicaltrials.gov/ct2/show/NCT00751842
   b) Diamyd European phase III trial:
      http://clinicaltrials.gov/ct2/show/NCT00723411
   c) Diabetes prevention - immune tolerance (DIAPREV-IT)
      http://clinicaltrials.gov/ct2/show/NCT01122446 ?term=diamyd&rank=6
Dong Z., et al., J. Biol. Chem. 272: 9962-9970; 1997.
Drouin S.M., et al., J. Immunol. 167: 4141-4145; 2001.
Drouin S.M., et al., J. Immunol. 169: 59-26-5933; 2002.
Drouin S.M., et al., Am. J. Respir. Crit. Care Med. 173: 852-857; 2006.
Ehrenstein M.R., et al., J. Exp. Med. 200: 277-285; 2004.
Elias D., et al., Proc. Natl. Acad. Sci. USA 87: 1576-1580; 1990.
Elias D., et al., Proc. Natl. Acad. Sci. USA 88: 3088-3091; 1991.
Eylar E., et al., Int. Immunol. 1: 97-101; 1993.
Ezekowitz R.A., et al., J. Exp. Med.150: 244-260; 1984.
Famulok M., et al., Curr. Top. Microbiol. Immunol. 243: 123-136; 1999.
Fang C., et al., Blood 114: 1005-1015; 2009.
Faria A.M., Weiner H.L., Immunol. Rev. 206: 232-259; 2005.
Feldmann M., Maini R.N., Nature Med. 9: 1245-1250; 2003.
Fiane A.E., et al., Xenotransplantation 6: 52-65; 1999.
Finch A.M., et al., J. Med. Chem. 42: 1965-1974; 1999.
Fletcher J.M., et al., Recent Pat. Inflamm. Allergy Drug Discov. 6: 22-34; 2012.
Fontenot J.D., et al., Nat. Immunol. 4: 330-336 ; 2003.
Francois C., et al., Patent WO2009046198.
Furst D.E., et al., J. Rheumatol. 14: 342-347; 1987.
Gail G., et al., Mol. Immunol. 46: 2837-2838; 2009.
Gao X., et al., Clin. Immunol. 140: 236-243; 2011.
Garren H., et al., Ann. Neurol. 63 : 611-620 ; 2008.
Gaskins H.M., et al., J. Clin. Invest. 90 : 2220-2227;1992.
Gerard N.P., Gerard C., Curr. Opin. Immunol. 14: 705-708; 2002.
Gatenby P., et al., Curr. Opin. Rheumatol. 25: 184-191; 2013.
Gessner A., et al., Immunobiology 201: 285-307; 2000.
Ghoreishi M., et al., J. Immunol. 182: 6071-6078; 2009.
Gilbert J.C., et al., J. Control. Release 5: 113-118; 1987.
Giulietti A., et al., Diabetologica 47: 451-462; 2004.
Godebu E., et al., J. Immunol. 181: 1798-1805; 2008.
Gogishvili T., et al., Int. Arch. Allergy Immunol. 142: 165-174; 2006.
Gohil U., et al., Global J. Pharmacol. 4 : 19-30 ; 2010.
Gong C.Y., et al., Int. J. Pharm. 365: 89-99; 2009 a.
Gong C.Y., et al., BMC Biotechnol. 9: 8; 2009 b.
Gorman S., et al., Immunol. 130: 181-192; 2010.
Goverman, J., Nature Rev. Immunol. 9: 393-407; 2009.
Grassetti D.R., Murray J.F., Arch. Biochem. Biophys. 119: 41-49; 1967.
Grinberg-Bleyer Y., et al., J. Clin. Invest. 120: 4558-4568; 2010.
Grunewald S.M., et al., J. Biol. Chem. 272: 1480-1483; 1997.
Grunewald S.M., et al., J. Immunl. 160: 404-4009; 1998.
Hagenaars N., et al., J. Control. Release 144: 17-24; 2010.
Hamano R., et al., Eur. J. Immunol. 41: 2010-2020; 2011.
Harkin D.W., et al., J. Vasc. Surg. 39: 196-205; 2004.
Harris S.S., J. Nutr. 135: 323-325; 2005.
Hart P.H., et al., Nat. Rev. Immunol. 11: 584-596; 2011.
Hartl D., et al., J. Allergy Clin. Immunol. 119: 1258-1266; 2007.
Hashimoto M., et al., J. Exp. Med. 207: 1135-1143; 2010.
Hayakawa M., et al., EMBO J. 22: 3356-3366; 2003.
Heeger P.S., Kemper C., Immunobiology 217: 216-224,2012.
Helbling-Leclerc A., et al., Biochim. Biophys. Acta 1418: 165-175; 1999.
Hering B.J., et al., Am. J. Transplant. 4: 390-401; 2004.
Hewison M., Mol. Cell. Endocrinol. 321: 103-111; 2010.
Higuchi K., et al., J. Rheumatol. 27 : 1038-1044 ; 2000.
Hogervorst E.J.M., et al., Internatl. Immunol 4: 719-727; 1992.
Holgate S.T., Polosa R., Nature Rev. Immunol 8: 218-230; 2008.
Homann D., von Herrath M., Clin. Immunol. 112: 202-209; 2004.
Huang X.W., et al., Clin. Cancer Res. 12: 2849-2855; 2006.
Huber-Lang M., et al., Am. J. Pathol. 161: 1849-1859; 2002.
Humbles A.A., et al., Nature 406: 998-1001; 2000.
Hyppönen E., et al., Lancet 358: 1500-1503; 2001.
Hyun H., et al., Biomacromolecules 8: 1093-1100; 2007.
Ichim T.E., et al., Expert Opin. Biol. Ther. 8: 191-199; 2008.
Iyer R.P., et al., J. Org. Chem. 55: 4693-4699; 1990.
Jacobsen L., et al., Allergy 62: 943-948; 2007.
Jaeckel E., et al., Horm. Metab. Res. 40: 126-136; 2008.
Jeannin P., et al., J. Exp. Med. 182: 1785-1792; 1995.
Jeffery L.E., et al., J. Immunol. 183: 5458-5467; 2009.
Jeong B., et al., Nature 388: 860-862, 1997.
Jirapongsananuruk O., et al., J. Allergy Clin. Immunol. 106: 981-985; 2000.
Juedes A.E., von Herrath M.G., Res. Rev. 20 : 446-451 ; 2004.
Jurynczyk M., et al., Ann. Neurol. 68 : 593-601 ; 2010.
Kamphuis S., et al., Lancet 366 : 50-56 ; 2005.
Kang Y.M., et al., Biomaterials 31: 2453-2460; 2010.
Karagiannidis C., et al., J. Allergy Clin. Immunol. 122: 617-624; 2004.
Kassiotis G., Kollias G., J. Exp. Med. 1993: 427-434; 2001.
Katragadda M., et al., . Med. Chem. 49: 4616-4622; 2006.
Kawamoto S., et al., J. Clin. Invest. 114: 399-407; 2004.
Kearley J., et al., J. Exp. Med. 202: 1539-1547; 2005.
Kearley, J., et al., J. Allergy Clin. Immunol. 122: 617-624; 2008.
Khodoun M., et al., J. Allergy Clin. Immunol. 123 : 342-351 ; 2009.
Kickler K., et al., PLoS ONE 7 : e48486.doi :10.1371/journal. pone.0048486 ; 2012.
Kimball S.M., et al., Am. J. Clin. Nutr. 86: 645-651; 2007.
Kioi M., et al., Cell. Immunol. 229: 41-51; 2004.
Kissmeyer A.-M., Binderup L., Biochem. Pharmacol.41: 1601-1606; 1991.
Kleijwegt P.S., et al., J. Immunol. 185: 1412-1418; 2010.
Knip M., et al., Diabetes 54: S125-S136; 2005.
Kodama T., et al., Immunobiology 217: 1200-1206; 2012.
Köhl J., et al., J. Clin. Invest. 116: 783-796; 2006.
Köhl J., Curr. Opin. Mol. Ther. 8: 529-538; 2006.
Kondo C., et al., Clin. Exp. Immunol. 124: 323-329; 2001.
Konteatis Z.D., et al., J. Immunol. 153: 4200-4205; 1994.
Kopp E., Ghosh S., Science 265: 956-959; 1994.
Korn T., et al., Nature Med. 13: 423-431; 2007.
Kragballe K., Pharmacol. Toxicol. 77: 241-246; 1995.
Kretschmer K., et al., Nat. Immunol. 6: 1219-1227 ; 2005.
Kruse N., et al., EMBO J. 11: 3237-3244; 1992.
Kruse N., et al., EMBO J. 12: 5121-5129; 1993.
Kukoc-Modun L., Radic N., Internatl. J. Analyt. Chem. 2011: article ID 140756; 2011.
Kukreja A., et al., J. Clin. Invest. 109: 131-140; 2002.
Kuo Y.-T., et al., J. Food Sci. 75: H200-H204; 2010.
Kwan W.-H., et al., Immunol. Res. DOI 10.1007/s12026-012-8327-1; 2012.
Landewe R.B.M., et al., Ann. Rheum. Dis. 69: 1655-1659; 2010.
Langier S., et al., Israel Med. Assoc. J. 14: 180-183; 2012.
Lappegard K.T., et al., Ann. Thorac. Surg. 79: 917-923; 2005.
Lappegard K.T., et al., J. Biomed. Mater. Res. A87: 129-135; 2008.
Larche M., et al., Nat. Rev. Immunol. 6: 761-771; 2006.
Leadbetter E.A., et al., J. Immunol. 161: 504-512; 1998.
Lee J.H., et al., Clin. Exp. Immunol. 148: 53-63; 2007.
Lee H., et al., Immunol. Cell Biol. 86 : 153-160 ; 2008.
Leroux-Roels G., Vaccine 285: C25-C36; 2010.
Letzelter F., et al., Eur. J. Biochem. 257: 11-20; 1998.
Li K., et al., Blood 112: 5084-5094; 2008.
Li C.-R., et al., J. Mol. Cell Biol. 4: 38-47; 2012.
Liang C.M., et al., J. Biol. Chem. 264: 13519-13523; 1989. Liang S.M., et al., J. Immunol. 146: 1909-1913; 1991.
Lin M., et al., Diabetes 59: 2247-2252; 2010.
Liu B., Hannun Y.A., J. Biol. Chem. 272: 16281-16287; 1997. Liu J., et al., J. Immunol. 180: 5882-5889; 2008.
Lu J., et al., US7897789 ; 2011.
Ludvigsson J., et al., N. Engl. J. Med. 359: 676-781; 2008.
MacroGenics press release (October 20, 2011), http://www.macrogenics.com/press_releases-284.html
Mahic M., et al., Eur. J. Immunol. 38 : 640-646 ; 2008.
Mahon B.D., et al., J. Cell. Biochem. 89: 922-932; 2003.
Majak P., et al., J. Allergy Clin. Immunol. 127 : 1294-1296 ; 2011.
Mantel P.-Y. et al., PLoS biology 5, e329: 2847-2861; 2007.
March D.R., et al., Mol. Pharmacol.65 : 868-879 ; 2004.
Markiewski M.M., et al., Nat. Immunol. 9 : 1225-1235 ; 2008.
Martinez-Forero I., et al., Eur. J. Immunol. 38: 576-586; 2008.
Mathieu C., et al., Diabetologica 37: 552-558; 1994.
Mazzeo D., et al., Eur. J. Immunol. 28: 3205-3213; 1998.
McGeachy M.J., et al., J. Immunol. 175: 3025-3032; 2005.
Moller C., et al., J. Allergy Clin. Immunol. 109: 251-256; 2002.
Mollnes T.E., et al., Trends Immunol. 23: 61-64; 2002.
Monk P.N., et al., Brit. J. Pharmacol. 152: 429-448; 2007.
Morgan B.P., Gasque, P., Clin. Exp. Immunol. 107: 1-7 ; 1997.
Morikis D., et al., Protein Science 7: 619-627; 1998.
Mortensen J.T., et al., J. Investig. Dermatol. Symp. Proc. 1: 60-63; 1996.
Muller T., et al., J. Mol. Biol. 237 : 423-436, 1994.
Munegowda M.A., et al., J. Clin. Immunol. 31: 811-826; 2011.
Mutwiri G., et al., Adv. Drug Deliv. Rev. 61: 226-232; 2009.
Nadkarni S., et al., J. Exp. Med. 204: 33-39: 2007.
Nakano Y., et al., J. Allergy Clin. Immunol. 112: 525-530; 2003.
Ni Choileain S.N., Astier A.L., Immunobiology 217: 169-175; 2012.
Nicholls E.F., et al., Ann. N.Y. Acad. Sci. 1213: 46-61; 2010.
Nie S., et al., Internatl. J. Nanomed. 6: 151-166; 2011.
Nilsson B., et al., Blood 92: 1661-1667; 1998.
Nomura T., et al., J. Control. Release 149: 8-14; 2011.
Notley C.A., et al., J. Exp. Med. 205: 2491-2497; 2008.
O'Connor R.A., et al., J. Immunol. 179: 958-966; 2007.
Ojwang J.O., et al., Biochemistry 36 : 6033-6045 ; 1997a.
Ojwang J.O., et al., Antisense Nucleic Acid Drug Dev. 7 : 447-459 ; 1997b.
Ojwang J.O., Rando R.F.. METHODS: A Companion to Methods in Enzymology 18: 244-251; 1999.
Oral H., et al., J. Biol. Chem. 272: 4836-4842; 1997.
Østergaard J.A., et al., Diabetes 60: e7-e8; 2011.
Pai S.S., et al., Am. Assoc. Pharmac. Sci. J. 11: 88-98; 2009.
Pendyala L., Creaven P.J., Cancer Epidemiol. Biomarkers Prevention 4: 245-251; 1995.
Peng Q., et al., J. Immunol. 176: 3330-3341; 2006.
Peng Q., et al., Blood 111: 2452-2461; 2008.
Peng K.-T., et al., Biomaterials 31: 5227-5236; 2010.
Penna G., Adorini, L., J. Immunol. 164: 2405-2411; 2000.
Penna G., et al., Blood 106: 3490-3497; 2005.
Pichler J., et al., Pediatr. Res. 52: 12-18; 2002.
Plum L.A., DeLuca, H.F., Nat. Rev. Drug Discov. 9: 941-955; 2010.
Pratt J.R., et al., Nat. Med. 8: 582-587 ; 2002.
Proctor L.M., et al., Brit. J. Pharmacol. 142: 756-764; 2004.
Qiao M. et al., Int. J. Pharm. 294: 103-112; 2005.
Qu H., et al., Mol. Immunol. 47: 185-195; 2009.
Raedler H., et al., Am. J. Transplant. 9: 1784-1795; 2009.
Ramagopalan S.V., et al., PLoS Genet. 5: e1000369; 2009.
Reichel H., et al., Proc. Natl. Acad. Sci. USA 84: 3385-3389; 1987.
Ricklin D., Lambris J.D., Adv. Exp. Med. Biol. 632: 273-292; 2008.
Ricklin D., et al., Nat. Immunol. 11: 785-797; 2010.
Rolland J.M., et al., Pharmacol. Ther. 121: 273-284; 2009.
Ross P.C., et al., J. Liposome Res. 8: 499-520; 1998.
Ruel-Gariepy E., Leroux J-C., Eur. J. Pharmaceutics Biopharmaceutics 58: 409-426; 2004.
Sahu A., et al., J. Immunol. 157: 884-891; 1996.
Sahu A., et al., Mol. Immunol. 39: 557-566; 2003.
Sandor N., et al., Mol. Immunol. 47: 438-448; 2009.
Sandstrom P.A., et al., J. Leukocyte Biol. 55: 221-226; 1994.
Schmidt S., et al., J. Biomed. Mater. Res. A66: 491-499; 2003.
Schmidt-Weber C., Blaser K. Curr. Opin. Allergy Clin. Immunol. 5: 525-530; 2005.
Schmidt-Weber C., Blaser K. Inflamm. Allergy Drug Targets 5: 15-21; 2006.
Schnatbaum K., et al., Bioorg. Med. Chem. Lett. 16: 5088-5092; 2006.
Sen C.K., Nutritional Biochem. 8: 660-672, 1997.
Sen C.K., et al., Free Radical Biol. Med. 22: 1241-1257; 1997.
Shephard, R.M., DeLuca, H.F., Arch. Biochem. Biophys. 202: 43-53; 1980.
Shibata H., et al., J. Biol. Chem. 283: 998-1007; 2008.
Shibata H., et al., Biomaterials 30: 6638-6647; 2009.
Siegmund K., et al., J. Immunol. 182: 2124-2130; 2009.
Silasi-Mansat R., et al., Blood 116: 1002-1010; 2010.
Simpson S. Jr, et al., J. Neurol. Neurosurg. Psychiatry 82: 1132-1141; 2011.
Sinha V.R., et al., Int. J. Pharm. 278: 1-23; 2004.
Skyler J.S., et al., Diabetes Care 28: 1068-1076; 2005.
Smyth L.J., et al., Chest 138: 905-912, 2010.
Soulika A.M., et al., Clin. Immunol. 96: 212-221; 2000.
Staal F.J., et al., Proc. Natl. Acad. Sci. USA 87: 9943-9947; 1990.
Stäger S., et al., Nat. Med. 9: 1287-1292; 2003.
Steed P.M., et al., Science 301: 1895-1898; 2003.
Steinman L., Zamvil S.S., Ann. Neurol. 68: 567-569; 2010.
Stene L.C., Joner G., Am. J. Clin. Nutr. 78: 1128-1134; 2003.
Strainic M.G., et al., Immunity 28: 425-435; 2008.
Strainic M.G., et al., Nat. Immunol. 14: 162-171; 2013.
Strickland D.H., et al., J. Exp. Med. 203: 2649; 2006.
Sumichika H., et al., J. Biol. Chem. 277: 49403-49407; 2002.
Suresh M., et al., J. Immunol. 170: 788-794; 2003.
Suvannavejh G.C., et al., Cell. Immunol. 205: 24-33; 2000.
Tagliabue A, Rappuoli R., Hum. Vaccin. 4: 347-349; 2008.
Taher Y.A., et al., J. Immunol. 180: 5211-5221; 2008.
Tang J., et al., J. Immunol. 182: 4624-4632; 2009.
Taurog J.D., et al., Methods Enzymol. 162: 339-355; 1988.
The EURODIAB Substudy 2 Study Group. Diabetologica 42: 51-54; 1999.
The Lenercept Multiple Sclerosis Study Group and The University of British Columbia MS/MRI Analysis Group. Neurology 53: 457-465; 1999.
Thole J.E.R., et al., Infect. Immun. 55:1466-1475; 1987.
Thommesen L., Laegreid A., J. Biochem. Mol. Biol. 38: 281-289; 2005.
Thorburn A.N., Hansbro P.M., Am. J. Respir. Cell Mol. Biol. 43: 511-519; 2010.
Ting E., et al., Br. J. Pharmacol. 153: 1043-1053; 2008.
Tirouvanziam R., et al., Proc. Natl. Acad. Sci. USA 103: 4628-4633; 2006.
Tjernberg J., et al., Transplantation 85: 1193-1199; 2008.
Tolerx, Inc. and GlaxoSmithKline (Novewmber 29, 2011),
   http://www.biospace.com/news_story.aspx?StoryID=21361&full =1
Tony H.P., et al., Eur. J. Biochem. 225: 659-665; 1994.
Tsai Y.-G., et al., J. Immunol. 184: 7229-7237; 2010.
Tsai Y.-G., Lin C.-Y., J. All. Ther. S7: oo5.doi:10.4172/ 2155-6121.S7-005; 2011.
Tsuboi I., et al., Cytokine 7: 372-379; 1995.
Urry Z., et al., J. Clin. Invest. 119: 387-398; 2009.
Valencia X., et al.; Blood 108:253-261; 2006.
van der Aar A.M.G., et al., J. Allergy Clin. Immunol. 127: 1532-1540; 2011.
van der Zee R., et al., Eur. J. Immunol. 19: 43-47; 1989.
van Eden W., et al., Nature 331: 171-173; 1988.
van Mierlo G.J., et al., J. Immunol. 180: 2747-2751; 2008.
Verschoor A., et al., J. Immunol. 171: 5363-5371; 2003.
Vieyra M., et al., Am. J. Pathol. 179: 766-744; 2011.
Viglietta V., et al., J. Exp. Med. 199: 971-979; 2004.
Von Haehling S., et al., Basic Res. Cardiol. 99: 18-28; 2004.
Wagner E., Frank M.M., Nature Rev. 9: 43-56; 2010.
Walker M.R., et al., J. Clin. Invest. 112: 1437-1443; 2003.
Warren K.G., et al., Eur. J. Neurol. 13: 887-895; 2006.
Waters S.M., et al., J. Biol. Chem. 280: 40617-40623; 2005.
Wei X, et al., Pharm Res. 23: 1251-1264. doi: 10.1007/s11095-006-0082-3; 2006.
Wenzel S., et al., Lancet 370: 1422-1431; 2007.
Wenzel S.E., et al., Am. J. Respir. Crit. Care Med. 179: 549-558; 2009.
Wingerchuk D.M., et al., J. Neurol. Neurosurg. Psychiatry 76: 1294-1296; 2005.
Wittke A., et al., J. Immunol. 173: 3432-3436; 2004.
Woodruff T.M., et al., Arthritis Rheum. 46: 2476-2485; 2002.
Woodruff T.M., et al., J. Pharmacol. Exp. Ther. 314: 811-817; 2005.
Woodruff T.M., et al., FASEB J. 20: 1407-1417; 2006.
Wu Y., et al., Biomaterials 33: 2351-3260; 2012.
Xu Y.Q., et al., J. Asthma 47: 367-373; 2010.
Xystrakis E., et al., J. Clin. Invest. 116: 146-155; 2006.
Yalcindag A., et al., J. Allergy Clin. Immunol. 117: 1455-1461; 2006.
Yamazaki S., et al., J. Exp. Med. 198: 235-247; 2003.
Yan A.C., et al., Frontiers Biosci. 10: 1802-1827; 2005.
Yang X.D., et al., Clin. Exp. Immunol. 81: 189-194; 1990.
Yiamouyiannis C.A., et al., Am. J. Pathol. 154 : 1911-1921; 1999.
Yu A., Malek T.R., J. Immunol. 177 : 5115-5121 ; 2006.
Zaharoff D.A., et al., Vaccine 25: 2085-2094; 2007.
Zalevsky J., et al., J. Immunol. 179 : 1872-1883 ; 2007.
Zella J.B., et al., Arch. Biochem. Biophys. 417: 77-80; 2003.
Zhang J., et al., Biomacromolecules 7: 2492-2500; 2006.
Zhang X, Köhl J., Expert Rev. Clin. Immunol. 6: 269-277; 2010.
Zhou W., Immunobiology 217: 225-234; 2012.
Zozulya A.L., Wiendl H., Hum. Immunol. 69: 797-804; 2008.

## Claims

1. Pharmaceutical composition for local modulation of T cell and B cell responses at the site of allergen or antigen presentation, made of one or more preparations comprising one or more antigens or allergens, and a therapeutically effective dose of two or more low molecular weight immune modulators selected from four groups of tolerance-inducing therapeutics comprising inhibitors of complement-mediated functions, inhibitors of tumor necrosis factor receptor 1 (TNFR1)-mediated functions, inhibitors of interleukin 4- and interleukin 13-mediated functions, and therapeutic agents suitable for vitamin D3 supplementation.

2. Composition according to claim 1, wherein two or more low molecular weight immune modulators and one or more antigens or allergens are coated or adsorbed on or embedded in a matrix, wherein the matrix is selected as to enable sustained release of one or more antigens or allergens and two or more immune modulators.

3. Composition according to claim 1, wherein two or more low molecular weight immune modulators are coated or adsorbed on or embedded in a first matrix, wherein the first matrix is selected as to enable sustained release of two or more low molecular weight immune modulators, and wherein one or more antigens or allergens are coated or adsorbed on or embedded in a second matrix, different from the first matrix, wherein the second matrix is an adjuvant providing a depot effect for antigen or allergen presentation, wherein the first and the second matrices are provided in separate preparations.

4. Composition according to any of the claims 1 to 3, wherein the matrix of claim 2 and the first matrix of claim 3 is a biodegradable or biostable polymer, preferably biodegradable, more preferably thermogelling, even more preferably reverse thermogelling, in particular selected from the group consisting of polyethylene, polypropylene, polyethylene oxide (PEO), polypropylene oxide (PPO), polyurethane, polyurea, polyamides, polycarbonates, polyaldehydes, polyorthoesters, polyiminocarbonates, poly caprolactone (PCL), poly-D,L-lactic acid (PDLLA), poly-L-lactic acid (PLLA), lactides of said lactic acids, polyphosphazenes, polyglycolic acids, albumin, monomethoxypoly(ethylene glycol) (MPEG), trimethylated chitosan derivatives, or copolymers or mixtures of any of the above including poly(lactic-co-glycolic acid) (PLGA), copolymers of L-lactide and D,L-lactide, polyester copolymers, diblock copolymers consisting of MPEG and PCL, MPEG and PCL-ran-PLLA, MPEG and PLGA, PEO and PLLA, trimethylated chitosan and α,β-glycerophosphate, triblock copolymers consisting of PEO and PLLA, PLGA-PEG-PLGA, PEG-PLGA-PEG, PEG-PCL-PEG, and PEO-PPO-PEO (Poloxamers), wherein the polymer is preferably reverse thermogelling and wherein the gelling temperature is between 20°C and 40°C, preferably between 25°C and 35°C, and/or wherein the 90% degradation of the polymer weight in body environment and/or 90% release of one or more antigens or allergens and/or of two or more immune modulators is completed within 1 to 10 days, preferably within 1 to 3 days.

5. Composition according to claim 3, wherein the second matrix is selected from the group consisting of aluminium salts, oil in water or water in oil emulsions such as Montanide, Adjuvant 65 and Lipovant, liposomes, polymeric microsphere adjuvants, and virosomes, preferably selected from aluminium phosphate or aluminium hydroxide gels.

6. Composition according to any of the claims 1 to 5, wherein preferred low molecular weight immune modulators have a molecular weight smaller than 5000 Da, wherein preferred low molecular weight immune modulators are soluble in aqueous solutions to allow for efficient access to the immunological synapse formed between the antigen presenting cells and the T cells, and wherein preferred low molecular weight immune modulators exhibit a short plasma half-life to minimize potential systemic side effects upon diffusion away from the delivery site.

7. Composition according to any of the claims 1 to 6, wherein low molecular weight complement inhibitors are selected from three groups of inhibitors, wherein the inhibitors of the first group include but are not limited to C3 inhibitors based on cyclic peptides or those based on nucleic acids or derivatives thereof, wherein inhibitors of the second group include but are not limited to low molecular weight C3aR antagonists based on nucleic acids or derivatives thereof, and those with scaffolds featuring an arginine moiety (arginine derivatives) or an amino-piperidine linker and a pyridine moiety (amino-piperidine derivatives), and wherein inhibitors of the third group include but are not limited to low molecular weight C5aR antagonists based on linear or cyclic peptidomimetics, those based on nucleic acids or derivatives thereof, and low molecular weight C5aR antagonists which are not based on peptides.

8. Composition according to any of the claims 1 to 6, wherein low molecular weight inhibitors of TNFR1-mediated functions are selected from two groups of inhibitors, wherein the inhibitors of the first group including but not limited to TNFR1-specific antisense oligonucleotides and anti-TNFR1 aptamers, allow specific inhibition of TNFR1 while leaving TNFR2 signaling intact, wherein inhibitors of the second group including but not limited to glutathione precursors such as N-acetyl-L-cysteine, glutathione and derivatives thereof such as S-methylglutathione, and salicylates such as acetylsalicylic acid and sodium salicylate, allow selective inhibition of TNFR1-mediated functions.

9. Composition according to any of the claims 1 to 6, wherein low molecular weight compounds suitable for vitamin D3 supplementation are selected from three groups of therapeutics, wherein the first groups includes cholecaliferol, calcidiol and calcitriol, wherein the second group includes vitamin D 3 derivatives with reduced calcemic side effects such as calcipotriol, and wherein the third group includes non-secosteroidal vitamin D receptor modulators.

10. Composition according to any of the claims 1 to 9, wherein one or more moderate molecular weight immune modulators with a molecular weight larger than 5 kDa but smaller than 20 kDa are used in combination with one or more low molecular weight immune modulators of claims 6-9, wherein preferred moderate molecular weight immune modulators are soluble in aqueous solutions to allow for efficient access to the immunological synapse formed between the antigen presenting cells and the T cells, and wherein preferred moderate molecular weight immune modulators exhibit a short plasma half-life to minimize potential systemic side effects upon diffusion away from the delivery site.

11. Composition according to claim 10, wherein moderate molecular weight immune modulators are selected from two groups of tolerance-inducing therapeutics comprising inhibitors of interleukin 4 (IL-4)- and interleukin 13 (IL-13)-mediated functions, preferably a human IL-4 mutant with one to three mutations, most preferably in at least one of the positions R121, Y124, and S125, and inhibitors of tumor necrosis factor receptor 1 (TNFR1)-mediated functions, preferably antagonistic TNF mutants with specificity for TNFR1 or dominant-negative sTNF mutants which allow specific inhibition of sTNF while leaving the mTNF-TNFR1 and mTNF-TNFR2 interactions intact.

12. Use of a composition according to one of the claims 1 to 11 for local modulation of T cell and B cell responses at the site of allergen or antigen presentation in an organism, preferably a human, in need thereof, in particular for the treatment of T cell-mediated diseases, preferably selected from the group consisting of, but not limited to, allergy, allergic asthma, and autoimmune disease including but not limited to type 1 diabetes, rheumatoid arthritis, multiple sclerosis, wherein the pharmaceutical composition is administered in a therapeutically effective dose.

13. Use of a composition according to one of the claims 1 to 12 for local modulation of T cell and B cell responses at the site of allergen or antigen presentation, wherein for the treatment of allergy or allergic asthma preferred compositions comprise one or more inhibitor of IL-4/IL-13-mediated functions and one or more inhibitor of complement, and, optionally, one or more vitamin D3 supplementing agent and/or one or more inhibitor of TNFR1-mediated functions, wherein for the treatment of type 1 diabetes preferred compositions comprise one or more inhibitor of complement and one or more vitamin D3 supplementing agent, and, optionally, one or more inhibitor of TNFR1-mediated functions and/or one or more inhibitor of IL-4/IL-13-mediated functions, wherein for the treatment of rheumatoid arthritis preferred compositions comprise one or more inhibitor of TNFR1-mediated functions and one or more vitamin D3 supplementing agent, and, optionally, one or more inhibitor of complement and/or one or more inhibitor of IL-4/IL-13-mediated functions, and wherein for the treatment of multiple sclerosis preferred compositions comprise one or more vitamin D3 supplementing agent and one or more inhibitor of TNFR1-mediated functions, and, optionally, one or more inhibitor of complement and/or one or more inhibitor of IL-4/IL-13-mediated functions.

14. Method for the manufacture of a pharmaceutical composition according to one of the claims 1 to 13, wherein all components are mixed in a therapeutically effective quantity as a single preparation, or wherein the first matrix according to claim 4 and two or more immune modulators in a therapeutically effective quantity are mixed as a first preparation and the second matrix according to claim 5 and one or more antigen or allergen in a therapeutically effective quantity are mixed as a second preparation, and wherein the composition is galenically prepared for administration by injection or by implantation, either subcutaneously, intradermally, intramuscularly, nasally, transbucally, transmucosally, sublingually, rectally, vaginally, intraocularly, or topically.
